# EUROPEAN PATENT APPLICATION

(11) **EP 1 621 537 A1**
(43) Date of publication of application: **01.02.2006**
(21) Application number: 04728462.5
(22) Date of filing: 20.04.2004
(51) Int. Cl.: C07D 401/14, C07D 401/06, C07D 413/06, C07D 413/14, A61K 31/497, A61K 31/5377, A61K 31/4439, A61K 31/501, A61K 31/553, A61K 31/499, A61K 31/4545, A61P 7/02, A61P 9/10

(54) **FIVE-MEMBERED HETEROCYCLIC DERIVATIVE**

(30) Priority: 21.04.2003 JP 2003115204; 19.02.2004 JP 2004042859
(71) Applicant: DAIICHI PHARMACEUTICAL CO., LTD., Chuo-ku, Tokyo 103-8234 (JP)
(72) Inventor: OKAYAMA, Toru, TAKAOKA-SHI, Toyama 9338511 (JP); UOTO, Kouichi, EDogawa-ku, Tokyo 1348630 (JP); ISHIYAMA, Takashi, EDogawa-ku, Tokyo 1348630 (JP); KANAYA, Naoaki, EDogawa-ku, Tokyo 1348630 (JP); KIMURA, Youichi, EDogawa-ku, Tokyo 1348630 (JP); ISHIHARA, Hiroaki, EDogawa-ku, Tokyo 1348630 (JP); WATANABE, Toshiyuki, EDogawa-ku, Tokyo 1348630 (JP); FUJII, Kunihiko, EDogawa-ku, Tokyo 1348630 (JP)
(74) Representative: Hartz, Nikolai
(86) International application number: PCT/JP2004/005605
(87) International publication number: WO 2004/094407

(57) **Abstract**

The present invention relates to a compound represented by formula (I): 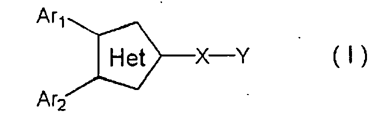 a salt of the compound, or a solvate of the compound or the salt; a drug containing any of the compounds, the salts, and the solvates; a preventive and/or therapeutic agent for an ischemic disease containing any of the compounds, the salts, and the solvates; and a platelet coagulation inhibitor containing any of the compounds, the salts, and the solvates. The compound of the present invention is useful as a strong platelet coagulation inhibitor without inhibiting COX-1 or COX-2.

## Description

### Technical Field

The present invention relates to 5-membered heterocyclic ring derivatives endowed with platelet coagulation-inhibiting activity.

### Background Art

Platelets play an important role in stopping hemorrhage caused by damage to blood vessel through aggregation to form thrombi. On the other hand, it has been known that, when vascular endothelium is injured or the blood vessel is narrowed as in the case of arteriosclerosis, platelets aggregate and trigger thrombus or embolus formation; causing ischemic diseases such as myocardial infarction, angina pectoris, ischemic cerebrovascular disorder, and peripheral vascular disease. Therefore, platelet aggregation inhibitors are administered for prevention and treatment of such ischemic diseases. Among them, aspirin has long been used as one of such platelet coagulation inhibitors, and their effects were demonstrated by APT (Antiplatelet Trialists' Collaboration) in which clinical test results obtained by administering aspirin to 1,000,000 patients had been subjected to metaanalysis (BMJ, vol.308, pages 81-106, 1994). Aspirin, however, is known to cause side effects such as hemorrhage in gastrointestine or like organs, namely, the so-called "aspirin-induced ulcer," and the side effect occurs at a rate of about 1 per 100 patients independently of its dose (BMJ, vol.321, pages 1183-1187, 2000).

The inhibitory effect of aspirin on platelet aggregation is known to be based on the activity to inhibit the activity of cyclooxygenase. Cyclooxygenases include cyclooxygenase-1 (COX-1) and cyclooxygenase-2 (COX-2), and aspirin specifically inhibits COX-1 at a low dose, resulting in inhibition of platelet aggregation. The inhibition of COX-1 also causes the aspirin-induced ulcer (Neurology, vol.57, Suppl.2, pages S5-S7, 2001 and Drugs Today, vol.35, pages 251-265, 1999). In addition, nonsteroidal antiinflammatory drugs are known to exhibit antiinflammatory action by selectively inhibiting COX-2.

As described above, although aspirin is useful as a platelet aggregation inhibitor, its use could lead to a side effect of gastrointestinal dysfunction attributable to the COX-1-inhibiting activity, which is mechanism of an action for platelet aggregation inhibition, and there is a strong demand for new platelet coagulation inhibitors having no COX-1-inhibiting activity.

Meanwhile, there have been compounds known as pyrazole derivatives having antithrombotic activity, such as compound (A) (Japanese Patent No. 2586713 and Chem. Pharm. Bull., vol.45, pages 987-995, 1997) and compound (B) (WO9729774).

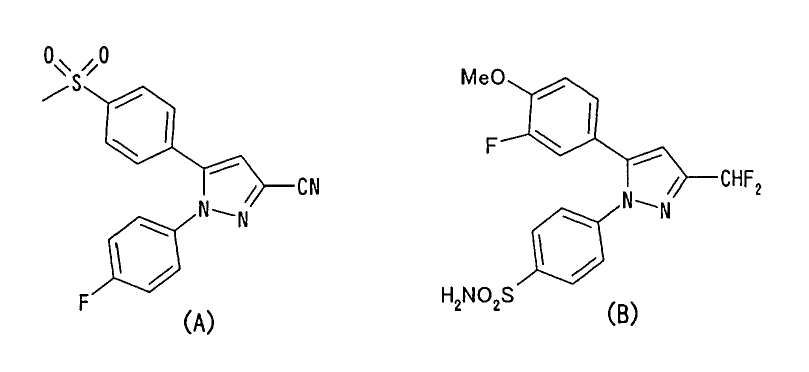

Compound (A), however, exhibits an IC₅₀ value of 5.3 × 10⁻⁶ M against collagen-induced platelet aggregation, with even stronger inhibitory activity against COX-2 (IC₅₀, 2.4 × 10⁻⁷ M). Similarly, the platelet aggregation inhibitory activity of compound (B) is not so strong compared with its inhibitory activity against COX-2.

There has been known, as a compound endowed with anti-platelet activity, a thiazole derivative (C) (J. Med. Chem., Vol. 37, pages 1189-1199, 1994).

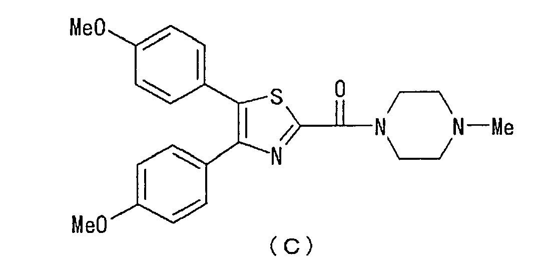

However, the thiazole derivative (C) also inhibits COX inhibiting effect, and the platelet aggregation inhibiting action of the derivative (C) is based on this COX inhibiting effect.

### Disclosure of the Invention

In view of the above, an object of the present invention is to provide a strong platelet coagulation inhibitor without inhibiting COX-1 or COX-2.

The present inventors have made an extensive study in search of such a platelet aggregation inhibitor, and found that 5-membered heterocyclic ring derivatives represented by the following formulas (I) and (II) exhibit strong platelet coagulation inhibitory activity without inhibiting COX-1 or COX-2, thereby completing the invention.

Accordingly, the present invention provides a compound represented by formula (I): 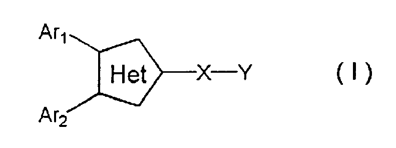
wherein the group represented by formula (1) : 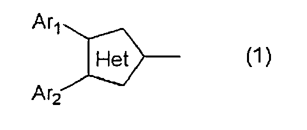 is any of the groups represented by formula (a) to (c): 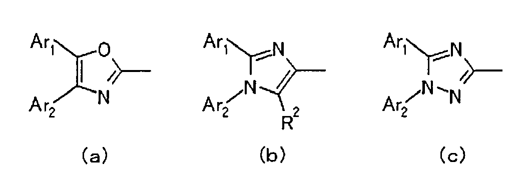
(wherein Ar₁ and Ar₂ each independently represent a 6-membered aromatic heterocyclic group which may have a substituent or a phenyl group which may have a substituent; and R² represents a group selected from among a hydrogen atom, a halogeno group, a hydroxyl group, a lower alkoxy group, and a lower alkyl group which may have a substituent);
X represents a carbonyl group or a thiocarbonyl group; and
Y represents a group represented by formula (2): 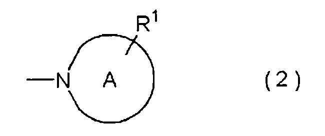 (wherein the ring structure A represents a 4- to 7-membered ring which may have, in addition to N shown in formula (2), one hetero atom selected from among N, O, and S; and the ring structure A may have a substituent represented by R¹, wherein R¹ represents 1 to 4 groups, which are identical to or different from one another, selected from among a hydroxyl group, a cyano group, an oxo group, a halogeno group, a lower alkyl group which may have a substituent, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be a substituted by a lower alkyl group, an aminosulfonyl group which may be substituted by a lower alkyl group, a 3-to 6-membered spiro-alicyclic alkyl group which may have a substituent, and a 4- to 7-membered alicyclic heterocyclic group which may have a substituent), a salt of the compound, or a solvate of the compound or the salt.

The present invention also provides a drug containing a compound represented by formula (I), a salt of the compound, or a solvate of the compound or the salt.

The present invention also provides a preventive and/or therapeutic agent for an ischemic disease, containing a compound represented by formula (I), a salt of the compound, or a solvate of the compound or the salt.

The present invention also provides a platelet aggregation inhibitor, containing a compound represented by formula (I), a salt of the compound, or a solvate of the compound or the salt.

The present invention also provides a drug composition containing a compound represented by formula (I), a salt of the compound, or a solvate of the compound or the salt and a pharmacologically acceptable carrier therefor.

The present invention also provides use of a compound represented by formula (I), a salt of the compound, or a solvate of the compound or the salt in production of a drug.

The present invention also provides use of a compound represented by formula (I), a salt of the compound, or a solvate of the compound or the salt in production of a preventive and/or therapeutic agent for an ischemic disease.

The present invention also provides use of a compound represented by formula (I), a salt of the compound, or a solvate of the compound or the salt in production of a platelet aggregation inhibitor.

The present invention also provides a method for treating an ischemic disease, characterized by comprising administering an effective amount of a compound represented by formula (I), a salt of the compound, or a solvate of the compound or the salt.

The compound (I) of the present invention, salts of the compound, and solvates of the compounds or the salts potently inhibit platelet aggregation, and accordingly, also inhibit thrombogenesis without inhibiting COX-1 or COX-2. Therefore, they are useful as preventive and/or therapeutic agents for ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting (CABG), percutaneous transluminal coronary angioplasty (PTCA), stent placement, etc.), diabetic retinopathy and nephropathy, and embolism after replacement with an artificial heart valve, and are also useful as preventive and/or therapeutic agents for thrombus and embolus associated with vascular operation, blood extracorporeal circulation, and the like.

Moreover, the compound (I) of the present invention, salts thereof, and solvates of the compounds or the salts are useful for ameliorating ischemic conditions such as ulcer, pain, and chill, which accompany chronic arteriosclerosis obliterans.

### Best Modes for Carrying Out the Invention

Substitutions and structural parts in formula (I) will next be described.

Ar₁ and Ar₂ each independently represent a 6-membered aromatic heterocyclic group which may have a substituent or a phenyl group which may have a substituent. Examples of the 6-membered aromatic heterocyclic group include pyridyl, pyridazinyl, pyrimidinyl, and pyrazinyl. Of these 6-membered aromatic heterocyclic groups, pyridyl is preferred.

The substituent of Ar₁ or Ar₂ will next be described.

Examples of the substituent of Ar₁ or Ar₂ include lower alkyl, halogeno, hydroxyl, cyano, lower alkoxy, aralkyloxy, lower thioalkoxy, lower alkoxycarbonyl, carboxyl, lower alkylsulfonyl, amino which may have a substituent, carbamoyl which may be substituted by a lower alkyl, aminosulfonyl which may be substituted by a lower alkyl, and 4- to 7-membered alicyclic heterocyclic group which may have a substituent.

The substituent of Ar₁ or Ar₂ will next be described in more detail.
(1) The lower alkyl group is a C1-C6 linear, branched, or cyclic alkyl group. Examples thereof include methyl, ethyl, propyl, isopropyl, butyl, isobutyl, tert-butyl, pentyl, isopentyl, cyclopropyl, cyclopentyl, cyclohexyl, cyclopropylmethyl, and cyclopentylmethyl. Among them, methyl, ethyl, and propyl are preferred, methyl and ethyl are more preferred, and methyl is still more preferred.
(2) Examples of the halogeno group include fluoro, chloro, and bromo. Among them, fluoro and chloro are preferred, with fluoro being more preferred.
(3) The lower alkoxy is an alkoxy group having any of the above lower alkyl group. Examples thereof include methoxy, ethoxy, propoxy, isopropoxy, butoxy, isobutoxy, pentoxy, and cyclopentyloxy. Among them, methoxy and ethoxy are preferred, with methoxy being more preferred.
(4) The aralkyl group in the aralkyloxy group is a group corresponding to the above lower alkoxy group which has been substituted by a substituted or non-substituted aryl group. Examples thereof include benzyloxy, phenethyloxy, 4-methoxybenzyloxy, and 4-methylbenzyloxy. Among them, benzyloxy, 4-methoxybenzyloxy, and 4-methylbenzyloxy are preferred, with benzyloxy being more preferred.
(5) The lower thioalkoxy is a thioalkoxy group having the above lower alkyl group. Examples thereof include methylthio, ethylthio, propylthio, isopropylthio, butylthio, isobutylthio, pentylthio, and cyclopentylthio. Among them, methylthio and ethylthio are preferred, with methylthio being more preferred.
(6) The lower alkoxycarbonyl is a C2-C6 linear or branched lower alkoxycarbonyl group. Examples thereof include methoxycarbonyl, ethoxycarbonyl, and propoxycarbonyl. Among them, methoxycarbonyl and ethoxycarbonyl are preferred.
(7) The lower alkylsulfonyl group is an alkylsulfonyl group having the above lower alkyl. Examples thereof include methanesulfonyl, ethanesulfonyl, and trifluoromethanesulfonyl. Among them, methanesulfonyl is preferred.
(8) The amino group which may have a substituent is a non-substituted amino, a (mono- or di-lower alkyl)amino, a lower alkanoylamino, a lower alkoxycarbonylamino, a (mono- or di-lower alkyl)sulfonylamino, a ureido, or a (mono- or di-lower alkyl)ureido.
   In these amino groups, the (mono- or di-lower alkyl)amino group is an amino group which has been substituted by one C1-C6 linear, branched, or cyclic alkyl group or two C1-C6 linear, branched, or cyclic alkyl groups which are identical to or different from each other. Examples thereof include methylamino, ethylamino, propylamino, isopropylamino, cyclopropylamino, butylamino, isobutylamino, cyclopentylmethylamino, dimethylamino, diethylamino, dipropylamino, dibutylamino, N-methyl-N-ethylamino, N-ethyl-N-propylamino, and N-methyl-N-cyclopentylmethylamino. Among them, methylamino, ethylamino, dimethylamino, and diethylamino are preferred, with dimethylamino and diethylamino being more preferred.
   The lower alkanoylamino group is an amino group which has been substituted by a C1-C6 linear or branched alkanoyl group. Examples thereof include formylamino, acetylamino, and propionylamino. Among them, formylamino and acetylamino are preferred.
   The lower alkoxycarbonylamino group is an amino group which has been substituted by a C2-C6 linear or branched alkoxycarbonyl group. Examples thereof include methoxycarbonylamino, ethoxycarbonylamino, and propoxycarbonylamino. Among them, methoxycarbonylamino and ethoxycarbonylamino are preferred.
   The (mono- or di-lower alkyl)sulfonylamino group is an alkylsulfonylamino group which has one C1-C6 linear, branched, or cyclic alkyl group or two C1-C6 linear, branched, or cyclic alkyl groups which are identical to or different from each other. Examples thereof include methanesulfonylamino, ethanesulfonylamino, propanesulfonylamino, isopropanesulfonylamino, n- to tert-butanesulfonylamino, cyclopropanesulfonylamino, cyclobutanesulfonylamino, cyclopentanesulfonylamino, cyclohexanesulfonylamino, and cyclopentylmethanesulfonylamino. Among them, methanesulfonylamino, ethanesulfonylamino, and propanesulfonylamino are preferred, with methanesulfonylamino and ethanesulfonylamino being more preferred.
   The (mono- or di-lower alkyl)ureido group is a ureido group which has been substituted by one C1-C6 linear, branched, or cyclic alkyl group or two C1-C6 linear, branched, or cyclic alkyl groups which are identical to or different from each other. Examples thereof include N¹⁻methylaminocarbonylamino, N¹-ethylaminocarbonylamino, N³⁻methylaminocarbonylamino, N¹,N¹-dimethylaminocarbonylamino, N¹,N³-dimethylaminocarbonylamino, and N¹-methyl-N³⁻ethylaminocarbonylamino. Among them, N¹⁻methylaminocarbonylamino, N³-ethylaminocarbonylamino, and N³,N³-dimethylaminocarbonylamino are preferred, with N³⁻methylaminocarbonylamino and N³,N³-dimethylaminocarbonylamino being more preferred.
(9) The carbamoyl group which may be substituted by a lower alkyl group is a non-substituted carbamoyl group or a carbamoyl group which has been substituted by one C1-C6 linear, branched, or cyclic alkyl group or two C1-C6 linear, branched, or cyclic alkyl groups which are identical to or different from each other. Examples thereof include methylcarbamoyl, ethylcarbamoyl, dimethylcarbamoyl, and methylethylcarbamoyl. Among them, carbamoyl, methylcarbamoyl, ethylcarbamoyl, and dimethylcarbamoyl are preferred, with carbamoyl, methylcarbamoyl, and dimethylcarbamoyl being more preferred.
(10) The aminosulfonyl group which may be substituted by a lower alkyl group is a non-substituted aminosulfonyl or an aminosulfonyl group which has been substituted by one C1-C6 linear, branched, or cyclic alkyl group or two C1-C6 linear, branched, or cyclic alkyl groups which are identical to or different from each other. Examples thereof include methylaminosulfonyl, ethylaminosulfonyl, propylaminosulfonyl, dimethylaminosulfonyl, diethylaminosulfonyl, and methylethylaminosulfonyl. Among them, aminosulfonyl, methylaminosulfonyl, ethylaminosulfonyl, dimethylaminosulfonyl, and diethylaminosulfonyl are preferred, with aminosulfonyl, methylaminosulfonyl, dimethylaminosulfonyl, and diethylaminosulfonyl being more preferred.
(11) In the 4- to 7-membered alicyclic heterocyclic group which may have a substituent, examples of a 4- to 7-membered non-substituted alicyclic heterocyclic group include azetidino, pyrrolidino, piperidino, piperazino, morpholino, and homopiperazino.

The 4- to 7-membered alicyclic heterocyclic group which has a substituent is a 4- to 7-membered alicyclic heterocyclic group which has been substituted by one substituent or two substituents which are identical to or different from each other, the substituent(s) being selected from among hydroxyl, lower alkyl, lower alkoxy, alkyl-substituted or non-substituted amino, and alkyl-substituted or non-substituted carbamoyl.

The lower alkyl group is a C1-C6 linear, branched, or cyclic alkyl group. Examples thereof include methyl, ethyl, propyl, isopropyl, butyl, isobutyl, tert-butyl, pentyl, isopentyl, cyclopropyl, cyclopentyl, cyclohexyl, cyclopropylmethyl, and cyclopentylmethyl. The lower alkoxy group is the alkoxy group having the above lower alkyl. Examples thereof include methoxy, ethoxy, propoxy, isopropoxy, butoxy, isobutoxy, pentoxy, and cyclopentyloxy.

The alkyl-substituted amino group is an alkyl group which has been substituted by one lower alkyl group or two lower alkyl groups which are identical to or different from each other. Examples thereof include methylamino, ethylamino, propylamino, isopropylamino, cyclopropylamino, butylamino, isobutylamino, cyclopentylmethylamino, dimethylamino, diethylamino, dipropylamino, dibutylamino, N-methyl-N-ethylamino, N-ethyl-N-propylamino, and N-methyl-N-cyclopentylmethylamino.

The alkyl-substituted carbamoyl group is a carbamoyl which has been substituted by one of the above lower alkyl groups or two of the above lower alkyl groups which are identical to or different from each other. Examples thereof include methylcarbamoyl, ethylcarbamoyl, dimethylcarbamoyl, and methylethylcarbamoyl.

Accordingly, examples of the 4- to 7-membered alicyclic heterocyclic group which may have a substituent include azetidino, pyrrolidino, piperidino, piperazino, morpholino, homopiperazino, 3-aminoazetidin-1-yl, 3-methylaminoazetidin-1-yl, 3-dimethylaminoazetidin-1-yl, 2-carbamoylazetidin-1-yl, 2-methylcarbamoylazetidin-1-yl, 2-dimethylcarbamoylazetidin-1-yl, 3-carbamoylazetidin-1-yl, 3-methylcarbamoylazetidin-1-yl, 3-dimethylcarbamoylazetidin-1-yl, 3-hydroxypyrrolidino, 3-methoxymethylpyrrolidino, 2-carbamoylpyrrolidino, 2-methylcarbamoylpyrrolidino, 2-dimethylcarbamoylpyrrolidino, 3-carbamoylpyrrolidino, 3-methylcarbamoylpyrrolidino, 3-dimethylcarbamoylpyrrolidino, 3-aminopiperidino, 4-aminopiperidino, 3-methylaminopiperidino, 4-methylaminopiperidino, 3-dimethylaminopiperidino, 4-dimethylaminopiperidino, 2-methylpiperidino, 3-methylpiperidino, 4-methylpiperidino, 2,2-dimethylpiperidino, 3,3-dimethylpiperidino, 4,9-dimethylpiperidino, 2-carbamoylpiperidino, 3-carbamoylpiperidino, 4-carbamoylpiperidino, 2-methylcarbamoylpiperidino, 3-methylcarbamoylpiperidino, 4-methylcarbamoylpiperidino, 2-dimethylcarbamoylpiperidino, 3-dimethylcarbamoylpiperidino, 4-dimethylcarbamoylpiperidino, 4-methylpiperazino, 4-cyclopropylpiperazino, 4-carbamoylpiperazino, 2,2-dimethylmorpholino, and 3,3-dimethylmorpholino. Among them, preferred are azetidino, pyrrolidino, piperidino, piperazino, morpholino, homopiperazino, 3-aminoazetidin-1-yl, 3-carbamoylazetidin-1-yl, 3-dimethylcarbamoylazetidin-1-yl, 4-methylpiperazino, and 4-carbamoylpiperazino, with azetidino, pyrrolidino, piperidino, piperazino, and morpholino being more preferred.

R² will be next described.

Examples of the halogeno group and lower alkoxy group are the same as those mentioned above for substituents of the Ar₁ and Ar₂.

The lower alkyl group which may have a substituent is a C1-C6 linear or branched lower alkyl group which may be substituted by a hydroxyl group, a halogeno group, a lower alkoxy group, a carboxyl group, a lower alkoxycarbonyl group, a substituted or non-substituted amino group, a substituted or non-substituted carbamoyl group, a lower alkylsulfonyl group, a substituted or non-substituted aminosulfonyl group, or a similar group. Examples of the lower alkyl group include methyl, ethyl, propyl, isopropyl, butyl, isobutyl, tert-butyl, pentyl, isopentyl, cyclopropyl, cyclopentyl, cyclohexyl, cyclopropylmethyl, and cyclopentylmethyl. Of these, methyl, ethyl, and propyl are preferred, with methyl and ethyl being more preferred.

The lower alkyl group substituted by a hydroxyl group is a group corresponding to the above lower alkyl group which has been substituted by one hydroxyl group. Examples thereof include hydroxymethyl, 2-hydroxyethyl, 3-hydroxypropyl, 2-hydroxypropyl, 4-hydroxybutyl, 2-hydroxybutyl, and 5-hydroxypentyl. Among them, hydroxymethyl, 2-hydroxyethyl, 3-hydroxypropyl, and 2-hydroxypropyl are preferred, with hydroxymethyl, 2-hydroxyethyl, and 3-hydroxypropyl being more preferred.

The lower alkyl group substituted by a halogeno group is a group corresponding to the above lower alkyl group which has been substituted by 1 to 3 substituents selected from among fluoro, chloro, and bromo. Examples thereof include fluoromethyl, difluoromethyl, trifluoromethyl, chloromethyl, dichloromethyl, trichloromethyl, 2-fluoroethyl, 2-chloroethyl, 2-bromoethyl, 3-fluoropropyl, 3-chloropropyl, and 3-bromopropyl. Among them, 2-fluoroethyl, 2-chloroethyl, 3-fluoropropyl, and 3-chloropropyl are preferred, with 2-fluoroethyl and 2-chloroethyl being more preferred.

The lower alkyl group substituted by a lower alkoxy group is a group corresponding to the above lower alkyl group which has one alkoxy group having a C1-C3 linear lower alkyl group. Examples thereof include methoxymethyl, 2-methoxyethyl, 2-ethoxyethyl, 2-propoxyethyl, 3-methoxypropyl, 3-ethoxypropyl, 2-methoxypropyl, 4-methoxybutyl, 2-methoxybutyl, and 5-methoxypentyl. Among them, methoxymethyl, 2-methoxyethyl, 2-ethoxyethyl, and 3-methoxypropyl are preferred, with methoxymethyl, 2-methoxyethyl, and 3-methoxypropyl being more preferred.

The lower alkyl group substituted by a carboxyl group is a group corresponding to the above lower alkyl group which has been substituted by one carboxyl group. Examples thereof include carboxymethyl, 1-carboxyethyl, 2-carboxyethyl, 3-carboxypropyl, 1-carboxypropyl, 2-carboxypropyl, 4-carboxybutyl, 2-carboxybutyl, and 5-carboxypentyl. Among them, carboxymethyl, 1-carboxyethyl, and 2-carboxyethyl are preferred, with carboxymethyl and 2-carboxyethyl being more preferred.

The lower alkyl group substituted by a lower alkoxycarbonyl group is a group corresponding to the above lower alkyl group which has been substituted by one alkoxycarbonyl group having a C1-C3 linear lower alkyl group. Examples include methoxycarbonylmethyl, 1-methoxycarbonylethyl, 2-methoxycarbonylethyl, 1-methoxycarbonylpropyl, 2-methoxycarbonylpropyl, 3-methoxycarbonylpropyl, 4-methoxycarbonylbutyl, 2-methoxycarbonylbutyl, 5-methoxycarbonylpentyl, 2-ethoxycarbonylethyl, 3-ethoxycarbonylpropyl, and 2-ethoxycarbonylpropyl. Among them, methoxycarbonylmethyl, 1-methoxycarbonylethyl, and 2-methoxycarbonylethyl are preferred, with methoxycarbonylmethyl and 2-methoxycarbonylethyl being more preferred.

The lower alkyl group which has been substituted by a substituted or non-substituted amino group is, in addition to an aminoalkyl group corresponding to the above lower alkyl group which has been substituted by one amino group, a lower alkylaminoalkyl group corresponding to the above aminoalkyl group whose nitrogen atom has been substituted by one C1-C3 linear lower alkyl group or two C1-C3 linear lower alkyl groups which are identical to or different from each other, a lower alkanoylaminoalkyl group corresponding to the above aminoalkyl group whose nitrogen atom has been substituted by one C1-C3 lower alkanoyl group, or a lower alkoxycarbonylaminoalkyl group corresponding to the above aminoalkyl group whose nitrogen atom has been substituted by one C1-C3 lower alkoxycarbonyl group. Examples thereof include 2-aminoethyl, 3-aminopropyl, 2-aminopropyl, 4-aminobutyl, 2-aminobutyl, 5-aminopentyl, 2-methylaminoethyl, 3-methylaminopropyl, 2-methylaminopropyl, 2-dimethylaminoethyl, 3-dimethylaminopropyl, 2-dimethylaminopropyl, 2-ethylaminoethyl, 2-diethylaminoethyl, 2-formylaminoethyl, 2-acetylaminoethyl, 2-propionylaminoethyl, 2-methoxycarbonylaminoethyl, 2-ethoxycarbonylaminoethyl, and 3-methoxycarbonylaminopropyl. Among them, preferred are 2-aminoethyl, 3-aminopropyl, 2-methylaminoethyl, 3-methylaminopropyl, 2-dimethylaminoethyl, 3-dimethylaminopropyl, 2-diethylaminoethyl, 2-formylaminoethyl, 2-acetylaminoethyl, 2-methoxycarbonylaminoethyl, and 2-ethoxycarbonylaminoethyl, with 2-aminoethyl, 3-aminopropyl, 2-dimethylaminoethyl, 3-dimethylaminopropyl, 2-diethylaminoethyl, 2-acetylaminoethyl, 2-methoxycarbonylaminoethyl, and 2-ethoxycarbonylaminoethyl being more preferred.

The lower alkyl group which has been substituted by a substituted or non-substituted carbamoyl group is, in addition to a carbamoylalkyl group corresponding to the above lower alkyl group which has been substituted by one carbamoyl group, or a lower alkylcarbamoylalkyl group corresponding to the above carbamoylalkyl group which has been substituted by one C1-C3 linear lower alkyl group or two C1-C3 linear lower alkyl groups which are identical to or different from each other. Examples thereof include carbamoylmethyl, 2-carbamoylethyl, 3-carbamoylpropyl, 2-carbamoylpropyl, 4-carbamoylbutyl, 2-carbamoylbutyl, 5-carbamoylpentyl, 2-(methylcarbamoyl)ethyl, 3-(methylcarbamoyl)propyl, 2-(methylcarbamoyl)propyl, 2-(dimethylcarbamoyl)ethyl, 3-(dimethylcarbamoyl)propyl, 2-(dimethylcarbamoyl)propyl, 2-(ethylcarbamoyl) ethyl, and 2-(diethylcarbamoyl)ethyl. Among them, carbamoylmethyl, 2-carbamoylethyl, 2-(methylcarbamoyl)ethyl, 3-(methylcarbamoyl)propyl, 2-(methylcarbamoyl)propyl, 2-(dimethylcarbamoyl)ethyl, 3-(dimethylcarbamoyl)propyl, 2-(dimethylcarbamoyl)propyl, 2-(ethylcarbamoyl)ethyl, and 2-(diethylcarbamoyl)ethyl are preferred, with carbamoylmethyl, 2-carbamoylethyl, 2-(dimethylcarbamoyl)ethyl, 3-(dimethylcarbamoyl)propyl, 2-(dimethylcarbamoyl)propyl, and 2-(diethylcarbamoyl)ethyl being more preferred.

The lower alkyl group substituted by a lower alkylsulfonyl group is a lower alkylsulfonylalkyl group corresponding to a C1-C6 linear or branched lower alkyl group which has been substituted by one C1-C3 linear alkylsulfonyl group. Examples thereof include 2-methylsulfonylethyl, 3-methylsulfonylpropyl, 2-methylsulfonylpropyl, 4-methylsulfonylbutyl, 2-methylsulfonylbutyl, 5-methylsulfonylpentyl, 2-ethylsulfonylethyl, and 3-ethylsulfonylpropyl. Among them, 2-methylsulfonylethyl, 3-methylsulfonylpropyl, 2-methylsulfonylpropyl, 2-ethylsulfonylethyl, and 3-ethylsulfonylpropyl are preferred, with 2-methylsulfonylethyl, 3-methylsulfonylpropyl, and 2-ethylsulfonylethyl being more preferred.

The lower alkyl group substituted by a substituted or non-substituted aminosulfonyl group is, in addition to an aminosulfonylalkyl group corresponding to a C1-C6 linear or branched lower alkyl group which has been substituted by an aminosulfonyl group, or a lower alkylaminosulfonylalkyl group corresponding to the above aminosulfonylalkyl group which has been substituted by one C1-C3 linear alkyl group or two C1-C3 linear alkyl groups which are identical to or different from each other. Examples thereof include 2-(aminosulfonyl)ethyl, 3-(aminosulfonyl)propyl, 2-(aminosulfonyl)propyl, 4-(aminosulfonyl)butyl, 2-(aminosulfonyl)butyl, 5-(aminosulfonyl)pentyl, 2-(methylaminosulfonyl)ethyl, 3-(methylaminosulfonyl)propyl, 2-(methylaminosulfonyl)propyl, 4-(methylaminosulfonyl)butyl, 2-(methylaminosulfonyl)butyl, 5-(methylaminosulfonyl)pentyl, 2-(ethylaminosulfonyl)ethyl, and 3-(ethylaminosulfonyl)propyl. Among them, 2-(aminosulfonyl)ethyl, 3-(aminosulfonyl)propyl, 2-(aminosulfonyl)propyl, 2-(methylaminosulfonyl)ethyl, 3-(methylaminosulfonyl)propyl, 2-(methylaminosulfonyl)propyl, 2-(ethylaminosulfonyl)ethyl, and 3-(ethylaminosulfonyl)propyl are preferred, with 2-(aminosulfonyl)ethyl, 3-(aminosulfonyl)propyl, 2-(methylaminosulfonyl)ethyl, 3-(methylaminosulfonyl)propyl, 2-(methylaminosulfonyl)propyl, 2-(ethylaminosulfonyl)ethyl, and 3-(ethylaminosulfonyl)propyl being more preferred.

In formula (I), X, which represents a carbonyl group or a thiocarbonyl group, is preferably a carbonyl group.

Next will be described the following formula (2).

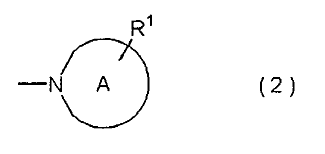

Examples of the ring structure A include azetidine ring, pyrrolidine ring, imidazolidine ring, pyrazoline ring, piperidine ring, piperazine ring, morpholine ring, thiomorpholine ring, hexahydropyridazine ring, hexahydropyrimidine ring, homopiperazine ring, azepane ring, and 1,4-oxazepane ring.

Next will be described substituents (R¹).
(1) Examples of the halogeno group include fluoro, chloro, and bromo. Among them, fluoro and chloro are preferred, with fluoro being more preferred. A carbon atom of the ring may be substituted by a plurality of identical halogeno groups.
(2) Examples of the lower alkyl group which may have a substituent are the same as those mentioned above for R².
(3) Examples of the lower alkoxy group are the same as those mentioned above for a substituent of Ar₁ or Ar₂.
(4) The aralkyl group in the aralkyloxy group is a group corresponding to the above lower alkoxy group which has been substituted by a substituted or non-substituted aryl group. Examples thereof include benzyloxy, phenethyloxy, 4-methoxybenzyloxy, and 4-methylbenzyloxy. Among them, benzyloxy, 4-methoxybenzyloxy, and 4-methylbenzyloxy are preferred, with benzyloxy being more preferred.
(5) The lower thioalkoxy group is a thioalkoxy group having the above lower alkyl group. Examples thereof include methylthio, ethylthio, propylthio, isopropylthio, butylthio, isobutylthio, pentylthio, and cyclopentylthio. Among them, methylthio and ethylthio are preferred, with methylthio being more preferred.
(6) The lower alkoxycarbonyl group is a C2-C6 linear or branched alkoxycarbonyl group. Examples thereof include methoxycarbonyl, ethoxycarbonyl, propoxycarbonyl, and tert-butoxycarbonyl. Among them, methoxycarbonyl and ethoxycarbonyl are preferred, with methoxycarbonyl being more preferred.
(7) The aralkyloxycarbonyl group is a group formed of the above aralkyloxy group and a carbonyl group. Examples of the aralkyloxycarbonyl group include benzyloxycarbonyl, phenethyloxycarbonyl, 4-methoxybenzyloxycarbonyl, and 4-methylbenzyloxycarbonyl. Among them, benzyloxycarbonyl, 4-methoxybenzyloxycarbonyl, and 4-methylbenzyloxycarbonyl are preferred, with benzyloxycarbonyl being more preferred.
(8) The lower acyl group is a C1-C6 linear or branched acyl group. Examples thereof include formyl, acetyl, and propionyl. Among them, formyl and acetyl are preferred, with acetyl being more preferred.
(9) The alkoxyimino group is an alkoxyimino group corresponding to a hydroxyimino group which has been substituted by a C1-C6 linear, branched, or cyclic alkyl group. Examples thereof include methoxyimino, ethoxyimino, propoxyimino, isopropoxyimino, cyclopropyloxyimino, and cyclopropylmethyloxyimino. Among them, methoxyimino, ethoxyimino, and cyclopropyloxyimino are preferred, with methoxyimino being more preferred.
(10) The lower alkylsulfonyl group is a C1-C6 linear or branched alkylsulfonyl group. Examples thereof include methylsulfonyl, ethylsulfonyl, propylsulfonyl, and isopropylsulfonyl. Among them, methylsulfonyl and ethylsulfonyl are preferred, with methylsulfonyl being more preferred.
(11) The amino group which may have a substituent is, in addition to a non-substituted amino group, a (mono- or di-lower alkyl)amino group, lower alkanoylamino group, a lower alkoxycarbonylamino group, a (mono- or di-lower alkyl)sulfonylamino group, a ureido group, or a (mono- or di-lower alkyl)ureido group.
   In these amino groups, the (mono- or di-lower alkyl)amino group is an amino group which has been substituted one C1-C6 linear, branched, or cyclic alkyl group or two C1-C6 linear, branched, or cyclic alkyl groups which are identical to or different from each other. Examples thereof include methylamino, ethylamino, propylamino, isopropylamino, cyclopropylamino, butylamino, isobutylamino, cyclopentylmethylamino, dimethylamino, diethylamino, dipropylamino, dibutylamino, N-methyl-N-ethylamino, N-ethyl-N-propylamino, and N-methyl-N-cyclopentylmethylamino. Among them, methylamino, ethylamino, dimethylamino, and diethylamino are preferred, with dimethylamino and diethylamino being more preferred.
   The lower alkanoylamino group is an amino group which has been substituted by a C1-C6 linear or branched alkanoyl group. Examples thereof include formylamino, acetylamino, and propionylamino. Among them, formylamino and acetylamino are preferred.
   The lower alkoxycarbonylamino group is an amino group which has been substituted by a C2-C6 linear or branched alkoxycarbonyl group. Examples thereof include methoxycarbonylamino, ethoxycarbonylamino, and propoxycarbonylamino. Among them, methoxycarbonylamino and ethoxycarbonylamino are preferred.
   The (mono- or di-lower alkyl)sulfonylamino group is an alkylsulfonylamino group which has one C1-C6 linear, branched, or cyclic alkyl group or two C1-C6 linear, branched, or cyclic alkyl groups which are identical to or different from each other. Examples thereof include methanesulfonylamino, ethanesulfonylamino, propanesulfonylamino, isopropanesulfonylamino, n- to tert-butanesulfonylamino, cyclopropanesulfonylamino, cyclobutanesulfonylamino, cyclopentanesulfonylamino, cyclohexanesulfonylamino, and cyclopentylmethanesulfonylamino. Among them, methanesulfonylamino, ethanesulfonylamino, and propanesulfonylamino are preferred, with methanesulfonylamino and ethanesulfonylamino being more preferred.
   The (mono- or di-lower alkyl)ureido group is a ureido group which has been substituted by one C1-C6 linear, branched, or cyclic alkyl group or two C1-C6 linear, branched, or cyclic alkyl groups which are identical to or different from each other. Examples thereof include N¹⁻methylaminocarbonylamino, N¹-ethylaminocarbonylamino, N³⁻methylaminocarbonylamino, N¹,N¹-dimethylaminocarbonylamino, N¹,N³-dimethylaminocarbonylamino, and N¹-methyl-N³⁻ethylaminocarbonylamino. Among them, N¹⁻methylaminocarbonylamino, N³-ethylaminocarbonylamino, and N³,N³-dimethylaminocarbonylamino are preferred, with N³⁻methylaminocarbonylamino and N³,N³-dimethylaminocarbonylamino being more preferred.
(12) The carbamoyl group which may be substituted by a lower alkyl group is a non-substituted carbamoyl group or a carbamoyl group which has been substituted by one C1-C6 linear, branched, or cyclic alkyl group or two C1-C6 linear, branched, or cyclic alkyl groups which are identical to or different from each other. Examples thereof include methylcarbamoyl, ethylcarbamoyl, dimethylcarbamoyl, and methylethylcarbamoyl. Among them, carbamoyl, methylcarbamoyl, ethylcarbamoyl, and dimethylcarbamoyl are preferred, with carbamoyl, methylcarbamoyl, and dimethylcarbamoyl being more preferred.
(13) The aminosulfonyl group which may be substituted by a lower alkyl group is a non-substituted aminosulfonyl group or an aminosulfonyl group which has been substituted by one C1-C6 linear, branched, or cyclic alkyl group or two C1-C6 linear, branched, or cyclic alkyl groups which are identical to or different from each other. Examples thereof include methylaminosulfonyl, ethylaminosulfonyl, propylaminosulfonyl, dimethylaminosulfonyl, diethylaminosulfonyl, and methylethylaminosulfonyl. Among them, aminosulfonyl, methylaminosulfonyl, ethylaminosulfonyl, dimethylaminosulfonyl, and diethylaminosulfonyl are preferred, with aminosulfonyl, methylaminosulfonyl, dimethylaminosulfonyl, and diethylaminosulfonyl being more preferred.
(14) In the 3- to 6-membered spiro-alicyclic alkyl group, examples of a non-substituted 3- to 6-membered spiro-alicyclic alkyl group include cyclopropanespiro, cyclobutanespiro, cyclopentanespiro, and cyclohexanespiro.
   The 3- to 6-membered spiro-alicyclic alkyl group which has a substituent is a 3- to 6-membered spiro-alicyclic alkyl group which has been substituted by one substituent or two substituents which are identical to or different from each other, the substituent(s) being selected from among hydroxyl, oxo, lower alkyl, halogeno, and alkylsubstituted or non-substituted amino. The lower alkyl group is a C1-C6 linear, branched, or cyclic alkyl group. Examples thereof include methyl, ethyl, propyl, isopropyl, butyl, isobutyl, tert-butyl, pentyl, isopentyl, cyclopropyl, cyclopentyl, cyclohexyl, cyclopropylmethyl, and cyclopentylmethyl.
   The alkyl-substituted amino group is an amino group which has been substituted one of the above lower alkyl groups or two of the above lower alkyl groups which are identical to or different from each other. Examples thereof include methylamino, ethylamino, propylamino, isopropylamino, cyclopropylamino, butylamino, isobutylamino, cyclopentylmethylamino, dimethylamino, diethylamino, dipropylamino, dibutylamino, N-methyl-N-ethylamino, N-ethyl-N-propylamino, and N-methyl-N-cyclopentylmethylamino.
   Examples of the 3- to 6-membered spiro-alicyclic alkyl group which may have a substituent include cyclopropanespiro, cyclobutanespiro, cyclopentanespiro, cyclohexanespiro, 1-methylcyclopropanespiro, 2-methylcyclobutanespiro, 3-methylcyclopentanespiro, 4-methylcyclohexanespiro, 1-fluorocyclopropanespiro, 2-fluorocyclobutanespiro, 3-fluorocyclopentanespiro, 4-fluorocyclohexanespiro, 1-oxocyclopropanespiro, 2-oxocyclobutanespiro, 3-oxocyclopentanespiro, 4-oxocyclohexanespiro, 1-fluorocyclopropanespiro, 2-fluorocyclobutanespiro, 1-hydroxycyclopropanespiro, 2-hydroxycyclobutanespiro, 3-hydroxycyclopentanespiro, 4-hydroxycyclohexanespiro, 1-aminocyclopropanespiro, 2-aminocyclobutanespiro, 3-aminocyclopentanespiro, and 4-aminocyclohexanespiro. Among them, cyclopropanespiro, cyclobutanespiro, cyclopentanespiro, cyclohexanespiro, 1-methylcyclopropanespiro, 1-fluorocyclopropanespiro, 1-oxocyclopropanespiro, 1-fluorocyclopropanespiro, and 1-aminocyclopropanespiro are preferred, with cyclopropanespiro, cyclobutanespiro, cyclopentanespiro, and cyclohexanespiro being more preferred.
(15) In the 4- to 7-membered alicyclic heterocyclic group which may have a substituent, examples of a non-substituted 4- to 7-membered alicyclic heterocyclic group include azetidino, pyrrolidino, piperidino, piperazino, morpholino, homopiperazino, and 1,4-oxazepan-4-yl.

The 4- to 7-membered alicyclic heterocyclic group which has a substituent is a 4- to 7-membered alicyclic heterocyclic group which has been substituted by one substituent or two substituents which are identical to or different from each other, the substituent(s) being selected from among hydroxyl, lower alkyl, lower alkoxy, alkylsubstituted or non-substituted amino, and alkylsubstituted or non-substituted carbamoyl.

The lower alkyl group is a C1-C6 linear, branched, or cyclic alkyl group. Examples thereof include methyl, ethyl, propyl, isopropyl, butyl, isobutyl, tert-butyl, pentyl, isopentyl, cyclopropyl, cyclopentyl, cyclohexyl, cyclopropylmethyl, and cyclopentylmethyl.

The lower alkoxy group is an alkoxy group having the above lower alkyl. Examples thereof include methoxy, ethoxy, propoxy, isopropoxy, butoxy, isobutoxy, pentoxy, and cyclopentyloxy.

The alkyl-substituted amino group is an amino group which has been substituted by one of the above lower alkyl groups or two of the above lower alkyl groups which are identical to or different from each other. Examples thereof include methylamino, ethylamino, propylamino, isopropylamino, cyclopropylamino, butylamino, isobutylamino, cyclopentylmethylamino, dimethylamino, diethylamino, dipropylamino, dibutylamino, N-methyl-N-ethylamino, N-ethyl-N-propylamino, and N-methyl-N-cyclopentylmethylamino.

The alkyl-substituted carbamoyl group is a carbamoyl group which has been substituted by one of the above lower alkyl groups or two of the above lower alkyl groups which are identical to or different from each other. Examples thereof include methylcarbamoyl, ethylcarbamoyl, dimethylcarbamoyl, and methylethylcarbamoyl.

Examples of the 4- to 7-membered alicyclic heterocyclic group which may have a substituent include azetidino, pyrrolidino, piperidino, piperazino, morpholino, homopiperazino, 1,4-oxazepan-4-yl, 3-aminoazetidin-1-yl, 3-methylaminoazetidin-1-yl, 3-dimethylaminoazetidin-1-yl, 2-carbamoylazetidin-1-yl, 2-methylcarbamoylazetidin-1-yl, 2-dimethylcarbamoylazetidin-1-yl, 3-carbamoylazetidin-1-yl, 3-methylcarbamoylazetidin-1-yl, 3-dimethylcarbamoylazetidin-1-yl, 3-hydroxypyrrolidino, 3-methoxymethylpyrrolidino, 2-carbamoylpyrrolidino, 2-methylcarbamoylpyrrolidino, 2-dimethylcarbamoylpyrrolidino, 3-carbamoylpyrrolidino, 3-methylcarbamoylpyrrolidino, 3-dimethylcarbamoylpyrrolidino, 3-aminopiperidino, 4-aminopiperidino, 3-methylaminopiperidino, 4-methylaminopiperidino, 3-dimethylaminopiperidino, 4-dimethylaminopiperidino, 2-methylpiperidino, 3-methylpiperidino, 4-methylpiperidino, 2,2-dimethylpiperidino, 3,3-dimethylpiperidino, 4,4-dimethylpiperidino, 2-carbamoylpiperidino, 3-carbamoylpiperidino, 4-carbamoylpiperidino, 2-methylcarbamoylpiperidino, 3-methylcarbamoylpiperidino, 4-methylcarbamoylpiperidino, 2-dimethylcarbamoylpiperidino, 3-dimethylcarbamoylpiperidino, 4-dimethylcarbamoylpiperidino, 4-methylpiperazino, 4-cyclopropylpiperazino, 4-carbamoylpiperazino, 2,2-dimethylmorpholino, and 3,3-dimethylmorpholino. Among them, azetidino, pyrrolidino, piperidino, piperazino, morpholino, homopiperazino, 3-aminoazetidin-1-yl, 3-carbamoylazetidin-1-yl, 3-dimethylcarbamoylazetidin-1-yl, 4-methylpiperazino, and 4-carbamoylpiperazino are preferred, with azetidino, pyrrolidino, piperidino, piperazino, morpholino, homopiperazino, and 1,4-oxazepan-4-yl being more preferred.

Specific examples of the group represented by formula (2) will next be described.

Examples of the group represented by formula (2) include azetidin-1-yl, 3-oxoazetidin-1-yl, 2-oxoazetidin-1-yl, 3-aminoazetidin-1-yl, 3-methylaminoazetidin-1-yl, 3-dimethylaminoazetidin-1-yl, 2-methylazetidin-1-yl, 3-methylazetidin-1-yl, 2,2-dimethylazetidin-1-yl, 2,4-dimethylazetidin-1-yl, 3,3-dimethylazetidin-1-yl, 2,2-dimethyl-3-dimethylaminoazetidin-1-yl, 2-dimethylaminomethylazetidin-1-yl, 3-dimethylaminomethylazetidin-1-yl, 3-methoxyazetidin-1-yl, 2-hydroxymethylazetidin-1-yl, 3-hydroxymethylazetidin-1-yl, 2-methoxymethylazetidin-1-yl, 3-methoxymethylazetidin-1-yl, 3-hydroxyazetidin-1-yl, 2-carboxyazetidin-1-yl, 3-carboxyazetidin-1-yl, 2-carbamoylazetidin-1-yl, 2-methylcarbamoylazetidin-1-yl, 2-dimethylcarbamoylazetidin-1-yl, 3-carbamoylazetidin-1-yl, 3-methylcarbamoylazetidin-1-yl, 3-dimethylcarbamoylazetidin-1-yl, 3-methoxyazetidin-1-yl, 3-fluoroazetidin-1-yl, 3,3-difluoroazetidin-1-yl, 2-fluoromethylazetidin-1-yl, 3-fluoromethylazetidin-1-yl, pyrrolidino, 2-oxopyrrolidino, 3-oxopyrrolidino, 2,5-dioxopyrrolidino, 3-aminopyrrolidino, 3-methylaminopyrrolidino, 3-dimethylaminopyrrolidino, 2-methylpyrrolidino, 3-methylpyrrolidino, 2,2-dimethylpyrrolidino, 3,3-dimethylpyrrolidino, 2,5-dimethylpyrrolidino, 2,2-dimethyl-3-dimethylaminopyrrolidino, 2-hydroxymethylpyrrolidino, 3-hydroxymethylpyrrolidino, 2-methoxymethylpyrrolidino, 3-methoxymethylpyrrolidino, 2-carboxypyrrolidino, 3-carboxypyrrolidino, 2-carbamoylpyrrolidino, 2-methylcarbamoylpyrrolidino, 2-dimethylcarbamoylpyrrolidino, 3-carbamoylpyrrolidino, 3-methylcarbamoylpyrrolidino, 3-dimethylcarbamoylpyrrolidino, 3-methoxypyrrolidino, 3-fluoropyrrolidino, 3,3-difluoropyrrolidino, 2-fluoromethylpyrrolidino, 3-fluoromethylpyrrolidino, imidazolidin-1-yl, 3-methylimidazolidin-1-yl, 2-oxoimidazolidin-1-yl, 4-oxoimidazolidin-1-yl, 3-methyl-2-oxoimidazolidin-1-yl, 3-methyl-4-oxoimidazolidin-1-yl, 2,2-dimethylimidazolin-1-yl, pyrazolidin-1-yl, 2-methylpyrazolidin-1-yl, 3-oxopyrazolidin-1-yl, 3,5-dioxopyrazolidin-1-yl, piperidino, 2-oxopiperidino, 3-oxopiperidino, 4-oxopiperidino, 3-oxopiperidino, 4-oxopiperidino, 3-hydroxypiperidino, 4-hydroxypiperidino, 2-hydroxyiminopiperidino, 3-hydroxyiminopiperidino, 4-hydroxyiminopiperidino, 2-methoxyiminopiperidino, 3-methoxyiminopiperidino, 4-methoxyiminopiperidino, 3-aminopiperidino, 4-aminopiperidino, 3-methylaminopiperidino, 4-methylaminopiperidino, 3-dimethylaminopiperidino, 4-dimethylaminopiperidino, 2-methylpiperidino, 3-methylpiperidino, 4-methylpiperidino, 2,2-dimethylpiperidino, 3,3-dimethylpiperidino, 4,4-dimethylpiperidino, 2-hydroxymethylpiperidino, 3-hydroxymethylpiperidino, 4-hydroxymethylpiperidino, 2-carboxypiperidino, 3-carboxypiperidino, 4-carboxypiperidino, 2-carbamoylpiperidino, 3-carbamoylpiperidino, 4-carbamoylpiperidino, 2-methylcarbamoylpiperidino, 3-methylcarbamoylpiperidino, 4-methylcarbamoylpiperidino, 2-dimethylcarbamoylpiperidino, 3-dimethylcarbamoylpiperidino, 4-dimethylcarbamoylpiperidino, 2-carboxymethylpiperidino, 3-carboxymethylpiperidino, 4-carboxymethylpiperidino, 2-methoxymethylpiperidino, 3-methoxymethylpiperidino, 4-methoxymethylpiperidino, 2-aminomethylpiperidino, 3-aminomethylpiperidino, 4-aminomethylpiperidino, 2-methylaminomethylpiperidino, 3-methylaminomethylpiperidino, 4-methylaminomethylpiperidino, 2-dimethylaminomethylpiperidino, 3-dimethylaminomethylpiperidino, 4-dimethylaminomethylpiperidino, 2-aminoethylpiperidino, 3-aminoethylpiperidino, 4-aminoethylpiperidino, 2-methylaminoethylpiperidino, 3-methylaminoethylpiperidino, 4-methylaminoethylpiperidino, 2-dimethylaminoethylpiperidino, 3-dimethylaminoethylpiperidino, 4-dimethylaminoethylpiperidino, 4-methoxypiperidino, 4-fluoropiperidino, 3,3-difluoropiperidino, 4,4-difluoropiperidino, piperazino, 2-oxopiperazino, 3-oxopiperazino, 2-oxo-4-methylpiperazino, 3-oxo-4-methylpiperazino, 2,3-dioxopiperazino, 3,5-dioxopiperazino, 2,6-dioxopiperazino, 2,3-dioxo-4-methylpiperazino, 3,5-dioxo-4-methylpiperazino, 2,6-dioxo-4-methylpiperazino, 2-methylpiperazino, 3-methylpiperazino, 4-methylpiperazino, 2-ethylpiperazino, 3-ethylpiperazino, 4-ethylpiperazino, 2-isopropylpiperazino, 3-isopropylpiperazino, 4-isopropylpiperazino, 2-cyclopropylpiperazino, 3-cyclopropylpiperazino, 4-cyclopropylpiperazino, 4-cyclobutylpiperazino, 2-cyclopropanespiropiperazino, 3-cyclopropanespiropiperazino, 2,2-dimethylpiperazino, 3,3-dimethylpiperazino, 2,3-dimethylpiperazino, 2,4-dimethylpiperazino, 3,4-dimethylpiperazino, 3,5-dimethylpiperazino, 2,6-dimethylpiperazino, 2-ethyl-4-methylpiperazino, 3-ethyl-4-methylpiperazino, 2-isopropyl-4-methylpiperazino, 3-isopropyl-4-methylpiperazino, 2-cyclopropyl-4-methylpiperazino, 3-cyclopropyl-4-methylpiperazino, 2-methyl-4-tert-butyloxycarbonylpiperazino, 3-methyl-4-benzylpiperazino, 4-phenylpiperazino, 4-(2-pyridyl)piperazino, 1,2,6-trimethylpiperazino, 3,4,5-trimethylpiperazino, 2,2,4-trimethylpiperazino, 3,3,4-trimethylpiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiropiperazino, 3-cyclopropanespiro-4-methyl-5-oxopiperazino, 4-acetylpiperazino, 4-acetyl-3-cyclopropanespiropiperazino, 2-hydroxymethylpiperazino, 3-hydroxymethylpiperazino, 2-methoxymethylpiperazino, 3-methoxymethylpiperazino, 2-hydroxyethylpiperazino, 3-hydroxyethylpiperazino, 4-hydroxyethylpiperazino, 2-hydroxymethyl-4-methylpiperazino, 3-hydroxymethyl-4-methylpiperazino, 2-methoxymethyl-4-methylpiperazino, 3-methoxymethyl-4-methylpiperazino, 2-hydroxyethyl-4-methylpiperazino, 3-hydroxyethyl-4-methylpiperazino, 2-methoxyethyl-4-methylpiperazino, 3-methoxyethyl-4-methylpiperazino, 2-carbamoylpiperazino, 3-carbamoylpiperazino, 4-carbamoylpiperazino, 2-methylcarbamoylpiperazino, 3-methylcarbamoylpiperazino, 4-methylcarbamoylpiperazino, 2-dimethylcarbamoylpiperazino, 3-dimethylcarbamoylpiperazino, 4-dimethylcarbamoylpiperazino, 2-carbamoylmethylpiperazino, 3-carbamoylmethylpiperazino, 4-carbamoylmethylpiperazino, 2-methylcarbamoylmethylpiperazino, 3-methylcarbamoylmethylpiperazino, 4-methylcarbamoylpiperazino, 2-dimethylcarbamoylmethylpiperazino, 3-dimethylcarbamoylmethylpiperazino, 2-carbamoyl-4-methylpiperazino, 3-carbamoyl-4-methylpiperazino, 4-carbamoylpiperazino, 2-methylcarbamoyl-4-methylpiperazino, 3-methylcarbamoyl-4-methylpiperazino, 4-methylcarbamoylpiperazino, 2-dimethylcarbamoyl-4-methylpiperazino, 3-dimethylcarbamoyl-4-methylpiperazino, 4-dimethylcarbamoylpiperazino, 2-carbamoylmethyl-4-methylpiperazino, 3-carbamoylmethyl-4-methylpiperazino, 4-carbamoylmethylpiperazino, 2-methylcarbamoylmethyl-4-methylpiperazino, 3-methylcarbamoylmethyl-4-methylpiperazino, 4-methylcarbamoylpiperazino, 2-dimethylcarbamoylmethyl-4-methylpiperazino, 3-dimethylcarbamoylmethyl-4-methylpiperazino, 2-carboxypiperazino, 3-carboxypiperazino, 2-methoxycarboxypiperazino, 3-methoxycarboxypiperazino, 2-ethoxycarboxypiperazino, 3-ethoxycarboxypiperazino, 2-carboxymethylpiperazino, 3-carboxymethylpiperazino, 4-carboxymethylpiperazino, 2-carboxyethylpiperazino, 3-carboxyethylpiperazino, 4-carboxyethylpiperazino, 4-carboxytert-butylpiperazino, 2-methoxycarbonylmethylpiperazino, 3-methoxycarbonylmethylpiperazino, 2-methoxycarbonylmethylpiperazino, 3-methoxycarbonylmethylpiperazino, 4-methoxycarbonylmethylpiperazino, 2-ethoxycarbonylmethylpiperazino, 3-ethoxycarbonylmethylpiperazino, 4-ethoxycarbonylmethylpiperazino, 2-carboxy-4-methylpiperazino, 3-carboxy-4-methylpiperazino, 2-carboxymethyl-4-methylpiperazino, 3-carboxymethyl-4-methylpiperazino, 2-methoxycarbonylmethyl-4-methylpiperazino, 3-methoxycarbonylmethyl-4-methylpiperazino, 2-methoxycarbonylmethyl-4-methylpiperazino, 3-methoxycarbonylmethyl-4-methylpiperazino, 2-ethoxycarbonylmethyl-4-methylpiperazino, 3-ethoxycarbonylmethyl-4-methylpiperazino, 4-benzyloxycarbonylpiperazino, 2-aminomethylpiperazino, 3-aminomethylpiperazino, 2-methylaminomethylpiperazino, 3-methylaminomethylpiperazino, 2-dimethylaminomethylpiperazino, 3-dimethylaminomethylpiperazino, 2-aminoethylpiperazino, 3-aminoethylpiperazino, 4-aminoethylpiperazino, 2-methylaminoethylpiperazino, 3-methylaminoethylpiperazino, 4-methylaminoethylpiperazino, 2-dimethylaminoethylpiperazino, 3-dimethylaminoethylpiperazino, 4-dimethylaminoethylpiperazino, 2-aminomethyl-4-methylpiperazino, 3-aminomethyl-4-methylpiperazino, 2-methylaminomethyl-4-methylpiperazino, 3-methylaminomethyl-4-methylpiperazino, 2-dimethylaminomethyl-4-methylpiperazino, 3-dimethylaminomethyl-4-methylpiperazino, 2-aminoethyl-4-methylpiperazino, 3-aminoethyl-4-methylpiperazino, 2-methylaminoethyl-4-methylpiperazino, 3-methylaminoethyl-4-methylpiperazino, 2-dimethylaminoethyl-4-methylpiperazino, 3-dimethylaminoethyl-4-methylpiperazino, 4-methanesulfonylpiperazino, 4-aminosulfonylpiperazino, 4-(azetidin-1-yl)piperazino, 4-pyrrolidinopiperazino, 4-piperidinopiperazino, morpholino, 2-methylmorpholino, 3-methylmorpholino, 2-ethylmorpholino, 3-ethylmorpholino, 2-cyclopropanespiromorpholino, 3-cyclopropanespiromorpholino, 2,2-dimethylmorpholino, 3,3-dimethylmorpholino, 2-hydroxymethylmorpholino, 3-hydroxymethylmorpholino, 2-methoxymethylmorpholino, 3-methoxymethylmorpholino, 2-hydroxyethylmorpholino, 3-hydroxyethylmorpholino, 2-methoxyethylmorpholino, 3-methoxyethylmorpholino, 2-carbamoylmorpholino, 3-carbamoylmorpholino, 2-methylcarbamoylmorpholino, 3-methylcarbamoylmorpholino, 2-dimethylcarbamoylmorpholino, 3-dimethylcarbamoylmorpholino, 2-carbamoylmethylmorpholino, 3-carbamoylmethylmorpholino, 2-methylcarbamoylmethylmorpholino, 3-methylcarbamoylmethylmorpholino, 2-dimethylcarbamoylmethylmorpholino, 3-dimethylcarbamoylmethylmorpholino, 2-carbamoylethylmorpholino, 3-carbamoylethylmorpholino, 2-methylcarbamoylethylmorpholino, 3-methylcarbamoylethylmorpholino, 2-dimethylcarbamoylethylmorpholino, 3-dimethylcarbamoylethylmorpholino, 2-carboxymorpholino, 3-carboxymorpholino, 2-methoxycarbonylmorpholino, 3-methoxycarbonylmorpholino, 2-carboxymethylmorpholino, 3-carboxymethylmorpholino, 2-methoxycarbonylmethylmorpholino, 3-methoxycarbonylmethylmorpholino, 2-ethoxycarbonylmethylmorpholino, 3-ethoxycarbonylmethylmorpholino, 2-aminomethylmorpholino, 3-aminomethylmorpholino, 2-methylaminomethylmorpholino, 3-methylaminomethylmorpholino, 2-dimethylaminomethylmorpholino, 3-dimethylaminomethylmorpholino, 2-aminoethylmorpholino, 3-aminoethylmorpholino, 2-methylaminoethylmorpholino, 3-methylaminoethylmorpholino, 2-dimethylaminoethylmorpholino, 3-dimethylaminoethylmorpholino, thiomorpholino, 3-oxothiomorpholino, 1,1-dioxothiomorpholino, 2-methylthiomorpholino, 3-methylthiomorpholino, 2-ethylthiomorpholino, 3-ethylthiomorpholino, 2-cyclopropanespirothiomorpholino, 3-cyclopropanespirothiomorpholino, 2,2-dimethylthiomorpholino, 3,3-dimethylthiomorpholino, 2-hydroxymethylthiomorpholino, 3-hydroxymethylthiomorpholino, 2-methoxymethylthiomorpholino, 3-methoxymethylthiomorpholino, 2-hydroxyethylthiomorpholino, 3-hydroxyethylthiomorpholino, 2-methoxyethylthiomorpholino, 3-methoxyethylthiomorpholino, 2-carbamoylthiomorpholino, 3-carbamoylthiomorpholino, 2-methylcarbamoylthiomorpholino, 3-methylcarbamoylthiomorpholino, 2-dimethylcarbamoylthiomorpholino, 3-dimethylcarbamoylthiomorpholino, 2-carbamoylmethylthiomorpholino, 3-carbamoylmethylthiomorpholino, 2-methylcarbamoylmethylthiomorpholino, 3-methylcarbamoylmethylthiomorpholino, 2-dimethylcarbamoylmethylthiomorpholino, 3-dimethylcarbamoylmethylthiomorpholino, 2-carbamoylethylthiomorpholino, 3-carbamoylethylthiomorpholino, 2-methylcarbamoylethylthiomorpholino, 3-methylcarbamoylethylthiomorpholino, 2-dimethylcarbamoylethylthiomorpholino, 3-dimethylcarbamoylethylthiomorpholino, 2-carboxythiomorpholino, 3-carboxythiomorpholino, 2-methoxycarbonylthiomorpholino, 3-methoxycarbonylthiomorpholino, 2-carboxymethylthiomorpholino, 3-carboxymethylthiomorpholino, 2-methoxycarbonylmethylthiomorpholino, 3-methoxycarbonylmethylthiomorpholino, 2-ethoxycarbonylmethylthiomorpholino, 3-ethoxycarbonylmethylthiomorpholino, 2-aminomethylthiomorpholino, 3-aminomethylthiomorpholino, 2-methylaminomethylthiomorpholino, 3-methylaminomethylthiomorpholino, 2-dimethylaminomethylthiomorpholino, 3-dimethylaminomethylthiomorpholino, 2-aminoethylthiomorpholino, 3-aminoethylthiomorpholino, 2-methylaminoethylthiomorpholino, 3-methylaminoethylthiomorpholino, 2-dimethylaminoethylthiomorpholino, 3-dimethylaminoethylthiomorpholino, hexahydropyridazin-1-yl, 3-oxohexahydropyridazin-1-yl, 6-oxohexahydropyridazin-1-yl, 4-aminohexahydropyridazin-1-yl, 4-methylaminohexahydropyridazin-1-yl, 4-dimethylaminohexahydropyridazin-1-yl, 2-methylhexahydropyridazin-1-yl, 3-methylhexahydropyridazin-1-yl, 4-methylhexahydropyridazin-1-yl, 2,3-dimethylhexahydropyridazin-1-yl, 3,3-dimethylhexahydropyridazin-1-yl, 4,4-dimethylhexahydropyridazin-1-yl, 3-hydroxymethylhexahydropyridazin-1-yl, 4-hydroxymethylhexahydropyridazin-1-yl, 5-hydroxymethylhexahydropyridazin-1-yl, 6-hydroxymethylhexahydropyridazin-1-yl, 2-carbamoylhexahydropyridazin-1-yl, 3-carbamoylhexahydropyridazin-1-yl, 4-carbamoylhexahydropyridazin-1-yl, 5-carbamoylhexahydropyridazin-1-yl, 6-carbamoylhexahydropyridazin-1-yl, 2-methylcarbamoylhexahydropyridazin-1-yl, 3-methylcarbamoylhexahydropyridazin-1-yl, 4-methylcarbamoylhexahydropyridazin-1-yl, 5-methylcarbamoylhexahydropyridazin-1-yl, 6-methylcarbamoylhexahydropyridazin-1-yl, 2-dimethylcarbamoylhexahydropyridazin-1-yl, 3-dimethylcarbamoylhexahydropyridazin-1-yl, 4-dimethylcarbamoylhexahydropyridazin-1-yl, 5-dimethylcarbamoylhexahydropyridazin-1-yl, 6-dimethylcarbamoylhexahydropyridazin-1-yl, 3-carboxyhexahydropyridazin-1-yl, 4-carboxyhexahydropyridazin-1-yl, 5-carboxyhexahydropyridazin-1-yl, 6-carboxyhexahydropyridazin-1-yl, 2-carboxymethylhexahydropyridazin-1-yl, 3-carboxymethylhexahydropyridazin-1-yl, 4-carboxymethylhexahydropyridazin-1-yl, 5-carboxymethylhexahydropyridazin-1-yl, 6-carboxymethylhexahydropyridazin-1-yl, 3-methoxycarboxyhexahydropyridazin-1-yl, 4-methoxycarboxyhexahydropyridazin-1-yl, 5-methoxycarboxyhexahydropyridazin-1-yl, 6-methoxycarboxyhexahydropyridazin-1-yl, 2-methoxycarbonylmethylhexahydropyridazin-1-yl, 3-methoxycarbonylmethylhexahydropyridazin-1-yl, 4-methoxycarbonylmethylhexahydropyridazin-1-yl, 5-methoxycarbonylmethylhexahydropyridazin-1-yl, 6-methoxycarbonylmethylhexahydropyridazin-1-yl, 3-methoxymethylhexahydropyridazin-1-yl, 4-methoxymethylhexahydropyridazin-1-yl, 5-methoxymethylhexahydropyridazin-1-yl, 6-methoxymethylhexahydropyridazin-1-yl, 2-aminoethylhexahydropyridazin-1-yl, 3-aminoethylhexahydropyridazin-1-yl, 4-aminoethylhexahydropyridazin-1-yl, 5-aminoethylhexahydropyridazin-1-yl, 6-aminoethylhexahydropyridazin-1-yl, 2-methylaminoethylhexahydropyridazin-1-yl, 3-methylaminoethylhexahydropyridazin-1-yl, 4-methylaminoethylhexahydropyridazin-1-yl, 5-methylaminoethylhexahydropyridazin-1-yl, 6-methylaminoethylhexahydropyridazin-1-yl, 3-aminomethylhexahydropyridazin-1-yl, 4-aminomethylhexahydropyridazin-1-yl, 5-aminomethylhexahydropyridazin-1-yl, 6-aminomethylhexahydropyridazin-1-yl, 3-methylaminomethylhexahydropyridazin-1-yl, 4-methylaminomethylhexahydropyridazin-1-yl, 5-methylaminomethylhexahydropyridazin-1-yl, 6-methylaminomethylhexahydropyridazin-1-yl, 3-dimethylaminomethylhexahydropyridazin-1-yl, 4-dimethylaminomethylhexahydropyridazin-1-yl, 5-dimethylaminomethylhexahydropyridazin-1-yl, 6-dimethylaminomethylhexahydropyridazin-1-yl, 2-dimethylaminoethylhexahydropyridazin-1-yl, 3-dimethylaminoethylhexahydropyridazin-1-yl, 4-dimethylaminoethylhexahydropyridazin-1-yl, 5-dimethylaminoethylhexahydropyridazin-1-yl, 6-dimethylaminoethylhexahydropyridazin-1-yl, hexahydropyrimidin-1-yl, 2-oxohexahydropyrimidin-1-yl, 4-oxohexahydropyrimidin-1-yl, 5-oxohexahydropyrimidin-1-yl, 6-oxohexahydropyrimidin-1-yl, 2-methylhexahydropyrimidin-1-yl, 3-methylhexahydropyrimidin-1-yl, 4-methylhexahydropyrimidin-1-yl, 4-methylhexahydropyrimidin-1-yl, 2,2-dimethylhexahydropyrimidin-1-yl, 4,4-dimethylhexahydropyrimidin-1-yl, 5,5-dimethylhexahydropyrimidin-1-yl, 6,6-dimethylhexahydropyrimidin-1-yl, 2-hydroxymethylhexahydropyrimidin-1-yl, 4-hydroxymethylhexahydropyrimidin-1-yl, 5-hydroxymethylhexahydropyrimidin-1-yl, 6-hydroxymethylhexahydropyrimidin-1-yl, 2-carboxyhexahydropyrimidin-1-yl, 4-carboxyhexahydropyrimidin-1-yl, 5-carboxyhexahydropyrimidin-1-yl, 6-carboxyhexahydropyrimidin-1-yl, 2-carbamoylhexahydropyrimidin-1-yl, 3-carbamoylhexahydropyrimidin-1-yl, 4-carbamoylhexahydropyrimidin-1-yl, 5-carbamoylhexahydropyrimidin-1-yl, 6-carbamoylhexahydropyrimidin-1-yl, 2-methylcarbamoylhexahydropyrimidin-1-yl, 3-methylcarbamoylhexahydropyrimidin-1-yl, 4-methylcarbamoylhexahydropyrimidin-1-yl, 5-methylcarbamoylhexahydropyrimidin-1-yl, 6-methylcarbamoylhexahydropyrimidin-1-yl, 2-dimethylcarbamoylhexahydropyrimidin-1-yl, 3-dimethylcarbamoylhexahydropyrimidin-1-yl, 4-dimethylcarbamoylhexahydropyrimidin-1-yl, 5-dimethylcarbamoylhexahydropyrimidin-1-yl, 6-dimethylcarbamoylhexahydropyrimidin-1-yl, 2-carboxymethylhexahydropyrimidin-1-yl, 3-carboxymethylhexahydropyrimidin-1-yl, 4-carboxymethylhexahydropyrimidin-1-yl, 5-carboxymethylhexahydropyrimidin-1-yl, 6-carboxymethylhexahydropyrimidin-1-yl, 2-methoxycarbonylmethylhexahydropyrimidin-1-yl, 3-methoxycarbonylmethylhexahydropyrimidin-1-yl, 4-methoxycarbonylmethylhexahydropyrimidin-1-yl, 5-methoxycarbonylmethylhexahydropyrimidin-1-yl, 6-methoxycarbonylmethylhexahydropyrimidin-1-yl, 3-methoxymethylhexahydropyrimidin-1-yl, 4-methoxymethylhexahydropyrimidin-1-yl, 5-methoxymethylhexahydropyrimidin-1-yl, 6-methoxymethylhexahydropyrimidin-1-yl, 2-aminoethylhexahydropyrimidin-1-yl, 3-aminoethylhexahydropyrimidin-1-yl, 4-aminoethylhexahydropyrimidin-1-yl, 5-aminoethylhexahydropyrimidin-1-yl, 6-aminoethylhexahydropyrimidin-1-yl, 2-methylaminoethylhexahydropyrimidin-1-yl, 3-methylaminoethylhexahydropyrimidin-1-yl, 4-methylaminoethylhexahydropyrimidin-1-yl, 5-methylaminoethylhexahydropyrimidin-1-yl, 6-methylaminoethylhexahydropyrimidin-1-yl, 2-dimethylaminoethylhexahydropyrimidin-1-yl, 3-dimethylaminoethylhexahydropyrimidin-1-yl, 4-dimethylaminoethylhexahydropyrimidin-1-yl, 5-dimethylaminoethylhexahydropyrimidin-1-yl, 6-dimethylaminoethylhexahydropyrimidin-1-yl, homopiperazino, 2-oxohomopiperazino, 3-oxohomopiperazino, 5-oxohomopiperazino, 6-oxohomopiperazino, 7-oxohomopiperazino, 2-oxo-4-methylhomopiperazino, 3-oxo-4-methylhomopiperazino, 5-oxo-4-methylhomopiperazino, 6-oxo-4-methylhomopiperazino, 7-oxo-4-methylhomopiperazino, 2,3-dioxohomopiperazino, 2,7-dioxohomopiperazino, 3,5-dioxohomopiperazino, 3,7-dioxohomopiperazino, 2,3-dioxo-4-methylhomopiperazino, 2,7-dioxo-4-methylhomopiperazino, 3,5-dioxo-4-methylhomopiperazino, 3,7-dioxo-4-methylhomopiperazino, 2-methylhomopiperazino, 3-methylhomopiperazino, 4-methylhomopiperazino, 5-methylhomopiperazino, 6-methylhomopiperazino, 7-methylhomopiperazino, 2-ethylhomopiperazino, 3-ethylhomopiperazino, 4-ethylhomopiperazino, 5-ethylhomopiperazino, 6-ethylhomopiperazino, 7-ethylhomopiperazino, 4-cyclopropylhomopiperazino, 2-cyclopropanespirohomopiperazino, 3-cyclopropanespirohomopiperazino, 5-cyclopropanespirohomopiperazino, 6-cyclopropanespirohomopiperazino, 7-cyclopropanespirohomopiperazino, 2-cyclopropanespiro-4-methylhomopiperazino, 3-cyclopropanespiro-4-methylhomopiperazino, 5-cyclopropanespiro-4-methylhomopiperazino, 6-cyclopropanespiro-4-methylhomopiperazino, 7-cyclopropanespiro-4-methylhomopiperazino, 2-cyclopropanespiro-4-methyl-3-oxohomopiperazino, 2-cyclopropanespiro-4-methyl-5-oxohomopiperazino, 2-cyclopropanespiro-4-methyl-7-oxohomopiperazino, 3-cyclopropanespiro-4-methyl-2-oxohomopiperazino, 3-cyclopropanespiro-4-methyl-5-oxohomopiperazino, 3-cyclopropanespiro-4-methyl-7-oxohomopiperazino, 5-cyclopropanespiro-4-methyl-2-oxohomopiperazino, 5-cyclopropanespiro-4-methyl-3-oxohomopiperazino, 5-cyclopropanespiro-4-methyl-7-oxohomopiperazino, 6-cyclopropanespiro-4-methyl-2-oxohomopiperazino, 6-cyclopropanespiro-4-methyl-3-oxohomopiperazino, 6-cyclopropanespiro-4-methyl-5-oxohomopiperazino, 6-cyclopropanespiro-4-methyl-7-oxohomopiperazino, 7-cyclopropanespiro-4-methyl-2-oxohomopiperazino, 7-cyclopropanespiro-4-methyl-3-oxohomopiperazino, 7-cyclopropanespiro-4-methyl-5-oxohomopiperazino, 2,2-dimethylhomopiperazino, 3,3-dimethylhomopiperazino, 5,5-dimethylhomopiperazino, 6,6-dimethylhomopiperazino, 7,7-dimethylhomopiperazino, 2,3-dimethylhomopiperazino, 2,4-dimethylhomopiperazino, 3,4-dimethylhomopiperazino, 3,5-dimethylhomopiperazino, 3,4,5-trimethylhomopiperazino, 2-hydroxymethylhomopiperazino, 3-hydroxymethylhomopiperazino, 5-hydroxymethylhomopiperazino, 6-hydroxymethylhomopiperazino, 7-hydroxymethylhomopiperazino, 2-hydroxymethyl-4-methylhomopiperazino, 3-hydroxymethyl-4-methylhomopiperazino, 5-hydroxymethyl-4-methylhomopiperazino, 6-hydroxymethyl-4-methylhomopiperazino, 7-hydroxymethyl-4-methylhomopiperazino, 2-methoxymethylhomopiperazino, 3-methoxymethylhomopiperazino, 5-methoxymethylhomopiperazino, 6-methoxymethylhomopiperazino, 7-methoxymethylhomopiperazino, 2-methoxymethyl-4-methylhomopiperazino, 3-methoxymethyl-4-methylhomopiperazino, 5-methoxymethyl-4-methylhomopiperazino, 6-methoxymethyl-4-methylhomopiperazino, 7-methoxymethyl-4-methylhomopiperazino, 2-hydroxyethylhomopiperazino, 3-hydroxyethylhomopiperazino, 4-hydroxyethylhomopiperazino, 5-hydroxyethylhomopiperazino, 6-hydroxyethylhomopiperazino, 7-hydroxyethylhomopiperazino, 2-hydroxyethyl4-methylhomopiperazino, 3-hydroxyethyl-4-methylhomopiperazino, 5-hydroxyethyl-4-methylhomopiperazino, 6-hydroxyethyl-4-methylhomopiperazino, 7-hydroxyethyl-4-methylhomopiperazino, 2-methoxyethylhomopiperazino, 3-methoxyethylhomopiperazino, 4-methoxyethylhomopiperazino, 5-methoxyethylhomopiperazino, 6-methoxyethylhomopiperazino, 7-methoxyethylhomopiperazino, 2-methoxyethyl-4-methylhomopiperazino, 3-methoxyethyl-4-methylhomopiperazino, 5-methoxyethyl-4-methylhomopiperazino, 6-methoxyethyl-4-methylhomopiperazino, 7-methoxyethyl-4-methylhomopiperazino, 2-carbamoylhomopiperazino, 3-carbamoylhomopiperazino, 4-carbamoylhomopiperazino, 5-carbamoylhomopiperazino, 6-carbamoylhomopiperazino, 7-carbamoylhomopiperazino, 2-carbamoyl-4-methylhomopiperazino, 3-carbamoyl-4-methylhomopiperazino, 4-carbamoylhomopiperazino, 5-carbamoyl-4-methylhomopiperazino, 6-carbamoyl-4-methylhomopiperazino, 7-carbamoyl-4-methylhomopiperazino, 2-methylcarbamoylhomopiperazino, 3-methylcarbamoylhomopiperazino, 4-methylcarbamoylhomopiperazino, 5-methylcarbamoylhomopiperazino, 6-methylcarbamoylhomopiperazino, 7-methylcarbamoylhomopiperazino, 2-methylcarbamoyl-4-methylhomopiperazino, 3-methylcarbamoyl-4-methylhomopiperazino, 5-methylcarbamoyl-4-methylhomopiperazino, 6-methylcarbamoyl-4-methylhomopiperazino, 7-methylcarbamoyl-4-methylhomopiperazino, 2-dimethylcarbamoylhomopiperazino, 3-dimethylcarbamoylhomopiperazino, 4-dimethylcarbamoylhomopiperazino, 5-dimethylcarbamoylhomopiperazino, 6-dimethylcarbamoylhomopiperazino, 7-dimethylcarbamoylhomopiperazino, 2-dimethylcarbamoyl-4-methylhomopiperazino, 3-dimethylcarbamoyl-4-methylhomopiperazino, 5-dimethylcarbamoyl-4-methylhomopiperazino, 6-dimethylcarbamoyl-4-methylhomopiperazino, 7-dimethylcarbamoyl-4-methylhomopiperazino, 2-carboxyhomopiperazino, 3-carboxyhomopiperazino, 5-carboxyhomopiperazino, 6-carboxyhomopiperazino, 7-carboxyhomopiperazino, 2-carboxy-4-methylhomopiperazino, 3-carboxy-4-methylhomopiperazino, 5-carboxy-4-methylhomopiperazino, 6-carboxy-4-methylhomopiperazino, 7-carboxy-4-methylhomopiperazino, 2-carboxymethylhomopiperazino, 3-carboxymethylhomopiperazino, 4-carboxymethylhomopiperazino, 5-carboxymethylhomopiperazino, 6-carboxymethylhomopiperazino, 7-carboxymethylhomopiperazino, 2-carboxymethyl-4-methylhomopiperazino, 3-carboxymethyl-4-methylhomopiperazino, 5-carboxymethyl-4-methylhomopiperazino, 6-carboxymethyl-4-methylhomopiperazino, 7-carboxymethyl-4-methylhomopiperazino, 2-methoxycarbonylmethylhomopiperazino, 3-methoxycarbonylmethylhomopiperazino, 4-methoxycarbonylmethylhomopiperazino, 5-methoxycarbonylmethylhomopiperazino, 6-methoxycarbonylmethylhomopiperazino, 7-methoxycarbonylmethylhomopiperazino, 2-methoxycarbonylmethyl-4-methylhomopiperazino, 3-methoxycarbonylmethyl-4-methylhomopiperazino, 5-methoxycarbonylmethyl-4-methylhomopiperazino, 6-methoxycarbonylmethyl-4-methylhomopiperazino, 7-methoxycarbonylmethyl-4-methylhomopiperazino, 2-ethoxycarbonylmethylhomopiperazino, 3-ethoxycarbonylmethylhomopiperazino, 4-ethoxycarbonylmethylhomopiperazino, 5-ethoxycarbonylmethylhomopiperazino, 6-ethoxycarbonylmethylhomopiperazino, 7-ethoxycarbonylmethylhomopiperazino, 2-ethoxycarbonylmethyl-4-methylhomopiperazino, 3-ethoxycarbonylmethyl-4-methylhomopiperazino, 5-ethoxycarbonylmethyl-4-methylhomopiperazino, 6-ethoxycarbonylmethyl-4-methylhomopiperazino, 7-ethoxycarbonylmethyl-4-methylhomopiperazino, 2-carbamoylmethylhomopiperazino, 3-carbamoylmethylhomopiperazino, 4-carbamoylmethylhomopiperazino, 5-carbamoylmethylhomopiperazino, 6-carbamoylmethylhomopiperazino, 7-carbamoylmethylhomopiperazino, 2-carbamoylmethyl-4-methylhomopiperazino, 3-carbamoylmethyl-4-methylhomopiperazino, 5-carbamoylmethyl-4-methylhomopiperazino, 6-carbamoylmethyl-4-methylhomopiperazino, 7-carbamoylmethyl-4-methylhomopiperazino, 2-methylcarbamoylmethylhomopiperazino, 3-methylcarbamoylmethylhomopiperazino, 4-methylcarbamoylhomopiperazino, 5-methylcarbamoylhomopiperazino, 6-methylcarbamoylhomopiperazino, 7-methylcarbamoylhomopiperazino, 2-methylcarbamoylmethyl-4-methylhomopiperazino, 3-methylcarbamoylmethyl-4-methylhomopiperazino, 5-methylcarbamoyl-4-methylhomopiperazino, 6-methylcarbamoyl-4-methylhomopiperazino, 7-methylcarbamoyl-4-methylhomopiperazino, 2-dimethylcarbamoylmethylhomopiperazino, 3-dimethylcarbamoylmethylhomopiperazino, 4-dimethylcarbamoylmethylhomopiperazino, 5-dimethylcarbamoylmethylhomopiperazino, 6-dimethylcarbamoylmethylhomopiperazino, 7-dimethylcarbamoylmethylhomopiperazino, 2-dimethylcarbamoylmethyl-4-methylhomopiperazino, 3-dimethylcarbamoylmethyl-4-methylhomopiperazino, 5-dimethylcarbamoylmethyl-4-methylhomopiperazino, 6-dimethylcarbamoylmethyl-4-methylhomopiperazino, 7-dimethylcarbamoylmethyl-4-methylhomopiperazino, 2-aminomethylhomopiperazino, 3-aminomethylhomopiperazino, 5-aminomethylhomopiperazino, 6-aminomethylhomopiperazino, 7-aminomethylhomopiperazino, 2-aminomethyl-4-methylhomopiperazino, 3-aminomethyl-4-methylhomopiperazino, 5-aminomethyl-4-methylhomopiperazino, 6-aminomethyl-4-methylhomopiperazino, 7-aminomethyl-4-methylhomopiperazino, 2-methylaminomethylhomopiperazino, 3-methylaminomethylhomopiperazino, 4-methylaminomethylhomopiperazino, 5-methylaminomethylhomopiperazino, 6-methylaminomethylhomopiperazino, 7-methylaminomethylhomopiperazino, 2-methylaminomethyl-4-methylhomopiperazino, 3-methylaminomethyl-4-methylhomopiperazino, 5-methylaminomethyl-4-methylhomopiperazino, 6-methylaminomethyl-4-methylhomopiperazino, 7-methylaminomethyl-4-methylhomopiperazino, 2-dimethylaminomethylhomopiperazino, 3-dimethylaminomethylhomopiperazino, 4-dimethylaminomethylhomopiperazino, 5-dimethylaminomethylhomopiperazino, 6-dimethylaminomethylhomopiperazino, 7-dimethylaminomethylhomopiperazino, 2-dimethylaminomethyl-4-methylhomopiperazino, 3-dimethylaminomethyl-4-methylhomopiperazino, 5-dimethylaminomethyl-4-methylhomopiperazino, 6-dimethylaminomethyl-4-methylhomopiperazino, 7-dimethylaminomethyl-4-methylhomopiperazino, 2-aminoethylhomopiperazino, 3-aminoethylhomopiperazino, 4-aminoethylhomopiperazino, 5-aminoethylhomopiperazino, 6-aminoethylhomopiperazino, 7-aminoethylhomopiperazino, 2-aminoethyl-4-methylhomopiperazino, 3-aminoethyl-4-methylhomopiperazino, 5-aminoethyl-4-methylhomopiperazino, 6-aminoethyl-4-methylhomopiperazino, 7-aminoethyl-4-methylhomopiperazino, 2-methylaminoethylhomopiperazino, 3-methylaminoethylhomopiperazino, 4-methylaminoethylhomopiperazino, 5-methylaminoethylhomopiperazino, 6-methylaminoethylhomopiperazino, 7-methylaminoethylhomopiperazino, 2-methylaminoethyl-4-methylhomopiperazino, 3-methylaminoethyl-4-methylhomopiperazino, 5-methylaminoethyl-4-methylhomopiperazino, 6-methylaminoethyl-4-methylhomopiperazino, 7-methylaminoethyl-4-methylhomopiperazino, 2-dimethylaminoethylhomopiperazino, 3-dimethylaminoethylhomopiperazino, 4-dimethylaminoethylhomopiperazino, 5-dimethylaminoethylhomopiperazino, 6-dimethylaminoethylhomopiperazino, 7-dimethylaminoethylhomopiperazino, 2-dimethylaminoethyl-4-methylhomopiperazino, 3-dimethylaminoethyl-4-methylhomopiperazino, 5-dimethylaminoethyl-4-methylhomopiperazino, 6-dimethylaminoethyl-4-methylhomopiperazino, 7-dimethylaminoethyl-4-methylhomopiperazino, 4-methanesulfonylhomopiperazino, 4-methanesulfonylaminohomopiperazino, 4-(azetidin-1-yl)homopiperazino, 4-pyrrolidinohomopiperazino, 4-piperidinohomopiperazino, [1,4]oxazepan-4-yl, spiro[azetidine-3,2'-1'-methylazetidine]-1-yl, spiro[piperidine-4,2'-1'-methylazetidine]-1-yl, spiro[piperidine-2,3'-1'-methylazetidine]-1-yl, spiro[piperidine-2,3'-1'-methylpyrrolidine]-1-yl, spiro[morpholine-3,3'-1'-methylazetidine]-4-yl, spiro[morpholine-3,3'-1'-methylpyrrolidine]-4-yl, spiro[piperazine-3-cyclopropane]-1-yl, and spiro[4-methylpiperazine-3-cyclopropane]-1-yl.

Among these groups, the following groups are preferred.

Preferred examples of the group represented by formula (2) include azetidin-1-yl, 3-dimethylaminoazetidin-1-yl, 2-methylazetidin-1-yl, 3-methylazetidin-1-yl, 2,2-dimethylazetidin-1-yl, 2,4-dimethylazetidin-1-yl, 3,3-dimethylazetidin-1-yl, 2,2-dimethyl-3-dimethylaminoazetidin-1-yl, 2-hydroxymethylazetidin-1-yl, 3-hydroxymethylazetidin-1-yl, 2-methoxymethylazetidin-1-yl, 3-methoxymethylazetidin-1-yl, 2-carbamoylazetidin-1-yl, 2-methylcarbamoylazetidin-1-yl, 2-dimethylcarbamoylazetidin-1-yl, 3-methoxyazetidin-1-yl, 3-fluoroazetidin-1-yl, 2-fluoromethylazetidin-1-yl, pyrrolidino, 2-oxopyrrolidino, 2,5-dioxopyrrolidino, 2-methylpyrrolidino, 3-methylpyrrolidino, 2,2-dimethylpyrrolidino, 3,3-dimethylpyrrolidino, 2-hydroxymethylpyrrolidino, 3-hydroxymethylpyrrolidino, 2-methoxymethylpyrrolidino, 3-methoxymethylpyrrolidino, 2-carbamoylpyrrolidino, 2-methylcarbamoylpyrrolidino, 2-dimethylcarbamoylpyrrolidino, 2-fluoromethylpyrrolidino, 3-fluoromethylpyrrolidino, 3-fluoropyrrolidino, 3-methoxypyrrolidino, 2-oxoimidazolidin-1-yl, 4-oxoimidazolidin-1-yl, 3-methyl-2-oxoimidazolidin-1-yl, 3-methyl-4-oxoimidazolidin-1-yl, 2-methylpyrazolidin-1-yl, 3-oxopyrazolidin-1-yl, 3,5-dioxopyrazolidin-1-yl, piperidino, 2-oxopiperidino, 3-oxopiperidino, 4-oxopiperidino, 2-hydroxyiminopiperidino, 3-hydroxyiminopiperidino, 4-hydroxyiminopiperidino, 2-methoxyiminopiperidino, 3-methoxyiminopiperidino, 4-methoxyiminopiperidino, 2-methylpiperidino, 3-methylpiperidino, 4-methylpiperidino, 2,2-dimethylpiperidino, 3,3-dimethylpiperidino, 4,4-dimethylpiperidino, 2,5-dimethylpiperidino, 2-hydroxymethylpiperidino, 2-carbamoylpiperidino, 2-methylcarbamoylpiperidino, 2-dimethylcarbamoylpiperidino, 2-carboxymethylpiperidino, 2-methoxymethylpiperidino, 2-aminomethylpiperidino, 2-methylaminomethylpiperidino, 2-dimethylaminomethylpiperidino, 2-aminoethylpiperidino, 2-methylaminoethylpiperidino, 2-dimethylaminoethylpiperidino, 4-methoxypiperidino, 4-fluoropiperidino, 4,4-difluoropiperidino, 2-oxo-4-methylpiperazino, 3-oxo-4-methylpiperazino, 2,3-dioxo-4-methylpiperazino, 3,5-dioxo-4-methylpiperazino, 2,6-dioxo-4-methylpiperazino, 4-methylpiperazino, 4-ethylpiperazino, 4-isopropylpiperazino, 2,4-dimethylpiperazino, 3,4-dimethylpiperazino, 2-ethyl-4-methyl-piperazino, 3-ethyl-4-methylpiperazino, 2-isopropyl-4-methylpiperazino, 3-isopropyl-4-methylpiperazino, 2-cyclopropyl-4-methylpiperazino, 3-cyclopropyl-4-methylpiperazino, 3,4,5-trimethylpiperazino, 2,2,4-trimethylpiperazino, 3,3,4-trimethylpiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiro-4-methyl-5-oxopiperazino, 4-acetyl-3-cyclopropanespiropiperazino, 2-hydroxymethyl-4-methylpiperazino, 3-hydroxymethyl-4-methylpiperazino, 2-methoxymethyl-4-methylpiperazino, 3-methoxymethyl-4-methylpiperazino, 2-hydroxyethyl-4-methylpiperazino, 3-hydroxyethyl-4-methylpiperazino, 2-methoxyethyl-4-methylpiperazino, 3-methoxyethyl-4-methylpiperazino, 2-carbamoyl-4-methylpiperazino, 3-carbamoyl-4-methylpiperazino, 4-carbamoylpiperazino, 2-methylcarbamoyl-4-methylpiperazino, 3-methylcarbamoyl-4-methylpiperazino, 4-methylcarbamoylpiperazino, 2-dimethylcarbamoyl-4-methylpiperazino, 3-dimethylcarbamoyl-4-methylpiperazino, 4-dimethylcarbamoylpiperazino, 2-carbamoylmethyl-4-methylpiperazino, 3-carbamoylmethyl-4-methylpiperazino, 4-carbamoylmethylpiperazino, 2-methylcarbamoylmethyl-4-methylpiperazino, 3-methylcarbamoylmethyl-4-methylpiperazino, 4-methylcarbamoylpiperazino, 2-dimethylcarbamoylmethyl-4-methylpiperazino, 3-dimethylcarbamoylmethyl-4-methylpiperazino, 2-carboxy-4-methylpiperazino, 2-carboxymethyl-4-methylpiperazino, 2-methoxycarbonylmethyl-4-methylpiperazino, 3-methoxycarbonylmethyl-4-methylpiperazino, 2-ethoxycarbonylmethyl-4-methylpiperazino, 3-ethoxycarbonylmethyl-4-methylpiperazino, 2-aminomethyl-4-methylpiperazino, 2-methylaminomethyl-4-methylpiperazino, 2-dimethylaminomethyl-4-methylpiperazino, 2-aminoethyl-4-methylpiperazino, 2-methylaminoethyl-4-methylpiperazino, 2-dimethylaminoethyl-4-methylpiperazino, morpholino, 2-methylmorpholino, 3-methylmorpholino, 2-ethylmorpholino, 3-ethylmorpholino, 2-cyclopropanespiromorpholino, 3-cyclopropanespiromorpholino, 2,2-dimethylmorpholino, 3,3-dimethylmorpholino, 3-hydroxymethylmorpholino, 3-methoxymethylmorpholino, 3-hydroxyethylmorpholino, 3-methoxyethylmorpholino, 3-carbamoylmorpholino, 3-methylcarbamoylmorpholino, 3-dimethylcarbamoylmorpholino, 3-carbamoylmethylmorpholino, 3-methylcarbamoylmethylmorpholino, 3-dimethylcarbamoylmethylmorpholino, 3-carbamoylethylmorpholino, 3-methylcarbamoylethylmorpholino, 3-dimethylcarbamoylethylmorpholino, 3-methoxycarbonylmorpholino, 3-methoxycarbonylmethylmorpholino, 3-ethoxycarbonylmethylmorpholino, 3-aminomethylmorpholino, 3-methylaminomethylmorpholino, 3-dimethylaminomethylmorpholino, 3-aminoethylmorpholino, 3-methylaminoethylmorpholino, 3-dimethylaminoethylmorpholino, thiomorpholino, 3-oxothiomorpholino, 1,1-dioxothiomorpholino, 2-methylthiomorpholino, 3-methylthiomorpholino, 2-ethylthiomorpholino, 3-ethylthiomorpholino, 2-cyclopropanespirothiomorpholino, 3-cyclopropanespirothiomorpholino, 2,2-dimethylthiomorpholino, 3,3-dimethylthiomorpholino, 3-hydroxymethylthiomorpholino, 3-methoxymethylthiomorpholino, 3-hydroxyethylthiomorpholino, 3-methoxyethylthiomorpholino, 3-carbamoylthiomorpholino, 3-methylcarbamoylthiomorpholino, 3-dimethylcarbamoylthiomorpholino, 3-carbamoylmethylthiomorpholino, 3-methylcarbamoylmethylthiomorpholino, 3-dimethylcarbamoylmethylthiomorpholino, 3-carbamoylethylthiomorpholino, 3-methylcarbamoylethylthiomorpholino, 3-dimethylcarbamoylethylthiomorpholino, 3-methoxycarbonylthiomorpholino, 3-methoxycarbonylmethylthiomorpholino, 3-ethoxycarbonylmethylthiomorpholino, 3-oxohexahydropyridazin-1-yl, 6-oxohexahydropyridazin-1-yl, 2,3-dimethylhexahydropyridazin-1-yl, 3-hydroxymethylhexahydropyridazin-1-yl, 5-hydroxymethylhexahydropyridazin-1-yl, 6-hydroxymethylhexahydropyridazin-1-yl, 2-carbamoylhexahydropyridazin-1-yl, 2-methylcarbamoylhexahydropyridazin-1-yl, 2-dimethylcarbamoylhexahydropyridazin-1-yl, 2-oxohexahydropyrimidin-1-yl, 4-oxohexahydropyrimidin-1-yl, 6-oxohexahydropyrimidin-1-yl, 2-methylhexahydropyrimidin-1-yl, 3-methylhexahydropyrimidin-1-yl, 3-carbamoylhexahydropyrimidin-1-yl, 3-methylcarbamoylhexahydropyrimidin-1-yl, 3-dimethylcarbamoylhexahydropyrimidin-1-yl, 2-oxo-4-methylhomopiperazino, 3-oxo-4-methylhomopiperazino, 5-oxo-4-methylhomopiperazino, 6-oxo-4-methylhomopiperazino, 7-oxo-4-methylhomopiperazino, 2,3-dioxohomopiperazino, 2,7-dioxohomopiperazino, 3,5-dioxohomopiperazino, 3,7-dioxohomopiperazino, 2,3-dioxo-4-methylhomopiperazino, 2,7-dioxo-4-methylhomopiperazino, 3,5-dioxo-4-methylhomopiperazino, 3,7-dioxo-4-methylhomopiperazino, 4-methylhomopiperazino, 4-ethylhomopiperazino, 4-cyclopropylhomopiperazino, 2-cyclopropanespirohomopiperazino, 3-cyclopropanespirohomopiperazino, 5-cyclopropanespirohomopiperazino, 6-cyclopropanespirohomopiperazino, 7-cyclopropanespirohomopiperazino, 2,4-dimethylhomopiperazino, 3,4-dimethylhomopiperazino, 3,4,5-trimethylhomopiperazino, 2-hydroxymethyl-4-methylhomopiperazino, 7-hydroxymethyl-4-methylhomopiperazino, 2-methoxymethyl-4-methylhomopiperazino, 3-methoxymethyl-4-methylhomopiperazino, 5-methoxymethyl-4-methylhomopiperazino, 6-methoxymethyl-4-methylhomopiperazino, 7-methoxymethyl-4-methylhomopiperazino, 2-hydroxyethyl4-methylhomopiperazino, 7-hydroxyethyl-4-methylhomopiperazino, 2-methoxyethyl-4-methylhomopiperazino, 3-methoxyethyl-4-methylhomopiperazino, 5-methoxyethyl-4-methylhomopiperazino, 6-methoxyethyl-4-methylhomopiperazino, 7-methoxyethyl-4-methylhomopiperazino, 2-carbamoyl-4-methylhomopiperazino, 7-carbamoyl-4-methylhomopiperazino, 2-methylcarbamoyl-4-methylhomopiperazino, 7-methylcarbamoyl-4-methylhomopiperazino, 2-dimethylcarbamoylhomopiperazino, 7-dimethylcarbamoylhomopiperazino, 2-carboxyhomopiperazino, 7-carboxyhomopiperazino, 2-carboxy-4-methylhomopiperazino, 7-carboxy-4-methylhomopiperazino, 2-carboxymethyl-4-methylhomopiperazino, 7-carboxymethyl-4-methylhomopiperazino, and [1,4]oxazepan-4-yl.

Among them, particularly preferred are the following.

Particularly preferred examples of the group represented by formula (2) include azetidin-1-yl, 2-methylazetidin-1-yl, 3-dimethylaminoazetidin-1-yl, 2,2-dimethyl-3-dimethylaminoazetidin-1-yl, 2-hydroxymethylazetidin-1-yl, 2-carbamoylazetidin-1-yl, 2-methylcarbamoylazetidin-1-yl, 2-dimethylcarbamoylazetidin-1-yl, 3-methoxyazetidin-1-yl, 3-fluoroazetidin-1-yl, 2-fluoromethylazetidin-1-yl, pyrrolidino, 2-oxopyrrolidino, 2,5-dioxopyrrolidino, 2-methylpyrrolidino, 3-methylpyrrolidino, 2,2-dimethylpyrrolidino, 3,3-dimethylpyrrolidino, 2-hydroxymethylpyrrolidino, 2-methoxymethylpyrrolidino, 2-carbamoylpyrrolidino, 2-methylcarbamoylpyrrolidino, 2-dimethylcarbamoylpyrrolidino, 2-fluoromethylpyrrolidino, 3-fluoromethylpyrrolidino, 3-methoxypyrrolidino, 3-methyl-2-oxoimidazolidin-1-yl, piperidino, 2-oxopiperidino, 2-hydroxymethylpiperidino, 2-carbamoylpiperidino, 2-methylcarbamoylpiperidino, 2-dimethylcarbamoylpiperidino, 2-methoxymethylpiperidino, 2-aminomethylpiperidino, 2-methylaminomethylpiperidino, 2-dimethylaminomethylpiperidino, 2-aminoethylpiperidino, 2-methylaminoethylpiperidino, 2-dimethylaminoethylpiperidino, 4-methoxypiperidino, 4-fluoropiperidino, 4,4-difluoropiperidino, 2-oxo-4-methylpiperazino, 3-oxo-4-methylpiperazino, 2,3-dioxopiperazino, 3,5-dioxopiperazino, 2,6-dioxopiperazino, 4-methylpiperazino, 4-ethylpiperazino, 4-isopropylpiperazino, 4-cyclopropylpiperazino, 2,4-dimethylpiperazino, 3,4-dimethylpiperazino, 3,4,5-trimethylpiperazino, 2,2,4-trimethylpiperazino, 3,3,4-trimethylpiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiro-4-methyl-5-oxopiperazino, 4-acetyl-3-cyclopropanespiropiperazino, 2-hydroxymethyl-4-methylpiperazino, 3-hydroxymethyl-4methylpiperazino, 2-methoxymethyl-4-methyl-piperazino, 3-methoxymethyl-4-methylpiperazino, 2-hydroxyethyl-4-methylpiperazino, 3-hydroxyethyl-4methylpiperazino, 2-methoxyethyl-4-methylpiperazino, 3-methoxyethyl-4-methylpiperazino, 2-carbamoyl-4-methylpiperazino, 2-methylcarbamoyl-4-methylpiperazino, 2-dimethylcarbamoyl-4-methylpiperazino, 2-carbamoylmethyl-4-methylpiperazino, 2-methylcarbamoylmethyl-4-methylpiperazino, 2-dimethylcarbamoylmethyl-4-methylpiperazino, 2-methoxycarbonylmethyl-4-methylpiperazino, 2-ethoxycarbonylmethyl-4-methylpiperazino, 2-aminomethyl-4-methylpiperazino, 2-methylaminomethyl-4-methylpiperazino, 2-dimethylaminomethyl-4-methylpiperazino, 2-aminoethyl-4-methylpiperazino, 2-methylaminoethyl-4-methylpiperazino, 2-dimethylaminoethyl-4-methylpiperazino, morpholino, 2-cyclopropanespiromorpholino, 3-cyclopropanespiromorpholino, 2,2-dimethylmorpholino, 3,3-dimethylmorpholino, 3-hydroxymethylmorpholino, 3-methoxymethylmorpholino, 3-hydroxyethylmorpholino, 3-methoxyethylmorpholino, 3-carbamoylmorpholino, 3-methylcarbamoylmorpholino, 3-dimethylcarbamoylmorpholino, 3-aminomethylmorpholino, 3-methylaminomethylmorpholino, 3-dimethylaminomethylmorpholino, 3-aminoethylmorpholino, 3-methylaminoethylmorpholino, 3-dimethylaminoethylmorpholino, thiomorpholino, 3-oxothiomorpholino, 1,1-dioxothiomorpholino, 3-hydroxymethylthiomorpholino, 3-hydroxyethylthiomorpholino, 3-oxohexahydropyridazin-1-yl, 2-methylhexahydropyridazin-1-yl, 2-oxohexahydropyrimidin-1-yl, 4-oxohexahydropyrimidin-1-yl, 3-methylhexahydropyrimidin-1-yl, 6-hydroxymethylhexahydropyrimidin-1-yl, 2-oxo-4-methylhomopiperazino, 3-oxo-4-methylhomopiperazino, 5-oxo-4-methylhomopiperazino, 7-oxo-4-methylhomopiperazino, 2,3-dioxohomopiperazino, 2,7-dioxohomopiperazino, 3,5-dioxohomopiperazino, 3,7-dioxohomopiperazino, 4-methylhomopiperazino, 4-ethylhomopiperazino, 4-cyclopropylhomopiperazino, 2-cyclopropanespiro-4-methylhomopiperazino, 3-cyclopropanespiro-4-methylhomopiperazino, 5-cyclopropanespiro-4-methylhomopiperazino, and 7-cyclopropanespiro-4-methylhomopiperazino. Still more preferred Examples thereof include 3-dimethylaminoazetidin-1-yl, 2,2-dimethyl-3-dimethylaminoazetidin-1-yl, 2-hydroxymethylazetidin-1-yl, 2-carbamoylazetidin-1-yl, 2-oxopyrrolidino, 2-hydroxymethylpyrrolidino, 2-carbamoylpyrrolidino, 2-hydroxymethylpiperidino, 2-carbamoylpiperidino, 2-methylcarbamoylpiperidino, 2-dimethylcarbamoylpiperidino, 3-oxo-4-methylpiperazino, 4-methylpiperazino, 4-ethylpiperazino, 4-isopropylpiperazino, 4-cyclopropylpiperazino, 2,4-dimethylpiperazino, 3,4-dimethylpiperazino, 3-cyclopropyl-4-methylpiperazino, 3,4,5-trimethylpiperazino, 2,2,4-trimethylpiperazino, 3,3,4-trimethylpiperazino, 2-cyclopropanespiro-4-methylpiperazino, morpholino, 3-carbamoylmorpholino, 1,1-dioxothiomorpholino, 3-oxo-4-methylhomopiperazino, 5-oxo-4-methylhomopiperazino, 4-methylhomopiperazino, 4-ethylhomopiperazino, 4-cyclopropylhomopiperazino, and 1,4-oxazepan-4-yl.

When the group represented by formula (1) is the group represented by formula (b), R¹ in formula (2) is preferably 1 to 4 groups selected from among a cyano group, an oxo group, a halogeno group, a lower alkyl group substituted by a halogeno group, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3- to 6-membered spiro-alicyclic alkyl group which may have a substituent, and a 4-to 7-membered alicyclic heterocyclic group which may have a substituent.

Preferred is the case where, in formula (2), the ring structure A is a ring selected from among azetidine, pyrrolidine, piperidine, piperazine, morpholine, homopiperazine, and oxazepane, and R¹ is 1 to 4 groups, which are identical to or different from one another, selected from among a hydroxyl group, a cyano group, an oxo group, a halogeno group, a lower alkyl group which may have a substituent, lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3- to 6-membered alicyclic spiro-alkyl group which may have a substituent, and a 4- to 7-membered alicyclic heterocyclic group which may have a substituent.

More preferred is the case where, in formula (2), the ring structure A is a ring selected from among azetidine, pyrrolidine, piperidine, piperazine, morpholine, homopiperazine, and oxazepane, and R¹ is 1 to 4 groups, which are identical to or different from one another, selected from among a hydroxyl group, a cyano group, an oxo group, a halogeno group, a lower alkyl group which has been substituted by a halogeno group, lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3-to 6-membered alicyclic spiro-alkyl group which may have a substituent, and a 4- to 7-membered alicyclic heterocyclic group which may have a substituent.

Still more preferred is the case where, in formula (2), the ring structure A is a ring selected from among azetidine, pyrrolidine, piperidine, piperazine, morpholine, homopiperazine, and oxazepane, and R¹ is 1 to 4 groups, which are identical to or different from one another, of halogeno groups and lower alkyl groups which has been substituted by a halogeno group.

All the compounds (I) of the present invention do not necessarily form salts. However, when the compound (I) has a carboxyl group, an amino group, or a like group, and/or when Ar₁ or Ar₂ is a pyridine ring or an analogous ring, the compound can form a salt, and in some cases, the salt may form a solvate. Examples of the salt include salts of inorganic acids such as hydrochloric acid, hydrobromic acid, sulfuric acid, and nitric acid; salts of organic acids such as methanesulfonic acid, p-toluenesulfonic acid, fumaric acid, and trifluoroacetic acid; and salts of alkali metal ions or alkaline earth metal ions, such as sodium ion, potassium ion, or calcium ion.

Next will be described a representative method for producing the compound (I) of the present invention.

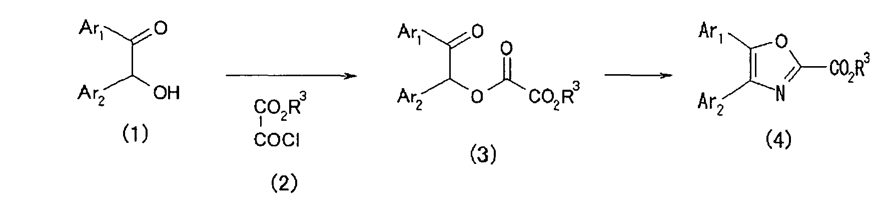

In the above reaction scheme, Ar₁ and Ar₂ have the same meanings as described above, and R³ represents a C1-C6 linear alkyl group.

An oxazole compound (4) is produced. Specifically, a benzoin compound (1) is dissolved in a solvent such as tetrahydrofuran, and the solution is reacted with an acid chloride (2) in the presence of an organic amine such as triethylamine or diisopropylethylamine, to thereby give an ester compound (3). The reaction temperature is preferably - 20 to 50°C. Subsequently, the ester compound (3) is dissolved in a solvent such as acetic acid, and ammonium acetate is added to the solution, followed by reflux, to thereby give an oxazole compound (4).

Alternatively, the oxazole compound (4) may be produced through the following [Method A] or [Method B].

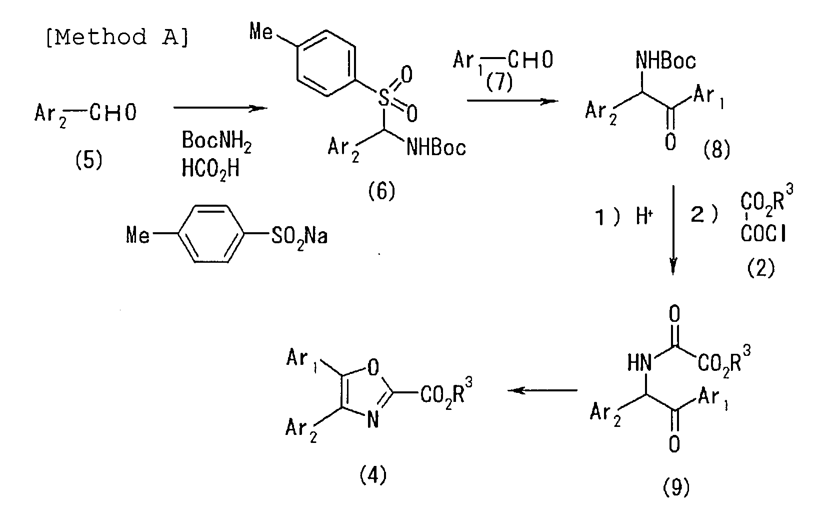

In the above reaction scheme, Ar₁, Ar₂, and R³ have the same meanings as described above, and Boc represents a tert-butoxycarbonyl group (Me₃COCO-).

Specifically, an aldehyde compound (5) is dissolved in a solvent such as a solvent mixture of tetrahydrofuran and water. Carbamic acid tert-butyl ester, sodium p-toluenesulfinate, and formic acid are added to the solution, followed by stirring, to thereby give a compound (6). The aldehyde compound (5) may be a commercially available product or may be produced through a method described in Referential Examples or a similar method.

Subsequently, the compound (6) is dissolved in a solvent such as methylene chloride. 3-Benzyl-5-(2-hydroxyethyl)-4-methylthiazolium chloride, an aldehyde compound (7), and triethylamine are added to the solution, and the mixture was stirred, to thereby give a compound (8). The reaction temperature is preferably 0 to 50°C. Conditions and reagents employed in this reaction may be determined appropriately based on a common knowledge of organic chemistry. The aldehyde compound (7) may be a commercially available product or may be produced through a method described in Referential Examples or a similar method.

The compound (8) is treated with trifluoroacetic acid, and the obtained compound is dissolved in a solvent such as methylene chloride. An organic amine such as triethylamine is added to the solution, and the mixture is reacted with an acid chloride (2) at -20 to 50°C, to thereby give an oxamide compound (9). The deprotection of the compound (8) may be carried out through use of acid/solvent such as trifluoroacetic acid/methylene chloride, HCl/ethyl acetate, or HCl/dioxane. The reaction temperature of the deprotection reaction is -50 to 50°C, preferably 0°C to room temperature. Notably, the tert-butoxycarbonyl group employed as a protecting group for the nitrogen atom in the above step may be replaced by a similar group capable of protecting nitrogen. However, tert-butoxycarbonyl is preferred in view of, among other factors, reaction yield, deprotection conditions, and convenience.

Transformation of the oxamide compound (9) to an oxazole compound (4) may be performed through the following process: triphenylphosphine is dissolved in a solvent such as methylene chloride; hexachloroethane, triethylamine, and the oxamide compound (9) are sequentially added to the solution; and the mixture is stirred.

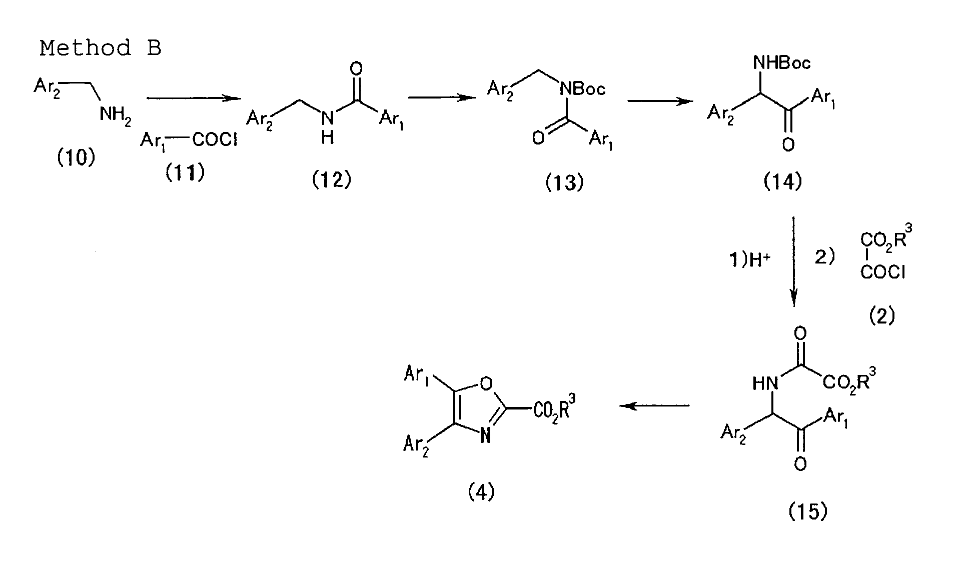

In the above reaction scheme, Ar₁, Ar₂, R³, and Boc have the same meanings as described above.

Specifically, a benzylamine compound (10) is dissolved in a solvent such as methylene chloride, and an organic amine such as triethylamine or diisopropylethylamine is added to the solution. The resultant mixture is reacted with an acid chloride (11), to thereby yield an amide compound (12). The reaction temperature is preferably -20 to 50°C. Each of the benzylamine compound (10) and the acid chloride (11) may be a commercially available product or may be produced through a method described in Referential Examples or a similar method.

Thereafter, the amide compound (12) is dissolved in acetonitrile, and the solution is treated with di-tert-butylcarbonate in the presence of an organic amine such as dimethylaminopyridine, to thereby yield a carbamic acid compound (13). The reaction temperature is preferably -20 to 50°C.

Transformation of the carbamic acid compound (13) into a compound (14) may be performed through the following process: diisopropylamine and N,N'-dimethylpropylurea are dissolved in an inert solvent such as tetrahydrofuran and then treated with n-butyllithium, to thereby produce a reagent; the carbamic acid compound (13) is added to the reagent; and the mixture is stirred. The reaction temperature is preferably -100 to 0°C, more preferably -100 to -50°C .

The compound (14) is treated with an appropriate acid such as hydrochloric acid and then dissolved in a solvent such as methylene chloride. The solution is treated with an acid chloride (2) at -20 to 50°C in the presence of an organic amine such as triethylamine, to thereby give an oxamide compound (15). The deprotection of the compound (14) may be performed through use of an acid/solvent such as HCl/ethanol, HCl/ethyl acetate, HCl/dioxane, or trifluoroacetic acid/methylene chloride. The reaction temperature is -50 to 50°C, preferably 0°C to room temperature. As a group for protecting the nitrogen atom in the above step, tert-butoxycarbonyl is preferred in view of, among other factors, reaction yield, deprotection conditions, and convenience. However, no particular limitation is imposed on the protecting group, and any suitable protecting group may be selected based on a common knowledge of organic chemistry.

Transformation of the oxamic acid compound (15) into an oxazole compound (4) may be performed through reflux of a solution of the oxamide compound (15) in phosphorus oxychloride.

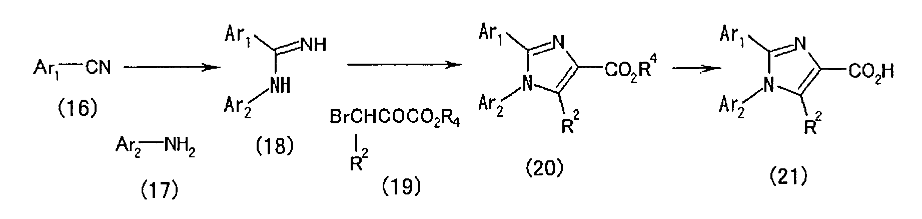

In the above reaction scheme, Ar₁, Ar₂, and R² have the same meanings as described above, and R⁴ represents a C1-C6 linear alkyl group.

A compound (16) is dissolved in an inert solvent such as tetrahydrofuran, and the solution is treated with sodium bis(trimethylsilyl)amide at -20 to 40°C under argon or under nitrogen flow. Subsequently, an amine compound (17) is added to the reaction mixture, to thereby give an amidine compound (18). Each of the compound (16) and the amine compound (17) may be a commercially available product or may be produced through a method described in Referential Examples or a similar method.

Subsequently, the amidine compound (18) is dissolved in ethanol, and a bromopyruvic acid alkyl ester (19) and potassium carbonate are added to the solution at room temperature. The mixture is refluxed, and a suitable amount of acetic acid is added to the mixture, followed by reflux, to thereby give an imidazole compound (20).

In the above process for producing the imidazole compound (20), an imidazole compound (20) may be produced by, without adding acetic acid, refluxing a solution of the amidine compound (18) and a bromopyruvic acid alkyl ester (19) in ethanol. In some cases, neither potassium carbonate nor acetic acid may be added.

The imidazole compound (20) is hydrolyzed through a common method, to thereby give an imidazole compound (21). The hydrolysis reaction in this step may be performed in the presence of a base or a Lewis acid. Examples of the base include hydroxides of alkaline metals (e.g., lithium, sodium, and potassium). Examples of the Lewis acid include boron tribromide. The reaction temperature is preferably -20 to 100°C, more preferably -10 to 50°C.

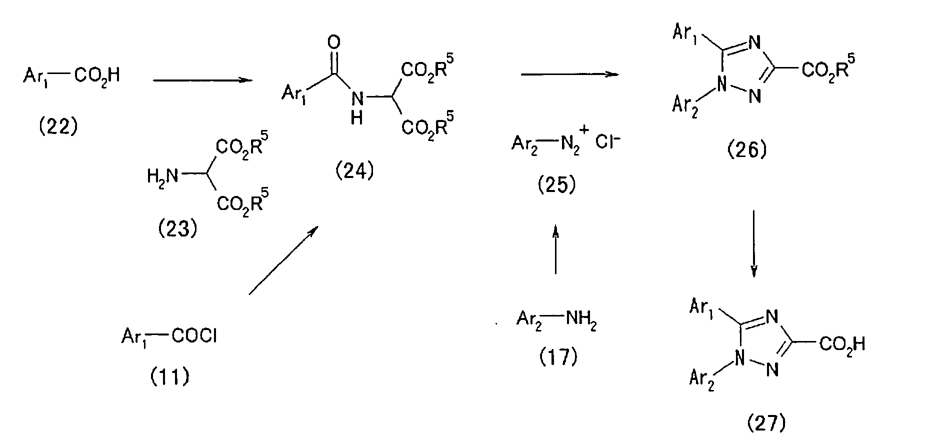

In the above reaction scheme, Ar₁ and Ar₂ have the same meanings as described above, and R⁵ represents a C1-C6 linear alkyl group.

A carboxylic acid (22) and an amine compound (23) are condensed together, to thereby yield an amide compound (24). The amide compound (24) may be produced by dissolving an acid chloride (11) in methylene chloride and then treating the solution with an amine compound (23) in the presence of an organic amine such as triethylamine.

The condensation process described above may be performed through a method generally used for peptide synthesis. Examples of the method for peptide synthesis include the azide method, the acid chloride method, the acid anhydride method, the DCC (dicyclohexylcarbodiimide) method, the active ester method, the carbodiimidazole method, the DCC/HOBT(1-hydroxybenzotriazole) method, a method using water-soluble carbodiimide, and a method using diethyl cyanophosphate. These methods are described in, for example, M. Bodanszky, Y.S. Klausner, and M.A. Ondetti, "Peptide Synthesis," A Wiley-interscience publication, New York, 1976; G.R. Pettit, "Synthetic Peptides," Elsevier Scientific Publication Company, New York, 1976; and Japanese Society of Chemistry ed. "Lectures on Experimental Chemistry 4th ed., vol. 22, Organic Synthesis IV," Maruzen Co., Ltd, 1991. Examples of a solvent used in the fusion reaction include N,N-dimethylformamide, pyridine, chloroform, methylene chloride, tetrahydrofuran, dioxane, acetonitrile, and a solvent mixture thereof. The reaction temperature is preferably -20 to 50°C, more preferably -10 to 30°C. Each of the carboxylic acid (22) and the acid chloride (11) may be a commercially available product or may be produced through any of the methods described in Referential Examples or a similar method.

An amine compound (17) is dissolved in acetic acid and concentrated hydrochloric acid, and the solution is treated with sodium nitrite to thereby give a solution of a diazonium salt (25). To the solution, an amide compound (24) in acetone and potassium carbonate are gradually added. Without being subjected to any further isolation and purification steps, the resultant addition compound is dissolved in anhydrous methanol. The solution is treated with sodium methoxide, to thereby yield a triazole compound (26). The reaction temperature is preferably -30 to 20°C. The triazole compound (26) is hydrolyzed through a common method, to therby yield a triazole compound (27). The above triazole ring formation reaction may be performed in accordance with the method described in Helv. Chim. Acta., Vol. 73, p. 1701 (1990).

The above hydrolysis reaction may be performed in the presence of a base or a Lewis acid. Examples of the base include hydroxides of alkaline metals (e.g., lithium, sodium, and potassium). Examples of the Lewis acid include boron tribromide. The reaction temperature is preferably -20 to 100°C, more preferably -5 to 50°C.

The compound (I) of the present invention may be produced through reaction of an oxazole compound (4) obtained in the above production process with an amine compound (28).

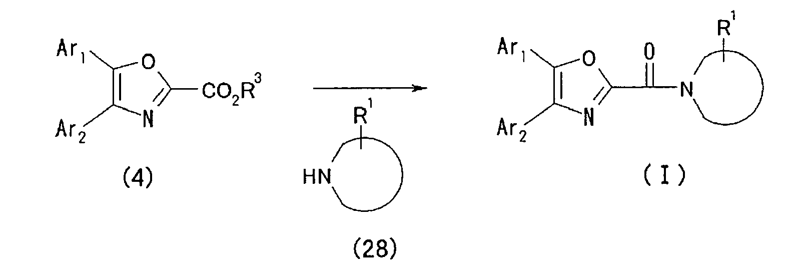

In the above reaction scheme, Ar₁, Ar₂, R¹, and R³ have the same meanings as described above.

Specifically, an oxazole compound (4) and an amine compound (28) are stirred under heating with no use of a solvent, to thereby yield the compound (I) of the present invention. The reaction temperature is preferably 50 to 120°C. The amine compound (28) may be a commercially available product or may be produced through any of the methods described in Referential Examples or a similar method.

Alternatively, the compound (I) of the present invention may be produced through reaction of a triazole compound (26) obtained in the above production process with an amine compound (28).

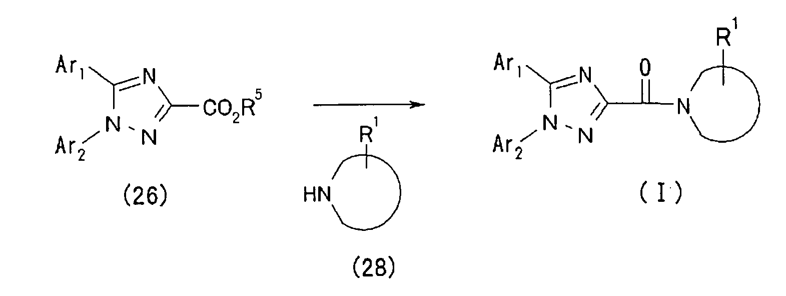

In the above reaction scheme, Ar₁, Ar₂, R¹, and R⁵ have the same meanings as described above.

Specifically, a triazole compound (26) and an amine compound (28) are stirred under heating with no use of a solvent, to thereby yield the compound (I) of the present invention. The reaction temperature is preferably 50 to 120°C. The amine compound (28) may be a commercially available product or may be produced through any of the methods described in Referential Examples or a similar method.

Alternatively, the compound (I) of the present invention may be produced by use of an imidazole compound (21) or a triazole compound (27) and an amine compound (28) through the corresponding one of the following processes.

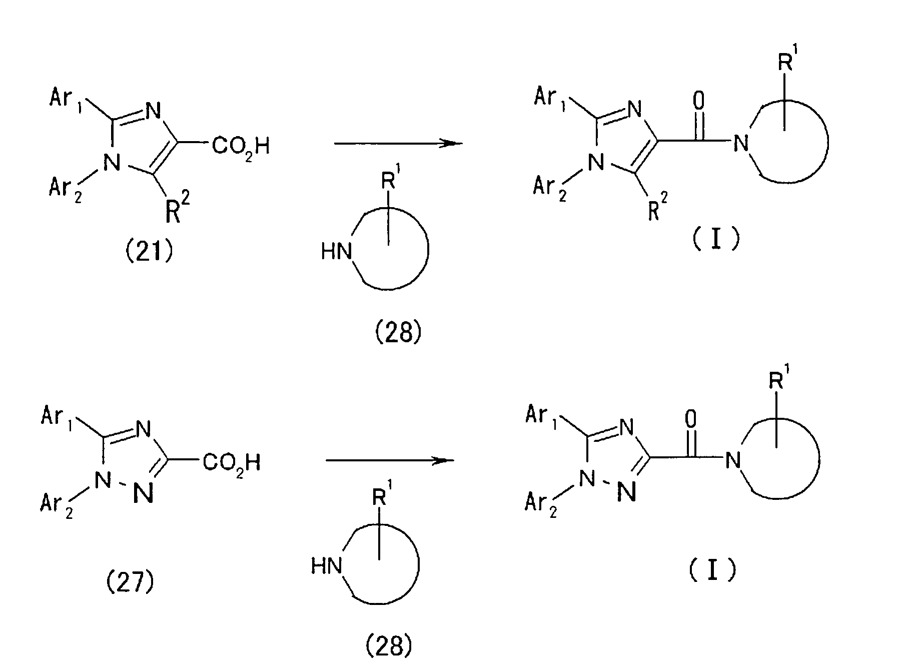

In the above reaction schemes, Ar₁, Ar₂, R¹ and R² have the same meanings as described above.

Specifically, an imidazole compound (21) or a triazole compound (27) is condensed with an amine compound (28), to thereby give the imidazole or triazole compound (I) of the present invention.

The condensation process described above may be performed through a method generally used for peptide synthesis. Examples of the method for peptide synthesis include the azide method, the acid chloride method, the acid anhydride method, the DCC (dicyclohexylcarbodiimide) method, the active ester method, the carbodiimidazole method, the DCC/HOBT(1-hydroxybenzotriazole) method, a method using water-soluble carbodiimide, and a method using diethyl cyanophosphate. These methods are described in, for example, M. Bodanszky, Y.S. Klausner, and M.A. Ondetti, "Peptide Synthesis," A Wiley-interscience publication, New York, 1976; G.R. Pettit, "Synthetic Peptides," Elsevier Scientific Publication Company, New York, 1976; and Japanese Society of Chemistry ed. "Lectures on Experimental Chemistry 4th ed., vol. 22, Organic Synthesis IV," Maruzen Co., Ltd., 1991. Examples of a solvent used in the fusion reaction include N,N-dimethylformamide, pyridine, chloroform, methylene chloride, tetrahydrofuran, dioxane, acetonitrile, and a solvent mixture thereof. The reaction temperature is preferably -20 to 50°C, more preferably -10 to 30°C.

When the amine compound (28) used in the condensation reaction described above has a functional group such as a hydroxyl group, an amino group, or a carboxyl group, the functional group may have to be protected in advance by use of a suitable protective group. Examples of a typical protective group for a hydroxyl group include a tert-butyl group and a benzyl group. Examples of a typical protective group for an amino group include a trifluoroacetyl group, a tert-butoxycarbonyl group, and a benzyloxycarbonyl group. When the functional group is a carboxyl group, the amine compound (10) may be transformed to a methyl ester or a tert-butyl ester prior to the fusion reaction. Such protective groups can be removed under suitable conditions, which vary depending on the type of protective group.

A compound (I) of the present invention produced through any of the above three processes can be transformed to another compound (I) of the present invention through chemical modifications based on a common knowledge of organic chemistry.

The compound (I) of the present invention, salts or solvates thereof, and solvates of the salts are endowed with potent anti-platelet activity, and they exhibited effectiveness in a high shear stress-induced thrombosis model. Therefore, the compound (I) of the present invention, salts or solvates thereof, or solvates of the salts are useful as preventive and/or therapeutic agents for ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting (CABG), percutaneous transluminal coronary angioplasty (PTCA), stent placement, etc.), diabetic retinopathy and nephropathy, and embolism after replacement with an artificial heart valve, and also, as a preventive and/or therapeutic agent for thrombus and embolus associated with vascular operation, blood extracorporeal circulation, and the like.

Moreover, the compounds (I) of the present invention, salts thereof, and solvates of the compounds or the salts are useful for ameliorating ischemic conditions such as ulcer, pain, and chill, which accompany chronic arteriosclerosis obliterans.

When the compound (I) of the present invention, a salt or a solvate of the compound, or a solvate of the salt is used as a drug, the daily dose for an adult, which varies depending on the age, sex, symptoms of the patient, etc., is preferably 0.1 mg to 1 g, more preferably 0.5 mg to 500 mg. The drug may be administered once a day or several times a day in a divided manner. If necessary, the compound/salt/solvate may be administered at a dose exceeding the above daily dose.

No particular limitation is imposed on the administration route and the dosage form of a drug containing the compound (I) of the present invention, a salt of the compound, or a solvate of the compound or the salt, and the drug may be administered via any route and in any dosage form as desired. The dosage form may be determined appropriately depending on the administration route. The drug preparation may be produced through a common drug preparation method by incorporating a pharmacologically acceptable carrier as desired.

Examples of oral preparations include solid preparations such as tablets, powders, granules, pills, and capsules, as well as liquid preparations such as solution, syrup, elixir, suspension, and emulsion.

An injection may be prepared by filling a container with a solution of a compound (I), a salt of the compound, or a solvate of the compound or the salt. A solid prepared by, for example, freeze-drying such a solution may also be used as an injection which is rehydrated before use.

In the production of such drug preparation, one or more pharmaceutically acceptable additives selected, in accordance with needs, from among a binder, a disintegrant, a dissolution promoter, a lubricant, a filler, an excipient, and similar additives may be incorporated into the drug preparation.

Next will be described methods for producing exemplary compounds of the present invention and tests carried out through use of some representative compounds, which reveal that the compounds of the present invention have strong platelet coagulation inhibitory activity without inhibiting COX-1 or COX-2.

### Examples

### [Referential Example 1] 2-Phenyl-1-(3-pyridyl)-1H-imidazole-4-carboxylic acid

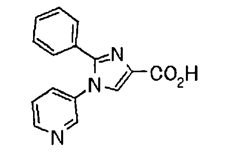

### 1) N-(3-Pyridyl)benzamidine

3-Aminopyridine (8.55 g) in tetrahydrofuran (40 mL) was added dropwise at room temperature to sodium bis(trimethylsilyl)amide (17.5 g) in tetrahydrofuran (100 mL) over 20 minutes, followed by stirring for 20 minutes. Benzonitrile (9.84 g) in tetrahydrofuran (50 mL) was added dropwise to the reaction mixture over 20 minutes, followed by stirring for 3 hours. The reaction solvent was evaporated under reduced pressure. Water and tetrahydrofuran were added to the residue, and the crystals that precipitated were collected by filtration, washed with a hexane-diethyl ether (7:3) solvent mixture, and then dried, to thereby give benzamidine compound (14.0 g, 74.1%).
¹H-NMR(300MHz,DMSO-d₆)δ:6.57(2H,br s), 7.23-7.45(5H,m), 7.96-8.17(4H, m).
MS(FAB)m/z:198(M+H)⁺.

### 2) 2-Phenyl-1-(3-pyridyl)-1H-imidazole-4-carboxylic acid ethyl ester

Ethyl bromopyruvate (8.67 g) and potassium carbonate (2.76 g) were added to the above-obtained benzamidine compound (3.94 g) in ethanol (80 mL). The resultant mixture was refluxed for 3 hours, followed by cooling in air. The reaction solvent was evaporated under reduced pressure, and the residue was dissolved in acetic acid (80 mL). The resultant mixture was refluxed for 3 hours, followed by cooling in air. The reaction solvent was evaporated under reduced pressure. Ethyl acetate and water were added for partitioning the residue. The aqueous layer was further extracted with chloroform, the organic layers were combined. The combined organic layer was dried over magnesium sulfate anhydrate. After a filtration step, the solvent was evaporated under reduced pressure, and the residue was purified by silica gel column chromatography (hexane - ethyl acetate), to thereby give 2-phenyl-1-(3-pyridyl)-1H-imidazole-4-carboxylic acid ethyl ester as a solid product (1.20 g, 20.5%).
¹H-NMR (300MHz, CDCl₃)δ: 1 . 42 (3H, t, J=7.07Hz), 4.44(2H,q,J=7.07Hz), 7.28-7.41(6H,m), 7.52-7.56(1H,m), 7.86(1H, s), 8.58(1H,d,J=2.57Hz), 8.68(2H,dd,J=4.77,2.57).
MS (FAB)m/z:294 (M+H)⁺.

### 3) Title compound

2N Aqueous sodium hydroxide (17.0 mL) was added at room temperature to the above-obtained 2-phenyl-1-(3-pyridyl)-1H-imidazole-4-carboxylic acid ethyl ester (1.0 g) in ethanol (20 mL), followed by stirring for 3 hours. The reaction solvent was evaporated under reduced pressure. 6N HCl was added to the residue for acidification, followed by extraction with chloroform. The organic layer was dried over magnesium sulfate anhydrate. After a filtration step, the solvent was evaporated under reduced pressure, to thereby give the title compound as a solid product (0.636 g, 70.3%).
¹H-NMR (300MHz, DMSO-d₆) δ:7.30-7.38 (5H, m), 7.49-7.54(1H,m), 7.80-7.85(1H,m), 8.23 (1H,s), 8.57 (1H,d,J=2.39Hz), 8.64 (1H, dd, J=4.77, 1.47Hz).
MS(FAB)m/z:266(M+H)⁺.

### [Referential Example 2] 2-Phenyl-1-(4-pyridyl)-1H-imidazole-4-carboxylic acid

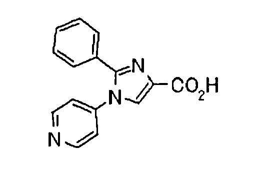

### 1) N-(4-Pyridyl)benzamidine

In a manner similar to that employed in step 1) of Referential Example 1, N-(4-pyridyl)benzamidine in solid form (15.9 g, 42.1%) was prepared from 4-aminopyridine (17.1 g).
¹H-NMR (300MHz, DMSO-d₆) δ: 6.67 (2H, br s), 6. 79 (2H, d, J=5.51Hz), 7.40-7.48(3H, m), 7.90 (2H,m), 8.34 (2H, d, J=6.06Hz) .
MS (FAB)m/z: 198(M+H)⁺.

### 2) 2-Phenyl-1-(4-pyridyl)-1H-imidazole-4-carboxylic acid ethyl ester

In a manner similar to that employed in step 2) of Referential Example 1, 2-phenyl-1-(4-pyridyl)-1H-imidazole-4-carboxylic acid ethyl ester in solid form (1.30 g, 5.8%) was prepared from the above-obtained N-(4-pyridyl)benzamidine (15.0 g) and ethyl bromopyruvate (33.0 g).
¹H-NMR (300MHz, CDCl₃) δ:1.42 (3H, t, J=7.07Hz), 4.44 (2H, q, J=7.07Hz), 7.15 (2H, dd, J=4.59, 1.65Hz), 7.29-7.43 (5H,m), 7. 89 (1H, s), 8. 68 (2H, dd, J=4.59, 1.65Hz) .
MS (FAB) m/z:294 (M+H)⁺.

### 3) Title compound

In a manner similar to that employed in step 3) of Referential Example 1, the title compound in solid form (0.377 g, 41.7%) was prepared from the above-obtained 2-phenyl-1-(4-pyridyl)-1H-imidazole-4-carboxylic acid ethyl ester (1.0 g).
¹H-NMR (300MHz, CDCl₃) δ:7.32-7.41(7H, m), 8.26 (1H, s), 8.65 (2H, dd, J=5.09, 1.47Hz) .
MS(FAB)m/z:266(M+H)⁺.

### [Referential Example 3] 5-Methyl-1,2-diphenyl-1H-imidazole-4-carboxylic acid

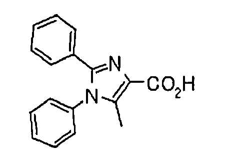

### 1) N-tert-Butoxycarbonyl-L-threonine methyl ester

Potassium carbonate (34.7 g) and dimethyl sulfate (29.3 g) were added to N-tert-butoxycarbonyl-L-threonine (50.0 g) in acetone (250 mL). The resultant mixture was refluxed for 1 hour, followed by cooling in air. The solid was separated by filtration, and the filtrate solvent was evaporated under reduced pressure, to thereby give L-threonine methyl ester compound as an oily product (54.6 g, 100%).
¹H-NMR (300MHz, DMSO-d₆)δ:1.26(3H, d, J=6.42Hz), 1.46(9H,s), 2.02 (1H, d, J=5. 14Hz), 3.78(3H,s), 4.28(1H,m), 5.29 (1H,m) .
MS (FAB)m/z:234 (M+H)⁺.

### 2) 2-tert-Butoxycarbonylamino-3-methanesulfonyloxybutanoic acid methyl ester

Under cooling on ice, N,N-diisopropylamine (54.4 g) was added to the above-obtained L-threonine methyl ester compound (54.5 g) in methylene chloride (400 mL), and then methanesulfonyl chloride (48.2 g) in methylene chloride (100 mL) was added dropwise to the resultant mixture, followed by stirring for 1 hour. The resultant mixture was stirred at room temperature for 1 hour. The reaction mixture was partitioned between water and methylene chloride. The organic layer was washed with saturated brine, followed by drying over magnesium sulfate anhydrate. After a filtration step, the solvent was evaporated under reduced pressure, and the residue was purified by silica gel column chromatography (hexane - ethyl acetate), to thereby give 3-methanesulfonyloxybutanoic acid methyl ester compound as an oily product (66.3 g, 91.0%).
¹H-NMR (300MHz, CDCl₃) δ: 1.47(9H, s), 1.50(3H,d,J=6.42Hz), 2.98(3H,s), 3.80(3H, s), 4.49-4.52(1H, m), 5.22-5.29(1H, m).
MS (FAB) m/z: 312(M+H)⁺.

### 3) 2-tert-Butoxycarbonylamino-2-butenoic acid methyl ester

Under cooling on ice, 0.1N aqueous potassium hydroxide (2.26 L) was added to the above-obtained 3-methanesulfonyloxybutanoic acid methyl ester compound (64.0 g) in methanol (1000 mL), followed by stirring for 1 hour. The reaction solvent was evaporated under reduced pressure. Ethyl acetate and water were added for partitioning the residue. The organic layer was washed with saturated brine, followed by drying over magnesium sulfate anhydrate. After a filtration step, the solvent was evaporated under reduced pressure, to thereby give 2-butenoic acid methyl ester compound as a solid product (38.1 g, 85.9%).
¹H-NMR(300MHz,CDCl₃)δ:1.47(9H,s), 1.81(3H,dd,J=7.16,0.55Hz), 3. 77 (3H, s), 6. 68 (1H, q, J=7.16Hz) .
MS (FAB)m/z:216 (M+H)⁺.

### 3) 3-Bromo-2-tert-butoxycarbonylamino-2-butenoic acid methyl ester

N-Bromosuccinimide (31.4 g) was added to the above-obtained 2-butenoic acid methyl ester compound (38.0 g) in chloroform (950 mL) under cooling on ice, followed by stirring at room temperature for 1 hour. Subsequently, triethylamine (17.9 g) was added to the reaction mixture under cooling on ice, followed by stirring at room temperature for 2 hours. The reaction mixture was partitioned between water and chloroform. The organic layer was washed with saturated brine, followed by drying over magnesium sulfate anhydrate. After a filtration step, the solvent was evaporated under reduced pressure, to thereby give 3-bromo-2-butenoic acid methyl ester compound as a solid product (51.9 g, 99.7%).
¹H-NMR(300MHz,CDCl₃)δ:1.46(9H,s), 2.40 and 2.52(1/2×3H,each s), 3.82 and 3.83(1/2x3H,each s).
MS (FAB)m/z:294 (M+H)⁺.

### 4) 3-Bromo-2-oxobutanoic acid methyl ester

95% Aqueous trifluoroacetic acid (200 mL) was added to the above-obtained 3-bromo-2-butenoic acid methyl ester compound (51.7 g) in chloroform (200 mL) under cooling on ice, followed by stirring at room temperature for 1 hour. The reaction mixture was partitioned between water and chloroform. The organic layer was washed with saturated brine, followed by drying over magnesium sulfate anhydrate. After a filtration step, the solvent was evaporated under reduced pressure, to thereby give 3-bromo-2-oxobutanoic acid methyl ester as an oily product (32.9 g, 96.0%).
¹H-NMR(300MHz,CDCl₃)δ:1.82(3H,d,J=6.79Hz), 3.94(3H,s), 5.18(1H,q,J=6.79Hz) .

### 5) 5-Methyl-1,2-diphenyl-1H-imidazole-4-carboxylic acid methyl ester

In a manner similar to that employed in step 2) of Referential Example 1, 5-methyl-1,2-diphenyl-1H-imidazole-4-carboxylic acid methyl ester in oily form (0.535 g, 35.9%) was prepared from N-phenylbenzamidine (1.00 g) and the above-obtained 3-bromo-2-oxobutanoic acid methyl ester (7.95 g).
¹H-NMR(300MHz, CDCl₃) δ:2.42 (3H, s), 3.96(3H,s), 7.17-7.24(5H, m), 7.34-7.37(2H,m), 7.47-7.49(3H,m).
MS (FAB)m/z:293 (M+H)⁺.

### 6) Title compound

2N Aqueous sodium hydroxide (8.56 mL) was added to the above-obtained 5-methyl-1,2-diphenyl-1H-imidazole-4-carboxylic acid methyl ester (0.50 g) in methanol (10 mL), followed by stirring at room temperature for 7 hours. The reaction solvent was evaporated under reduced pressure. 6N HCl was added to the residue for acidification. Chloroform was added for partitioning the resultant mixture. The aqueous layer was further extracted with tetrahydrofuran, and the organic layers were combined. The combined organic layer was dried over magnesium sulfate anhydrate. After a filtration step, the solvent was evaporated under reduced pressure, to thereby give the title compound as an amorphous product (0.423 g, 88.9%).
¹H-NMR(300MHz,CDCl₃)δ:2.44(3H,s), 7.33-7.56(10H,m).
MS (FAB)m/z:279 (M+H)⁺.

### [Referential Example 4] 2-(6-Methoxy-3-pyridyl)-1-phenyl-1H-imidazole-4-carboxylic acid

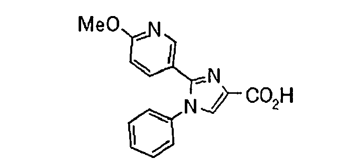

### 1) 5-Bromo-2-methoxypyridine

Sodium methoxide (55.2 g) was added to 2,5-dibromopyridine (50.0 g) in methanol (100 mL). The resultant mixture was refluxed for 17 hours, followed by cooling in air. The formed salt was separated by filtration, and the filtrate solvent was evaporated under reduced pressure. 5% Aqueous sodium hydrogencarbonate and diethyl ether were added thereto for partitioning the residue. The organic layer was washed with saturated brine, followed by drying over magnesium sulfate anhydrate. After a filtration step, the solvent was evaporated under reduced pressure, to thereby give 5-bromo-2-methoxypyridine as an oily product (31.2 g, 78.6%).
¹H-NMR (300MHz, CDCl₃) δ:3.91 (3H, s), 6.66 (1H, d, J=8.81Hz), 7.63 (1H, dd, J=8.81, 2.39Hz), 8.20 (1H, d, J=2.39Hz).

### 2) 6-Methoxynicotinonitrile

Copper cyanide (24.6 g) was added to the above-obtained 5-bromo-2-methoxypyridine (31.0 g) in N,N-dimethylformamide (600 mL). The resultant mixture was stirred at 120°C for 19 hours, and at 140°C for 22 hours, followed by cooling in air. The reaction mixture was subjected to filtration, and then the filtrate was partitioned between water and methylene chloride. The organic layer was washed with saturated brine, followed by drying over magnesium sulfate anhydrate. After a filtration step, the solvent was removed under reduced pressure. The residue still contained N,N-dimethylformamide, therefore water and ethyl acetate were added thereto for partitioning the residue again. The organic layer was dried over magnesium sulfate anhydrate. After a filtration step, the solvent was removed under reduced pressure, and the residue was purified by silica gel column chromatography (hexane - ethyl acetate), to thereby give 6-methoxynicotinonitrile as a solid product (12.5 g, 56.5%).
¹H-NMR(300MHz,CDCl₃)δ:4.00(3H,s), 6.82(1H,d,J=8.81Hz), 7.77(1H,dd,J=8.81,2.39Hz), 8.49(1H,d,J=2.39Hz).

### 3) 6-Methoxy-N-phenylnicotinamidine

In a nitrogen atmosphere, aniline (5.29 g) in tetrahydrofuran (20 mL) was added dropwise to sodium bis(trimethylsilyl)amide (10.4 g) in tetrahydrofuran (100 mL) over 10 minutes, followed by stirring for 20 minutes. The above-obtained 6-methoxynicotinonitrile (8.00 g) in tetrahydrofuran (20 mL) was added dropwise to the reaction mixture over 10 minutes, followed by stirring at room temperature for 15 hours. The crystals that precipitated were collected by filtration, washed with hexane-diethyl ether, and dried, to thereby give 6-methoxy-N-phenylnicotinamidine (6.79 g). Hexane was further added to the filtrate, and the crystals that precipitated were collected by filtration, washed with hexane-diethyl ether, and dried, to thereby give 6-methoxy-N-phenylnicotinamidine (4.95 g). In total, 6-methoxy-N-phenylnicotinamidine was obtained in an amount of 11.7 g (88.6%).
¹H-NMR(300MHz,CDCl₃)δ:3.99(3H,s), 6.81(1H,d,J=8.72Hz), 6.96-7.10(3H,m), 7.43-7.39(2H,m), 8.18(1H,dd,J=8.72,2.39Hz), 8.61(1H,d,J=2.39Hz).
MS (FAB)m/z:228 (M+H)⁺.

### 4) 2-(6-Methoxy-3-pyridyl)-1-phenyl-1H-imidazole-4-carboxylic acid ethyl ester

Ethyl bromopyruvate (12.4 g) and potassium carbonate (3.95 g) were added to the above-obtained 6-methoxy-N-phenylnicotinamidine (6.50 g) in ethanol (100 mL), followed by refluxing for 3 hours. Ethyl bromopyruvate (12.4 g) and potassium carbonate (3.95 g) were further added to the reaction mixture. The resultant mixture was refluxed for 4 hours, followed by cooling in air. Insoluble matter was removed by filtration, and the filtrate solvent was evaporated under reduced pressure. Acetic acid (100 mL) was added to the residue. The resultant mixture was refluxed for 1 hour, followed by cooling in air. The reaction solvent was removed under reduced pressure, and the residue was purified by silica gel column chromatography (hexane - ethyl acetate), to thereby give 2-(6-methoxy-3-pyridyl)-1-phenyl-1H-imidazole-4-carboxylic acid ethyl ester as an oily product (0.833 g, 9.0%).
¹H-NMR (300MHz, CDCl₃) δ: 1.41(3H, t, J=7.16Hz), 3.90(3H, s), 4.43(2H, q, J=7.16Hz), 6.67(1H, d, J=8.75Hz), 7.22-7.27(2H,m), 7.45-7.47(3H, m), 7.73(1H, dd, J=8.75, 2.39Hz), 7. 83 (1H, s), 8.11 (1H, d, J=2.39Hz).
MS(FAB)m/z:324 (M+H)⁺.

### 5) Title compound

2N Aqueous sodium hydroxide (12.4 mL) was added to the above-obtained 2-(6-methoxy-3-pyridyl)-1-phenyl-1H-imidazole-4-carboxylic acid ethyl ester (0.80 g) in ethanol (15 mL), followed by stirring at room temperature for 4 hours. The reaction solvent was removed under reduced pressure, and water and ethyl acetate were added thereto for partitioning the residue. The aqueous layer was acidified with 6N HCl, followed by extraction with chloroform. The organic layer was dried over magnesium sulfate anhydrate. After a filtration step, the solvent was removed under reduced pressure, to thereby give the title compound as an amorphous product (0.345 g, 47.3%).
¹H-NMR (300MHz, CDCl₃)δ: 3.91 (3H, s), 6. 68 (1H, d, J=8.63Hz), 7.47-7.52 (3H, m), 7.69 (1H, dd, J=8 . 63, 2 . 39Hz), 7 . 90 (1H, s), 8.13(1H,d,J=2.39Hz).
MS (FAB)m/z:296 (M+H)⁺.

### [Referential Example 5] Alternative synthesis method of 2-(6-Methoxy-3-pyridyl)-1-phenyl-1H-imidazole-4-carboxylic acid

### 1) 2-(6-Methoxy-3-pyridyl)-1-phenyl-1H-imidazole-4-carboxylic acid ethyl ester

Ethyl bromopyruvate (12.9 g) was added to 6-methoxy-N-phenylnicotinamidine (9.0 g) obtained in step 3) of the above Referential Example 4 in tetrahydrofuran (180 mL), followed by stirring at room temperature for 20 minutes. The resultant mixture was refluxed for 2 hours, followed by cooling in air. The reaction solvent was removed under reduced pressure, and the residue was purified by silica gel column chromatography (hexane - ethyl acetate), to thereby give 2-(6-methoxy-3-pyridyl)-1-phenyl-1H-imidazole-4-carboxylic acid ethyl ester as a solid product (4.45 g, 34.8%).

### 2) Title compound

2N Aqueous sodium hydroxide (68.0 mL) was added to the above obtained 2-(6-methoxy-3-pyridyl)-1-phenyl-1H-imidazole-4-carboxylic acid ethyl ester (4.40 g) in ethanol (80 mL), followed by stirring at room temperature for 3.5 hours. The reaction solvent was removed under reduced pressure. 6N HCl was added to the residue for acidification. The resultant mixture was extracted with chloroform. The organic layer was dried over magnesium sulfate anhydrate. After a filtaraiton step, the solvent was removed under reduced pressure, to thereby give the title compound as a solid product (3.82 g, 95.1%).

### [Referential Example 6] 2-(6-Methoxy-3-pyridyl)-1-(4-methylphenyl)-1H-imidazole-4-carboxylic acid

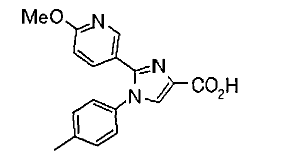

### 1) 6-Methoxy-N-(4-methylphenyl)nicotinamidine

In a manner similar to that employed in step 3) of Referential Example 4, a nicotinamidine compound in solid form (9.15 g, 63.5%) was prepared from p-toluidine (6.09 g) and 6-methoxynicotinonitrile obtained in step 2) of Referential Example 4 (8.00 g).
¹H-NMR (300MHz, CDCl₃) δ: 2.34(3H, s), 3.99(3H,s), 6.80(1H, d, J=8.63Hz), 6.86-6.91(2H,m), 7.15-7.20 (2H, m), 8.17(1H, dd, J=8.63, 2.39Hz), 8.60(1H, d, J=2.39Hz).
MS (FAB)m/z: 242(M+H)⁺.

### 2) 2-(6-Methoxy-3-pyridyl)-1-(4-methylphenyl)-1H-imidazole-4-carboxylic acid ethyl ester

In a manner similar to that employed in step 4) of Referential Example 4, 2-(6-methoxy-3-pyridyl)-1-(4-methylphenyl)-1H-imidazole-4-carboxylic acid ethyl ester in oily form (1.43 g, 23.3%) was prepared from the above-obtained nicotinamidine compound (4.38 g) and ethyl bromopyruvate (15.7 g).
¹H-NMR (300MHz, CDCl₃) δ:1. 41(3H, t, J=7.07Hz), 2.41(3H,s), 3.90(3H,s), 4.43(2H,q,J=7.07Hz), 6.67(1H,d,J=8.63Hz), 7.08-7.12(2H,m), 7.24(2H,d,J=8.08Hz), 7.76 (1H, dd, J=8.08, 2.57Hz), 7.79 (1H, s), 8.10 (1H, d, J=2.57Hz).
FAB-MS(FAB)m/z:338(M+H)⁺.

### 3) Title compound

In a manner similar to that employed in step 5) of Referential Example 4, the title compound in amorphous form (0.815 g, 63.5%) was prepared from the above-obtained 2-(6-methoxy-3-pyridyl)-1-(4-methylphenyl)-1H-imidazole-4-carboxylic acid ethyl ester (1.40 g).
¹H-NMR(300MHz,CDCl₃)δ:2.42(3H,s), 3.92(3H,s), 6 . 69 (1H, d, J=8.63Hz), 7.12-7.27(4H, m), 7. 73 (1H, dd, J=8. 63, 2.7Hz), 7. 86 (1H, s), 8. 13 (1H, d, J=2.57Hz) .
MS(FAB)m/z:310(M+H)⁺.

### [Referential Example 7] 1-(4-Fluorophenyl)-2-(6-methoxy-3-pyridyl)-1H-imidazole-4-carboxylic acid

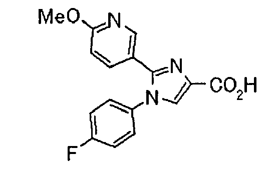

### 1) N-(4-Fluorophenyl)-6-methoxynicotinamidine

In a manner similar to that employed in step 3) of Referential Example 4, N-(4-fluorophenyl)-6-methoxynicotinamidine in solid form (10.7 g, 73.5%) was prepared from p-fluoroaniline (6.64 g) and 6-methoxynicotinonitrile (8.00 g) obtained in step 2) of Referential Example 4.
¹H-NMR (300MHz, CDCl₃) δ:3.99 (3H, s), 6.81 (1H, d, J=8.63Hz), 6.90-6.95(2H,m), 7.04-7.12(2H,m), 8.15 (1H, dd, J=8.63, 2.39Hz), 8.60 (1H, d, J=2.39Hz).
MS(FAB)m/z:246(M+H)⁺.

### 2) 1-(4-Fluorophenyl)-2-(6-methoxy-3-pyridyl)-1H-imidazole-4-carboxylic acid ethyl ester

(Method A) In a manner similar to that employed in step 4) of Referential Example 4, 1-(4-fluorophenyl)-2-(6-methoxy-3-pyridyl)-1H-imidazole-4-carboxylic acid ethyl ester in amorphous form (1.58 g, 17.5%) was prepared from the above-obtained N-(4-fluorophenyl)-6-methoxynicotinamidine (6.50 g) and ethyl bromopyruvate (23.0 g).
(Method B) Ethyl bromopyruvate (7.23 mL) was added to the above-obtained N-(4-fluorophenyl)-6-methoxynicotinamidine (10.6 g) in tetrahydrofuran (210 mL). The resultant mixture was refluxed for 2 hours, followed by cooling in air. The reaction mixture was partitioned between saturated aqueous sodium hydrogencarbonate and chloroform. The organic layer was dried over magnesium sulfate anhydrate. After a filtration step, the solvent was removed under reduced pressure, and the residue was purified by silica gel column chromatography (hexane - ethyl acetate), to thereby give 1-(4-fluorophenyl)-2-(6-methoxy-3-pyridyl)-1H-imidazole-4-carboxylic acid ethyl ester as a solid product (6.90 g, 46.8%).
   ¹H-NMR (300MHz, CDCl₃) δ: 1.41(3H, t, J=7 . 07Hz), 3.91(3H,s), 4.43(2H,q,J=7.07Hz), 6.68(1H,dd,J=8.63,0.73Hz), 7.12-7.18(2H,m), 7.21-7.26(2H,m), 7.74(1H,dd,J=8.63,2.39Hz), 7.80(1H,s), 8.07(1H,dd,J=2.39,0.73Hz).
   MS (FAB)m/z:342 (M+H)⁺.

### 3) Title compound

In a manner similar to that employed in step 5) of Referential Example 4, the title compound in amorphous form (5.71 g, 90.7%) was prepared from the above-obtained 1-(4-fluorophenyl)-2-(6-methoxy-3-pyridyl)-1H-imidazole-4-carboxylic acid ethyl ester (6.85 g).
¹H-NMR (300MHz, CDCl₃) δ:3.92(3H, s), 6.70 (1H, d, J=8.63Hz), 7.14-7.20(2H,m), 7.24-7 .29 (2H,m), 7.72 (1H, dd, J=8.63, 2.8Hz), 7.87(1H,s), 8.10(1H,d, J=1.84Hz).
MS(FAB)m/z:314 (M+H)⁺.

### [Referential Example 8] 2-(4-Methylphenyl)-1-(4-pyridyl)-1H-imidazole-4-carboxylic acid

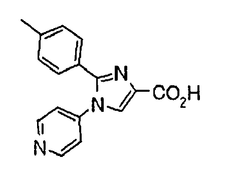

### 1) 4-Methyl-N-(4-pyridyl)benzamidine

In a manner similar to that employed in step 3) of Referential Example 4, 4-methyl-N-(4-pyridyl)benzamidine in solid form (27.4 g, 70.9%) was prepared from 4-aminopyridine (17.1 g) and p-tolunitrile (22.4 g).
¹H-NMR (300MHz, CDCl₃) δ:2.41(3H, s), 4.98(2H,br s), 6.90(2H, d, J=3.85Hz), 7.26 (2H,d, J=6. 97Hz), 7.76 (2H, d, J=6. 97Hz), 8.47(2H, s) .
MS (FAB)m/z:212 (M+H)⁺.

### 2) 2-(4-Methylphenyl)-1-(4-pyridyl)-1H-imidazole-4-carboxylic acid ethyl ester

In a manner similar to that employed in step 4) of Referential Example 4, 2-(4-methylphenyl)-1-(4-pyridyl)-1H-imidazole-4-carboxylic acid ethyl ester in amorphous form (0.717 g, 2.5%) was prepared from the above-obtained 4-methyl-N-(4-pyridyl)benzamidine (20.0 g) and ethyl bromopyruvate (41.0 g).
¹H-NMR (300MHz, CDCl₃) δ:1.41(3H, t, J=7.07Hz), 2.35(3H,s), 4.94(2H,q,J=7.07Hz), 7.10-7.16(4H,m), 7.27-7.30(2H,m), 7.87(1H,s), 8.67(2H, dd, J=4.59,1.65Hz).
MS(FAB)m/z:308(M+H)⁺.

### 3) Title compound

In a manner similar to that employed in step 5) of Referential Example 4, the title compound in solid form (0.350 g, 64.3%) was prepared from the above-obtained 2-(4-methylphenyl)-1-(4-pyridyl)-1H-imidazole-4-carboxylic acid ethyl ester (0.600 g).
¹H-NMR(300MHz,CD₃OD)δ:2.40(3H,s), 7.31(2H,d,J=8.08Hz), 7.39 (2H, d, J=8.26Hz), 7.75(2H,dd,J=5.05,1.56Hz), 8.51 (1H,s), 8. 83 (2H, d, J=6.79Hz) .
MS (FAB)m/z:280 (M+H)⁺.

### [Referential Example 9] 1,3,3-Trimethylpiperazine hydrochloride

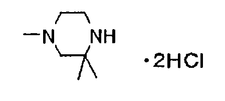

### 1) [N-[N'-(2-Benzyloxycarbonyl)amino-2-methylpropionyl]-N-methylamino]acetic acid ethyl ester

Sarcosine ethyl ester hydrochloride (7.77 g), 1-hydroxybenzotriazole (6.83 g), triethylamine (7.08 mL), and 1-ethyl-3-(3-dimethylaminopropyl)carbodiimide hydrochloride (9.70 g) were added at room temperature to N-(2-benzyloxycarbonyl)amino-2-methylpropionic acid (10.0 g) in N,N-dimethylformamide (200 mL), followed by stirring for 20 hours. 1N HCl and ethyl acetate were added thereto for partitioning the reaction mixture. The organic layer was sequentially washed with saturated brine, saturated aqueous sodium hydrogencarbonate, and saturated brine, and then dried over magnesium sulfate anhydrate. After a filtration step, the solvent was removed under reduced pressure, and the residue was purified by silica gel column chromatography (hexane - ethyl acetate), to thereby give [N-(N'-(2-benzyloxycarbonyl)amino-2-methylpropionyl)-N-methylamino]acetic acid ethyl ester as an oily product (13.1 g, 92.3%).
¹H-NMR (300MHz, CDCl₃) δ:1.26 (3H, t, J=7.16Hz), 1.65(6H, s), 3.16(3H, br s), 4.08(1H, br s), 4.18 (2H, q, J=7.16Hz), 5.09(2H,s), 5.55(1H, br s), 7.30-7.38(5H, m).
MS (FAB) m/z: 337 (M+H)⁺.

### 2) 1,3,3-Trimethylpiperazine-2,5-dione

5% Palladium-carbon (wet, 6.0 g) was added to the above-obtained [N-(N'-(2-benzyloxycarbonyl)amino-2-methylpropionyl)-N-methylamino]acetic acid ethyl ester (12.0 g) in ethanol (120 mL), followed by stirring in a hydrogen atmosphere for 6 hours. The catalyst was removed from the reaction mixture by filtration. The filtrate solvent was removed under reduced pressure, to thereby give 1,3,3-trimethylpiperazine-2,5-dione as an amorphous product (5.16 g, 92.5%).
¹H-NMR(30OMHz,CDC1₃)6:1.50(6H,s), 2.99(3H,s), 4.00(2H,s), 6.95(1H, br s).
MS (FAB)m/z: 157 (M+H)⁺.

### 3) Title compound

The above-obtained 1,3,3-trimethylpiperazine-2,5-dione (0.40 g) in tetrahydrofuran (5 mL) was added dropwise to 1M borane-tetrahydrofuran complex in tetrahydrofuran (7.25 mL) over 10 minutes in a nitrogen atmosphere under cooling on ice, followed by refluxing for 12 hours. Methanol (1 mL) and 4N HCl-dioxane (2 mL) were added to the reaction mixture under cooling on ice. The resultant mixture was refluxed for 1 hour, followed by cooling in air. The crystals that precipitated were collected by filtration, to thereby give the title compound (0.529 g, 100%).
[Referential NMR data: as 1,3,3-trimethylpiperazine ¹H-NMR(300MHz, CDCl₃)δ: 1.53 (6H, s), 2.30(3H,s), 2.42(2H,s), 2. 66 (2H, t, J=5. 14Hz), 3.20 (2H, t, J=5.14Hz).]
MS(FAB)m/z:129(M+H)⁺.

### [Referential Example 10] 2-(6-Methyl-3-pyridyl)-1-phenyl-1H-imidazole-4-carboxylic acid

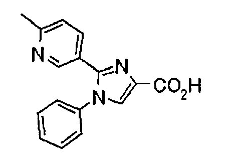

### 1) 6-Methylnicotinamide

In a manner similar to that employed in step 1) of Referential Example 9, 6-methylnicotinamide in solid form (1.98 g, 40%) was prepared from 6-methylnicotinic acid (5.0 g) and 28% aqueous ammonia (7.4 mL).
¹H-NMR(300MHz, CDCl₃)δ:2.58(3H, s), 3.32(2H,br s), 7.39 (1H, d, J=8.26Hz), 8.16 (1H, d, J=8.26Hz), 8.88(3H,s).
MS(FAB)m/z:137(M+H)⁺.

### 2) 6-Methylnicotinonitrile

Phosphorus oxychloride (10.9 mL) was added to the above-obtained 6-methylnicotinamide (1.6 g) in benzene (50 mL). The resultant mixture was refluxed for 2 hours, followed by cooling in air. The reaction solvent was removed under reduced pressure. Saturated aqueous sodium hydrogencarbonate, water, and chloroform were added thereto for partitioning the residue. The organic layer was dried over magnesium sulfate anhydrate. After a filtration step, the solvent was removed under reduced pressure, to thereby give 6-methylnicotinonitrile as a solid product (1.15 g, 83%).
¹H-NMR (300MHz, CDCl₃) δ: 2.65(3H,s), 7.30(1H,d,J=8.07Hz), 7.85(1H,dd,J=8.07,2.20Hz), 8.78(1H,d,J=2.02Hz).

### 3) 6-Methyl-N-phenylnicotinamidine

Tetrahydrofuran (50 mL) was added to a solution (26.6 mL) of 35% sodium bis(trimethylsilyl)amide in a mixture of tetrahydrofuran and cumene (3:1). Aniline (5.0 g) in tetrahydrofuran (10 mL) was added dropwise to the resultant mixture at room temperature, followed by stirring for 30 minutes. 6-Methylnicotinonitrile (6.0 g) in tetrahydrofuran (20 mL) was added dropwise to the reaction mixture, followed by stirring for 17 hours. The reaction solvent was removed under reduced pressure. Water was added to the residue, followed by stirring for 5 minutes. The crystals that precipitated were collected by filtration, and dried, to thereby give 6-methyl-N-phenylnicotinamidine (2.85 g, 28%).
¹H-NMR (300MHz, CD₃OD) δ:2.62 (3H, s), 6.98(2H,d,J=7.34Hz), 7.05-7.11(1H, m), 7.25-7.35(1H, m), 7.37(2H,t,J=7.71Hz), 8.14 (1H, dd, J=8.07, 2.20Hz), 8.94 (1H, s).
MS(FAB)m/z:212 (M+H)⁺.

### 4) 2-(6-Methyl-3-pyridyl)-1-phenyl-1H-imidazole-4-carboxylic acid ethyl ester

In a manner similar to that employed in step 2) of Referential Example 1, .2-(6-methyl-3-pyridyl)-1-phenyl-1H-imidazole-4-carboxylic acid ethyl ester in amorphous form (389 mg, 9.5%) was prepared from the above-obtained 6-methyl-N-phenylnicotinamidine (3.0 g) and ethyl bromopyruvate (9.23 g).
¹H-NMR (300MHz, CDCl₃) δ: 1.41 (3H, t, J=7.16Hz), 2.53(3H,s), 4.44 (2H, q, J=7.16Hz), 7.09(1H, d, J=8.07Hz), 7.22-7.26(2H,m), 7.44-7.47(3H,m), 7.74(1H,dd,J=8.07,2.20Hz), 7.85(1H,s), 8.42 (1H, d, J=2.20Hz) .
MS(FAB)m/z:308(M+H)⁺.

### 5) Title compound

In a manner similar to that employed in step 3) of Referential Example 1, the title compound in amorphous form (0.150 g, 54.8%) was prepared from the above-obtained 2-(6-methyl-3-pyridyl)-1-phenyl-1H-imidazole-4-carboxylic acid ethyl ester (0.30 g).
¹H-NMR(300MHz,CD₃OD)δ:2.58(3H,s), 7.13(1H,d,J=8.26Hz), 7.24-7.29(2H,m), 7.45-7.48(3H,m), 7.74-7.78(1H,m), 7.92(1H,s), 8.55(1H, d, J=1 .84Hz).
MS(FAB)m/z:280(M+H)⁺.

### [Referential Example 11] 1-(4-Methylphenyl)-2-(6-methyl-3-pyridyl)-1H-imidazole-4-carboxylic acid

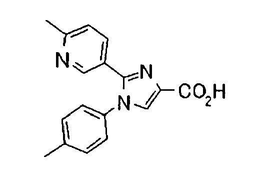

### 1) 6-Methyl-N-p-tolylnicotinamidine

In a manner similar to that employed in step 3) of Referential Example 10, 6-methyl-N-p-tolylnicotinamidine in solid form (13.5 g, 88.5%) was prepared from p-toluidine (6.9 g) and 6-methylnicotinonitrile (8.0 g) obtained in step 2) of Referential Example 10.
¹H-NMR(300MHz,CDCl₃)δ:2.34(3H,s), 2.62(3H,s), 6.88(2H, d, J=7.89Hz), 7.16-7.26 (4H,m), 8.14(1H, d, J=6.42Hz), 8.92 (1H, s).
MS(FAB)m/z:226(M+H)⁺.

### 2) 1-(4-Methylphenyl)-2-(6-methyl-3-pyridyl)-1H-imidazole-4-carboxylic acid ethyl ester

In a manner similar to that employed in step 2) of Referential Example 1, 1-(4-methylphenyl)-2-(6-methyl-3-pyridyl)-1H-imidazole-4-carboxylic acid ethyl ester in amorphous form (1.21 g, 56.6%) was prepared from the above-obtained 6-methyl-N-p-tolylnicotinamidine (1.50 g) and ethyl bromopyruvate.
¹H-NMR(300MHz,CDCl₃)δ:1.41(3H,t,J=7.16Hz), 2.41(3H,s), 2.53(1H,s), 4.44(2H,q,J=7.16Hz), 7.08-7.12(3H,m), 7.22-7.26(2H,m), 7.79 (1H, dd, J=8.07, 2.20Hz), 7.81 (1H, s), 8.40 (1H,d, J=2.20Hz).
MS (FAB) m/z:322(M+H)⁺.

### 3) Title compound

In a manner similar to that employed in step 3) of Referential Example 1, the title compound in amorphous form (1.7 g, 58.7%) was prepared from the above-obtained 1-(4-methylphenyl)-2-(6-methyl-3-pyridyl)-1H-imidazole-4-carboxylic acid ethyl ester (3.5 g).
¹H-NMR(300MHz,CD₃OD)δ:2.41(3H,s), 2.56(3H,s), 7.10-7.14(3H,m), 7.23-7.27(2H,m), 7.75(1H, dd, J=8.07, 2.20Hz), 7 . 88 ( 1H, s ), 8.51(1H, d, J=8.07, 2.20Hz).
MS(FAB)m/z:294 (M+H)⁺.

### [Referential Example 12] 1,2-Dimethylpiperazine trifluoroacetic acid salt

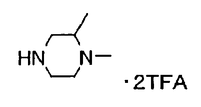

### 1) 3-Methylpiperazine-1-carboxylic acid tert-butyl ester

Di-tert-butyl dicarbonate (21.7 g) was added at room temperature to 2-methylpiperazine (10.0 g) in methanol (200 mL), followed by stirring for 24 hours. The reaction solvent was removed under reduced pressure, and the residue was purified by silica gel column chromatography (chloroform - methanol), to thereby give 3-methylpiperazine-1-carboxylic acid tert-butyl ester as an oily product (19.3 g, 96.5%).
¹H-NMR (300MHz, CDCl₃) δ: 1.04 (3H, d, J=6.24Hz), 1.46(9H,s), 2.39(1H,br s), 2.70-2.77(3H,m), 2.94(1H,br s), 3.93(2H,br s).
MS(FAB)m/z:201(M+H)⁺.

### 2) 3,4-Dimethylpiperazine-1-carboxylic acid tert-butyl ester

36% Aqueous formalin (833 µL) and sodium triacetoxyborohydride (3.18 g) were added at room temperature to the above-obtained 3-methylpiperazine-1-carboxylic acid tert-butyl ester (2.0 g) in methylene chloride (40 mL), followed by stirring for 3 hours. Saturated aqueous sodium hydrogencarbonate was added thereto for partitioning the reaction mixture. The organic layer was washed with brine, and dried over magnesium sulfate anhydrate. After a filtration step, the solvent was removed under reduced pressure, to thereby give a 3,4-dimethylpiperazine compound as an oily product (1.95 g, 91%).
¹H-NMR(300MHz, CDCl₃) δ:1.05 (3H, d, J=6.24Hz), 1.49(9H,s), 1.96-2.05(2H,m), 2.11-2.24(1H, m), 2.28(3H,s), 2.72(1H, d, J=11.75Hz), 3.00(1H, t, J=11.20Hz), 3.81(2H,br s).
MS (FAB)m/z:215 (M+H)+.

### 3) Title compound

The above-obtained 3,4-dimethylpiperazine compound (1.7 g) in trifluoroacetic acid (10 mL) was stirred at room temperature for 3 hours. The reaction solvent was removed under reduced pressure, to thereby give the title compound as an amorphous product (3.5 g, 100%).
¹H-NMR(300MHz,CD₃OD)δ:1.36(3H,d,J=6.24Hz), 2.90(3H,s), 3.13-3.74 (7H, m) .
MS (FAB) m/ z : 115 (M+H)⁺ .

### [Referential Example 13] 1-Methylhexahydropyrimidine

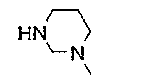

N-Methyl-1,3-diaminopropane (15.0 g) in toluene (20 mL) was added dropwise to 36% formalin (14.2 mL) in toluene (20 mL) at 0°C. The resultant mixture was refluxed by use of a Dean-Stark apparatus for 3 hours, followed by cooling in air. The reaction solvent was removed under reduced pressure, and the residue was purified through distillation (boiling point 98°C/2 mmHg), to thereby give the title compound as an oily product (1.9 g, 11.2%).
¹H-NMR (300MHz, CDCl₃) δ:1.66 (2H, t, J=5.69Hz), 2.20(3H,s), 2.43(3H,br s), 2.57(2H,br s), 3.14 (2H, br s).
MS(FAB)m/z:101(M+H)⁺.

### [Referential Example 14] 1-Methylhexahydropyridazine

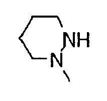

### 1) Benzyl ethyl hydrazine-1,2-dicarboxylate

Triethylamine (100 mL) and benzyl chloroformate (103 mL) were added to ethyl carbazate (50.0 g) in methylene chloride (400 mL) at 0°C, followed by stirring at room temperature for 18 hours. The reaction mixture was partitioned between saturated aqueous sodium hydrogencarbonate and chloroform. The organic layer was washed with brine, and dried over magnesium sulfate anhydrate. After a filtration step, the solvent was removed under reduced pressure, and the residue was purified by silica gel column chromatography (hexane - ethyl acetate), to thereby give benzyl ethyl hydrazine-1,2-dicarboxylate as an oily product (31.7 g, 27.7%).
¹H-NMR(300MHz, CDCl₃) δ:1.25 (3H, t, J=7.16Hz), 4.12 (2H, q, J=7.16Hz), 5.16 (2H, s), 7.28-7.36 (5H, m).

### 2) Benzyl ethyl azo-1,2-dicarboxylate

tert-Butyl hypochlorite (19.1 mL) was added at room temperature to the above-obtained benzyl ethyl hydrazine-1,2-dicarboxylate (31.0 g) in ethyl acetate (150 mL), followed by stirring for 3 hours. The reaction mixture was partitioned through addition of saturated aqueous sodium carbonate and water. The organic layer was dried over magnesium sulfate anhydrate. After a filtration step, the solvent was removed under reduced pressure, to thereby give an azo compound as an oily product (28.7 g, 93.4%).
¹H-NMR (300MHz, CDCl₃)δ: 1.39 (3H, t, J=7.16Hz), 4.46(2H,q,J=7.16Hz), 5.41(2H,s), 7.30-7.53(5H,m).

### 3) 3,6-Dihydropyridazine-1,2-dicarboxylic acid 1-benzyl ester 2-ethyl ester

1,3-Butadiene (64.0 g) was bubbled into the above-obtained azo compound (28.0 g) in benzene (100 mL) at -10°C, followed by stirring at room temperature for 18 hours. The reaction solvent was removed under reduced pressure, to thereby give crude 3,6-dihydropyridazine-1,2-dicarboxylic acid 1-benzyl ester 2-ethyl ester, with impurities, as an oily product (32 g).
MS(FAB)m/z:291(M+H)⁺.

### 4) Tetrahydropyridazine-1-carboxylic acid ethyl ester

10% Palladium-carbon (3.2 g) was added to the above-obtained oily product (32 g) in ethanol (100 mL). The resultant mixture was stirred in a hydrogen atmosphere at 40°C for 24 hours, followed by cooling in air. The reaction mixture was subjected to filtration. The filtrate solvent was removed under reduced pressure, and the residue was purified through distillation (boiling point 81°C/1 mmHg), to thereby give tetrahydropyridazine-1-carboxylic acid ethyl ester as an oily product (5.96 g, 31.1% in two steps).
¹H-NMR(30OMHz,CDC1₃)5:1.29(3H,t,J=7.16Hz),. 1.65(4H, brs), 2.92(2H, t, J=5.69Hz), 3.57(2H, t, J=5.69Hz), 4.19(2H, q, J=7.16Hz).

### 5) Title compound

The above-obtained tetrahydropyridazine-1-carboxylic acid ethyl ester (5.5 g) in diethyl ether (20 mL) was added dropwise at room temperature to a suspension of lithium aluminum hydride (2.64 g) in diethyl ether (50 mL) over 1 hour, followed by refluxing for 4 hours. 40% Aqueous potassium hydroxide solution (100 mL) was slowly added dropwise to the reaction mixture at -10°C. Diethyl ether was added thereto for partitioning the resultant mixture. The organic layer was washed with brine, and dried over magnesium sulfate anhydrate. After a filtration step, the solvent was removed under reduced pressure, to thereby give the title compound as an oily product (1.75 g, 50.3%).
¹H-NMR(300MHz,CDCl₃)δ:1.42(2H,br s), 1.73-1.81(2H,m), 2.38 (3H, s), 2.48 (2H,br s), 3.02 (2H, t, J=5.51Hz) .

### [Referential Example 15] 4-(6-Methoxy-3-pyridyl)-5-(3-pyridyl)oxazole-2-carboxylic acid ethyl ester

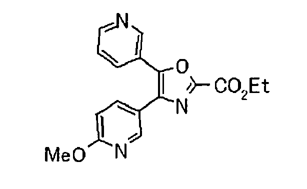

### 1) 6-Methoxy-3-pyridinecarboxaldehyde

Methyl 6-methoxynicotinate (3.1 g) in tetrahydrofuran (20 mL) was added to a suspension of lithium aluminum hydride (1.4 g) in tetrahydrofuran (30 mL) under cooling on ice, and the mixture was stirred for 1.5 hours. Water, 15% aqueous sodium hydroxide, and water were sequentially added to the reaction mixture, and the resultant mixture was stirred for 1 hour at room temperature, followed by filtration. Ethyl acetate was added to the filtrate for partitioning the mixture, and the organic layer was washed with saturated brine and then dried over sodium sulfate anhydrate. After a filtration step, the solvent was removed under reduced pressure, to thereby give 5-hydroxymethyl-2-methoxypyridine. The product was dissolved in methylene chloride (100 mL), and manganese dioxide (8 g) was added to the solution in a nitrogen atmosphere at room temperature, followed by stirring for 88 hours. The reaction mixture was filtrated through Celite, and the solvent of the filtrate was removed under reduced pressure. The residue was purified through silica gel column chromatography (hexane - ethyl acetate), to thereby give an aldehyde compound as a solid product (2.4 g, 94%). ¹H-NMR(400MHz, CDCl₃) δ:4.04(3H, s), 6.85 (1H, dd, J=8.5, 1.8Hz), 8.08 (1H, dd, J=8.5, 2.2Hz), 8.64 (1H, d, J=3.0Hz), 9.96(1H, s).

### 2) N-[C-(6-Methoxy-3-pyridyl)-C-(p-toluenesulfonyl)methyl]carbamic acid tert-butyl ester

At room temperature, sodium p-toluenesulfinate (0.36 g), the above 6-methoxy-3-pyridinecarboxaldehyde (0.3 g), and formic acid (0.48 mL) were added to a suspension of carbamic acid tert-butyl ester (0.23 g) in tetrahydrofuran (0.8 mL) and water (0.2 mL), and the mixture was stirred for 18.5 hours. The precipitates in the reaction mixture were collected through filtration, washed with water, and then dried, to thereby give a carbamic acid tert-butyl ester compound as a solid product (0.52 g, 66%).
¹H-NMR(400MHz, CDCl₃) δ:1.26(9H,s), 2.43(3H,s), 3.95(3H,s), 5.63(1H,br s), 5.84(1H, d, J=9.8Hz), 6.79 (1H, d, J=8.6Hz), 7.34 (2H, d, J=8.1Hz), 7.68 (1H, dd, J=8.5, 2.7Hz), 7.78 (2H, d, J=8.3Hz), 8.16 (1H, d, J=2.2Hz).
MS(FAB)m/z:393(M+H)⁺.

### 3) N-[1-(6-Methoxy-3-pyridyl)-2-oxo-2-(3-pyridyl)ethyl]carbamic acid tert-butyl ester

In a nitrogen atmosphere, 3-pyridinecarboxaldehyde (0.31 mL) was added to a solution of the N-[C-(6-methoxy-3-pyridyl)-C-(p-toluenesulfonyl)methyl]carbamic acid tert-butyl ester (1.2 g) and 3-benzyl-5-(2-hydroxyethyl)-4-methylthiazolium chloride (81 mg) in methylene chloride (20 mL). Triethylamine (6.3 mL) was added to the reaction mixture, and the resultant mixture was stirred for 23 hours at 35°C, followed by cooling in air. Water was added thereto for partitioning the reaction mixture. Subsequently, the aqueous layer was extracted with chloroform, and the organic layers were combined, washed with saturated brine, and dried over sodium sulfate anhydrate. After a filtration step, the solvent was removed under reduced pressure, and the residue was purified through silica gel column chromatography (hexane - ethyl acetate), to thereby give N-[1-(6-methoxy-3-pyridyl)-2-oxo-2-(3-pyridyl)ethyl]carbamic acid tert-butyl ester as an oil (1.0 g, 95%).
¹H-NMR(400MHz,CDCl₃)δ:1.44(9H,s), 3.89(3H,s), 5.94(1H,br s), 6.19(1H,d,J=7.1Hz), 6.69(1H,d, J=8.5Hz), 7.36-7.39(1H,m), 7.53(1H,d,J=7.8Hz), 8.18-8.21(2H,m), 8.73(1H,dd,J=4.9,1.5Hz), 9.14(1H,d,J=2.2Hz).

### 4) N-[1-(6-Methoxy-3-pyridyl)-2-oxo-2-(3-pyridyl)ethyl]oxamic acid ethyl ester

Under cooling on ice, trifluoroacetic acid (5 mL) was added to a solution of the above N-[1-(6-methoxy-3-pyridyl)-2-oxo-2-(3-pyridyl)ethyl]carbamic acid tert-butyl ester (1.0 g) was dissolved in methylene chloride (20 mL), and the mixture was stirred for 15 hours at room temperature. Ice-water was added to the reaction mixture, and the resultant mixture was neutralized with sodium hydrogencarbonate. Chloroform was added thereto for partitioning the mixture. The organic layer was sequentially washed with water and saturated brine and dried over sodium sulfate anhydrate. After a filtration step, the solvent was removed under reduced pressure, and the residue was dissolved in methylene chloride (30 mL). Under cooling on ice, triethylamine (0.61 mL) and ethyloxalyl chloride (0.36 mL) were sequentially added to the solution, and the mixture was stirred for 1 hour at room temperature. Saturated aqueous sodium hydrogencarbonate was added to the reaction mixture for partitioning the resultant mixture. The aqueous layer was extracted with chloroform, and the organic layers were combined, washed with saturated brine, and dried over sodium sulfate anhydrate. After a filtration step, the solvent was removed under reduced pressure, and the residue was purified through silica gel column chromatography (hexane - ethyl acetate), to thereby give an oxamic acid ethyl ester compound as an oil (0.45 g, 45%).
¹H-NMR (400MHz, CDCl₃) δ: 1.38 (3H, t, J=7.1Hz), 3.89(3H,s), 4.36 (2H, q, J=7.1Hz), 6.43 (1H, d, J=7.3Hz), 6.71(1H, d, J=8.6Hz), 7.40 (1H, dd, J=8.1,4.9Hz), 7.57(1H, dd, J=8.5,2.4Hz), 8.22-8.25(2H,m), 8.41(1H,br s), 8.76(1H,d,J=4.4Hz), 9 .16 (1H, d, J=2 . 4Hz) .

### 5) Title compound

In a nitrogen atmosphere and at room temperature, hexachloroethane (0.69 g), triethylamine (0.98 mL), and a solution of the above oxamic acid ethyl ester compound (0.4 g) in methylene chloride (10 mL) were sequentially added to triphenylphosphine (0.92 g) in methylene chloride (20 mL), and the mixture was stirred for 20 hours. The reaction solvent was removed under reduced pressure, and the residue was partitioned between ethyl acetate and 1N HCl. The organic layer was extracted with 1N HCl, and the aqueous layers were combined and then neutralized with 1N aqueous sodium hydroxide. The mixture was partitioned between saturated aqueous sodium hydrogencarbonate and ethyl acetate.

The aqueous layer was extracted with ethyl acetate, and the organic layers were combined, washed with saturated brine, and dried over sodium sulfate anhydrate. After a filtration step, the solvent was removed under reduced pressure, and the residue was purified through silica gel column chromatography (hexane - ethyl acetate), to thereby give the title compound as an oil (0.25 g, 66%).
¹H-NMR (400MHz, CDCl₃) δ:1.47 (3H, t, J=7.3Hz), 3.97(3H,s), 4.54(2H,q,J=7.3Hz), 6.82 (1H, d, J=8.8Hz), 7.38 (1H, dd, J=8.1, 4.9Hz), 7.86 (1H, dd, J=8.5, 2.4Hz), 7.98 (1H, d, J=8.1Hz), 8.42 (1H, d, J=2.4Hz), 8.65 (1H, d, J=4.8Hz), 8.91 (1H, d, J=2.2Hz).

### [Referential Example 16] 5-(4-Fluorophenyl)-4-(3-pyridyl)oxazole-2-carboxylic acid ethyl ester

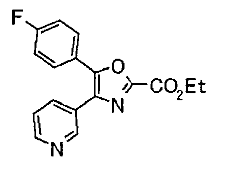

### 1) 4-Fluoro-N-(3-pyridylmethyl)benzamide

Triethylamine (10.5 mL) and 4-fluorobenzoyl chloride (8.7 g) in methylene chloride (20 mL) were added to 3-(aminomethyl)pyridine (5.4 g) in methylene chloride (100 mL) under cooling on ice, and the mixture was stirred for 1 hour at room temperature. Saturated aqueous sodium hydrogencarbonate was added thereto for partitioning the reaction mixture. The aqueous layer was extracted with chloroform, and the organic layers were combined, sequentially washed with water and saturated brine, and dried over sodium sulfate anhydrate. After a filtration step, the solvent was removed under reduced pressure, and the residue was purified through silica gel column chromatography (chloroform - methanol), to thereby give a benzamide compound as an oil (11.3 g, 98%).
¹H-NMR(400MHz,CDCl₃)δ:4.66(2H,d,J=5.6Hz), 6.60(1H,br s), 7.12(2H,d,J=8.1Hz), 7.26-7.30(1H,m), 7.71(1H,d,J-7.2Hz), 7.79-7.83(2H,m), 8.55(1H,d,J=4.6Hz), 8.60(1H,s).

### 2) N-(4-Fluorobenzoyl)-N-(3-pyridylmethyl)carbamic acid tert-butyl ester

Di-tert-butylcarbonate (16.1 g) in acetonitrile (20 mL) was added, at room temperature, to a solution of the above benzamide compound (11.3 g) and 4-dimethylaminopyridine (0.6 g) in acetonitrile (50 mL), and the mixture was stirred for 19 hours. The reaction solvent was removed under reduced pressure, and the residue was purified through silica gel column chromatography (hexane - ethyl acetate), to thereby give a carbamic acid tert-butyl ester compound as an oil (14.9 g, 92%).
¹H-NMR(400MHz,CDCl₃)δ:1.18(9H,s), 4.97(2H,s), 7.07(2H,t,J=8.5Hz), 7.25-7.29(1H,m), 7.51-7.54(2H,m), 7.78(1H,d,J=7.9Hz), 8.53(1H,d,J=4.6Hz), 8.70(1H,s).

### 3) N-[2-(4-fluorophenyl)-2-oxo-1-(3-pyridyl)ethyl]carbamic acid tert-butyl ester

In a nitrogen atmosphere, a 1.56M solution (9.6 mL) of n-butyllithium in hexane was added at -78°C to a solution of diisopropylamine (2.1 mL) in a mixture of N,N'-dimethylpropyleneurea (0.9 mL) and tetrahydrofuran (10 mL), and the resultant mixture was stirred for 1 hour. A solution of the above carbamic acid tert-butyl ester compound (1.65 g) in tetrahydrofuran (20 mL) was added to the reaction mixture, and the resultant mixture was stirred for 1 hour. The reaction mixture was partitioned between saturated aqueous ammonium chloride and ethyl acetate. The organic layer was washed with saturated brine and dried over sodium sulfate anhydrate. After a filtration step, the solvent was removed under reduced pressure, and the residue was purified through silica gel column chromatography (hexane - ethyl acetate), to thereby give N-[2-(4-fluorophenyl)-2-oxo-1-(3-pyridyl)ethyl]carbamic acid tert-butyl ester as a solid product (1.0 g, 61%).
¹H-NMR (400MHz, CDCl₃) δ:1.43 (9H, s), 6.10(H,br s), 6.26 (1H, d, J=7.1Hz), 7.09 (2H, t, J=7.8Hz), 7.22-7.26 (1H, m), 7.64 (1H, d, J=7.8Hz), 7.95-7.99 (2H, m), 8.52 (1H, d, J=1.5Hz), 8.65 (1H, s).

### 4) 2-Amino-1-(4-fluorophenyl)-2-(3-pyridyl)ethanone hydrochloride

The above N-[2-(4-fluorophenyl)-2-oxo-1-(3-pyridyl)ethyl]carbamic acid tert-butyl ester (1.0 g) was dissolved in saturated HCl-ethanol (10 mL), and the mixture was stirred for 14 hours at room temperature. The reaction solvent was removed under reduced pressure, to thereby give an ethanone hydrochloride compound as a solid product (0.9 g, quantitative amount). The compound was used in the next reaction step without being subjected to any further purification step.
¹H-NMR (400MHz, DMSO-d₆) δ: 6.45(1H, br s), 7.35(2H, t, J=8.8Hz), 7.51(1H, m), 7.92(1H, br s), 8.10-8.14(2H, m), 8.60(1H, d, J=4.9Hz), 8.82(1H, s), 8.99(3H,br s).

### 5) N-[2-(4-Fluorophenyl)-2-oxo-1-(3-pyridyl)ethyl]oxamic acid ethyl ester

Triethylamine (1.4 mL) and ethyloxalyl chloride (0.44 mL) were added, at room temperature, to a suspension of the above ethanone hydrochloride compound (1.0 g) in methylene chloride (20 mL) under cooling on ice, and the mixture was stirred for 1 hour. Saturated aqueous sodium hydrogencarbonate was added thereto for partitioning the reaction mixture. The aqueous layer was extracted with chloroform, and the organic layers were combined, washed with saturated brine, and dried over sodium sulfate anhydrate. After a filtration step, the solvent was removed under reduced pressure, and the residue was purified through silica gel column chromatography (hexane - ethyl acetate), to thereby give an oxamic acid ethyl ester compound as an oil (1.1 g, quantitative amount).
¹H-NMR(400MHz, CDCl₃) δ:1.38 (3H, t, J=7.3Hz), 4.36 (2H, q, J=7.3Hz), 6.49 (1H, d, J=7.4Hz), 7.12 (2H, t, J=8.3Hz), 7.25-7.28 (1H, m), 7.71 (1H, dd, J=5.9, 2.2Hz), 7.98-8.02(2H,m), 8.54-8.56 (2H, m), 8.71 (1H, d, J=1.5Hz).

### 6) Title compound

Phosphorus oxychloride (0.85 mL) was added to the above oxamic acid ethyl ester compound (1.0 g), and the mixture was stirred for 1 hour at 105°C, followed by cooling in air. The reaction mixture was poured into ice-water, and the mixture was neutralized with sodium hydrogencarbonate. Ethyl acetate was added thereto for partitioning the mixture. The organic layer was washed with saturated brine and then dried over sodium sulfate anhydrate. After a filtration step, the solvent was removed under reduced pressure, and the residue was purified through silica gel column chromatography (hexane - ethyl acetate), to thereby give the title compound as an oil (0.22 g, 23%).
¹H-NMR (400MHz, CDCl₃) δ: 1.47 (3H, t, J=7.1Hz), 4.55 (2H, q, J=7.1Hz), 7.13(2H,t,J=8.3Hz), 7.35-7.38(1H,m), 7.63-7.67(2H,m), 8.00(1H,dd,J=7.8,4.9Hz), 8.63(1H,d,J=4.9Hz), 8.88(1H,s).

### [Referential Example 17] 4,5-Diphenyloxazole-2-carboxylic acid ethyl ester

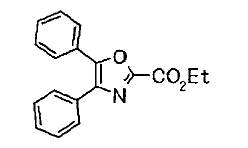

Triethylamine (4.93 mL) and ethyloxalyl chloride (2.90 mL) were added to benzoin (5.0 g) in tetrahydrofuran (100 mL) at 0°C, and the mixture was stirred for 4 hours at room temperature. Insoluble matter in the reaction mixture was removed through filtration, and the solvent of the filtrate was removed through filtration. The residue was dissolved in acetic acid (100 mL), and ammonium acetate (9.08 g) was added to the solution at room temperature. The mixture was refluxed for 14 hours and then cooled in air. The solvent was removed under reduced pressure, and the residue was partitioned between chloroform and saturated aqueous sodium hydrogencarbonate. The organic layer was washed with saturated brine and dried over sodium sulfate anhydrate. After a filtration step, the solvent was removed under reduced pressure, and the residue was purified through silica gel column chromatography (hexane - ethyl acetate), to thereby give the title compound as a solid product (1.365 g, 20%).
¹H-NMR (400MHz, CDCl₃) δ: 1 .46 (3H, t, J=7. 1Hz), 4 .52 (2H, q, J=7. 1Hz), 7.35-7.41(6H,m), 7.65-7.70(4H,m).
MS (EI)m/z:293 (M⁺) .

### [Referential Example 18] 2-(6-Methoxy-3-pyridyl)-1-(2-pyridyl)-1H-imidazole-4-carboxylic acid

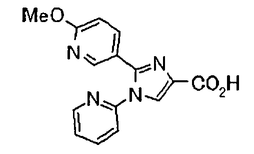

### 1) 6-Methoxy-N-(2-pyridyl)nicotinamidine

2-Aminopyridine (5.47 g) and 6-methoxynicotinonitrile (8.20 g) were treated in a manner similar to that employed in step 3) of Referential Example 10, to thereby give a nicotinamidine compound as a solid product (9.13 g, 69.2%).
¹H-NMR (300MHz, CDCl₃) δ : 3.99 (3H,s), 6.81(1H,d,J=8.7Hz), 6.94(1H,t,J=6.2Hz), 7.26(1H,d,J=2.1Hz), 7.65(1H,dt,J=7.7,2.1Hz), 8.19(1H,dd,J=8.7,2.7Hz), 8.30(1H,dd,J=6.2,3.9Hz), 8.68(1H,d,J=2.1Hz).
MS (FAB) m/z:229 (M+H)⁺.

### 2) 2-(6-Methoxy-3-pyridyl)-1-(2-pyridyl)-1H-imidazole-4-carboxylic acid ethyl ester

The above nicotinamidine compound (1.14 g) and ethyl bromopyruvate (1.3 g) were dissolved in tetrahydrofuran (25 mL), and, under reflux conditions, triethylamine (0.84 mL) was added to the solution. The mixture was refluxed for 1 hour and then cooled in air. Ethyl bromopyruvate (1.3 g) was added to the reaction mixture, and the resultant mixture was refluxed for 7 hours and then cooled in air. Ethyl bromopyruvate (1.3 g) and tetrahydrofuran (25 mL) were added to the reaction mixture, and the resultant mixture was refluxed for 16 hours and then cooled in air. The reaction solvent was removed under reduced pressure, and the residue was partitioned between saturated aqueous sodium hydrogencarbonate and methylene chloride. The organic layer was dried over magnesium sulfate anhydrate. After a filtration step, the solvent was removed under reduced pressure, and the residue was purified through silica gel column chromatography (hexane - ethyl acetate), to thereby give a 1H-imidazole-4-carboxylic acid ethyl ester compound as an oil (302 mg, 18.6%).
¹H-NMR (300MHz, CDCl₃) δ:1.41 (3H, t, J=6.9Hz), 3.92 (3H,s), 4.43(2H,q,J=6.9Hz), 6.71(1H,d,J-8.7Hz), 7.07(1H,d,J=8.1Hz), 7.38(1H,m), 7.76(2H,m), 8.12(1H,s), 8.13(1H,d,J=2.7Hz), 8.59(1H,m).
MS(FAB)m/z:325(M+H)⁺.

### 3) Title compound

The above 1H-imidazole-4-carboxylic acid ethyl ester compound (600 mg) was treated in a manner similar to that employed in step 3) of Referential Example 1, to thereby give the title compound as an amorphous product (555 mg, quantitative amount).
¹H-NMR (300MHz, CDCl₃) δ: 3. 94 (1H, s), 6.73 (1H, d, J=9.3Hz), 7.12(1H,d,J=7.8Hz), 7.41(1H,m), 7.73(1H,dd,J=8.7,2.4Hz), 7.80(1H,dt,J=7.8,2.4Hz), 8.16(1H,dd,J=9.3Hz,1.5Hz), 8.60(1H,m).
MS(FAB)m/z:297(M+H)⁺.

### [Referential Example 19] 1,4-Oxazepane

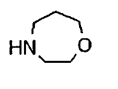

### 1) 1,4-Oxazepan-5-one

Under cooling on ice, sodium azide (17.8 g) was added to a solution of tetrahydro-4H-pyran-4-one (9.80 g) in concentrated hydrochloric acid (50 mL) over 40 minutes, and the mixture was stirred for 30 minutes and then at room temperature for 16 hours. Under cooling on ice, sodium carbonate was added to the reaction mixture to make the pH condition to 8 to 9, and chloroform was added thereto for partitioning the mixture. The organic layer was washed with saturated brine and then dried over magnesium sulfate anhydrate. After a filtration step, the solvent was removed under reduced pressure, to thereby give [1,4]oxazepan-5-one as a solid product (5.34 g, 47.4%).
¹H-NMR (300MHz, CDCl₃) δ:2.70-2.74 (2H, m), 3.32-3.37(2H,m), 3.75-3.83(4H,m), 6.31 (1H, br s).
MS(FAB)m/z:116(M+H)⁺.

### 2) Title compound

Under nitrogen flow and under cooling on ice, a solution of the above 1,4-oxazepan-5-one (1.6 g) in tetrahydrofuran (64 mL) was added to a 1.0M solution (18.2 mL) of borane-tetrahydrofuran complex in tetrahydrofuran over 20 minutes, and the mixture was stirred at room temperature for 30 minutes and then refluxed for 2 hours, followed by cooling in air. 4N HCl-Dioxane (13 mL) and methanol (6 mL) were added to the reaction mixture, and the resultant mixture was refluxed for 1 hour and then cooled in air. 1N HCl (60 mL), water, and ethyl acetate were added thereto for partitioning the reaction mixture. The aqueous layer was alkalified with 4N aqueous sodium hydroxide, and the mixture was extracted with ethyl acetate. The aqueous layer was further extracted with chloroform. The organic layers were combined and then dried over sodium sulfate anhydrate. After a filtration step, the solvent was removed under reduced pressure, to thereby give the title compound as an oil (1.19 g, 88.4%).
¹H-NMR (300MHz, CDCl₃) δ:1.83-1.91 (2H,m), 2.93-2.99(4H,m), 3.70-3.83 (4H,m).

### [Referential Example 20] 3-Methylpiperazine-1-carboxylic acid tert-butyl ester

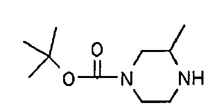

2-Methylpiperazine (3.19 g) was added at 0°C to 2-(tert-butylcarbonyloxyimino)-2-phenylacetonitrile (7.87 g) in tetrahydrofuran (100 mL), and the mixture was stirred for 2 hours. The reaction solvent was removed under reduced pressure, and the residue was purified through silica gel column chromatography (chloroform - 7N ammonia/methanol), to thereby give the title compound as an oil (5.70 g, 89%).
¹H-NMR(400MHz,CDCl₃)δ:1.05(3H,d,J=6.4Hz), 1.46(9H,s), 2.40 (1H,br), 2.65-2.84(3H,m), 2.90-3.00 (1H,br), 3.94(2H,br).
MS(ESI)m/z:201(M+H)⁺.

### [Referential Example 21] 3,4-Dimethylpiperazine-1-carboxylic acid tert-butyl ester

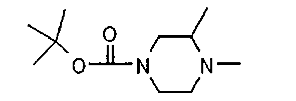

10% Palladium-carbon (0.59 g), 35% aqueous formalin (9.7 mL), and 1M HCl-ethanol (31.3 mL) were added to a solution of 3-methylpiperazine-1-carboxylic acid tert-butyl ester (5.70 g) obtained in Referential Example 20 in methanol (100 mL) at room temperature, and the mixture was stirred for 15 hours in a hydrogen atmosphere. The reaction mixture was purged with nitrogen, and insoluble matter was removed through filtration. The solvent of the filtrate was removed under reduced pressure, and chloroform-methanol (9%) was added to the residue. The mixture was alkalified with aqueous sodium hydroxide and then partitioned. The aqueous layer was extracted with chloroform - methanol (9%), and the organic layers were combined, washed with saturated brine, and dried over sodium sulfate anhydrate. After a filtration step, the solvent was removed under reduced pressure, and the residue was purified through silica gel column chromatography (chloroform - methanol), to thereby give the title compound as an oil (3.10 g, 51%).
¹H-NMR (400MHz, CDCl₃) δ: 1.04 (3H, d, J=6. 3Hz), 1.46(9H,s), 1.95-2.20(2H,m), 2.28(3H,s), 2.50-2.78(2H,br), 2. 90-3.05 (1H,br), 3.88 (1H, br).
MS (ESI)m/z:215 (M+H)⁺.

### [Referential Example 22] 1,2-Dimethylpiperazine trifluoroacetic acid salt

Trifluoroacetic acid (15 mL) was added to a solution of 3,4-dimethylpiperazine-1-carboxylic acid tert-butyl ester (3.10 g) obtained in Referential Example 21 in methylene chloride (30 mL) at room temperature, and the mixture was stirred for 1 hour. The reaction solvent was removed under reduced pressure, and the residue was crystallized from chloroform - ether and then collected through filtration, to thereby give the title compound (2.756 g, 56%).
¹H-NMR(400MHz,DMSO-d₆)δ:1.24(3H,d,J=6.4Hz), 2.30-3.70(10H,br).
MS(ESI)m/z:115(M+H)⁺.

### [Referential Example 23] 1-(6-Methoxy-3-pyridyl)-5-phenyl-1H-1,2,4-triazole-3-carboxylic acid

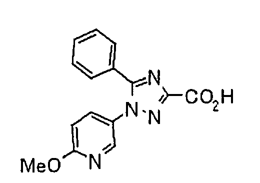

### 1) 2-Benzoylaminomalonic acid diethyl ester

Benzoic acid (5.0 g) in thionyl chloride (10 mL) was refluxed for 1 hour and then cooled in air. The reaction solvent was removed under reduced pressure, and the residue was dissolved in methylene chloride (50 mL). Aminomalonic acid diethyl ester hydrochloride (7.53 g) was added to the solution, and the mixture was refluxed for 17 hours and then cooled in air. The solid that precipitated was collected through filtration, and the filtrate was partitioned between water and chloroform. The organic layer was sequentially washed with water, saturated aqueous sodium hydrogencarbonate, and saturated brine, and then dried over magnesium sulfate anhydrate. After a filtration step, the solvent was removed under reduced pressure, and the residue was purified through silica gel column chromatography (hexane - ethyl acetate), to thereby give a 2-benzoyl compound as an amorphous product (8.26 g, 72%).
¹H-NMR (400MHz, CDCl₃) δ: 1.32(6H, t, J=7.1Hz), 4.25-4.38 (4H,m) , 5.35(1H, d, J=6.8Hz), 7.12(1H, d, J=6.1Hz), 7.43-7. 47(2H,m), 7.52-7.56(1H,m), 7.83-7.86(2H,m).LC-MSm/z:280(M+H)⁺.

### 2) 1-(6-Methoxy-3-pyridyl)-5-phenyl-1H-1,2,4-triazole-3-carboxylic acid methyl ester

At 0°C, sodium nitrite (828 mg) in water (5 mL) was added dropwise to a solution of 5-amino-2-methoxypyridine (1.5 g) in acetic acid (8 mL) and concentrated hydrochloric acid (2 mL), and the mixture was stirred for 15 minutes. At -15°C, a solution of the above 2-benzoyl compound (3.0 g) in acetone (20 mL) and a solution of potassium carbonate (15.2 g) in water (20 mL) were gradually added to the reaction mixture, and the resultant mixture was stirred for 1 hour at 0°C. Ethyl acetate was added thereto for partitioning the reaction mixture. The organic layer was sequentially washed with water, saturated aqueous sodium hydrogencarbonate, water, and saturated brine, and then dried over magnesium sulfate anhydrate. After a filtration step, the solvent was removed under reduced pressure, and the residue was dissolved in anhydrous methanol (50 mL). At room temperature, 28% sodium methoxide-methanol (414 mg) was added to the solution, and the mixture was stirred for 1.5 hours. The reaction solvent was removed under reduced pressure, and the residue was purified through NH-silica gel column chromatography (hexane - ethyl acetate), to thereby give a 1H-1,2,4-triazole-3-carboxylic acid methyl ester compound as an oil (1.56 g, 42%).
¹H-NMR(400MHz,CDCl₃) δ :4.01 (3H,s), 4.06(3H,s), 6.82 (1H, d, J=8.8Hz), 7.35-7.47(3H,m), 7.53-7.55(2H,m), 7.61(1H,dd,J=8.8,2.7Hz), 8.28(1H,d,J=2.7Hz).
MS(ESI)m/z:310(M⁺).

### 3) Title compound

1N Aqueous sodium hydroxide (5 mL) was added to a solution of the above 1H-1,2,4-triazole-3-carboxylic acid methyl ester compound (1.56 g) in a mixture of methanol (20 mL) and tetrahydrofuran (10 mL) at room temperature, and the resultant mixture was stirred for 6 hours. The reaction solvent was removed under reduced pressure, and 1N HCl was added to the residue to make the pH condition to 8. The resultant mixture was partitioned between ice-water and ethyl acetate. The aqueous layer was acidified with 1N HCl, and then extracted with chloroform. The organic layer was washed with saturated brine and then dried over magnesium sulfate anhydrate. After a filtration step, the solvent was removed under reduced pressure, to thereby give the title compound as an amorphous product (998 mg, 66%).
¹H-NMR(400MHz, DMSO-d₆)δ:3.90 (3H,s), 6.97(1H,d,J=8.8Hz), 7.43-7.48 (5H,m), 7.85(1H,br d,J=7.8Hz), 8.28(1H,s).
LC-MSm/z:297(M+H)⁺.

### [Referential Example 24] 5-(4-Fluorophenyl)-1-(6-methoxy-3-pyridyl)-1H-1,2,4-triazole-3-carboxylic acid

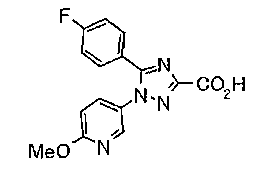

### 1) 2-(4-Fluorobenzoyl)aminomalonic acid diethyl ester

4-Fluorobenzoic acid (5.5 g) was treated in a manner similar to that employed in step 1) of Referential Example 23, to thereby give a 4-fluorobenzoyl compound as an amorphous product (7.87 g, 68%).
¹H-NMR (400MHz, CDCl₃) δ:1.33 (6H, t, J=7,1Hz), 4.26-4.38 (4H, m), 5.33 (1H, d, J=7.0Hz), 7.06 (1H, d, J=6.3Hz), 7.11-7.17(2H,m), 7.84-7.89(2H,m).
LC-MSm/z:298(M+H)⁺.

### 2) 5-(4-Fluorophenyl)-1-(6-methoxy-3-pyridyl)-1H-1,2,4-triazole-3-carboxylic acid methyl ester

5-Amino-2-methoxypyridine (1.39 g) and the above 4-fluorobenzoyl compound (3.0 g) were treated in a manner similar to that employed in step 2) of Referential Example 23, to thereby give a triazole-3-carboxylic acid methyl ester compound as an amorphous product (1.73 g, 53%).
¹H-NMR (400MHz, CDCl₃) δ:3.98 (3H, s), 4.06(3H,s), 6.83 (1H, d, J=8.8Hz), 7.07(2H,t,J=8.6Hz), 7.54-7.57(2H,m), 7.61 (1H, dd, J=2.9, 8.8Hz), 8.17 (1H, d, J=2.7Hz).
LC-MSm/z:329 (M+H)⁺.

### 3) Title compound

The above triazole-3-carboxylic acid methyl ester compound (1.73 g) was treated in a manner similar to that employed in step 3) of Referential Example 23, to thereby give the title compound as an amorphous product (1.27 g, 77%).
¹H-NMR (400MHz, CDC1₃) δ: 3.99(3H,s), 6.85(1H, d, J=8. 8Hz), 7.07-7.11(2H,m), 7.55-7.58(2H,m), 7.63(1H,br d,J=9.0Hz), 8.19(1H,s) .
LC-MSm/z:315(M+H)⁺.

### [Referential Example 25] 5-(6-Methoxy-3-pyridyl)-1-phenyl-1H-1,2,4-triazole-3-carboxylic acid

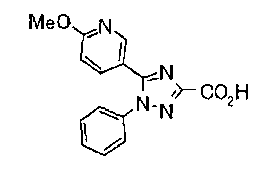

### 1) 6-Methoxynicotinic acid

1N Aqueous sodium hydroxide (45 mL) was added to methyl 6-methoxynicotinate (6.7 g) in methanol (60 mL) at room temperature, and the mixture was stirred for 7.5 hours. The reaction solvent was removed under reduced pressure, and 1N HCl was added to the residue to make the pH condition to 4. The solid that precipitated was collected through filtration and then dried, to thereby give 6-methoxynicotinic acid (5.19 g, 85%).
¹H-NMR (400MHz, DMSO-d₆) δ: 3.92 (3H, s), 6. 90 (1H, d, J=8. 6Hz), 8.14(1H,dd,J=8.6,2.2,Hz), 8.73 (1H, d, J=2.2Hz), 13.04 (1H, br s).
LC-MSm/z:154 (M+H)⁺.

### 2) 2-(6-Methoxy-3-nicotinoyl)aminomalonic acid diethyl ester 1-Ethyl-3-(3-dimethylaminopropyl)carbodiimide

hydrochloride (2.72 g) was added at 0°C to a solution of the above 6-methoxynicotinic acid (1.98 g), triethylamine (1.98 mL), aminomalonic acid diethyl ester hydrochloride (3.0 g), and 1-hydroxybenzotriazole (174 mg) in N,N-dimethylformamide (20 mL), and the mixture was stirred for 4 hours at room temperature. The reaction mixture was partitioned between water and ethyl acetate. The organic layer was sequentially washed with water, saturated aqueous sodium hydrogencarbonate, and saturated brine, and then dried over magnesium sulfate anhydrate. After a filtration step, the solvent was removed under reduced pressure, and the residue was purified through silica gel column chromatography (hexane - ethyl acetate). Diethyl ether-hexane was added to the product for solidification, and the product was collected through filtration and then dried, to thereby give an aminomalonic acid diethyl ester compound (3.39 g, 85%).
¹H-NMR (400MHz, CDCl₃) δ:1.29-1.34 (6H, m), 4.00(3H,s), 4.27-4.38(4H,m), 5.32(1H, d, J=6.8Hz), 6.79 (1H, d, J=8.5Hz), 7.02 (1H, d, J=6.5Hz), 8.02 (1H, dd, J=8.6, 2.5Hz), 8.69 (1H, d, J=2.5Hz).
MS (ESI) m/z:311 (M+H)⁺.

### 3) 5-(6-Methoxy-3-pyridyl)-1-phenyl-1H-1,2,4-triazole-3-carboxylic acid methyl ester

Aniline (484 µL) and the above aminomalonic acid diethyl ester compound (1.5 g) were treated in a manner similar to that employed in step 2) of Referential Example 23, to thereby give a triazole-3-carboxylic acid methyl ester compound as an amorphous product (1.12 g, 75%).
¹H-NMR(400MHz,CDCl₃)δ:3.94(3H,s), 4.06(3H,s), 6.73(1H,d,J=8.8Hz), 7.39-7.42(2H,m), 7.46-7.50(3H,m), 7.79(1H,dd,J=8.8,2.4Hz), 8.29 (1H, d, J=2.4Hz) .
MS(ESI)m/z:311(M+H)⁺.

### 4) Title compound

The above triazole-3-carboxylic acid methyl ester compound (1.1 g) was treated in a manner similar to that employed in step 3) of Example 23, to thereby give the title compound as a solid product (903 mg, 77%).
¹H-NMR (400MHz, CDCl₃) δ: 3. 95 (3H, s), 6.73 (1H, d, J=8.8Hz), 7.41-7.44(2H,m), 7.49-7.51 (3H,m), 7.79 (1H, d, J=8.6Hz), 8.30 (1H, s).
LC-MSm/z:297(M+H)⁺.

### [Referential Example 26] 1-(6-Methoxy-3-pyridyl)-5-(4-methylphenyl)-1H-1,2,4-triazole-3-carboxylic acid

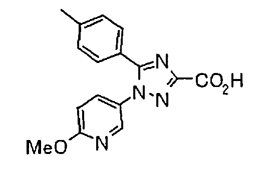

### 1) 2-(4-Methylbenzoyl)aminomalonic acid diethyl ester

4-Methylbenzoic acid (3.0 g) and aminomalonic acid diethyl ester hydrochloride (5.12 g) were treated in a manner similar to that employed in step 2) of Referential Example 25, to thereby give 2-(4-methylbenzoyl)aminomalonic acid diethyl ester as a solid product (2.45 g, 38%).
¹H-NMR(400MHz, CDCl₃) δ:1.32 (6H, t, J=7.1Hz), 2.41(3H,s), 4.25-4.37(4H,m), 5.34 (1H, d, J=5.2Hz), 7.08 (1H, d, J=6.6Hz), 7.26(2H, d, J=2.0Hz), 7.74(1H, d, J=8.1Hz).
LC-MSm/ z : 2 94 (M+H)⁺.

### 2) 1-(6-Methoxy-3-pyridyl)-5-(4-methylphenyl)-1H-1,2,4-triazole-3-carboxylic acid methyl ester

5-Amino-2-methoxypyridine (700 mg) and the above 2-(4-methylphenyl)aminomalonic acid diethyl ester (1.5 g) were treated in a manner similar to that employed in step 2) of Referential Example 23, to thereby give 1-(6-methoxy-3-pyridyl)-5-(4-methylphenyl)-1H-1,2,4-triazole-3-carboxylic acid methyl ester as a solid product (349 mg, 21%).
¹H-NMR(400MHz, CDCl₃)δ :2.37(3H,s), 3.98(3H,s), 4.05(3H,s), 6.81 (1H, dd, J=7.8, 0.7Hz), 7.17 (2H, dd, J=8.5, 0.5Hz), 7.43 (2H, dd, J=6.6, 1.7Hz), 7.61 (1H, dd, J=8.6, 2.7Hz), 8.18(1H, dd, J=2.7,0.7Hz).
MS (ESI)m/z:325 (M+H)⁺.

### 3) Title compound

The above 1-(6-methoxy-3-pyridyl)-5-(4-methylphenyl)-1H-1,2,4-triazole-3-carboxylic acid methyl ester (340 mg) was treated in a manner similar to that employed in step 3) of Referential Example 23, to thereby give the title compound as a solid product (257 mg, 79%).
¹H-NMR (400MHz, CDCl₃) δ: 2 .23 (3H, s), 3.95(3H,s), 6.75(1H,br d,J=8.1Hz), 6.72-6.95(2H,br m), 7.25-7.35(2H,br m), 7.61(1H,br d,J=7.8Hz), 8.14(1H,s).
MS(ESI)m/z:310(M⁺).

### [Referential Example 27] 5-(6-Methoxy-3-pyridyl)-1-(4-methylphenyl)-1H-1,2,4-triazole-3-carboxylic acid

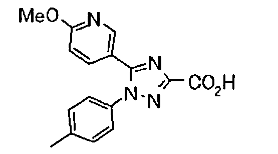

### 1) 5-(6-Methoxy-3-pyridyl)-1-(4-methylphenyl)-1H-1,2,4-triazole-3-carboxylic acid methyl ester

p-Toluidine (456 mg) and 2-(6-methoxy-3-nicotinoyl)aminomalonic acid diethyl ester (1.2 g) obtained in step 2) of Referential Example 25 were treated in a manner similar to that employed in step 3) of Referential Example 25, to thereby give 5-(6-methoxy-3-pyridyl)-1-(4-methylphenyl)-1H-1,2,4-triazole-3-carboxylic acid methyl ester as a solid product (550 mg, 44%).
¹H-NMR(400MHz,CDCl₃)δ:2.42(3H,s), 3.94(3H,s), 4.05(3H,s), 6.73(1H,d,J=8.8Hz), 7.26-7.29(4H,m), 7.81(1H,dd,J=8.6, 2.5Hz), 8.28(1H, d, J=2.4Hz).
LC-MSm/z:324(M+H)⁺.

### 2) Title compound

The above 5-(6-methoxy-3-pyridyl)-1-(4-methylphenyl)-1H-1,2,4-triazole-3-carboxylic acid methyl ester (550 mg) was treated in a manner similar to that employed in step 2) of Referential Example 23, to thereby give the title compound (489 mg, 93%) as a solid product.
¹H-NMR(400MHz, CDCl₃) δ:2.43 (3H,s), 3.95(3H,s), 6.75 (1H, dd, J=8.8, 0.7Hz), 7.26-7.32(4H,m), 7.83 (1H, dd, J=8.8, 2.5Hz), 8.31 (1H, dd, J=2.5, 0.8Hz).
LC-MSm/z:311(M+H)⁺.

### [Referential Example 28] 1-(4-Methylphenyl)-5-(6-methyl-3-pyridyl)-1H-1,2,4-triazole-3-carboxylic acid methyl ester

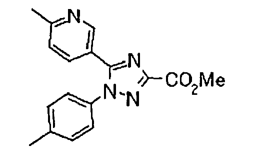

### 1) 2-(6-Methyl-3-nicotinoyl)aminomalonic acid diethyl ester

6-Methylnicotinic acid (2.0 g) and aminomalonic acid diethyl ester (3.49 g) were treated in a manner similar to that employed in step 2) of Referential Example 25, to thereby give 2-(6-methyl-3-nicotinoyl)aminomalonic acid diethyl ester as a solid product (2.5 g, 570).
¹H-NMR (400MHz, CDCl₃) δ:1.34 (6H, t, J=7.1Hz), 2.62(3H,s), 4.25-4.38(4H,m), 5.33 (1H, d, J=6.6Hz), 7.11 (1H, d, J=5.9Hz), 7.25 (1H, d, J=6.6Hz), 8.03 (1H, dd, J=8.1, 2.4Hz), 8.96(1H,d,J=2.4Hz).
LC-MSm/z:295(M+H)⁺.

### 2) Title compound

p-Toluidine (521 mg) and the above 2-(6-methyl-3-nicotinoyl)aminomalonic acid diethyl ester (1.3 g) were treated in a manner similar to that employed in step 3) of Referential Example 23, to thereby give the title compound as a solid product (777 mg, 57%).
¹H-NMR(400MHz,CDCl₃)δ:2.42(3H,s), 2.58 (3H,s), 4 .05(3H,s), 7.18(1H,d,J=8.1Hz), 7.26(4H,m), 7.84 (1H, dd, J=8. 1, 2.2Hz), 8.56 (1H,d, J=2.2Hz) .
MS (ESI)m/z:308 (M⁺) .

### [Referential Example 29] 1-(4-Methoxyphenyl)-5-(6-methyl-3-pyridyl)-1H-1,2,4-triazole-3-carboxylic acid

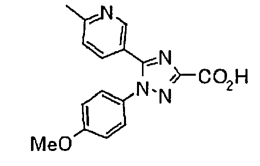

### 1) 1-(4-Methoxyphenyl)-5-(6-methyl-3-pyridyl)-1H-1,2,4-triazole-3-carboxylic acid methyl ester

p-Anisidine (461 mg) and 2-(6-methyl-3-nicotinoyl)aminomalonic acid diethyl ester (1.0 g) obtained in step 1) of Referential Example 28 were treated in a manner similar to that employed in step 3) of Referential Example 23, to thereby give 1-(4-methoxyphenyl)-5-(6-methyl-3-pyridyl)-1H-1,2,4-triazole-3-carboxylic acid methyl ester as a solid product (416 mg, 38%).
¹H-NMR(400MHz,CDCl₃)δ:2.58(3H,s), 3.86(3H,s), 4.06(3H,s), 6.94-6.98 (2H, m), 7.17 (1H, d, J=8.1Hz), 7.29-7.32 (2H, m), 7.85 (1H, dd, J=8.1, 2.2Hz), 8.58 (1H, d, J=1.7Hz).
MS (ESI) m/z:325 (M+H)⁺.

### 2) Title compound

The above 1-(4-methoxyphenyl)-5-(6-methyl-3-pyridyl)-1H-1,2,4-triazole-3-carboxylic acid methyl ester (200 mg) was treated in a manner similar to that employed in step 3) of Referential Example 23, to thereby give the title compound as a solid product (82 mg, 43%).
¹H-NMR (400MHz, DMSO-d₆) δ:2.46 (3H, s), 3.81(3H,s), 7.03-7.07(2H,m), 7.29 (1H, d, J=8.3Hz), 7.36-7.40(2H,m), 7.69 (1H, dd, J=8.0, 2.2Hz), 8.53 (1H, d, J=2.0Hz).

### [Referential Example 30] Azetidine-2-carboxylic acid dimethylamide hydrochloride

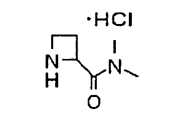

### 1) 1-Benzhydrylazetidine-2-carboxylic acid

1N Aqueous sodium hydroxide (30 mL) was added to 1-benzhydrylazetidine-2-carboxylic acid ethyl ester (6.0 g) in ethanol (60 mL) at room temperature, and the mixture was stirred for 4 hours. The reaction solvent was removed under reduced pressure, and 1N HCl was added to the residuce to make the pH condition to 7. The solid that precipitated was collected through filtration and then dried, to thereby give a carboxylic acid compound (4.8 g, 90%).
¹H-NMR(400MHz, CDCl₃)δ:2.26(1H,m), 2.51(1H,m), 3.15(1H,q, J=8. 8Hz), 3.64(1H, m), 3.97(1H,t, J=8.8Hz), 4. 69 (1H, s), 7.21-7.30(6H,m), 7.35-7.43 (4H,m) .
MS (ESI)m/z:268(M+H)⁺.

### 2) 1-Benzhydrylazetidine-2-carboxylic acid dimethylamide

1-Ethyl-3-(3-dimethylaminopropyl)carbodiimide hydrochloride (2.15 g) was added to the above carboxylic acid compound (2.5 g), a 2.0M solution (7.0 mL) of dimethylamine in tetrahydrofuran, and 1-hydroxybenzotriazole (126 mg) in N,N-dimethylformamide (30 mL) at 0°C, and the mixture was stirred for 19 hours at room temperature. The reaction mixture was partitioned between water and ethyl acetate. The organic layer was sequentially washed with saturated aqueous sodium hydrogencarbonate and saturated brine, and then dried over magnesium sulfate anhydrate. After a filtration step, the solvent was removed under reduced pressure, to thereby give 2-carboxylic acid dimethylamide compound as a solid product (2.4 g, 87%).
¹H-NMR (400MHz, CDCl₃) δ: 2.11(1H, m), 2.42(1H, m), 2.42(3H,s), 2.66(3H,s), 2.95(1H, q, J=8.3Hz), 3.43(1H, m), 4.13(1H, t, J=7.6Hz), 4.56(1H, s), 7.12-7.28(6H,m), 7.44-7.48(4H, m).
MS (ESI) m/z: 295(M+H)⁺.

### 3) Title compound

20% Palladium hydroxide (50% wet, 300 mg) was added to a solution of the above 2-carboxylic acid dimethylamide compound (800 mg) in ethanol (10 mL), and the mixture was stirred for 18.5 hours in a hydrogen atmosphere at room temperature. The reaction mixture was subjected to filtration, and 1N HCl in ethanol (3.6 mL) was added to the filtrate, followed by stirring for 30 minutes. The reaction solvent was removed under reduced pressure, to thereby give the title compound as a solid product (416 mg, 84%).
¹H-NMR(40OMHz,DMSO-d₆)δ:2.42(1H,quin., J=10.0Hz), 2.73(1H,m), 2.82(3H,d,J=1.8Hz), 2.89(3H,d,J=1.8Hz), 3.69(1H,m), 3.91(1H,q,J=8.3Hz), 5.26(1H,t,J=8.3Hz), 9.33(1H,br s).
MS (ESI)m/z:129 (M+H)⁺.

### [Referential Example 31] 2-Dimethylaminomethylazetidine hydrochloride

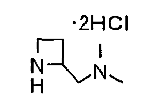

### 1) 1-Benzhydryl-2-dimethylaminomethylazetidine

A solution of 1-benzhydrylazetidine-2-carboxylic acid dimethylamide (1.2 g) obtained in step 2) of Referential Example 30 in a mixture of diethyl ether (10 mL) and tetrahydrofuran (20 mL) was added dropwise at 0°C to a suspension of lithium aluminum hydride (116 mg) in diethyl ether (10 mL), and the mixture was stirred for 2 hours at room temperature. Lithium aluminum hydride (77 mg) was further added to the reaction mixture, and the resultant mixture was stirred for 2 hours at room temperature. Water and 5N aqueous sodium hydroxide was added dropwise to the reaction mixture, and the resultant mixture was stirred for 20 minutes. The reaction mixture was subjected to filtration, and ethyl acetate was added to the filtrate for partitioning the resultant mixture. The organic layer was sequentially washed with saturated aqueous sodium hydrogencarbonate and saturated brine, and then dried over sodium sulfate anhydrate. After a filtration step, the solvent was removed under reduced pressure, and the residue was purified through NH-silica gel column chromatography (hexane - ethyl acetate), to thereby give 2-dimethylaminomethylazetidine compound as an oil (878 mg, 77%).
¹H-NMR(400MHz,CDCl₃)δ:1.45(1H,dd,J=12.3,2.7Hz), 1.94(1H,m), 1.96(6H, s), 2.10-2.20 (1H,m), 2.75(1H,q,J=8.9Hz), 3.31-3.39 (1H,m), 4.36 (1H, s), 7.16-7.31(6H,m), 7. 35 (2H, dd, J=8 . 1, 1 .2Hz), 7.42 (2H, dd, J=8. 3, 1 .2Hz) .

### 2) Title compound

The above 2-dimethylaminomethylazetidine compound (870 mg) was treated in a manner similar to that employed in step 3) of Referential Example 30, to thereby give the title compound as a solid product (490 mg, 84%).
¹H-NMR(400MHz, DMSO-d₆) δ:2.33 (1H, quin., J=8.5Hz), 2.46 (1H, m), 2.77(3H,s), 2.78(3H,s), 3.42 (1H, d, J=14.1Hz), 3.71 (1H, m), 3.87-3.93 (2H, m), 4.86 (1H, m), 9.54 (1H, br s).
MS (ESI) m/z:115 (M+H)⁺.

### [Referential Example 32] 1-methylpiperazin-2-one hydrochloride

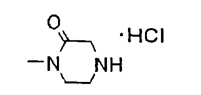

### 1) 3-Oxopiperazine-1-carboxylic acid tert-butyl ester

Triethylamine (3.83 mL) and di-tert-butoxydicarbonate (6.32 mL) were added to a solution of piperazin-2-one (2.5 g) in a mixture of tetrahydrofuran (50 mL) and methanol (50 mL) at room temperature, and the mixture was stirred for 4 hours. The reaction solvent was removed under reduced pressure, and the residue was partitioned between water and ethyl acetate. The organic layer was sequentially washed with water and saturated brine, and the aqueous layers obtained through washing were combined and then extracted with ethyl acetate. The organic layers were combined and then dried over magnesium sulfate anhydrate. After a filtration step, the solvent was removed under reduced pressure, and ethyl acetate - hexane was added to the residue for solidification, to thereby give 3-oxopiperazine-1-carboxylic acid tert-butyl ester (3.6 g, 72%).
¹H-NMR(400MHz,CDCl₃)δ:1.48(9H,s), 3.37-3.40(2H,m), 3.62-3.65(2H,m), 4.01(2H,s), 6.32(1H,br s).

### 2) 4-Methyl-3-oxopiperazine-1-carboxylic acid tert-butyl ester

60% Sodium hydride (960 mg) was added to the above 3-oxopiperazine-1-carboxylic acid tert-butyl ester (3.0 g) in N,N-dimethylformamide (50 mL) at 0°C, and methyl iodide (2.33 mL) was added to the reaction mixture, followed by stirring for 15 hours at room temperature. The reaction mixture was partitioned between water and ethyl acetate. The organic layer was sequentially washed with water and saturated brine, and the aqueous layers obtained through washing were combined and then extracted with ethyl acetate. The organic layers were combined and then dried over magnesium sulfate anhydrate. After a filtration step, the solvent was removed under reduced pressure, to thereby give 4-methyl-3-oxopiperazine-1-carboxylic acid tert-butyl ester as an oil (2.32 g, 72%).
¹H-NMR(40OMHz,CDCl₃)δ:1.47(9H,s), 3.01(3H,s), 3.34(2H, t, J=5.6Hz), 3.65 (2H, t, J=5.6Hz), 4.07 (2H, s ) .

### 3) Title compound

4N HCl-Dioxane (20 mL) was added to the above 4-methyl-3-oxopiperazine-1-carboxylic acid tert-butyl ester (2.06 g), and the mixture was stirred for 1 hour at room temperature. The reaction solvent was removed under reduced pressure, and toluene was added to the residue. The solvent was removed through azeotropy under reduced pressure, and the residue was dried, to thereby give the title compound as an oil (1.44 g, 99%).
¹H-NMR(400MHz,DMSO-d₆)δ:2.86(3H,s), 3.34(2H,br m), 3.50(2H,m), 3.64(2H,s).
MS(ESI)m/z:115(M+H)⁺.

### [Referential Example 33] 1-Cyclopropylpiperazine hydrochloride

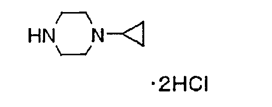

### 1) 1-Cyclopropylpiperazine-4-carboxylic acid tert-butyl ester

To a solution of piperazine-1-carboxylic acid tert-butyl ester (1.87 g), [(1-ethoxycyclopropyl)oxy]trimethylsilane (8.05 mL), and acetic acid (5.72 mL) in methanol (60 mL), at room temperature, sodium cyanoborohydride (1.89 g) was added, and the mixture was stirred for 5 days. The reaction solvent was removed under reduced pressure, and diethyl ether was added to the residue. Insoluble matter was removed through filtration. 1N Aqueous sodium hydroxide was added to the filtrate for partitioning the resultant mixture. The organic layer was washed with saturated brine and then dried over magnesium sulfate anhydrate. After a filtration step, the solvent was removed under reduced pressure, and the residue was purified through silica gel column chromatography (hexane - ethyl acetate), to thereby give 1-cyclopropylpiperazine-4-carboxylic acid tert-butyl ester as a solid product (1.62 g, 71%).
¹H-NMR (400MHz, CDCl₃) δ:0.41-0.48 (4H, m), 1.46(9H,s), 2.54-2.56 (4H,m), 3.37-3.44 (4H, m).
MS (ESI)m/z:268 (M+MeCN)⁺.

### 2) Title compound

The above 1-cyclopropylpiperazine-4-carboxylic acid tert-butyl ester (1.61 g, 7.11mmol) was treated in a manner similar to that employed in step 3) of Referential Example 32, to thereby give the title compound as a solid product (1.30 g, 93%).
¹H-NMR(400MHz,DMSO-d₅)δ:0.79-0.81(2H,m), 1.14(2H,br s), 3.52 (8H, br s), 9.94 (2H, br).

### [Referential Example 34] Azetidin-3-yldimethylamine hydrochloride

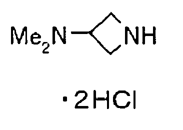

### 1) 1-Benzhydrylazetidin-3-one

Under cooling on ice, pyridinesulfonic acid (19.7 g) in dimethyl sulfoxide (84 mL) was added dropwise to 1-benzhydrylazetidin-3-ol (4.79 g) in triethylamine (27.9 mL), and the mixture was stirred for 40 minutes at 50°C. The reaction mixture was partitioned between ice-water and ethyl acetate. The organic layer was washed with saturated brine and then dried over magnesium sulfate anhydrate. After a filtration step, the solvent was removed under reduced pressure, and the residue was purified through silica gel column chromatography (hexane - ethyl acetate), to thereby give 1-benzhydrylazetidin-3-one as a solid product (2.85 g, 60%).
¹H-NMR(400MHz,CDCl₃)δ:4.00(4H,s), 4.59 (1H,s), 7.19-7.49 (10H,m) .

### 2) (1-Benzhydrylazetidin-3-yl)dimethylamine

5% Palladium-carbon (1.5 g) was added to a solution of the above 1-benzhydrylazetidin-3-one (1.50 g) and 40% aqueous dimethylamine (4 mL) in methanol (30 mL), and the mixture was stirred overnight in a hydrogen atmosphere. The catalyst was removed from the reaction mixture through filtration, and the solvent of the filtrate was removed under reduced pressure. The residue was purified through silica gel column chromatography (chloroform - methanol), to thereby give (1-benzhydrylazetidin-3-yl)dimethylamine as a solid product (1.55 g, 92%).
¹H-NMR(400MHz,CDCl₃)δ:2.08(6H,s), 2.80-2.87(3H,m), 3.36-3.42(2H,m), 4.37 (1H, s), 7.15-7.41 (10H, m).
MS (ESI)m/z:267 (M+H)⁺.

### 3) Title compound

20% Palladium hydroxide-carbon (533 mg) was added to a solution of the above (1-benzhydrylazetidin-3-yl)dimethylamine (533 mg) in ethanol (15 mL), and the mixture was stirred for 18 hours in a hydrogen atmosphere. The catalyst was removed from the reaction mixture through filtration. 1N HCl in ethanol (4 mL) was added to the solvent of the filtrate, and the solvent was removed under reduced pressure. Ether was added to the residue, and the solid that precipitated was collected through filtration, to thereby give the title compound (300 mg, 87%).
¹H-NMR (400MHz, DMSO-d₆) δ:2.70 (6H, m), 4.05-4.10 (2H, m), 4.25-4.31 (1H, m), 4.38-4.43 (2H, m).
LC-MSm/z:101 (M+H)⁺.

### [Referential Example 35] (2,2-Dimethylazetidin-3-yl)dimethylamine hydrochloride

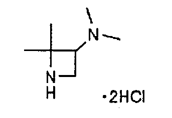

### 1) 3-Bromo-3-methylbutan-2-one

3 Drops of bromine was added to a solution of potassium chloride (2.1 g) and 3-methylbutan-2-one (30 mL) in water (20 mL) at 60°C, while the solution was irradiated with light from an incandescent lamp (250 W). After confirmation of decoloration, bromine (7.6 mL) was added dropwise to the mixture over 1 hour, while the mixture was irradiated with light from an incandescent lamp (100 W) at an internal temperature of 40 to 45°C, and the resultant mixture was stirred for 2 hours at 40°C and then cooled in air. The reaction mixture was partitioned between water and ethyl acetate. The organic layer was sequentially washed with water, saturated aqueous sodium bicarbonate, and saturated brine, and then dried over calcium chloride anhydrate. After a filtration step, the solvent was removed under reduced pressure, and the residue was distilled (boiling point: 120-130°C), to thereby give 3-bromo-3-methylbutan-2-one as an oil (5.88 g, 13%).
¹H-NMR(400MHz,CDCl₃)δ:1.86(6H,s), 2.44(3H,s) .

### 2) 3-(Benzhydrylamino)-3-methylbutan-2-one

Benzhydrylamine (5.0 mL) and triethylamine (7.5 mL) were added to a solution of the above 3-bromo-3-methylbutan-2-one (5.88 g) in methanol (30 mL), and the mixture was stirred for 24 hours at 70°C and then cooled in air. The reaction mixture was partitioned between water and ethyl acetate. The organic layer was sequentially washed with saturated aqueous sodium hydrogencarbonate and saturated brine, and then dried over magnesium sulfate anhydrate.
After a filtration step, the solvent was removed under reduced pressure, and diethyl ether was added to the produced solid. Insoluble matter was removed through filtration. The solvent of the filtrate was evaporated under reduced pressure, and the residue was purified through silica gel column chromatography (hexane - ethyl acetate), to thereby give 3-(benzhydrylamino)-3-methylbutan-2-one as an oil (3.3 g, 34%).
¹H-NMR(400MHz,CDCl₃)δ:1.18(6H,s), 2.09(3H,s), 4.76(1H,s), 7.17(2H,m), 7.25-7.29(4H,m), 7.37-7.39(4H,m).
LC-MSm/z : 2 68 (M+H)⁺.

### 3) 1-Benzhydryl-2,2-dimethylazetidin-3-one

HCl gas was bubbled into a solution of the above 3-(benzhydrylamino)-3-methylbutan-2-one (6.5 g) in acetic acid (20 mL) to thereby saturate the solution, and bromine (1.25 mL) was added thereto, followed by stirring for 3 hours. 20% Aqueous sodium hydroxide was added to the reaction mixture to adjust the pH to 14, and carbon tetrachloride was added to the mixture for partitioning the mixture. The organic layer was washed with water. The solvent was removed under reduced pressure, and N,N-dimethylformamide (30 mL) and saturated aqueous sodium hydrogencarbonate (7 mL) were added to the residue, followed by stirring for 3 minutes. The reaction mixture was partitioned between water and carbon tetrachloride. The organic layer was washed twice with saturated brine, and then dried over magnesium sulfate anhydrate. After a filtration step, the solvent was removed under reduced pressure, and the residue was purified through silica gel column chromatography, to thereby give 1-benzhydryl-2,2-dimethylazetidin-3-one as a solid product (754 mg, 12%).
¹H-NMR(400MHz,CDCl₃)δ:1.20(6H,s), 3.95(2H,s), 4.85(1H,s), 7.18(2H,m), 7.26-7.31(4H,m), 7.52-7.54(4H,m).

### 4) Title compound

2M Dimethylamine in tetrahydrofuran (3 mL) and 10% palladium-carbon (50% wet, 250 mg) were added to a suspension of the above 1-benzhydryl-2,2-dimethylazetidin-3-one (265 mg) in methanol (4 mL), and the mixture was stirred for 20 hours in a hydrogen atmosphere at room temperature. The reaction mixture was subjected to filtration, and the solvent was removed under reduced pressure. Ethanol (4 mL) and 20% palladium hydroxide (50% wet, 265 mg) were sequentially added to the residue, and the mixture was stirred for 22 hours in a hydrogen atmosphere at room temperature. The reaction mixture was subjected to filtration, and 1N HCl in ethanol (2.2 mL) was added to the reaction solvent, followed by stirring for 10 minutes. The reaction solvent was removed under reduced pressure, and diethyl ether-ethyl acetate was added to the residue for solidification, followed by filtration, to thereby give the title compound (60 mg, 30%).
¹H-NMR(400MHz,DMSO-d₆)δ:1.62(3H,s), 1.81(3H,s), 2.57(6H,m), 3.89(2H,m), 4.06(1H,m).
LC-MSm/z:129(M+H)⁺.

### [Referential Example 36] 4,7-Diazaspiro[2.5]octane hydrochloride

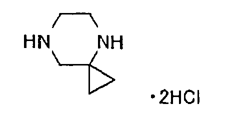

### 1) [(1-Benzyloxycarbonylaminocyclopropanecarbonyl)amino]acetic acid ethyl ester

Triethylamine (3.2 mL), glycine ethyl ester hydrochloride (3.22 g), and 1-hydroxybenzotriazole (283 mg) were added to 1-benzyloxycarbonylaminocyclopropanecarboxylic acid (4.9 g) in N,N-dimethylformamide (50 mL) at room temperature. At 0°C, 1-ethyl-3-(3-dimethylaminopropyl)carbodiimide hydrochloride (5.23 g) was gradually added to the mixture, and the resultant mixture was stirred for 16.5 hours. The reaction mixture was partitioned between ice-water and ethyl acetate. The organic layer was sequentially washed with saturated aqueous sodium hydrogencarbonate and saturated brine, and then dried over magnesium sulfate anhydrate. After a filtration step, the solvent of the filtrate was removed under reduced pressure, and the residue was purified through silica gel column chromatography (chloroform - methanol). Diethyl ether-hexane was added to the product for solidification, to thereby give [(1-benzyloxycarbonylaminocyclopropanecarbonyl)amino]acetic acid ethyl ester (6.13 g, 61%).
¹H-NMR (400MHz, CDCl₃)δ:1.09 (2H,m), 1.28(3H,t,J=7.1Hz), 1.62(2H,m), 4.03 (2H,m), 4.21(2H,d,J=7.1Hz), 5.14(2H,br s), 5.32(1H,br s), 6.88(1H,br s), 7.29-7.39(5H,m).
LC-MSm/z:321(M+H)⁺.

### 2) 4,7-Diazaspiro[2.5]octane-5,8-dione

10% Palladium-carbon (50% wet, 0.5 g) was added to a solution of the above [(1-benzyloxycarbonylaminocyclopropanecarbonyl)amino]acetic acid ethyl ester (5.18 g) in ethanol (50 mL), and the mixture was stirred for 2.5 hours in a hydrogen atmosphere at room temperature. The catalyst was removed from the reaction mixture through filtration, and the solvent of the filtrate was removed under reduced pressure. The residue was dissolved in toluene (120 mL), and 1,8-diazabicyclo[5.4.0]undec-7-ene (1.2 mL) was added to the solution. The mixture was refluxed for 16.5 hours and then cooled in air. The reaction solvent was removed under reduced pressure, and methanol was added to the residue for solidification, to thereby give 4,7-diazaspiro[2.5]octane-5,8-dione (1.85 g, 81%).
¹H-NMR(400MHz, DMSO-d₆)δ:0.89-0.92(2H,m), 1.14-1.17(2H,m), 3.85(2H,s), 8.02(1H, br s), 8.26(1H, br s).

### 3) Title compound

A 1.04 M solution (24.7 mL) of borane-tetrahydrofuran complex in tetrahydrofuran was added dropwise to a solution of the above 4,7-diazaspiro[2.5]octane-5,8-dione (1.2 g) in tetrahydrofuran (30 mL) over 30 minutes at 0°C, and the mixture was refluxed for 13 hours. At 0°C, methanol (4 mL) and 4N HCl-dioxane (8 mL) were added to the reaction mixture, and the resultant mixture was refluxed for 1 hour and then cooled in air. The solid that precipitated was collected through filtration and then washed with tetrahydrofuran, to thereby give a mixture (1.86 g) containing 4,7-diazaspiro[2.5]ocatne. The mixture (1.4 g) was dissolved in water (25 mL), and triethylamine (3.16 mL) was added to the solution. N-Carbobenzoxysuccinimide (4.7 g) in acetonitrile (15 mL) was added to the reaction mixture, and the resultant mixture was stirred for 24 hours at room temperature. The reaction mixture was partitioned between water and ethyl acetate. The aqueous layer was extracted with ethyl acetate, and the organic layers were combined, sequentially washed with saturated aqueous sodium hydrogencarbonate and saturated brine, and dried over magnesium sulfate anhydrate. After a filtration step, the solvent was removed under reduced pressure, and the residue was purified through silica gel column chromatography (hexane - ethyl acetate), to thereby give an N-benzyloxycarbonyl compound as an oil (1.4 g). The oil substance (1.4 g) was dissolved in ethanol (10 mL), and 10% palladium-carbon (50% wet, 100 mg) was added to the solution. In a hydrogen atmosphere, the mixture was stirred for 1.5 hours at room temperature. The reaction mixture was subjected to filtration, and 1N HCl in ethanol (5.78 mL) was added to the solvent at 0°C, followed by stirring for 1 hour. The solvent of the reaction mixture was removed under reduced pressure, and ethanol and ethyl acetate were added for solidification, followed by filtration, to thereby give the title compound (315 mg, 26%).
¹H-NMR (400MHz, DMSO-d₆)δ: 0.96-1. 03 (2H,m), 1.18-1.21 (2H,m), 3.30(2H,s), 8. 36 (4H,m) .
LC-MSm/z:113(M+H)⁺.

### [Referential Example 37] 5-(3-Fluoro-4-methylphenyl)-1-(6-methoxy-3-pyridyl)-1H-1,2,4-triazole-3-carboxylic acid

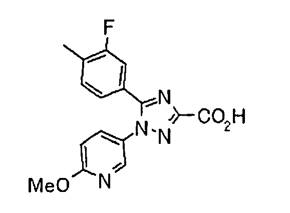

### 1) 2-(3-Fluoro-4-methylbenzoyl)aminomalonic acid diethyl ester

3-Fluoro-4-methylbenzoic acid (5.00 g) in thionyl chloride (50 mL) was refluxed for 2 hours and then cooled in air. The solvent was removed under reduced pressure, to thereby give an acid chloride. The acid chloride and aminomalonic acid diethyl ester hydrochloride (6.00 g) were treated in a manner similar to that employed in step 1) of Referential Example 23, to thereby give 2-(3-fluoro-4-methylbenzoyl)aminomalonic acid diethyl ester as a solid product (7.01 g, 80%).
¹H-NMR (400MHz, CDCl₃)δ:1.33(6H, t, J=7.3Hz) , 2.33(3H,d,J=1.7Hz), 4.27-4.37(4H,m), 5.32(1H,d,J=6.8Hz), 7.07(1H,d,J=6.8Hz), 7.25-7.29(1H,m), 7.46-7.52(2H,m).

### 2) 5-(3-Fluoro-4-methylphenyl)-1-(6-methoxy-3-pyridyl)-1H-1,2,4-triazole-3-carboxylic acid methyl ester

5-Amino-2-methoxypyridine (2.25 g) and the above 2-(3-fluoro-4-methylbenzoyl)aminomalonic acid diethyl ester (5.11 g) were treated in a manner similar to that employed in step 2) of Referential Example 23, to thereby give 1H-1,2,4-triazole-3-carboxylic acid methyl ester compound as a solid product (2.25 g, 40%).
¹H-NMR (400MHz, CDCl₃) : 2.30 (3H, d, J=1.7Hz), 3. 99 (3H, s), 4.06(3H,s), 6.84(1H, d, J=8.8Hz), 7.18-7.30(3H, m), 7.62 (1H, dd, J=8.8, 2.9Hz), 8.18(1H, d, J=2.9Hz).
LC-MSm/z:343(M+H)⁺.

### 3) Title compound

The above 1H-1,2,4-triazole-3-carboxylic acid methyl ester compound (2.00 g) was treated in a manner similar to that employed in step 3) of Referential Example 23, to thereby give the title compound as a solid product (1.78 g, 93%).
¹H-NMR(400MHz, DMSO-d₆)δ:2.25(3H,d,J=1.7Hz), 3.92(3H,s), 7.00(1H,d,J=8.9Hz), 7.18(1H,dd,J=7.8,1.7Hz), 7.27(1H,dd,J=10.5,1.7Hz), 7.37(1H,t,J=7.8Hz), 7.89(1H,dd,J=8.9,2.8Hz), 8.32(1H,d,J=2.8Hz), 13.61(1H,br).
LC-MSm/z:329(M+H)⁺.

### [Referential Example 38] 1-Methylpiperazin-2-one trifluoroacetic acid salt

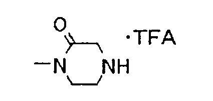

### 1) 3-Oxopiperazine-1-carboxylic acid tert-butyl ester

Triethylamine (3.9 mL) and di-tert-butyl dicarbonate (6.31 g) were added to a solution of 2-oxopiperazine (2.61 g) in a mixture of tetrahydrofuran (40 mL) and methanol (50 mL) at room temperature, and the mixture was stirred for 3 hours. The reaction solvent was removed under reduced pressure, and diethyl ether was added to the residue. The solid that precipitated was collected through filtration, to thereby give 3-oxopiperazine-1-carboxylic acid tert-butyl ester (4.54 g, 87%).
¹H-NMR(400MHz,DMSO-d₆)δ:1.40 (9H,s), 3.15(2H,br), 3.45(2H,br), 3.81 (2H,br) , 8.03 (1H,br) .
LC-MSm/z:201(M+H)⁺.

### 2) 4-Methyl-3-oxopiperazine-1-carboxylic acid tert-butyl ester

Sodium hydride (which was used after having been washed with pentane and then dried; 44.3 mg) was added to a solution of the above 3-oxopiperazine-1-carboxylic acid tert-butyl ester (0.303 g) in N,N-dimethylformamide (12 mL) at 0°C, and the mixture was stirred for 10 minutes. Methyl iodide (0.141 mL) was added to the reaction mixture, and the resultant mixture was stirred for 20 hours at room temperature. The reaction mixture was partitioned between water and ethyl acetate. The aqueous layer was extracted with ethyl acetate, and the organic layers were combined, washed with saturated brine, and dried over sodium sulfate anhydrate. After a filtration step, the solvent was removed under reduced pressure, to thereby give 4-methyl-3-oxopiperazine-1-carboxylic acid tert-butyl ester as an oil (0.308 g, 95%).
¹H-NMR(400MHz,CDCl₃)δ:1.46(9H,s), 2.99(3H,s), 3.34(2H,t-like,J=5.3Hz), 3.65(2H,t-like,J=5.3Hz), 4.07(2H, s).
MS(FAB)m/z:215(M+H)⁺.

### 3) Title compound

Trifluoroacetic acid (3 mL) was added to a solution of the above 4-methyl-3-oxopiperazine-1-carboxylic acid tert-butyl ester (0.308 g) in methylene chloride (6 mL) at room temperature, and the mixture was stirred for 1.5 hours. The reaction solvent was removed under reduced pressure, and the residue was dried, to thereby give the title compound (0.485 g, quantitative amount).
¹H-NMR(400MHz,CDCl₃-CD₃OD(15:1))δ:2.98 (3H, s), 3.39(2H,t-like, J=6. 1Hz), 3.54(2H,t-like,J=6.1Hz), 3.72(2H,s).
MS(EI)m/z:114(M⁺).

### [Referential Example 39] 4-Methoxypiperidine hydrochloride

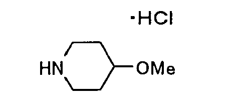

### 1) 4-Methoxypiperidine-1-carboxylic acid tert-butyl ester

In an argon atmosphere, 4-hydroxypiperidine-1-carboxylic acid tert-butyl ester (2.00 g) in N,N-dimethylformamide (20 mL) was added dropwise to a suspension of 60% sodium hydride (0.477 g) in N,N-dimethylformamide (20 mL) at room temperature. The mixture was stirred for 15 minutes, and methyl iodide (0.742 mL) was added thereto, followed by stirring for 2 hours. The reaction mixture was partitioned between water and ethyl acetate, and the organic layer was dried over sodium sulfate anhydrate. After a filtration step, the solvent was removed under reduced pressure, and the residue was purified through silica gel column chromatography (hexane - ethyl acetate), to thereby give 4-methoxypiperidine-1-carboxylic acid tert-butyl ester as an oil (1.43 g, 67%).
¹H-NMR (400MHz, CDCl₃)δ: 1.39-1 .54 (2H,m), 1.46(9H,s), 1.81-1.84(2H,m), 3.05-3.12(2H,m), 3.31-3.39(1H,m), 3.35(3H,s), 3.74-3.77(2H,m).

### 2) Title compound

4N HCl-Dioxane (10 mL) was added to a solution of the above 4-methoxypiperidine-1-carboxylic acid tert-butyl ester (5.34 g) in 1,4-dioxane (10 mL) at room temperature, and the mixture was stirred for 30 minutes. 4N HCl-Dioxane (20 mL) was further added thereto, and the resultant mixture was stirred for 30 minutes. The reaction solvent was removed under reduced pressure, and the obtained solid was washed with ethyl acetate, to thereby give the title compound (3.55 g) .
¹H-NMR (400MHz, DMSO-d₆) δ: 1.68 (2H,m), 1.93 (2H,m), 2.91 (2H, m), 3.08(2H,m), 3.23(3H,s), 3.42 (1H, q, J=3. 90Hz).

### [Referential Example 40] 4,4-Difluoropiperidine hydrochloride

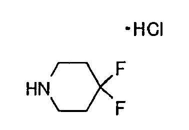

### 1) N-Benzyl-4,4-difluoropiperidine

In an argon atmosphere, diethylaminosulfur trifluoride (8.38 mL) was added dropwise to 1-benzyl-4-piperidone (5.00 g) in benzene (200 mL) at 0°C, and the mixture was stirred for 30 minutes and then refluxed for 18 hours. At 0°C, the reaction mixture was partitioned between saturated aqueous sodium hydrogencarbonate and ethyl acetate, and the organic layer was dried over sodium sulfate anhydrate. After a filtration step, the solvent was removed under reduced pressure, and the residue was purified through silica gel column chromatography (hexane - ethyl acetate), to thereby give N-benzyl-4,4-difluoropiperidine as an oil (4.67 g, 84%).
¹H-NMR (400MHz, CDCl₃) δ:1.93-2.04(4H,m), 2.53-2.55(4H,m), 3.54(2H, s), 7.24-7.34(5H, m).
MS (EI) m/z: 211(M⁺).

### 2) Title compound

In an argon atmosphere, 1-chloroethyl chloroformate (2.62 mL) was added dropwise to a solution of the above N-benzyl-4,4-difluoropiperidine (4.66 g) in methylene chloride (93 mL) at 0°C, and the mixture was stirred for 2 hours at 55°C and then cooled in air. The reaction solvent was removed under reduced pressure, and the residue was dissolved in methanol (93 mL). The solution was refluxed for 4 hours and cooled in air. The reaction solvent was removed under reduced pressure, to thereby give the title compound as a solid product (3.03 g, 87%).
MS (FAB) m/z : 122 (M+H)⁺.

### [Referential Example 41] 4-Fluoropiperidine hydrochloride

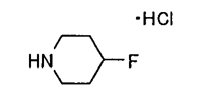

### 1) 4-Fluoropiperidine-N-carboxylic acid tert-butyl ester

In an argon atmosphere, [bis(2-methoxyethyl)amino]sulfur trifluoride (7.33 mL) was added dropwise to 4-hydroxy-1-piperidinecarboxylic acid tert-butyl ester (4.00 g) in methylene chloride (80 mL) at -78°C, and the mixture was stirred for 30 minutes, at 0°C for 30 minutes, and at room temperature for 2 hours. The reaction mixture was partitioned between saturated aqueous sodium hydrogencarbonate and chloroform, and the organic layer was dried over sodium sulfate anhydrate. After a filtration step, the solvent was removed under reduced pressure, and the residue was purified through silica gel column chromatography (chloroform - ethyl acetate), to thereby give 4-fluoropiperidine-N-carboxylic acid tert-butyl ester as an oil (1.77 g, 44%).
¹H-NMR (400MHz, CDCl₃) δ: 1.45(9H, s), 1.86-1.76(4H,m), 3.41-3.54(4H, m), 4.70-4.87(1H, m).
MS (EI) m/z: 203 (M⁺).

### 2) Title compound

The above 4-fluoropiperidine-N-carboxylic acid tert-butyl ester (1.74 g) was treated in a manner similar to that employed in step 2) of Referential Example 39, to thereby give the title compound as a solid product (0.87 g, 73%).
¹H-NMR(400MHz,DMSO-d₆)δ:2.13-1.92(4H,m), 3.01-3.12(4H,m), 4.83-4.97(1H,m).
MS(FAB)m/z:104(M+H)⁺.

### [Referential Example 42] 1-(6-Methoxy-3-pyridyl)-5-(2-pyridyl)-1H-1,2,4-triazole-3-carboxylic acid

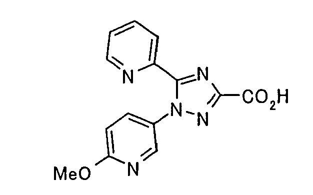

### 1) 2-[(Pyridine-2-carbonyl)amino]malonic acid dimethyl ester

Picolinoyl chloride hydrochloride (15.0 g) was added to a solution of aminomalonic acid dimethyl ester hydrochloride (18.56 g) and triethylamine (35.2 mL) in methylene chloride (210 mL) at 0°C, and the mixture was stirred for 4.5 hours at room temperature. The reaction mixture was partitioned between saturated aqueous sodium hydrogencarbonate and methylene chloride, and the organic layer was washed with saturated brine and then dried over sodium sulfate anhydrate. After a filtration step, the solvent was removed under reduced pressure, and the residue was purified through silica gel column chromatography (ethyl acetate - hexane), to thereby give 2-[(pyridine-2-carbonyl)amino]malonic acid dimethyl ester as a solid product (17.9 g, 84%).
¹H-NMR(400MHz,CDCl₃)δ:3.87(6H,s), 5.43(1H,d,J=9.2Hz), 7.43-7.53 (1H,m), 7.86(1H,td,J=7.7,1.7Hz), 8.16-8.19(1H,m), 8.94-9.00(1H,m), 8.58-8.63(1H,m).
MS (ESI)m/z:253 (M+H)⁺.

### 2) 1-(6-Methoxy-3-pyridyl)-5-(2-pyridyl)-1H-1,2,4-triazole-3-carboxylic acid methyl ester

Sodium nitrite (3.1 g) in water (20 mL) was gradually added dropwise to a solution of 5-amino-2-methoxypyridine (5.4 g) in a mixture of acetic acid (26 mL) and concentrated hydrochloric acid (6.5 mL) at 0°C, and the mixture was stirred for 15 minutes. The reaction mixture was cooled to - 15°C, and a solution of the above 2-[(pyridine-2-carbonyl)amino]malonic acid dimethyl ester (10 g) in acetone (90 mL) and a solution of potassium carbonate (54.7 g) in water (80 mL) were gradually added to the reaction mixture, followed by stirring for 30 minutes at 0°C. Ethyl acetate was added to the reaction mixture for partitioning the resultant mixture, and the organic layer was sequentially washed with water, saturated aqueous sodium hydrogencarbonate, water, and saturated brine, and then dried over sodium sulfate anhydrate. After a filtration step, the solvent was removed under reduced pressure, and the residue was dissolved in methanol (200 mL). Sodium methoxide (356 mg) was added to the solution at room temperature, and the mixture was stirred for 19 hours. The reaction solvent was removed under reduced pressure, and the obtained solid was collected through filtration and then washed with methanol, to thereby give 1-(6-methoxy-3-pyridyl)-5-(2-pyridyl)-1H-1,2,4-triazole-3-carboxylic acid methyl ester as a solid product (2.6 g, 21%).
¹H-NMR(400MHz, CDCl₃)δ:3.99 (3H,s), 4.04(3H,s), 6.82(1H, d, 8.8Hz), 7.33(1H, ddd, J=7.6, 4.8, 1.1Hz), 7.71(1H, dd, J=8.8, 2.7Hz), 7.83(1H, td, J=7.8, 1.8Hz), 8.22-8.24(2H,m), 8.43(1H, dq, J=4.7, 0.9Hz).

### 3) Title compound

1N Aqueous sodium hydroxide (10 mL) was added to a solution of the above 1-(6-methoxy-3-pyridyl)-5-(2-pyridyl)-1H-1,2,4-triazole-3-carboxylic acid methyl ester (2.4 g) in methanol (20 mL), and the mixture was stirred for 1 hour at room temperature. The reaction mixture was acidified with 1N HCl, and the solid that precipitated was collected through filtration and then dried, to thereby give the title compound as a solid product (1.84 g, 81%).
¹H-NMR (400MHz, DMSO-d₆) δ: 3.91(3H, s), 6.95(1H,d,J=8.5Hz), 7.48(1H,ddd,J=7.5, 4.8, 1.2Hz), 7.88(1H, dd, J=8.6, 2.7Hz), 8.00(1H, td, J=7.7, 1.7Hz), 8.11-8.14(1H, m), 8.30-8.34(1H, m), 8.41-8.43(1H, m).
MS (ESI) m/z: 298(M+H)⁺.

### [Referential Example 43] 5-(5-Cyano-2-pyridyl)-1-(6-methoxy-3-pyridyl)-1H-1,2,4-triazole-3-carboxylic acid

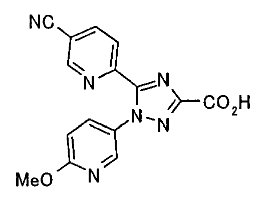

### 1) 5-Benzyloxy-2-methylpyridine

Benzyl bromide (10.9 mL) was added to a solution of 5-hydroxy-2-methylpyridine (10.0 g) and potassium carbonate (38.0 g) in acetonitrile (200 mL) at room temperature, and the mixture was stirred for 12 hours. The reaction mixture was partitioned between water and ethyl acetate, and the organic layer was dried over sodium sulfate anhydrate. After a filtration step, the solvent was removed under reduced pressure, and the residue was purified through silica gel column chromatography (ethyl acetate - hexane), to thereby give 5-benzyloxy-2-methylpyridine as an oil (4.14 g, 23%).
¹H-NMR(400MHz,CDCl₃)δ:2.48(3H,s), 5.08(2H,s), 7.05(1H,d,J=8.5Hz), 7.16(1H,dd,J=8.5,2.9Hz), 7.31-7.43(5H,m), 8.26 (1H, d, J=2.9Hz).
MS(EI)m/z:199(M⁺).

### 2) 2-[(5-Benzyloxypyridine-2-carbonyl)amino]malonic acid dimethyl ester

Selenium dioxide (40 g) was added to a solution of the above 5-benzyloxy-2-methylpyridine (40 g) in pyridine (200 mL), and the mixture was stirred for 24 hours and then cooled in air. The reaction mixture was partitioned between water and chloroform, and the organic layer was dried over sodium sulfate anhydrate. After a filtration step, the solvent was removed under reduced pressure, and toluene was added to the residue. The solvent was removed through azeotropy under reduced pressure. The residue was dissolved in N,N-dimethylformamide (1 L). Triethylamine (83.94 mL), 1-ethyl-3-(3-dimethylaminopropyl)carbodiimide hydrochloride (42.33 g), 1-hydroxybenzotriazole (29.8 g), and aminomalonic acid dimethyl ester hydrochloride (37 g) were added to the solution, and the mixture was stirred for 164 hours at room temperature. The reaction mixture was partitioned between water and methylene chloride-methanol (10:1) solvent mixture, and the organic layer was dried over sodium sulfate anhydrate. After a filtration step, the solvent was removed under reduced pressure, and the residue was purified through silica gel column chromatography (hexane - ethyl acetate). The crude crystals were recrystallized from methylene chloride - hexane, to thereby give 2-[(5-benzyloxypyridine-2-carbonyl)amino]malonic acid dimethyl ester (14.7 g).
¹H-NMR (400MHz, CDCl₃) δ:3.85 (6H, s), 5.17(2H,s), 5.42 (1H, d, J=7.4Hz), 7.33-7.44(6H,m), 8.11 (1H, d, J=9.1Hz), 8.32 (1H, d, J=6.1Hz), 8.72 (1H, d, J=7.4Hz).

### 3) 5-(5-Benzyloxy-2-pyridyl)-1-(6-methoxy-3-pyridyl)-1H-1,2,4-triazole-3-carboxylic acid methyl ester

Sodium nitrite (4.77 g) in water (17 mL) was added dropwise to a solution of 5-amino-2-methoxypyridine (5.9 g) in acetic acid (27 mL) and concentrated hydrochloric acid (6.75 mL) at 0°C, and the mixture was stirred for 15 minutes. At -15°C, a solution of the above 2-[(5-benzyloxypyridine-2-carbonyl)amino]malonic acid dimethyl ester (14.7 g) in acetone (68 mL) was added to the reaction mixture, and saturated aqueous potassium carbonate was gradually added to the reaction mixture to adjust the pH of the reaction mixture to 6. The resultant mixture was stirred for 30 minutes at 0°C, and ethyl acetate was added thereto for partitioning the mixture. The organic layer was sequentially washed with water, saturated aqueous sodium hydrogencarbonate, water, and saturated brine, and then dried over sodium sulfate anhydrate. After a filtration step, the solvent was removed under reduced pressure, and the residue was dissolved in methanol (200 mL). At room temperature, sodium methoxide (356 mg) was added to the solution, and the mixture was stirred for 19 hours. The reaction solvent was removed under reduced pressure, and the obtained crude crystals were collected through filtration and then washed with cold methanol, to thereby give 5-(5-benzyloxy-2-pyridyl)-1-(6-methoxy-3-pyridyl)-1H-1,2,4-triazole-3-carboxylic acid methyl ester (5.3 g, 31%).
¹H-NMR(400MHz,CDCl₃)δ:4.01(3H,s), 4.06(3H,s), 5.13(2H,s), 6.82 (1H, d, J=8.8Hz), 7.39-7.35 (6H, m), 7.70-7.68 (1H, m), 8.20-8.16(3H,m).

### 4) 5-(5-Hydroxy-2-pyridyl)-1-(6-methoxy-3-pyridyl)-1H-1,2,4-triazole-3-carboxylic acid methyl ester

The above 5-(5-benzyloxy-2-pyridyl)-1-(6-methoxy-3-pyridyl)-1H-1,2,4-triazole-3-carboxylic acid methyl ester (5.3 g) was dissolved in a mixture of methanol (200 mL), acetic acid (50 mL), and ethyl acetate (300 mL). 10% Palladium-carbon (5 g) was added to the solution, and the mixture was stirred for 24 hours in a hydrogen atmosphere at room temperature. The reaction mixture was subjected to filtration, and the solvent of the filtrate was removed under reduced pressure. The obtained crystals were recrystallized from ethyl acetate-hexane, to thereby give 5-(5-hydroxy-2-pyridyl)-1-(6-methoxy-3-pyridyl)-1H-1,2,4-triazole-3-carboxylic acid methyl ester (3.4 g, 81%).
¹H-NMR (400MHz, CDCl₃) δ:3.98 (3H, dd, J=19.6, 10.8Hz), 4.03 (3H, t, J=8.8Hz), 6.84 (1H, d, J=8.8Hz), 7.19 (1H, dd, J=8.3, 2.5Hz), 7.74 (1H, dd, J=8.8, 2.5Hz), 7.94 (1H, d, J=8.8Hz), 8.03 (1H, d, J=2.5Hz), 8.19 (1H, d, J=2.5Hz).

### 5) 5-(5-Cyano-2-pyridyl)-1-(6-methoxy-3-pyridyl)-1H-1,2,4-triazole-3-carboxylic acid methyl ester

Pyridine (1.63 mL) and trifluoromethane sulfonic acid anhydrate (2.04 mL) were added to a solution of the above 5-(5-hydroxy-2-pyridyl)-1-(6-methoxy-3-pyridyl)-1H-1,2,4-triazole-3-carboxylic acid methyl ester (3.3 g) in methylene chloride (50 mL) at room temperature, and the mixture was stirred for 2.5 hours. The reaction mixture was partitioned between saturated aqueous sodium hydrogencarbonate and methylene chloride, and the organic layer was dried over sodium sulfate anhydrate. After a filtration step, the solvent was removed under reduced pressure, and the residue was dissolved in 1,2-dichloroethane (240 mL). tetrakis (Triphenylphosphine) palladium (15.85 g) and tri-n-butyltin cyanide (2.89 g) were added to the solution, and the mixture was stirred for 23 hours at 80°C and then cooled in air. An excessive amount of potassium fluoride and methanol were added to the reaction mixture, and the resultant mixture was stirred for 5 hours at room temperature. Saturated aqueous sodium hydrogencarbonate was added to the reaction mixture, and the reaction solution was filtrated through Celite. Chloroform was added to the filtrate for partitioning the mixture, and the organic layer was dried over sodium sulfate anhydrate. After a filtration step, the solvent was removed under reduced pressure, and the residue was purified through silica gel column chromatography (ethyl acetate -chloroform), to thereby give 5-(5-cyano-2-pyridyl)-1-(6-methoxy-3-pyridyl)-1H-1,2,4-triazole-3-carboxylic acid methyl ester as a solid product (1.73 g, 56%).
¹H-NMR (400MHz, CDCl₃) δ: 4.00(3H, s), 4.06(3H,s), 6.84(1H, d, J=6.4Hz), 7.67(1H, dd, J=8.8, 2.9Hz), 8.12(1H, dd, J=8.3, 2.0Hz), 8.20(1H, d, J=2.0Hz), 8.46(1H, dd, J=8.3, 1.0Hz), 8.65-8.67(1H, m).

### 6) Title compound

The above 5-(5-cyano-2-pyridyl)-1-(6-methoxy-3-pyridyl)-1H-1,2,4-triazole-3-carboxylic acid methyl ester (1.6 g) was dissolved in tetrahydrofuran (30 mL) and water (15 mL), and, at room temperature, lithium hydroxide monohydrate (290 mg) was added to the solution, followed by stirring for 5.5 hours. 1N HCl was added to the reaction mixture, and the crystals that precipitated were collected through filtration, to thereby give the title compound as a solid product (1.07 g, 95%).
¹H-NMR (400MHz, DMSO-d₆) δ: 3.91(3H,s), 6.96(1H, d, J=8.8Hz), 7.90(1H, dd, J=8.8, 2.5Hz), 8.29(1H, dd, J=8.3, 1.0Hz), 8.34(1H, d, J=2.5Hz), 8.51(1H, dd, J=8.3, 2.0Hz), 8.86-8.88(1H, m).

### [Referential Example 44] (3S)-Fluoropyrrolidine hydrochloride

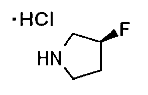

### 1) (3S)-Fluoropyrrolidine-1-carboxylic acid tert-butyl ester

Diethylaminosulfur trifluoride (2.22 mL) was added to (3R)-hydroxypyrrolidine-1-carboxylic acid tert-butyl ester (2.62 g) in methylene chloride (50 mL) at -78°C, and the mixture was stirred for 70 minutes at room temperature. The reaction mixture was poured to ice-water for partitioning the mixture, and the organic layer was dried over magnesium sulfate anhydrate. After a filtration step, the solvent was removed under reduced pressure, and the residue was purified through silica gel column chromatography (hexane - ethyl acetate), to thereby give (3S)-fluoropyrrolidine-1-carboxylic acid tert-butyl ester (676 mg, 26%) as an oil.
¹H-NMR(400MHz, CDCl₃) δ:1.45 (9H,s), 2.17-2.26 (1H,m), 3.52-3.68(5H, m), 5.20(1H, dt, J=52.7, 3.4Hz).

### 2) Title compound

4N HCl-Dioxane (5 mL) was added to a solution of the above (3S)-fluoropyrrolidine-1-carboxylic acid tert-butyl ester (600 mg) in methylene chloride (10 mL) at room temperature, and the mixture was stirred for 1 hour. Diethyl ether was added to the reaction mixture, and the solid that precipitated was collected through filtration, to thereby give the title compound (341 mg, 86%).
¹H-NMR(40OMHz, DMSO-d₆) δ:2.00-2.26 (2H, m) 3.15-3.56 (4H, m), 5.43 (1H, dt, J=52.9, 3.8Hz), 9.83(2H,br s).

### [Referential Example 45] 4-Fluoromethylpiperidine hydrochloride

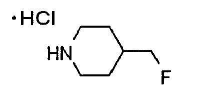

### 1) 4-Fluoromethylpiperidine-1-carboxylic acid tert-butyl ester

A solution of [bis(2-methoxyethyl)amino]sulfur trifluoride (1.2 mL) and 50% [bis(2-methoxyethyl)amino]sulfur trifluoride in tetrahydrofuran (3 mL) was added dropwise to 4-hydroxymethylpiperidine-1-carboxylic acid tert-butyl ester (1.17 g) in methylene chloride (8 mL) under cooling on ice, and the mixture was stirred for 17 hours at room temperature. Water, saturated aqueous sodium hydrogencarbonate, and ethyl acetate were added to the reaction mixture for partitioning the mixture, and the organic layer was dried over magnesium sulfate anhydrate. After a filtration step, the solvent was removed under reduced pressure, and the residue was purified through silica gel column chromatography (hexane - ethyl acetate), to thereby give 4-fluoromethylpiperidine-1-carboxylic acid tert-butyl ester as a solid product (597 mg, 51%).
¹H-NMR(400MHz,CDCl₃)δ:1.22(2H,m), 1.46(9H,s), 1.70(2H, d, J=12.94Hz), 1.83(1H,m), 2.71(2H,br), 4.13(2H, br), 4.21(1H,d,J=6.10Hz), 4.32(1H, d, J=6.10Hz).

### 2) Title compound

4-Fluoromethylpiperidine-1-carboxylic acid tert-butyl ester (635 mg) was treated in a manner similar to that employed in step 2) of Referential Example 39, to thereby give the title compound as a solid product (526 mg, quantitative amount).
¹H-NMR(400MHz, CDCl₃) δ:1.76(3H,m), 1.96(1H,d,J=13.4Hz), 2.90(2H,br), 3.54(2H,d,J=12.1Hz), 4.26(1H,d,J=6.10Hz), 4.37(1H,d,J=6.10Hz).

### [Referential Example 46] 5-(5-Fluoro-2-pyridyl)-1-(6-methoxy-3-pyridyl)-1H-1,2,4-triazole-3-carboxylic acid sodium salt

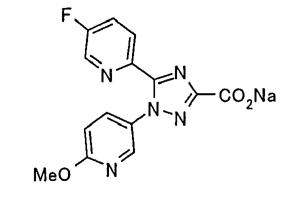

### 1) 5-Fluoropyridine-2-carbonitrile

5-Amino-2-cyanopyridine (24.5 g) was added to hydrogen fluoride-pyridine (100 mL) under cooling on ice, and the mixture was stirred for 10 minutes. Sodium nitrite (15.6 g) was added to the reaction mixture, and the resultant mixture was stirred at room temperature for 10 minutes and at 50°C for 2 hours and then cooled in air. The reaction mixture was partitioned between 20% aqueous sodium hydroxide and diethyl ether, and the organic layer was dried over sodium sulfate anhydrate. After a filtration step, the solvent was removed under reduced pressure, and the residue was purified through silica gel column chromatography (hexane - ethyl acetate), to thereby give 5-fluoropyridine-2-carbonitrile as a solid product (16.0 g, 64%).
¹H-NMR (400MHz, CDCl₃) δ:7.57 (1H, ddd, J=8.6, 8.6, 3.1Hz), 7.77 (1H, dd, J=8.6, 4.4Hz), 8.60 (1H, d, J=3.1Hz).
MS (EI)m/z:122 (M⁺) .

### 2) 2-[(5-Fluoropyridine-2-carbonyl)amino]malonic acid diethyl ester

The above 5-fluoropyridine-2-carbonitrile (11.8 g) in 6N HCl (100 mL) was refluxed for 4 hours and then cooled in air. NaCl was added to the reaction mixture to saturate the mixture, and ethyl acetate was added thereto for partitioning the mixture. The organic layer was dried over sodium sulfate anhydrate. After a filtration step, the solvent was removed under reduced pressure, and the residue was dissolved in N,N-dimethylformamide (140 mL). 1-Ethyl-3-(3-dimethylaminopropyl)carbodiimide hydrochloride (6.0 g), 1-hydroxybenzotriazole (770 mg), and aminomalonic acid diethyl ester hydrochloride (7.2 g) were added to the solution, and the mixture was stirred for 19.5 hours at room temperature. The reaction mixture was partitioned between water and ethyl acetate, and the organic layer was washed with saturated brine and then dried over sodium sulfate anhydrate. After a filtration step, the solvent was removed under reduced pressure, and the residue was purified through silica gel column chromatography (hexane - ethyl acetate), to thereby give 2-[(5-fluoropyridine-2-carbonyl)amino]malonic acid diethyl ester as a solid product (9.4 g, 53%).
¹H-NMR (400MHz, CDCl₃) δ:1.33 (6H, t, J=7.1Hz), 4.27-4.38(4H,m), 5.36 (1H, d, J=7.4Hz), 7.51-7.56 (1H, m), 8.20-8.21 (1H, m), 8.46 (1H, d, J=2.7Hz), 8.74 (1H, d, J=10.0Hz).

### 3) Title compound

5-Amino-2-methoxypyridine (3.23 g) and the above 2-[(5-fluoropyridine-2-carbonyl)amino]malonic acid diethyl ester (9.3 g) were treated in a manner similar to that employed in step 3) of Referential Example 43, to thereby give 5-(5-fluoro-2-pyridyl)-1-(6-methoxy-3-pyridyl)-1H-1,2,4-triazole-3-carboxylic acid ethyl ester. The ethyl ester compound was dissolved in methanol (150 mL), and sodium methoxide (170 mg) was added to the solution, followed by stirring for 60 hours at room temperature. The reaction solvent was removed under reduced pressure, and the obtained solid was washed with diethyl ether, to thereby give the title compound (9.5 g, 93%).
¹H-NMR(400MHz,DMSO-d₆) δ : 3.83(3H,s), 7.07 (1H,d,J=8.8Hz), 7.63-7.67(1H,m), 7.75-7.79 (3H,m), 8.03-8.05(2H,m).

### [Referential Example 47] 1-(6-Methoxy-3-pyridyl)-5-(5-methyl-2-pyridyl)-1H-1,2,4-triazole-3-carboxylic acid

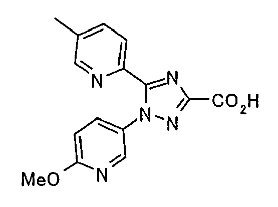

### 1) 2-[(5-Methylpyridine-2-carbonyl)amino]malonic acid diethyl ester

1-Ethyl-3-(3-dimethylaminopropyl)carbodiimide hydrochloride (10.15 g), 1-hydroxybenzotriazole (650 mg), aminomalonic acid diethyl ester hydrochloride (12.22 g), and triethylamine (20.1 mL) were added to 5-methylpyridine-2-carboxylic acid (6.6 g) in N,N-dimethylformamide (240 mL), and the mixture was stirred for 30.5 hours at room temperature. The reaction mixture was partitioned between water and chloroform, and the organic layer was dried over sodium sulfate anhydrate. After a filtration step, the solvent was removed under reduced pressure, and the residue was purified through silica gel column chromatography (hexane - ethyl acetate), to thereby give 2-[(5-methylpyridine-2-carbonyl)amino]malonic acid diethyl ester as an oil (7.0 g, 49%).
¹H-NMR (400MHz, CDCl₃) δ: 1.32 (6H, t, J=7.1Hz), 2.41 (3H, s), 4.29-4.34(4H,m), 5.38(1H, d, J=7.4Hz) , 7.64(1H, d, J=7.8Hz), 8.02-8.04(8H,m), 8.43(1H,s), 8.85(1H,d,J=7.4Hz).

### 2) Title compound

5-Amino-2-methoxypyridine (3.42 g) and the above 2-[(5-methylpyridine-2-carbonyl)amino]malonic acid diethyl ester (10.0 g) were treated in a manner similar to that employed in step 3) of Referential Example 43, to thereby give 1-(6-methoxy-3-pyridyl)-5-(5-methyl-2-pyridyl)-1H-1,2,4-triazole-3-carboxylic acid ethyl ester. The ethyl ester compound was dissolved in methanol (100 mL), and sodium methoxide (130 mg) was added to the solution, followed by stirring for 13 hours at room temperature. The reaction solvent was removed under reduced pressure, and the obtained solid was dissolved in tetrahydrofuran (120 mL) and water (120 mL). Lithium hydroxide monohydrate (1.03 g) was added to the solution, and the mixture was stirred for 1.5 hours at room temperature. The reaction mixture was partitioned between 1N HCl and chloroform-methanol (10:1) solvent mixture, and the organic layer was dried over sodium sulfate anhydrate. After a filtration step, the solvent was removed under reduced pressure, and the obtained solid was washed with diethyl ether, to thereby give the title compound (1.58 g, 21%).
¹H-NMR (400MHz, DMSO-d₆) δ:2.31 (3H,s), 3.91(3H,s), 6.93 (1H, d, J=8.8Hz), 7.80-7.86(2H,m), 7.99 (1H, d, J=7.8Hz), 8.26-8.29(2H,m).

### [Referential Example 48] (3R)-Fluoropiperidine hydrochloride

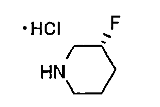

(2S)-Hydroxymethylpyrrolidine-1-carboxylic acid tert-butyl ester (3.0 g) and diethylaminosulfur trifluoride (2.95 mL) were treated in a manner similar to that employed in step 1) of Referential Example 44, to thereby give (3R)-fluoropiperidine-1-carboxylic acid tert-butyl ester as an oil (346 mg). The ester compound was dissolved in methylene chloride (20 mL), and, at room temperature, 4N HCl-dioxane (7 mL) was added to the solution, followed by stirring for 30 minutes. Diethyl ether was added to the reaction mixture, and the precipitate was collected through filtration, to thereby give the title compound as a solid product (162 mg, 8%).
1H-NMR (4 OOMHz, DMSO-d6) δ: 1 . 65-1 . 93 ( 4H, m), 3.03-3.20(4H, m), 4.97(1H, dd, J=45.7, 2.4Hz), 9.34(2H, br s).

### [Referential Example 49] 5-(5-Chloro-2-pyridyl)-1-(6-methoxy-3-pyridyl)-1H-1,2,4-triazole-3-carboxylic acid

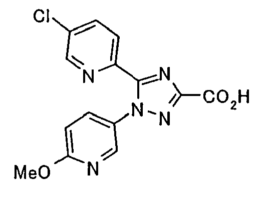

### 1) Pyridine-2,5-dicarboxylic acid dibenzyl ester

Thionyl chloride (250 mL) and N,N-dimethylformamide (10 mL) were added to 2,5-pyridinedicarboxylic acid (60 g) in methylene chloride (360 mL), and the mixture was refluxed for 5 hours and then cooled in air. The solvent of the reaction mixture was removed under reduced pressure, and toluene was added to the residue. The solvent was again removed through azeotropy under reduced pressure, and the residue was dissolved in methylene chloride (500 mL). At 0°C, benzyl alcohol (81.7 mL) in methylene chloride (200 mL) was added dropwise to the solution, and the mixture was stirred for 4 hours at room temperature. Water was added thereto for partitioning the reaction mixture, and the organic layer was washed with saturated aqueous sodium hydrogencarbonate and then dried over sodium sulfate anhydrate. After a filtration step, the solvent was removed under reduced pressure, to thereby give pyridine-2,5-dicarboxylic acid dibenzyl ester as a solid product (65 g, 52%).
¹H-NMR (400MHz, CDCl₃) δ: 5.42 (2H, s), 5.47(2H,s), 7.38-7.46(10H,m), 8.19(1H,d,J=8.3Hz), 8.44(1H,dd,J=8.2,2.1Hz), 9.35-9.36(1H,m).

### 2) Pyridine-2,5-dicarboxylic acid 5-benzylester

Copper(II) sulfate pentahydrate (46.7 g) was added to a suspension of pyridine-2,5-dicarboxylic acid dibenzyl ester (65 g) in methanol (500 mL), and the mixture was refluxed for 1 hour and then cooled in air. The precipitate was collected through filtration. The solid was suspended in dioxane, and hydrogen sulfide gas was bubbled into the suspension at room temperature. The reaction mixture was subjected to filtration, and the solvent of the filtrate was removed under reduced pressure, to thereby give pyridine-2,5-dicarboxylic acid 5-benzylester as a solid product (48.1 g, 74%).
¹H-NMR(400MHz,DMSO-d₆)δ:5.40(2H,s), 7.29-7.42(3H,m), 7.49-7.50(2H,m), 8.20(1H,br s), 8.47(1H,d,J=7.8Hz), 9.19(1H,br s), 10.64(1H,brs) .

### 3) 2-[(5-Benzyloxycarbonylpyridine-2-carbonyl)amino]malonic acid diethyl ester

Thionyl chloride (50 mL) and N,N-dimethylformamide (12 mL) were added to pyridine-2,5-dicarboxylic acid 5-benzylester (48.1 g) in methylene chloride (360 mL), and the mixture was refluxed for 3 hours and then cooled in air. The reaction solvent was removed under reduced pressure, and toluene was added to the residue. The solvent was removed through azeotropy under reduced pressure. The residue was dissolved in methylene chloride (500 mL), and, at 0°C, aminomalonic acid diethyl ester hydrochloride (47.49 g) was added to the solution, followed by stirring for 39 hours at room temperature. Water was added to the reaction mixture for partitioning the mixture, and the organic layer was washed with saturated aqueous sodium hydrogencarbonate and then dried over sodium sulfate anhydrate. After a filtration step, the solvent was removed under reduced pressure, and the residue was purified through silica gel column chromatography (hexane - ethyl acetate), to thereby give 2-[(5-benzyloxycarbonylpyridine-2-carbonyl)amino]malonic acid diethyl ester as an oil (22.5 g, 29%).
¹H-NMR (400MHz, CDCl₃) δ:1.32 (6H, t, J=7.1Hz), 4.30-4.33(4H,m), 5.37 (1H, d, J=7.4Hz), 5.42 (2H, s), 7.37-7.47 (5H, m), 8.23(1H, d, J=8.1Hz), 8.47 (1H, dd, J=8.1, 1.7Hz), 8.92 (1H, d, J=10.0Hz), 9.24 (1H, d, J=2.0Hz).

### 4) 2-[(5-carboxypyridine-2-carbonyl)amino]malonic acid diethyl ester

10% Palladium-carbon (2.2 g) was added to 2-[(5-benzyloxycarbonylpyridine-2-carbonyl)amino]malonic acid diethyl ester (22.0 g) in dioxane (250 mL), and the mixture was stirred for 76 hours in a hydrogen atmosphere at room temperature. The catalyst was removed through filtration, and the solvent of the filtrate was removed under reduced pressure, to thereby give 2-[(5-carboxypyridine-2-carbonyl)amino]malonic acid diethyl ester (17.0 g, quantitative amount).
¹H-NMR (400MHz, CDCl₃) δ: 1.33 (6H, t, J=7.1Hz), 4.31-4.35 (4H,m), 5.40(1H,d,J=7.6Hz), 8.27(1H, d, J = 8.1Hz), 8.49 (1H, dd, J=8.1, 2.0Hz), 8.96(1H, d, J=7.6Hz), 9.23-9.24(1H, m).

### 5) 2-[(5-Benzyloxycarbonylaminopyridine-2-carbonyl)amino]malonic acid diethyl ester

Triethylamine (5.43 mL), diphenylphosphorylazide (8.4 mL), and benzyl alcohol (7.68 mL) were added to 2-[(5-carboxypyridine-2-carbonyl)amino]malonic acid diethyl ester (12 g) in dioxane (70 mL), and the mixture was refluxed for 14.5 hours and then cooled in air. The reaction solvent was removed under reduced pressure, and the residue was partitioned between water and chloroform. The organic layer was dried over sodium sulfate anhydrate. After a filtration step, the solvent was removed under reduced pressure, and the residue was purified through silica gel column chromatography (hexane - ethyl acetate), to thereby give 2-[(5-benzyloxycarbonylaminopyridine-2-carbonyl)amino]malonic acid diethyl ester as a solid product (14.4 g, 91%).
¹H-NMR (400MHz, CDCl₃) δ: 1.32 (6H, t, J=7.1Hz), 4 . 26-4 . 34 (4H, m), 5.23(2H, s), 5.37(1H, d, J=7.6Hz), 7.27-7.40(5H,m), 8.10(2H, s), 8.53(1H,s), 8.73(1H,d,J=7.6Hz) .

### 6) 2-[(5-Aminopyridine-2-carbonyl)amino]malonic acid diethyl ester

10% Palladium-carbon (1.4 g) was added to 2-[(5-benzyloxycarbonylaminopyridine-2-carbonyl)amino]malonic acid diethyl ester (14.4 g) in dioxane (200 mL), and the mixture was stirred for 14 hours in a hydrogen atmosphere at room temperature. After a filtration step, the solvent of the filtrate was removed under reduced pressure, and the residue was purified through silica gel column chromatography (hexane - ethyl acetate), to thereby give 2-[(5-aminopyridine-2-carbonyl)amino]malonic acid diethyl ester as an oil (11.5 g, 80%).
¹H-NMR(400MHz,CDCl₃)δ:1.31(6H,t, J=7.1Hz), 4.04(2H,s), 4.26-4.34(4H,m), 5.37(1H,d,J=7.6Hz), 7.00(1H,dd,J=8.3,2.7Hz), 7.95(1H,d,J=8.6Hz), 8.01(1H, d, J=2.7Hz),8.64(1H, d, J=10.0Hz) .

### 7) Title compound

tert-Butyl nitrite (930 µL) and copper(II) chloride (750 mg) were added to 2-[(5-aminopyridine-2-carbonyl)amino]malonic acid diethyl ester (2.0 g) in acetonitrile (65 mL), and the mixture was stirred for 20 minutes at 65°C and cooled in air. The reaction mixture was partitioned between 1N HCl and chloroform, and the organic layer was dried over sodium sulfate anhydrate. After a filtration step, the solvent was removed under reduced pressure, and the residue was purified through silica gel column chromatography (hexane - ethyl acetate), to thereby give 2-[(5-chloropyridine-2-carbonyl)amino]malonic acid diethyl ester. Sodium nitrite (780 mg) in water (2.5 mL) was added dropwise to 5-amino-2-methoxypyridine (690 mg) in acetic acid (4.4 mL) and concentrated hydrochloric acid (1.1 mL) at 0°C, and the mixture was stirred for 15 minutes. At - 15°C, 2-[(5-chloropyridine-2-carbonyl)amino]malonic acid diethyl ester (2.1 g) in acetone (10 mL) and potassium carbonate (3.69 g) in water (10 mL) were slowly added to the reaction mixture, and the resultant mixture was stirred for 1.5 hours at 0°C. Ethyl acetate was added to the reaction mixture for partitioning the mixture, and the organic layer was sequentially washed with water, saturated aqueous sodium hydrogencarbonate, water, and saturated brine, and then dried over sodium sulfate anhydrate. After a filtration step, the solvent was removed under reduced pressure, and the residue was dissolved in methanol (50 mL). At room temperature, sodium methoxide (40 mg) was added to the solution, and the mixture was stirred for 13 hours. The reaction solvent was removed under reduced pressure, and the residue was acidified with 1N HCl. Ethyl acetate and NaCl were added to the mixture for partitioning the mixture. The organic layer was dried over sodium sulfate anhydrate. After a filtration step, the solvent was removed under reduced pressure, and the obtained solid was washed with diethyl ether, to thereby give the title compound (429 mg, 15%).
¹H-NMR (400MHz, DMSO-d₆) δ :3.92 (3H, s), 6.96 (1H, d, J=8.8Hz), 7.89 (1H,dd,J=8.8,2.7Hz), 8.15 (3H, t, J=1.7Hz), 8.33(1H,d,J=2.2Hz), 8.52(1H,dd,J-1.1,0.5Hz).

### [Example 1] 1-[2-Phenyl-1-(3-pyridyl)-1H-imidazole-4-carbonyl]-4-methylpiperazine

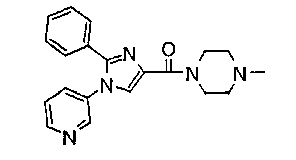

1-Hydroxybenzotriazole (367 mg), 1-ethyl-3-(3-dimethylaminopropyl)carbodiimide hydrochloride (520 mg), and 1-methylpiperazine (272 mg) were added at room temperature to 2-phenyl-1-(3-pyridyl)-1H-imidazole-4-carboxylic acid (600 mg) obtained in Referential Example 1 in N,N-dimethylformamide (20 mL), followed by stirring for 19 hours. The reaction mixture was partitioned between saturated aqueous sodium hydrogencarbonate and ethyl acetate. The organic layer was washed with saturated brine, and then dried over magnesium sulfate anhydrate. After a filtration step, the solvent was removed under reduced pressure, and the residue was purified by silica gel column chromatography (chloroform - methanol), to thereby give the title compound as an amorphous product (443 mg, 56.4%).
¹H-NMR (300MHz, CDCl₃) δ:2.35 (3H, s), 2.52 (4H, t, J=5.14Hz), 3.84(2H,br s), 4.43(2H,br s), 7.30-7.40(6H,m), 7.55-7.58 (1H, m), 7.78 (1H, s), 8.56 (1H, d, J=2.57Hz), 8.67 (1H, dd, J=4.86, 1.38Hz).
MS(FAB)m/z:348(M+H)⁺.

### [Example 2] 1-[2-Phenyl-1-(4-pyridyl)-1H-imidazole-4-carbonyl]-4-methylpiperazine

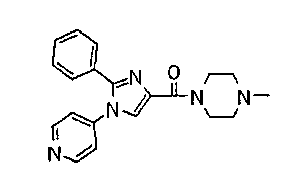

In a manner similar to that employed in Example 1, the title compound in amorphous form (364 mg, 81.9%) was prepared from 2-phenyl-1-(4-pyridyl)-1H-imidazole-4-carboxylic acid (340 mg) obtained in Referential Example 2 and N-methylpiperazine (154 mg).
¹H-NMR (300MHz, CDCl₃) δ: 2.34 (3H, s), 2.51 (4H, t, J=5. 05Hz), 3.83(2H,br s), 4.40(2H,br s), 7.15 (2H, dd, J=4.59, 1.65Hz), 7.31-7.41 (5H, m), 7.82 (1H,s), 8.67 (2H, dd, J=4.59, 1.65Hz).
MS (FAB)m/z:348 (M+H)⁺.

### [Example 3] 1-(5-Methyl-1,2-diphenyl-1H-imidazole-4-carbonyl)-4-methylpiperazine

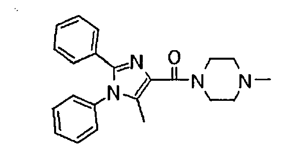

In a manner similar to that employed in Example 1, the title compound in solid form (140 mg, 27.0%) was prepared from 5-methyl-1,2-diphenyl-1H-imidazole-4-carboxylic acid (400 mg) obtained in Referential Example 3 and N-methylpiperazine (173 mg).
¹H-NMR(300MHz, CDCl₃)δ:2.31(3H,s), 2.35(3H,s), 2.53(4H,t,J=5.05Hz), 4.22(4H,br s), 7.16-7.23(5H,m), 7.30-7.33(2H,m), 7.46-7.48(3H,m).
MS (FAB)m/z:361 (M+H)⁺.

### [Example 4] 1-[2-(6-Methoxy-3-pyridyl)-1-phenyl-1H-imidazole-4-carbonyl]-4-methylpiperazine

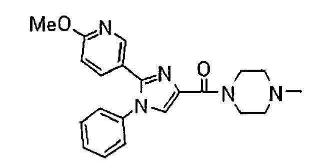

In a manner similar to that employed in Example 1, the title compound in solid form (136 mg, 33.4%) was prepared from 2-(6-methoxy-3-pyridyl)-1-phenyl-1H-imidazole-4-carboxylic acid (320 mg) obtained in Referential Example 4 and N-methylpiperazine (130 mg).
¹H-NMR(300MHz,CDCl₃)δ:2.37(3H,s), 2.55(4H,t,J=4.86Hz), 4.45(4H,br s), 3.91(3H, s), 6.65(1H, d, J=8.81Hz), 7.24-7.26(2H,m), 7.44-7.46(3H,m), 7.56(1H,dd, J=8.81,2.48Hz), 7.75(1H, s), δ.17(1H, d, J=2.02Hz).
MS(FAB)m/z:378(M+H)⁺.

### [Example 5] 1-[2-(6-Methoxy-3-pyridyl)-1-(4-methylphenyl)-1H-imidazole-4-carbonyl]-4-methylpiperazine

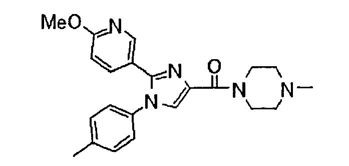

In a manner similar to that employed in Example 1, the title compound in solid form (625 mg, 61.6%) was prepared from 2-(6-methoxy-3-pyridyl)-1-(4-methylphenyl)-1H-imidazole-4-carboxylic acid (800 mg) obtained in Referential Example 6 and N-methylpiperazine (331 mg).
¹H-NMR (300MHz, CDCl₃) δ:2.34 (3H,s), 2.41 (3H,s), 2.52(4H,t,J=5.05Hz), 4.42(4H,br s), 3.91(3H,s), 6.66 (1H, d, J=8.63Hz), 7.13(2H,d,J=8.26Hz), 7.24(2H,d,J=8.26Hz), 7.60(1H, dd, J=8.63, 2.39Hz), 7.72 (1H, s) , 8.17(1H, d, J=2.39Hz).
MS (FAB) m/z:392 (M+H)⁺.

### [Example 6] 1-[1-(4-Fluorophenyl)-2-(6-methoxy-3-pyridyl)-1H-imidazole-4-carbonyl]-4-methylpiperazine

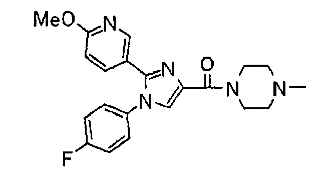

In a manner similar to that employed in Example 1, the title compound in solid form (557 mg, 64.9%) was prepared from 1-(4-fluorophenyl)-2-(6-methoxy-3-pyridyl)-1H-imidazole-4-carboxylic acid (680 mg) obtained in Referential Example 7 and N-methylpiperazine (261 mg).
'H-NMR(30OMHz,CDC1₃)5:2.35(3H,s), 2.52(4H,t,J=5.05Hz), 4.21(4H,br s), 3.92(3H,s), 6.66-6.69(1H,m), 7.12-7.27(4H,m), 7.58(1H, dd, J=8.72, 2.48Hz), 7.72(1H, s), 8.13-8.14(1H,m).
MS(FAB)m/z:396(M+H)⁺.

### [Example 7] 1-[2-(4-Methylphenyl)-1-(4-pyridyl)-1H-imidazole-4-carbonyl]-4-methylpiperazine

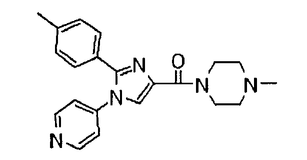

In a manner similar to that employed in Example 1, the title compound in solid form (120 mg, 27.7%) was prepared from 2-(4-methylphenyl)-1-(4-pyridyl)-1H-imidazole-4-carboxylic acid (335 mg) obtained in Referential Example 8 and N-methylpiperazine (144 mg).
¹H-NMR (300MHz, CDCl₃) δ:2.34 (3H, s), 2.36(3H,s), 2.51 (4H, t, J=5.05Hz), 3.83(2H,br s), 4.39(2H,br s), 7.12-7.16(4H,m), 7.25(2H,d,J=8.81Hz), 7.80(1H,s), 8.66 (2H, dd, J=4.50,1.56Hz).
MS (FAB)m/z:362 (M+H)⁺.

### [Example 8] 4-[2-(6-Methoxy-3-pyridyl)-1-phenyl-1H-imidazole-4-carbonyl]morpholine

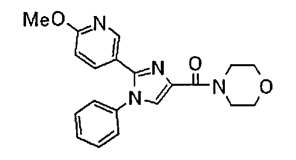

In a manner similar to that employed in Example 1, the title compound in solid form (564 mg, 91.5%) was prepared from 2-(6-methoxy-3-pyridyl)-1-phenyl-1H-imidazole-4-carboxylic acid (500 mg) obtained in Referential Example 4 and morpholine (177 mg).
¹H-NMR(300MHz,CDCl₃)δ:3.80(4H,m), 4.46(4H,br s), 3.91(3H,s), 6.65(1H,d,J=8.63Hz), 7.24-7.26(2H,m), 7.45-7.57(3H,m), 7.78 (1H, s), 8.17(1H, d, J=2 . 02Hz) .
MS(FAB)m/z:365(M+H)⁺.

### [Example 9] 1-[2-(6-Methoxy-3-pyridyl)-1-phenyl-1H-imidazole-4-carbonyl]-2,2,4-trimethylpiperazine

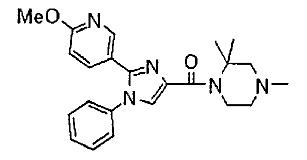

In a manner similar to that employed in Example 1, the title compound in solid form (244 mg, 32.4%) was prepared from 1,3,3-trimethylpiperazine hydrochloride (487 mg) obtained in Referential Example 9 and 2-(6-methoxy-3-pyridyl)-1-phenyl-1H-imidazole-4-carboxylic acid (550 mg) obtained in Referential Example 4.
¹H-NMR(300MHz,CDCl₃)δ:1.58(6H,s), 2.31(5H,s), 2.59(2H,t,J=5.14Hz), 3. 91 (3H, s), 4.13(2H,t,J=5.14Hz), 6.63(1H,d,J=8.63Hz), 7.22-7.25 (2H,m), 7.43-7.45(3H,m), 7.55(1H,dd,J=8.63,2.39Hz), 7.64(1H,s), 8.16(1H,d,J=2.39Hz).
MS(FAB)m/z:406(M+H)⁺.

### [Example 10] 1-[1-(4-Methylphenyl)-2-(6-methyl-3-pyridyl)-1H-imidazole-4-carbonyl]-3-dimethylaminoazetidine

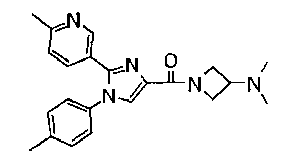

In a manner similar to that employed in Example 1, the title compound in solid form (260 mg, 73.3%) was prepared from 1-(4-methylphenyl)-2-(6-methyl-3-pyridyl)-1H-imidazole-4-carboxylic acid (277 mg) obtained in Referential Example 11 and azetidin-3-yldimethylamine hydrochloride (180 mg) obtained in Referential Example 34.
¹H-NMR (300MHz, CDCl₃) δ:2.23 (6H, s), 2.41 (1H, s), 2.53(3H,s), 3.18 (1H, m), 4.02-4.07 (1H, m), 4.19-4.25 (1H, m), 4.51-4.56 (1H, m), 4.78-4.84 (1H, m), 7.05-7.12(3H,m), 7.22-7.26(2H,m), 7.58 (1H, dd, J=7.98, 2.29Hz), 8.50 (1H, d, J=2.02Hz).
MS (FAB) m/z:376 (M+H)⁺.

### [Example 11] 4-[1-(4-Methylphenyl)-2-(6-methyl-3-pyridyl)-1H-imidazole-4-carbonyl]piperazine-1-carboxylic acid benzyl ester

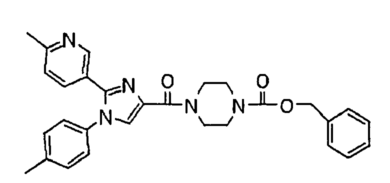

In a manner similar to that employed in Example 1, the title compound in solid form (290 mg, 57.2%) was prepared from 1-(4-methylphenyl)-2-(6-methyl-3-pyridyl)-1H-imidazole-4-carboxylic acid (300 mg) obtained in Referential Example 11 and piperazine-1-carboxylic acid benzyl ester (222 µL).
¹H-NMR (300MHz, CDCl₃)δ:2.41(3H,s), 2.53(3H,s), 3.63(4H,m), 3.78 (2H,br s), 4.40(2H,br s), 5.17(2H,s), 7.07-7.13(3H,m), 7.25-7.26(2H,m), 7.30-7.39(5H,m), 7.62(1H,d,J=8.26Hz), 7.76(1H,s), 8.42(1H,d,J=1.84Hz).
MS(FAB)m/z:496(M+H)⁺.

### [Example 12] 1-[2-(6-Methyl-3-pyridyl)-1-phenyl-1H-imidazole-4-carbonyl]-4-methylpiperazine

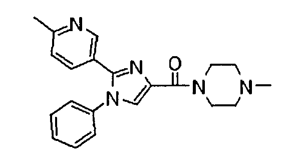

In a manner similar to that employed in Example 1, the title compound in solid form (212 mg, 55.4%) was prepared from 2-(6-methyl-3-pyridyl)-1-phenyl-1H-imidazole-4-carboxylic acid (280 mg) obtained in Referential Example 10 and N-methylpiperazine (127 mg).
¹H-NMR(300MHz, CDCl₃) δ:2.35 (3H, s), 2.53(3H,s), 7.07 (1H, d, J=8.08Hz), 7.23-7.26(2H,m), 7.43-7.46(3H,m), 7.58 (1H, dd, J=2.20, 8.08Hz), 7.77 (1H, s), 8.47 (1H, d, J=2.20Hz).
MS (FAB)m/z:362 (M+H)⁺.

### [Example 13] 1-[1-(4-Methylphenyl)-2-(6-methyl-3-pyridyl)-1H-imidazole-4-carbonyl]-4-methylpiperazine

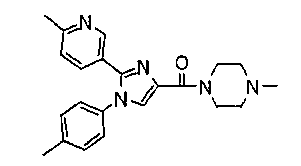

In a manner similar to that employed in Example 1, the title compound in solid form (650 mg, 45.9%) was prepared from 1-(4-methylphenyl)-2-(6-methyl-3-pyridyl)-1H-imidazole-4-carboxylic acid (1.0 g) obtained in Referential Example 11 and N-methylpiperazine (453 mg).
¹H-NMR (300MHz, CDCl₃) δ: 2.36 (3H, s), 2.41 (3H, s), 2.53(3H,s), 7.06-7.14 (3H,m), 7.23 (2H, d, J=8.07Hz), 7.62 (2H,dd, J=8.07, 2.20Hz), 7.74 (1H, s), 8.45 (1H, d, J=2.20Hz) .
MS(FAB)m/z:376(M+H)⁺.

### [Example 14] 4-[2-(6-Methoxy-3-pyridyl)-1-phenyl-1H-imidazole-4-carbonyl]piperazine-1-carboxylic acid benzyl ester

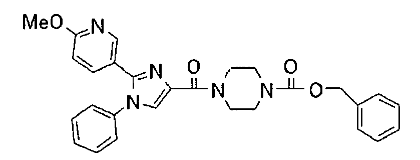

In a manner similar to that employed in Example 1, the title compound in solid form (821 mg, 97.6%) was prepared from 2-(6-methoxy-3-pyridyl)-1-phenyl-1H-imidazole-4-carboxylic acid (500 mg) obtained in Referential Example 4 and piperazine-1-carboxylic acid benzyl ester (447 mg).
¹H-NMR (300MHz, CDCl₃) δ:3.62-3.65 (4H,m), 3.79 (2H, br s), 3.91(3H,s), 4.43 (2H, br s), 5.17 (2H, s), 6.65(1H,d,J=8.81Hz), 7.23-7.26(2H,m), 7.32-7.39 (5H,m), 7.44-7.46(3H,m), 7.78(1H,s), 8.15(1H, d, J=2.57Hz).
MS (FAB) m/z:498 (M+H)⁺.

### [Example 15] 4-[2-(6-Methoxy-3-pyridyl)-1-phenyl-1H-imidazole-4-carbonyl]-3-methylpiperazine-1-carboxylic acid tert-butyl ester

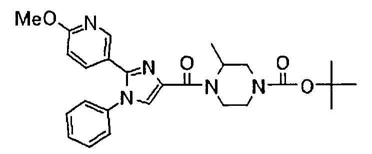

In a manner similar to that employed in Example 1, the title compound in solid form (940 mg, 83.1%) was prepared from 2-(6-methoxy-3-pyridyl)-1-phenyl-1H-imidazole-4-carboxylic acid (700 mg) obtained in Referential Example 4 and 3-methylpiperazine-1-carboxylic acid tert-butyl ester (568 mg) obtained in step 1) of Referential Example 12.
¹H-NMR(300MHz,CDCl₃)δ:1.34(3H,d,J=6.06Hz), 1.49(9H,s), 1.70(3H,br s), 3.01(1H,br s), 3.15(1H,br s), 3.92(5H,s), 7.23-7.27(2H,m), 7.44-7.53(3H,m), 7.76(1H,s), 8.18(1H,br s).
MS(FAB)m/z:392(M+H)⁺.

### [Example 16] 1-[2-(6-Methoxy-3-pyridyl)-1-phenyl-1H-imidazole-4-carbonyl]-2-methylpiperazine trifluoroacetic acid salt

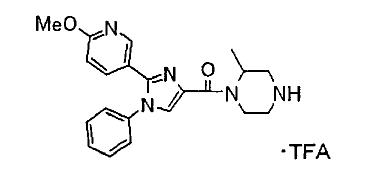

4-[2-(6-Methoxy-3-pyridyl)-1-phenyl-1H-imidazole-4-carbonyl]-3-methylpiperazine-1-carboxylic acid tert-butyl ester (700 mg) obtained in Example 15 in trifluoroacetic acid (10 mL) was stirred at room temperature for 3 hours. The reaction solvent was removed under reduced pressure, to thereby give the title compound as an amorphous product (890 mg, quantitative amount).
¹H-NMR(300MHz, CDCl₃) δ:1.55(3H,d,J=7.16Hz), 3.18-3.62(7H,m), 3.92(3H,s), 6.68(1H, d, J=8.81Hz), 7.24-7.28(2H,m), 7.46-7.51(3H,m), 7.56(1H, dd, J = 8.81, 2.20Hz), 7.81(1H, s), 8.17 (1H, d, J=2.20Hz).
MS (FAB)m/z:378 (M+H)⁺.

### [Example 17] 1-[2-(6-Methoxy-3-pyridyl)-1-phenyl-1H-imidazole-4-carbonyl]-2,4-dimethylpiperazine

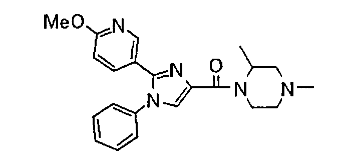

36% Formaldehyde (83 µL) and sodium triacetoxyborohydride (316 mg) were added to 1-[2-(6-methoxy-3-pyridyl)-1-phenyl-1H-imidazole-4-carbonyl]-2-methylpiperazinetrifluoroacetic acid (600 mg) obtained in Example 16 in methylene chloride (50 mL), followed by stirring at room temperature for 3 hours. The reaction mixture was partitioned between saturated aqueous sodium hydrogencarbonate and methylene chloride. The organic layer was washed with saturated brine, followed by drying over magnesium sulfate anhydrate. After a filtration step, the solvent was removed under reduced pressure, and the residue was purified by silica gel thin-layer chromatography (chloroform - methanol), to thereby give the title compound as an amorphous product (260 mg, 70%).
¹H-NMR (300MHz, CDCl₃) δ: 1.45(3H, d, J=6.06Hz), 2.10(2H, t, J=11.16Hz), 2.24-2.29(2H,m), 2.29(3H,s), 3.91(3H, s), 6.64(1H, d, J=8.81Hz), 7.24-7.28(2H,m), 7.42-7.46(3H, m), 7.74(1H, s), 8.19(1H, d, J=2.20Hz).
MS (FAB) m/z: 392 (M+H)⁺.

### [Example 18] 1-[2-(6-Methoxy-3-pyridyl)-1-phenyl-1H-imidazole-4-carbonyl]-3,4-dimethylpiperazine

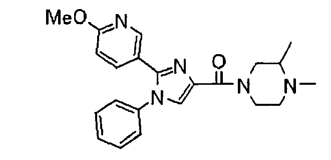

In a manner similar to that employed in Example 1, the title compound in solid form (230 mg, 43.6%) was prepared from 2-(6-methoxy-3-pyridyl)-1-phenyl-1H-imidazole-4-carboxylic acid (400 mg) obtained in Referential Example 4 and 1,2-dimethylpiperazine trifluoroacetic acid salt (602 mg) obtained in Referential Example 12.
¹H-NMR(300MHz,CDCl₃)δ:1.14(3H,d,J=6.24Hz), 1.76(1H,br s), 1.84 (1H,br s), 2.22 (1H,br s), 2.31(3H,s), 2.87(1H,d,J=11.74Hz), 3.91(3H,s), 4.51(1H,s), 5.20-5.33(1H,m), 6.64(1H,d,J=8.63Hz), 7.23-7.29(2H,m), 7.42-7.46(3H,m), 7.54(1H,br s), 7.75(1H,s), 8.18(1H,br s).
MS(FAB)m/z:392(M+H)⁺.

### [Example 19] 1-[1-(4-Methylphenyl)-2-(6-methyl-3-pyridyl)-1H-imidazole-4-carbonyl]piperidine

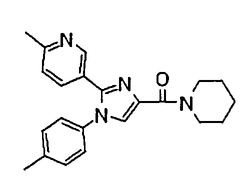

In a manner similar to that employed in Example 1, the title compound in solid form (171 mg, 62.9%) was prepared from 1-(4-methylphenyl)-2-(6-methyl-3-pyridyl)-1H-imidazole-4-carboxylic acid (200 mg) obtained in Referential Example 11 and piperidine (90 µL).
¹H-NMR (300MHz, CDCl₃) δ: 1.62-1.70(10H, m), 2.40(3H,s), 2.53(3H, s), 7.05-7.12(3H, m), 7.21-7.26(2H, m), 7.60-7.65(1H, m), 7.69(1H, s), 8.45(1H, d, J=1.84Hz).
MS (FAB) m/z: 361(M+H)⁺.

### [Example 20] 1-[2-(6-Methoxy-3-pyridyl)-1-phenyl-1H-imidazole-4-thiocarbonyl]-4-methylpiperazine

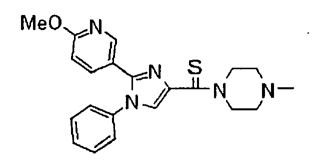

2,4-bis(4-Methoxyphenyl)-1,3,2,4-dithiadiphosphetan-2,4-disulfide (Lawson reagent, 300 mg) was added to 1-[2-(6-methoxy-3-pyridyl)-1-phenyl-1H-imidazole-4-carbonyl]-4-methylpiperazine (280 mg) obtained in Example 4 in benzene (50 mL). The resultant mixture was stirred at 85°C for 6 hours, followed by cooling in air. The reaction solvent was removed under reduced pressure, and the residue was purified by silica gel column chromatography (chloroform - methanol), to thereby give the title compound as an amorphous product (205 mg, 70.4%).
¹H-NMR (300MHz, CDCl₃) δ:2.39 (3H, s), 2.66(4H,br s), 3.91(3H,s), 4.48(4H,br s), 7.25-7.29(2H,m), 7.43-7.46(3H,m), 7.53(1H,dd,J=8.81,2.39Hz), 7.88(1H,s), 8.15(1H,d, J=1.84Hz).
MS (FAB)m/z:394 (M+H)⁺.

### [Example 21] 4-[1-(4-Methylphenyl)-2-(6-methyl-3-pyridyl)-1H-imidazole-4-carbonyl]morpholine

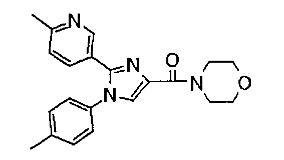

In a manner similar to that employed in Example 1, the title compound in solid form (112 mg, 45.3%) was prepared from 4-[1-(4-methylphenyl)-2-(6-methyl-3-pyridyl)-1H-imidazole-4-carboxylic acid (200 mg) obtained in Referential Example 11 and morpholine (79 µL).
¹H-NMR(300MHz,CDCl₃)δ:1.60(4H,br s), 2.41(3H,s), 2.53(3H,s), 3.80(4H,br s), 7.06-7.13(3H,m), 7.22-7.26(2H,m), 7.59-7.63(1H,m), 7.77(1H,s), 8.44(1H,d,J=2.02Hz).
MS (FAB)m/z:363 (M+H)⁺.

### [Example 22] 1-[2-(6-Methoxy-3-pyridyl)-1-phenyl-1H-imidazole-4-carbonyl]-4-methyl[1,4]diazepane

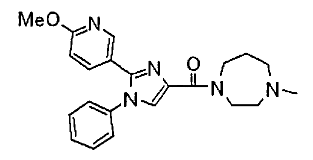

In a manner similar to that employed in Example 1, the title compound in solid form (238 mg, 59.6%) was prepared from 2-(6-methoxy-3-pyridyl)-1-phenyl-1H-imidazole-4-carboxylic acid (300 mg) obtained in Referential Example 4 and 1-methylhomopiperazine (152 µL).
¹H-NMR (300MHz, CDCl₃) δ:2.10 (2H, br s), 2.44(3H,s), 2.65-2.71 (1H, m), 2.77-2.81 (1H, m), 2.89-2.93 (1H, m), 3.77-3.86(2H,m), 3.92(3H,s), 4.27-4.37(2H,m), 6.64 (1H, d, J=8.76Hz), 7.24-7.27(2H,m), 7.42-7.58 (1H, m), 7.75 (1H, d, J=5.87Hz), 8.17-8.19 (2H, m).
MS (FAB)m/z:392 (M+H)⁺.

### [Example 23] 1-[2-(6-Methoxy-3-pyridyl)-1-phenyl-1H-imidazole-4-carbonyl]-3-methylhexahydropyrimidine

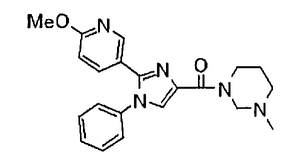

In a manner similar to that employed in Example 1, the title compound in solid form (331 mg, 86%) was prepared from 2-(6-methoxy-3-pyridyl)-1-phenyl-1H-imidazole-4-carboxylic acid (300 mg) obtained in Referential Example 4 and 1-methylhexahydropyrimidine (153 mg) obtained in Referential Example 13.
¹H-NMR(300MHz, CDCl₃)δ:1.74-1.84(4H,m), 2.37(3H,s), 2.75(2H,t,J=5.69Hz), 3.78(1H,br s), 3.91(3H,s), 7.23-7.26(2H,m), 7.42-7.54(3H,m), 7.57(1H,dd,J=8.81,2.39Hz), 7.77(1H,s), 8.17(1H,t,J=1.84Hz).
MS(FAB)m/z:378(M+H)⁺.

### [Example 24] 1-[2-(6-Methoxy-3-pyridyl)-1-phenyl-1H-imidazole-4-carbonyl]-2-methylhexahydropyridazine

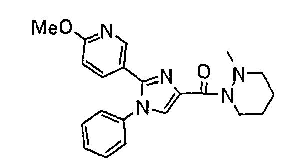

In a manner similar to that employed in Example 1, the title compound in solid form (212 mg, 41.2%) was prepared from 2-(6-methoxy-3-pyridyl)-1-phenyl-1H-imidazole-4-carboxylic acid (400 mg) obtained in Referential Example 4 and 1-methylhexahydropyridazine (204 mg) obtained in Referential Example 14.
¹H-NMR (300MHz,CDCl₃)δ: 1.39-1. 43 (1H,m), 1.70-1.79(1H,m), 1.97-2.02(1H,m), 2.75(3H,s), 2.87-2.92(1H,m), 3.09-3.18(1H,m), 3.89(3H,s), 4.52-4.57(1H,m), 6.66(1H,d,J=8.63Hz), 7.25-7.27(2H,m), 7.43-7.48(3H,m), 7.82 (1H,d, J=8. 63Hz), 7.95 (1H, s), 8.09(1H,d,J=2.2Hz).
MS (FAB)m/z:378 (M+H)⁺.

### [Example 25] 1-(4,5-diphenyloxazole-2-carbonyl)-4-methylpiperazine

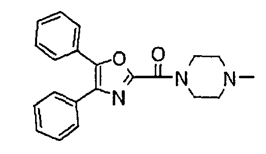

### 1) Title compound

4,5-Diphenyloxazole-2-carboxylic acid ethyl ester (200 mg) obtained in Referential Example 17 in N-methylpiperazine (2 mL) was stirred at 80°C for 3.5 hours, followed by cooling in air. The reaction solvent was removed under reduced pressure, and the residue was purified by silica gel thin-layer chromatography (chloroform-7N ammonia/methanol), to thereby give the title compound as an oily product (176 mg, 74%).
¹H-NMR (400MHz, CDCl₃) δ:2.33 (3H, s), 2.51(4H, t, J=4.7Hz), 3.85(2H, t, J=4.7Hz), 4.29(2H,t,J=4.7Hz), 7.33-7.40(6H,m), 7.64-7.67(4H, m).
MS (EI) m/z: 347 (M⁺).

### 2) Hydrochloric acid salt of the title compound

1M HCl-ethanol (0.55 mL) was added at room temperature to the above-obtained 1-(4,5-diphenyloxazole-2-carbonyl)-4-methylpiperazine (173 mg) in ethanol (2 mL), followed by stirring for 2 hours. Ether was added to the reaction mixture. The solid that precipitated was collected by filtration, and dried, to thereby give the title compound as a solid product (168 mg, 86%).
MS(EI)m/z:347(M⁺).
Elementary analysis: as C₂₁H₂₁N₃O₂ · 1 . 0HCl · 0 . 5H₂O
Calculated: C, 64.20 ; H, 5.90 ; N, 10.70 ; Cl, 9.02.
Found: C, 64.26 ; H, 5. 88 ; N, 10.75 ; Cl, 9. 11.

### [Example 26] 1-[4-(6-Methoxy-3-pyridyl)-5-(3-pyridyl)oxazole-2-carbonyl]-4-methylpiperazine

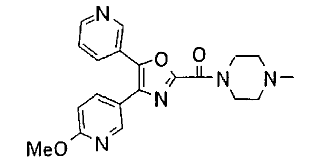

4-(6-Methoxy-3-pyridyl)-5-(3-pyridyl)oxazole-2-carboxylic acid ethyl ester (0.25 g) obtained in Referential Example 15 in N-methylpiperazine (2 mL) was stirred at 80°C for 2.5 hours, followed by cooling in air. The resultant mixture was partitioned between saturated aqueous sodium hydrogencarbonate and ethyl acetate. The organic layer was washed with saturated brine, followed by drying over sodium sulfate anhydrate. After a filtration step, the solvent was removed under reduced pressure, and the residue was purified by silica gel column chromatography (chloroform - methanol), to thereby give the title compound as a solid product (0.1 g, 34%).
¹H-NMR (400MHz, CDCl₃) δ:2.35 (3H, s), 2.53 (4H,br s), 3.87(2H,br s), 3.98(3H,s), 4.27(2H,br s), 6.81(1H,d,J=8.8Hz), 7.36 (1H, dd, J=7. 8, 4.7Hz), 7.80(1H,dd,J=8.6,2.5Hz), 7.98(1H,d,J=8.0Hz), 8.45(1H,s), 8.63(1H,d,J=4.7Hz), 8.89 (1H, s) .MS (EI)m/z:380 (M⁺) .
Elementary analysis: as C₂₀H₂₁N₅O₃
Calculated: C,63.31;H,5.45;N,18.46.
Found: C, 63.00; H, 5.52; N, 18.24.

### [Example 27] 1-[5-(4-Fluorophenyl)-4-(3-pyridyl)oxazole-2-carbonyl]-4-methylpiperazine

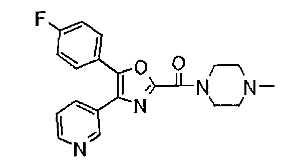

### 1) Title compound

In a manner similar to that employed in Example 26, the title compound in oily form (0.21 g, 85%) was prepared from 5-(4-fluorophenyl)-4-(3-pyridyl)oxazole-2-carboxylic acid ethyl ester (0.21 g) obtained in Referential Example 16 and N-methylpiperazine (2 mL).
¹H-NMR(400MHz, CDCl₃)δ: 2.35 (3H, s), 2.53(4H, t, J=5.1Hz), 3.86 (2H, br s), 4.28(2H,br s), 7.11 (2H, t, J=8.6Hz), 7.35(1H, dd, J=8.0,4.9Hz), 7.62-7.66(2H, m), 7.94(1H, d, J=8.0Hz), 8.61(1H,d,J=3.2Hz), 8.88(1H,d,J=2.2Hz).

### 2) Hydrochloric acid salt of the title compound

In a manner similar to that employed in step 2) of Example 25, a hydrochloric acid salt of the title compound in solid form (170 mg, 67%) was prepared from the above-obtained 1-[5-(4-fluorophenyl)-4-(3-pyridyl)oxazole-2-carbonyl]-4-methylpiperazine (0.21 g).
¹H-NMR (400MHz, DMSO-d₆) δ: 2.82 (3H, s), 3.53(4H,m), 4.57-4.61(2H,m), 5.11-5.15 (2H,m), 7.40(2H,t,J=8.8Hz), 7. 61 (1H,m), 7.67-7.70(2H,m), 8.11(1H,m), 8. 68 (1H, s), 8. 87 (1H, s) .
MS(FAB)m/z:367(M+H)⁺.
Elementary analysis: as C₂₀H₁₉FN₄O₂·2HCl·1.5H₂O
Calculated: C,51.51;H,5.19;N,12.01;F,4.07;Cl,15.20.
Found: C,51.83;H,5.10;N,11.99;F,4.11;Cl,15.01.

### [Example 28] 4-[2-(6-Methoxy-3-pyridyl)-1-phenyl-1H-imidazole-4-carbonyl]1,4-oxazepane

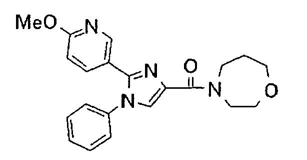

In a manner similar to that employed in Example 1, the title compound in solid form (341 mg, 76%) was prepared from 2-(6-methoxy-3-pyridyl)-1-phenyl-1H-imidazole-4-carboxylic acid (350 mg) obtained in Referential Example 4 and 1,4-oxazepane (206 mg) obtained in Referential Example 19.
¹H-NMR (300MHz, CDCl₃) δ:2.05-2.17 (2H, m), 3.82-3.86(5H,m), 3.91 (3H, s), 3.94-3.97 (1H, m), 4.34-4.40(2H,m), 6.64 (1H, dd, J=8.81, 0.73Hz), 7.26-7.27(2H,m), 7.43-7.47 (3H, m), 7.54 (1H, d, J=8.26Hz), 7.78(1H, s), 8.17(1H, d, J=6.42Hz).
MS(FAB)m/z:379(M+H)⁺.

### [Example 29] 1-[2-(6-Methoxy-3-pyridyl)-1-phenyl-1H-imidazole-4-carbonyl]-3-oxopiperazine

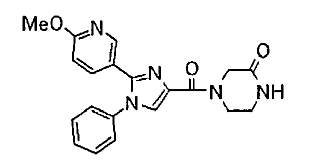

In a manner similar to that employed in Example 1, the title compound in solid form (461 mg, 81.6%) was prepared from 2-(6-methoxy-3-pyridyl)-1-phenyl-1H-imidazole-4-carboxylic acid (442 mg) obtained in Referential Example 4 and piperazin-2-one (150 mg).
¹H-NMR(300MHz, CDCl₃) δ:3.54 (2H, br s), 3.91(3H,s), 4.00(1H, br s), 4.45(1H, br s), 4.70(1H, br s), 5.12(1H, br s), 6.66(1H, d, J=8.81Hz), 7.24-7.27(3H,m), 7.44-7.48(3H,m), 7.81(1H, s), 8.13(1H, br s).MS(FAB)m/z:378(M+H)⁺.

### [Example 30] 1-[2-(6-Methoxy-3-pyridyl)-1-phenyl-1H-imidazole-4-carbonyl]-4-methyl-3-oxopiperazine

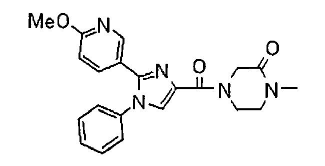

In a manner similar to that employed in Example 1, the title compound in solid form (448 mg, 67.7%) was prepared from 2-(6-methoxy-3-pyridyl)-1-phenyl-1H-imidazole-4-carboxylic acid (500 mg) obtained in Referential Example 4 and 1-methylpiperazin-2-one trifluoroacetic acid salt (425 mg) obtained in Referential Example 38.
¹H-NMR(300MHz,CDCl₃)δ:3.03(3H,s), 3.49(2H,br s), 3.91(3H,s), 4.02(1H,br s), 4.43(1H,br s), 4.71(1H,br s), 5.07(1H,s), 6.66(1H,d,J=8.63Hz), 7.24-7.26(2H,m), 7.45-7.48(3H,m), 7.67(1H,br s), 7.79(1H,s), 8.11(1H, br s).
MS (FAB)m/z:392 (M+H)⁺.

### [Example 31] 1-[1-(4-Methylphenyl)-2-(6-methyl-3-pyridyl)-1H-imidazole-4-carbonyl]-3-oxopiperazine

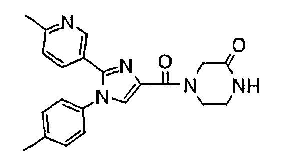

In a manner similar to that employed in Example 1, the title compound in solid form (603 mg, quantitative amount) was prepared from 1-(4-methylphenyl)-2-(6-methyl-3-pyridyl)-1H-imidazole-4-carboxylic acid (439 mg) obtained in Referential Example 11 and piperazin-2-one (150 mg).
¹H-NMR (300MHz, CDCl₃) δ: 2.41 (3H, s), 2.54 (3H, s), 3.55 (2H, br s), 4.01(1H.br s), 4.45 (1H, br s), 4.70 (1H, br s), 5.13 (1H, br s), 7.08-7.14 (3H,m), 7.23-7.28(3H,m), 7.80 (1H, s), 8.38 (1H,m) .
MS(FAB)m/z:376(M+H)⁺.

### [Example 32] 1-[1-(4-Methylphenyl)-2-(6-methyl-3-pyridyl)-1H-imidazole-4-carbonyl]-4-methyl-3-oxopiperazine

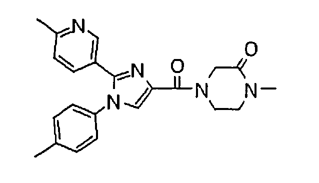

Under cooling on ice, 60% sodium hydride (63.9 mg) was added to 1-[1-(4-methylphenyl)-2-(6-methyl-3-pyridyl)-1H-imidazole-4-carbonyl]-3-oxopiperazine (500 mg) obtained in Example 31 in N,N-dimethylformamide (15 mL), followed by stirring for 10 minutes. Methyl iodide (124 µL) was added to the reaction mixture, followed by stirring for 1 hour. The reaction mixture was partitioned between water and ethyl acetate. The aqueous layer was further extracted with tetrahydrofuran, and the organic layers were combined. The combined organic layer was dried over magnesium sulfate anhydrate. After a filtration step, the filtrate solvent was removed under reduced pressure, and the residue was purified by silica gel column chromatography (chloroform - methanol), to thereby give the title compound as a solid product (283 mg, 54.6%) .
¹H-NMR(300MHz,CDCl₃)δ:2.41(3H,s), 2.54(3H,s), 3.03(3H,s), 3.50(2H,br s), 4.02(1H.br s), 4.42(1H,br s), 4.71(1H,br s), 5.07(1H,br s), 7.08-7.13(3H,m), 7.23-7.27(3H,m), 7.78(1H,s), 8.36(1H,m).
MS(FAB)m/z:390(M+H)⁺.

### [Example 33] 1-[1-(6-Methoxy-3-pyridyl)-5-phenyl-1H-1,2,4-triazole-3-carbonyl]-4-methylpiperazine hydrochloride

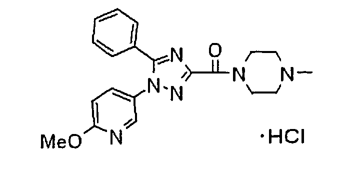

1-Ethyl-3-(3-dimethylaminopropyl)carbodiimide hydrochloride (127 mg) was added at 0°C to 1-(6-methoxy-3-pyridyl)-5-phenyl-1H-1,2,4-triazole-3-carboxylic acid (150 mg) obtained in Referential Example 23, triethylamine (107 µL), N-methylpiperazine (68 µL), and 1-hydroxybenzotriazole (90 mg) in N,N-dimethylformamide (4 mL), followed by stirring at room temperature for 13.5 hours. The reaction mixture was partitioned between water and ethyl acetate. The organic layer was washed with saturated brine, and then dried over magnesium sulfate anhydrate. After a filtration step, the filtrate solvent was removed under reduced pressure, and the residue was purified by silica gel column chromatography (chloroform - methanol). The obtained oily product was dissolved in diethyl ether (4 mL). 1N HCl-ethanol (0.61 mL) was added to the resultant mixture at 0°C, followed by stirring for 10 minutes. The solid that precipitated was collected by filtration, and then dried, to thereby give the title compound (101 mg, 48%).
¹H-NMR(400MHz, DMSO-d₆)δ:2.77 (3H,s), 3.12(2H,br m), 3.25-3.65(4H,br m), 3.90(3H,s), 4.58(2H,br m), 7.00 (1H, d, J=8.8Hz), 7.43-7.51(5H,m), 7.86(1H,dd,J=8.8,2.7Hz), 8.31(1H,d,J=2.7Hz), 11.30(1H,br s).
LC-MSm/z:379(M+H)⁺.
Elementary analysis: as C₂₀H₂₂N₆O₂·HCl
Calculated: C,57.90;H,5.59;N,20.26;Cl,8.55.
Found: C,57.72;H,5.60;N,20.04;Cl,8.27.

### [Example 34] 1-[5-(4-Fluorophenyl)-1-(6-methoxy-3-pyridyl)-1H-1,2,4-triazole-3-carbonyl]-4-methylpiperazine hydrochloride

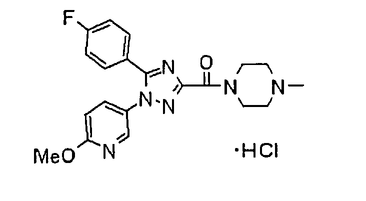

In a manner similar to that employed in Example 33, the title compound in solid form (219 mg, 79%) was prepared from 5-(4-fluorophenyl)-1-(6-methoxy-3-pyridyl)-1H-1, 2, 4-triazole-3-carboxylic acid (200 mg) obtained in Referential Example 24 and N-methylpiperazine (85 µL).
¹H-NMR (400MHz, DMSO-d₆)δ:2.77 (3H, s), 3.13 (2H,br m), 3.25-3.65(4H,br m), 3.90 (3H, s), 4.57 ( 2H, br m), 6.99 (1H, d, J=8.8Hz), 7.31(2H, t, J=8.6Hz), 7.56(2H, dd, J=8.8,5.6Hz), 7.86 (1H,dt,J=8.8,1.3Hz), 8.32 (1H, d, J=2.5Hz), 11.17(1H, br s).
MS (FAB)m/z:397 (M+H)⁺.

### [Example 35] 1-[1-(6-Methoxy-3-pyridyl)-5-phenyl-1H-1,2,4-triazole-3-carbonyl]-4-cyclopropylpiperazine

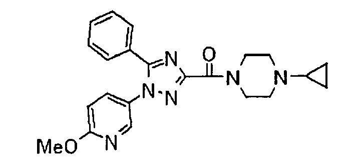

In a manner similar to that employed in Example 1, the title compound in solid form (165 mg, 88%) was prepared from 1-(6-methoxy-3-pyridyl)-5-phenyl-1H-1,2,4-triazole-3-carboxylic acid (150 mg) obtained in Referential Example 23 and N-cyclopropylpiperazine hydrochloride (112 mg) obtained in Referential Example 33.
¹H-NMR(400MHz, CDCl₃) δ:0.42-0.51 (4H,m), 2.68-2.73(4H,m), 3.82(2H,m), 3.91(2H,m), 3.98(3H,s), 6.81(1H,d,J-9.0Hz), 7.39(2H,m), 7.44(1H,m), 7.52(2H,m), 7.61(1H,dd,J=8.8,2.7Hz).
MS (EI)m/z:404 (M⁺) .
Elementary analysis: as C₂₂H₂₄N₆O₂·0.25H₂O
Calculated: C,64.61;H,6.04;N,20.54.
Found: C,64.47;H,5.96;N,20.40.

### [Example 36] 1-[5-(6-Methoxy-3-pyridyl)-1-phenyl-1H-1,2,4-triazole-3-carbonyl]-4-methylpiperazine hydrochloride

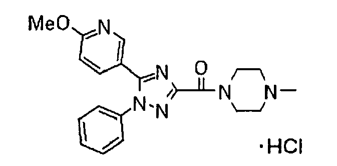

In a manner similar to that employed in Example 33, the title compound in solid form (140 mg, 66%) was prepared from 5-(6-methoxy-3-pyridyl)-1-phenyl-1H-1,2,4-triazole-3-carboxylic acid (150 mg) obtained in Referential Example 25 and N-methylpiperazine (68 µL).
¹H-NMR(400MHz,DMSO-d₆)δ:2.75(3H,s), 3.17(2H,br m), 3.25-3.45(4H,br m), 3.87(3H,s), 4.57(2H,br m), 6.89(1H,d,J=8.8Hz), 7.51-7.53(2H,m), 7.56-7.58(2H,m), 7.72(1H,dd,J=8.8,2.5Hz), 8.25(1H,d, J=2.2Hz), 10.89(1H, br s).
MS (ESI)m/z:379 (M+H)⁺.
Elementary analysis: as C₂₀H₂₂N₆O₂·HCl·H₂O
Calculated: C,55.49;H,5.82;N,19.41;Cl,8.19.
Found: C, 55.35; H, 5.72; N, 19.22; Cl, 8.02.

### [Example 37] 1-[1-(6-Methoxy-3-pyridyl)-5-(4-methylphenyl)-1H-1,2,4-triazole-3-carbonyl]-4-methylpiperazine hydrochloride

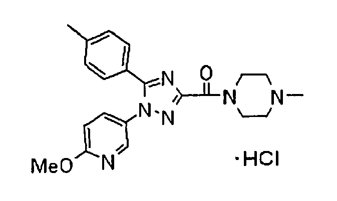

In a manner similar to that employed in Example 33, the title compound in solid form (167 mg, 81%) was prepared from 1-(6-methoxy-3-pyridyl)-5-(4-methylphenyl)-1H-1,2,4-triazole-3-carboxylic acid (150 mg) obtained in Referential Example 26 and N-methylpiperazine (64 µL).
¹H-NMR(400MHz, DMSO-d₆) δ:2.33 (3H, s), 2.76(3H,s), 3.14(2H,br m), 3.25-3.65(4H,br m), 3.91(3H,s), 4.57(2H,br m), 7.00 (1H, d, J=8.8Hz), 7.26(2H, d, J=8 .1Hz), 7.39(1H, d, J=8.3Hz), 7.86(1H, dd, J=8.8, 2.7Hz), 8.31(1H, d, J=2.7Hz), 11.01(1H,br s).
MS(ESI)m/z:393(M+H)⁺.
Elementary analysis: as C₂₁H₂₄N₆O₂·HCl
Calculated: C,58.81;H,5.87;N,19.59;Cl,8.27.
Found: C,58.47;H,5.89;N,19.28;Cl,8.19.

### [Example 38] 1-[5-(6-Methoxy-3-pyridyl)-1-(4-methylphenyl)-1H-1,2,4-triazole-3-carbonyl]-4-methylpiperazine hydrochloride

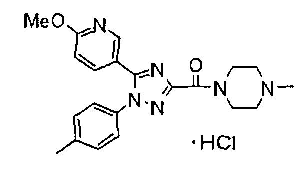

In a manner similar to that employed in Example 33, the title compound in solid form (148 mg, 72%) was prepared from 5-(6-methoxy-3-pyridyl)-1-(4-methylphenyl)-1H-1,2,4-triazole-3-carboxylic acid (150 mg) obtained in Referential Example 27 and N-methylpiperazine (64 µL).
¹H-NMR(400MHz, DMSO-d₆) δ:2.40 (3H,s), 2.77(3H,s), 3.15-3.45(4H,br m), 3.37(2H,br m), 3.87(3H,s), 4.59(2H,br m), 6.89(1H, d, J=8.5Hz), 7.35-7.41(4H,m), 7.73(1H, dd, J=8.8, 2.4Hz), 8.26 (1H, d, J=2.5Hz), 11.38 (1H, br s).
LC-MSm/z:393(M+H)⁺.
Elementary analysis: as C₂₁H₂₄N₆O₂·HCl
Calculated: C,58.81;H,5.87;N,19.59.
Found: C,58.66;H,5.91;N,19.28.

### [Example 39] 1-[1-(6-Methoxy-3-pyridyl)-5-(4-methylphenyl)-1H-1,2,4-triazole-3-carbonyl]-3-dimethylaminoazetidine

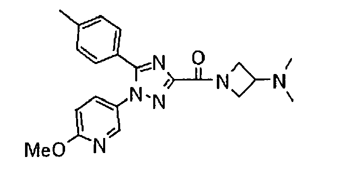

In a manner similar to that employed in Example 1, the title compound in solid form (96 mg, 61%) was prepared from 1-(6-methoxy-3-pyridyl)-5-(4-methylphenyl)-1H-1,2,4-triazole-3-carboxylic acid (100 mg) obtained in Referential Example 26 and 3-dimethylaminoazetidine hydrochloride (66 mg) obtained in Referential Example 34.
¹H-NMR(400MHz,CDCl₃)δ:2.22(6H,s), 2.37(3H,s), 3.17(1H,m), 3.98(3H,s), 4.10(1H,dd,J=10.6,5.9Hz), 4.28(1H,m), 4.48(1H,dd,J=10.5,5.9Hz), 4.71(1H,m), 6.80(1H,dd,J=8.8,0.5Hz), 7.17(2H,d,J=8.8Hz), 7.41(2H,dd,J=6.9,1.76Hz), 7.58(1H,dd,J=8.8,2.7Hz), 8.18(1H,d,J=0.5Hz).
MS (ESI)m/z:393 (M+H)⁺.
Elementary analysis: as C₂₁H₂₄N₆O₂
Calculated: C,64.27;H,6.16;N,21.41.
Found: C,63.92;H,6.16;N,21.21.

### [Example 40] 1-[5-(3-Fluoro-4-methylphenyl)-1-(6-methoxy-3-pyridyl)-1H-1,2,4-triazole-3-carbonyl]-4-methylpiperazine

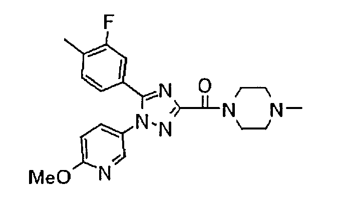

In a manner similar to that employed in Example 1, the title compound in solid form (145 mg, 77%) was prepared from 5-(3-fluoro-4-methylphenyl)-1-(6-methoxy-3-pyridyl)-1H-1,2,4-triazole-3-carboxylic acid (150 mg) obtained in Referential Example 37 and N-methylpiperazine (61 µL).
¹H-NMR (400MHz, CDCl₃) δ:2.29 (3H, s), 2.35(3H,s), 2.50-2.54(4H,br m), 3.88(2H,m), 3.95(2H,m), 3.99(3H,s), 6.83(1H,dd,J=8.8,0.7Hz), 7.16 (1H, s), 7.16-7.24 (2H, m), 7.60 (1H, dd, J=8.8, 2.7Hz), 8.17 (1H, dd, J=8.8, 0.7Hz).
MS (ESI) m/z:411 (M+H)⁺.
Elementary analysis: as C₂₁H₂₃FN₆O₂
Calculated: C,61.45;H,5.65;N,20.48;F,4.63.
Found: C, 61.07; H, 5.65; N, 20.16; F, 4.53.

### [Example 41] 1-[1-(4-Methylphenyl)-5-(6-methyl-3-pyridyl)-1H-1,2,4-triazole-3-carbonyl]-4-methylpiperazine hydrochloride

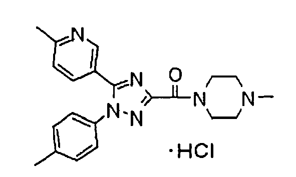

1-(4-Methylphenyl)-5-(6-methyl-3-pyridyl)-1H-1,2,4-triazole-3-carboxylic acid methyl ester (100 mg) obtained in Referential Example 28 in N-methylpiperazine (358 µL) was stirred at 80°C for 2.5 hours, followed by cooling in air. The reaction solvent was removed under reduced pressure, and the residue was purified by silica gel column chromatography (chloroform - methanol). The resultant oily product was dissolved in diethyl ether (4 mL). 1N HCl-ethanol (704 µL) was added to the resultant mixture at 0°C, followed by stirring for 10 minutes. The reaction solvent was removed under reduced pressure, to thereby give the title compound in solid form (113 mg, 79%).
¹H-NMR (400MHz, DMSO-d₆) δ:2.39 (3H, s), 2. 53 (3H, s), 2.79(3H,d,J=9.6Hz), 3.12(2H,m), 3.36(1H, m), 3.45 (1H, d, J=12.0Hz), 3.51 (1H, d, J=12.0Hz), 3.68 (1H, m), 4.61(2H,m), 7.34-7.44(5H,m), 7.81 (1H, dd, J=8.1, 2.2Hz), 8.56 (1H, d, J=1.9Hz), 11.22 (1H, br s).
MS (ESI)m/z:377 (M+H)⁺.

### [Example 42] 4-[1-(6-Methoxy-3-pyridyl)-5-(4-methylphenyl)-1H-1,2,4-triazole-3-carbonyl]morpholine

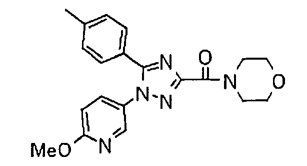

In a manner similar to that employed in Example 1, the title compound in solid form (148 mg, 81%) was prepared from 1-(6-methoxy-3-pyridyl)-5-(4-methylphenyl)-1H-1,2,4-triazole-3-carboxylic acid (150 mg) obtained in Referential Example 26 and morpholine (50 µL).
¹H-NMR(400MHz,CDCl₃)δ:2.37(3H,s), 3.75(2H,t,J=4.9Hz), 3.80(2H,t,J=5.1Hz), 3.86(2H, d, J=5.1Hz), 3. 98 (3H, s) , 4.02(2H,t,J=4.9Hz), 6.82(1H,d,J=9.1Hz), 7.18(2H,d,J=8.1Hz), 7.41(2H,d,J=8.1Hz), 7.59(1H,dd,J=8.8,2.7Hz), 8.18(1H,d,J=2.4Hz).
MS (ESI)m/z:380 (M+H)⁺.

### [Example 43] 7-[1-(6-Methoxy-3-pyridyl)-5-(4-methylphenyl)-1H-1,2,4-triazole-3-carbonyl]-4,7-diazaspiro[2.5]ocatne

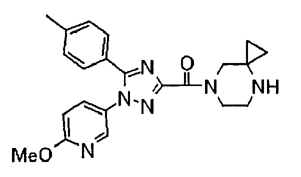

### 1) Title compound

In a manner similar to that employed in Example 1, the title compound in amorphous form (252 mg, 77%) was prepared from 1-(6-methoxy-3-pyridyl)-5-(4-methylphenyl)-1H-1,2,4-triazole-3-carboxylic acid (250 mg) obtained in Referential Example 26 and 4,7-diazaspiro[2.5]octane hydrochloride (150 mg) obtained in Referential Example 36.
¹H-NMR(400MHz,CDCl₃)δ:0.62-0.73(4H,m), 2.37(3H,s), 3.01-3.08(2H,m), 3.69 and 3.75(2H,each s), 3.82(1H,t,J=5.1Hz), 3.92(1H,t,J-5.1Hz), 3.98(3H,s), 6.80 and 6.81(1H,each d,J=8.8Hz), 7.17(2H,m), 7.38-7.43(2H,m), 7.57 and 7.58(1H,each dd,J=8.8,2.7Hz), 8.16 and 8.19(1H,each d, J=2.7Hz).
MS (ESI) m/z:405 (M+H)⁺.

### 2) Hydrochloric acid salt of the title compound

The above-obtained 7-[1-(6-methoxy-3-pyridyl)-5-(4-methylphenyl)-1H-1,2,4-triazole-3-carbonyl]-4,7-diazaspiro[2.5]octane (100 mg) was dissolved in diethyl ether (4 mL). 1N HCl-ethanol (297 µL) was added to the resultant mixture at 0°C, followed by stirring for 10 minutes. The reaction solvent was removed under reduced pressure, and then dried, to thereby give a hydrochloric acid salt of the title compound as a solid product (70 mg, 64%).
¹H-NMR(400MHz, DMSO-d₆) δ: 0.92 (2H,m), 1.12 (2H,m), 2.32 (3H, s), 3.22(4H,m), 3.92(3H,s), 4.00-4.24(2H,m), 6.96 (1H, d, J=8 . 8Hz), 7.25 (2H, d, J=8 .0Hz), 7 . 39 (2H, d, J=8 . 3Hz), 7.82 (1H, d, J=8 .2Hz), 8.27 (1H, s) .
MS (ESI)m/z:405 (M+H)⁺.
Elementary analysis: as C₂₂H₂₄N₆O₂·HCl·1.25H₂O
Calculated: C,57.02;H,5.98;N,18.13;Cl,7.65.
Found: C,57.16;H,5.97;N,18.13;Cl,7.59.

### [Example 44] 7-[1-(6-Methoxy-3-pyridyl)-5-(4-methylphenyl)-1H-1,2,4-triazole-3-carbonyl]-4-methyl-4,7-diazaspiro[2.5]octane hydrochloride

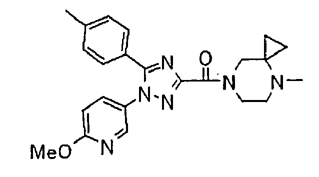

Sodium cyanoborohydride (186 mg) and 37% formaldehyde (246 µL) were added to 7-[1-(6-methoxy-3-pyridyl)-5-(4-methylphenyl)-1H-1,2,4-triazole-3-carbonyl]-4,7-diazaspiro[2.5]octane (148 mg) obtained in Example 43 in methanol (5 mL), followed by stirring at room temperature for 4 hours. The reaction solvent was removed under reduced pressure. Water and ethyl acetate were added thereto for partitioning the residue. The organic layer was sequentially washed with saturated aqueous sodium hydrogencarbonate and saturated brine, and then dried over magnesium sulfate anhydrate. After a filtration step, the filtrate solvent was removed under reduced pressure, and the residue was purified by silica gel column chromatography (chloroform - methanol). The resultant oily product was dissolved in diethyl ether (2 mL). At 0°C, 1N HCl-ethanol (200 µL) was added to the resultant mixture, followed by stirring for 10 minutes. The reaction solvent was removed under reduced pressure, and then dried, to thereby give the title compound as a solid product (54 mg, 32%).
¹H-NMR(400MHz,DMSO-d₆) δ:0.93 (2H, m), 1.20 (2H, m), 2.33 (3H, s), 2.82(3H,s), 3.29 (4H,m), 3.86 (2H, m), 3.92(3H,s), 6.96 (1H, d, J=8.8Hz), 7.25 (2H, d, J=8.1Hz), 7.38 (2H, m), 7.81 (1H, m), 8.27 (1H, s).
MS(ESI)m/z:419 (M+H)⁺.

### [Example 45] 1-[1-(6-Methoxy-3-pyridyl)-5-(4-methylphenyl)-1H-1,2,4-triazole-3-carbonyl]-4-methyl-3-oxopiperazine

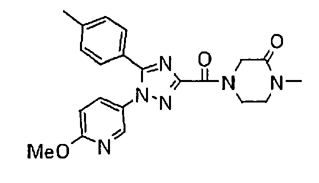

In a manner similar to that employed in Example 1, the title compound in solid form (45 mg, 28%) was prepared from 1-(6-methoxy-3-pyridyl)-5-(4-methylphenyl)-1H-1,2,4-triazole-3-carboxylic acid (124 mg) obtained in Referential Example 26 and 1-methylpiperazin-2-one hydrochloride (72 mg) obtained in Referential Example 32.
¹H-NMR (400MHz, CDCl₃) δ:2.37 (3H, s), 3.01 and 3.04(3H,each s), 3.51 (2H, m), 3.98(3H,s), 4.08 and 4.30 (2H, each m), 4.47 and 4.71 (2H, each s), 6.82 (1H, d, J=8.8Hz), 7.18 (2H, d, J=8.0Hz), 7.36-7.41 (2H, m), 7. 60 (1H, dd, J=8.8, 2.7Hz), 8.18 (1H, d, J=2.4Hz).
MS (ESI) m/z:406 (M⁺).

### [Example 46] 1-[1-(6-Methoxy-3-pyridyl)-5-(4-methylphenyl)-1H-1,2,4-triazole-3-carbonyl]-2,2-dimethyl-3-dimethylaminoazetidine hydrochloride

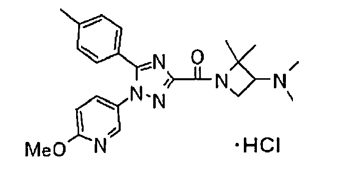

In a manner similar to that employed in Example 33, the title compound in solid form (190 mg, 87%) was prepared from 1-(6-methoxy-3-pyridyl)-5-(4-methylphenyl)-1H-1,2,4-triazole-3-carboxylic acid (150 mg) obtained in Referential Example 26 and (2,2-dimethylazetidin-3-yl)dimethylamine hydrochloride (116 mg) obtained in Referential Example 35.
¹H-NMR (400MHz, DMSO-d₆) δ:1.68 (3H, s), 1.70 (3H, s), 2.32 (3H, s), 2.70(3H,br s), 2.82(3H,br s), 3.91(3H,s), 3.99 (1H, m), 4.58 (1H, m), 4.80 (1H, t, J=9.7Hz), 6.99 (1H, d, J=8.8Hz), 7.25(2H,d,J=8.1Hz), 7.39(2H,d,J-8.0Hz), 7.83(1H,dd,J=8.8,2.7Hz), 8.31(1H,d,J=2.7Hz), 11.22(1H,br s).MS(ESI)m/z:421(M+H)⁺.
Elementary analysis: as C₂₃H₂₈RN₆O₂·HCl·0.75H₂O
Calculated: C,58.72;H,6.53;N,17.86;Cl,7.54.
Found: C,58.82;H,6.54;N,17.80;Cl,7.46.

### [Example 47] 1-[1-(6-Methoxy-3-pyridyl)-5-(4-methylphenyl)-1H-1,2,4-triazole-3-carbonyl]azetidine-2-carboxylic acid dimethylamide

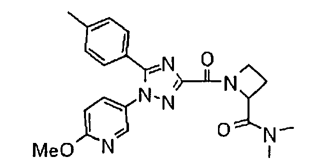

In a manner similar to that employed in Example 1, the title compound in solid form (158 mg, 78%) was prepared from 1-(6-methoxy-3-pyridyl)-5-(4-methylphenyl)-1H-1,2,4-triazole-3-carboxylic acid (150 mg) obtained in Referential Example 26 and azetidine-2-carboxylic acid dimethylamide (95 mg) obtained in Referential Example 30.
¹H-NMR(400MHz,DMSO-d₆)δ:2.02 and 2.10(1H,each m), 2.32(3H,s), 2.71(1H,m), 2.78 and 2.86(3H,each s), 2.87 and 2.98(3H,each s), 3.90(3H,s), 3.96 and 4.47(1H,each m), 4.02(1H,m), 5.27 and 5.83(1H,each dd,J=9.2,5.3Hz and 9.0,4.6Hz), 6.97 and 6.98(1H,each d,J=1.9Hz and 9.0Hz), 7.24-7.39(4H,m), 7.75 and 7.84(1H,each dd,J=8.8,2.7Hz and 9.1,2.9Hz), 8.20 and 8.28(1H,each d,J=2.7Hz).
MS (ESI)m/z:421 (M+H)⁺.

### [Example 48] 1-[1-(6-Methoxy-3-pyridyl)-5-(4-methylphenyl)-1H-1,2,4-triazole-3-carbonyl]-2-dimethylaminomethylazetidine hydrochloride

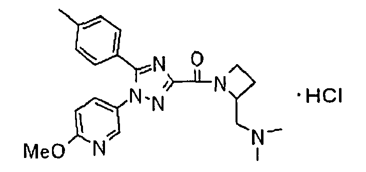

In a manner similar to that employed in Example 33, the title compound in solid form (181 mg, 85%) was prepared from 1-(6-methoxy-3-pyridyl)-5-(4-methylphenyl)-1H-1,2,4-triazole-3-carboxylic acid (150 mg) obtained in Referential Example 26 and 2-dimethylaminomethylazetidine hydrochloride (108 mg) obtained in Referential Example 31.
¹H-NMR(400MHz,DMSO-d₆)δ:2.24 and 2.53 (1H, each m), 2.32(3H,s), 2.66(1H,m), 2.86(6H,s), 3.40(1H,dd,J=13.0,5.6Hz), 3.71(1H,dd,J=13.0,7.2Hz), 3.90(3H,s), 4.05 and 4.52(2H,each t,J=7.8Hz), 4.94 and 5.26(1H,each t,J=7.6Hz), 6.99(1H,d,J=9.1Hz), 7.24-7.26(2H,m), 7.36-7.42(2H,m), 7.83 and 7.90(1H,each dd,J=8.8,2.7Hz), 8.28 and 8.31(1H,each d,J=2.9 and 2.7Hz).
MS (ESI)m/z:407 (M+H)⁺.

### [Example 49] 1-[1-(6-Methoxyphenyl)-5-(6-methyl-3-pyridyl)-1H-1,2,4-triazole-3-carbonyl]-2,2-dimethyl-3-dimethylaminoazetidine

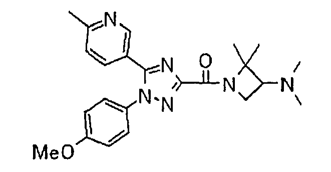

In a manner similar to that employed in Example 1, the title compound in solid form (37 mg, 68%) was prepared from 1-(4-methoxyphenyl)-5-(6-methyl-3-pyridyl)-1H-1,2,4-triazole-3-carboxylic acid (40 mg) obtained in Referential Example 29 and 2,2-dimethyl-3-dimethylaminoazetidine hydrochloride (31 mg) obtained in Referential Example 35.
¹H-NMR(400MHz,DMSO-d₆)δ:1.68(1H,s), 1.71(1H,s), 2.13(6H,s), 2.56(3H,s), 2.70(1H,t,J=7.5Hz), 3.85(3H,s), 4.21(2H, dd, J=9.8, 7.6Hz), 4.59 (2H, dd, J=9. 8, 7.8Hz), 6.94-6.97 (2H,m), 7.15(1H,d,J=8.3Hz), 7.26-7.30(2H,m), 7. 80 (1H, dd, J=8.0,2.1Hz), 8. 58 (1H, d, J=2.2Hz).
MS (ESI)m/z:421(M+H)⁺.
Elementary analysis: as C₂₃H₂₈N₆O₂·0.25H₂O
Calculated: C,65.00;H,6.76;N,19.77.
Found: C,65.06;H,6.68;N,19.72.

### [Example 50] 1-[2-(6-Methoxy-3-pyridyl)-1-(2-pyridyl)-1H-imidazole-4-carbonyl]piperidine

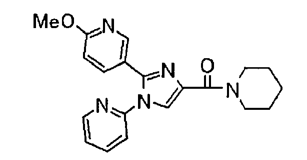

In a manner similar to that employed in Example 1, the title compound in solid form (204 mg, 66.9%) was prepared from 2-(6-methoxy-3-pyridyl)-1-(2-pyridyl)-1H-imidazole-4-carboxylic acid (250 mg) obtained in Referential Example 18 and piperidine (125 µL).
¹H-NMR(300MHz, CDCl₃) δ:1.55-1.75 (6H, m), 3.73(2H,br m), 3.93(3H,s), 4.11(2H, br m), 6.70 (1H, dd, J=8.4, 0. 6Hz), 7.16(1H, d, J=8.1Hz), 7.36 (1H, m), 7.65 (1H, dd, J=8.7, 2.4Hz), 7.79 (1H, ddd, J=8.1, 8.1, 1.8Hz), 7.90 (1H, s), 8.16 (1H, d, J=1.8Hz), 8.55(1H, dd, J=3.9, 0.9Hz).
MS (FAB)m/z:364 (M+H)⁺,

### [Example 51] 1-[5-(6-Methoxy-3-pyridyl)-1-(4-methylphenyl)-1H-1,2,4-triazole-3-carbonyl]-4-methyl-3-oxopiperazine

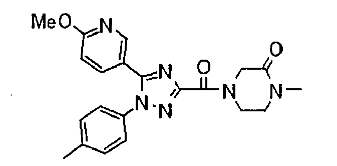

In a manner similar to that employed in Example 1, the title compound in solid form (115 mg, 46%) was prepared from 5-(6-methoxy-3-pyridyl)-1-(4-methylphenyl)-1H-1,2,4-triazole-3-carboxylic acid (182 mg) obtained in Referential Example 27 and 1-methyl-2-oxopiperazine trifluoroacetic acid salt (270 mg) obtained in Referential Example 38.
¹H-NMR(400MHz,CDCl₃)δ:2.43(3H,s), 3.03(3H,s), 3.48(2H,t,J=5.49Hz), 3.95(3H,s), 4.08 (1H, t, J=5.49Hz), 4.29 (1H, t, J=5.49Hz), 4.47 (1H,br), 4.71 (1H,br), 6.73 (1H, d, J=8.79Hz), 7.27(4H,m), 7.78 (1H, dd, J=8. 79, 2.44Hz), 8.29(1H,s).
MS(FAB)m/z:407(M+H)⁺.
Elementary analysis: as C₂₁H₂₂N₆O₃·0.6H₂O
Calculated: C,60.45;H,5.60;N,20.14.
Found: C,60.21;H,5.35;N,19.93.

### [Example 52] 4-[2-(6-Methoxy-3-pyridyl)-1-(2-pyridyl)-1H-imidazole-4-carbonyl]morpholine

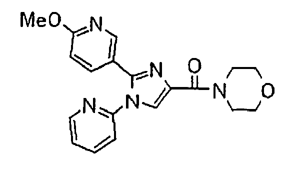

In a manner similar to that employed in Example 1, the title compound in solid form (215 mg, 58.9%) was prepared from 2-(6-methoxy-3-pyridyl)-1-(2-pyridyl)-1H-imidazole-4-carboxylic acid (250 mg) obtained in Referential Example 18 and morpholine (118 µL).
¹H-NMR (300MHz, CDCl₃) δ: 3.70-3.90(6H,m), 3.93(3H,s), 4.38(2H,br m), 6.71(1H,dd,J=8.7,0.9Hz), 7.16(1H,d,J=8.1Hz), 7.37(1H,m), 7.62(1H,dd,J=8.7,2.4Hz), 7.80(1H,ddd,J=7.8,7.8,1.8Hz), 7.99(1H,s), 8.15 (1H, d, J=1.8Hz), 8.56(1H,dd,J=5.1,1.2Hz).
MS (FAB)m/z:366 (M+H)⁺.

### [Example 53] 1-[2-(6-Methoxy-3-pyridyl)-1-(2-pyridyl)-1H-imidazole-4-carbonyl]-4-methyl-3-oxopiperazine

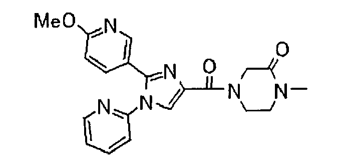

In a manner similar to that employed in Example 1, the title compound in solid form (194 mg, 54.3%) was prepared from 2-(6-methoxy-3-pyridyl)-1-(2-pyridyl)-1H-imidazole-4-carboxylic acid (270 mg) obtained in Referential Example 18 1-methylpiperazin-2-one trifluoroacetic acid salt (228 mg) obtained in Referential Example 38.
¹H-NMR(300MHz,CDCl₃)δ:3,03(3H,s), 3.49(2H,br m), 3.93(3H,s), 4.05(1H,br m), 4.30-4.70(2H,br m), 5.02(1H,br m), 6.71(1H,br d, J=8.4Hz), 7.16(1H,d,J=8.1Hz), 7.39(1H,m), 7.64(1H,m), 7.81(1H,ddd,J=8.4,8.4,2.1Hz), 8.03(1H,s), 8.15(1H,br m), 8.56(1H,dd,J=4.8,0.9Hz).
MS(FAB)m/z:393(M+H)⁺.
Elementary analysis: as C₂₀H₂₀N₆O₃·0.25HO
Calculated: C,60.52;H,5.21;N,21.17.
Found: C,60.56;H,5.20;N,20.93.

### [Example 54] 1-[2-(6-Methoxy-3-pyridyl)-1-(4-methylphenyl)-1H-imidazole-4-carbonyl]-4-methyl-3-oxopiperazine

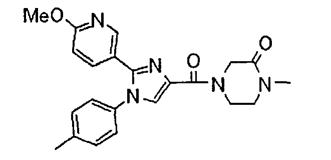

In a manner similar to that employed in Example 1, the title compound in solid form (430 mg, 65.4%) was prepared from 2-(6-methoxy-3-pyridyl)-1-(4-methylphenyl)-1H-imidazole-4-carboxylic acid (500 mg) obtained in Referential Example 6 and 1-methylpiperazin-2-one trifluoroacetic acid salt (407 mg) obtained in Referential Example 38.
¹H-NMR(300MHz, CDCl₃) δ:2.42(3H,s), 3.03(3H,s), 3.50(2H,br s), 3.91 (3H, s), 4.16 (1H,br s), 4.42 (1H,br s), 4.71 (1H,br s), 5.07 (1H, br s), 6.67 (1H, d, J=8.63Hz), 7.11-7.15 (2H, m), 7.23-7.27(2H,m), 7.61 (1H,br s), 7.75 (1H,br s), 8.13 (1H, br s).
MS (FAB) m/z : 406 (M+H)⁺.

### [Example 55] 1-[1-(4-Fluorophenyl)-2-(6-methoxy-3-pyridyl)-1H-imidazole-4-carbonyl]-4-methyl-3-oxopiperazine

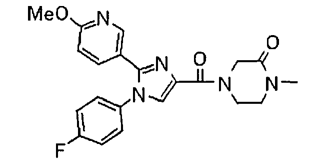

In a manner similar to that employed in Example 1, the title compound in solid form (513 mg, 78.3%) was prepared from 1-(4-fluorophenyl)-2-(6-methoxy-3-pyridyl)-1H-imidazole-4-carboxylic acid (500 mg) obtained in Referential Example 7 and 1-methylpiperazin-2-one trifluoroacetic acid salt (401 mg) obtained in Referential Example 38.
¹H-NMR (300MHz, CDCl₃) δ:3.03 (3H, s), 3.47-3.49(2H,m), 3.92(3H,s), 4.02 (1H, br s), 4.43 (1H, br s), 4.68 (1H, br s), 5.05 (1H, br s), 6.69 (1H, d, J=8.63Hz), 7.13-7.20 (2H, m), 7.22-7.27(2H,m), 7.70 (1H, br s), 7.75 (1H, s), 8.09 (1H, br s).
MS (FAB)m/z:410 (M+H)⁺.

### [Example 56] (25)-1-[2-(6-Methoxy-3-pyridyl)-1-(2-pyridyl)-1H-imidazole-4-carbonyl]pyrrolidine-2-carboxamide

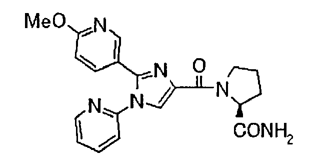

In a manner similar to that employed in Example 1, the title compound in amorphous form (385 mg, 83.2%) was prepared from 2-(6-methoxy-3-pyridyl)-1-(2-pyridyl)-1H-imidazole-4-carboxylic acid (350 mg) obtained in Referential Example 18 and L-prolinamide (162 mg).
¹H-NMR (300MHz, CDCl₃) δ:1.74 (1H, m), 1.90-2.25(3H, m), 2.42 (1H, m), 3.77(0.3H,m), 3.91(0.4H,m), 3.93(3H,br s), 4.25(1H, m), 4.87(0.5H,m), 5.35-5.55(1H, m), 6.71(1H,dd,J=8.7,0.6Hz), 7.15(1H, m), 7.38(1H, m), 7.65 (1H, m), 7.80 (1H, t, J=7.8Hz), 8.05(0.5H,m), 8.06(1H,s), 8.16(0.5H,m), 8.56(1H, d, J=3.6Hz).
[α]_{D}²⁶-73.8° (c=0.24, CHCl₃).
MS (FAB)m/z:393 (M+H)⁺.

### [Example 57] 1-[2-(6-Methoxy-3-pyridyl)-1-(2-pyridyl)-1H-imidazole-4-carbonyl]pyrrolidine

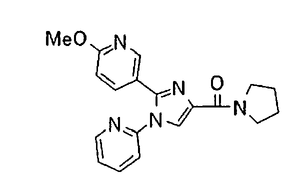

In a manner similar to that employed in Example 1, the title compound in solid form (302 mg, 73.3%) was prepared from 2-(6-methoxy-3-pyridyl)-1-(2-pyridyl)-1H-imidazole-4-carboxylic acid (350 mg) obtained in Referential Example 18 and pyrrolidine (125 µg).
¹H-NMR (300MHz, CDCl₃) δ:1.96 (4H, m), 3.69(2H,t,J=6.6Hz), 3.93(3H,s), 4.12(2H,t,J=6.6Hz), 6.70(1H,dd,J=8.7,0.6Hz), 7.17(1H,d,J=8.1Hz), 7.36(1H,m), 7.66(1H,dd,J=8.7,2.4Hz), 7.79 (1H, dt, J=7.8, 1 .8Hz), 8.00 (1H, s), 8.17(1H,d,J=1.8Hz), 8.55(1H,m).
MS (FAB)m/z:350 (M+H)⁺.

### [Example 58] (2S)-1-[2-(6-Methoxy-3-pyridyl)-1-phenyl-1H-imidazole-4-carbonyl]pyrrolidine-2-carboxamide

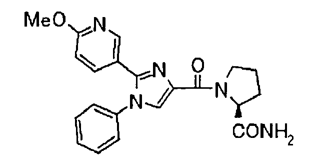

In a manner similar to that employed in Example 1, the title compound in solid form (456 mg, 86.0%) was prepared from 2-(6-methoxy-3-pyridyl)-1-phenyl-1H-imidazole-4-carboxylic acid (400 mg) obtained in Referential Example 4 and L-prolinamide (174 mg).
¹H-NMR(300MHz,CDCl₃)δ:2.01(2H,m), 2.17(1H,m), 2.42(1H,m), 3.77 and 3.89(1H, each m), 3.89 and 3.91(3H,each s), 4.30 (1H,m), 4.87 and 5.53 (1H, each m), 5.39,5.52,6.73 and 7.13(2H,each br s), 6.66(1H,d,J=8.63Hz), 7.22-7.26(2H,m), 7.45-7.48(3H,m), 7.59 and 7.68(1H,each d,J=8.63Hz), 7.83(1H,s), 8.04 and 8.17(1H,each s).
[α]_{D}²⁶-94.3°(c=0.2, CHCl₃).
MS (FAB)m/z:392 (M+H)⁺.

### [Example 59] (2S)-1-[2-(6-Methoxy-3-pyridyl)-1-(4-methylphenyl)-1H-imidazole-4-carbonyl]pyrrolidine-2-carboxamide

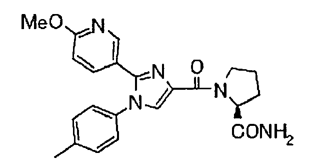

In a manner similar to that employed in Example 1, the title compound in solid form (262 mg, 50.0%) was prepared from 2-(6-methoxy-3-pyridyl)-1-(4-methylphenyl)-1H-imidazole-4-carboxylic acid (400 mg) obtained in Referential Example 6 and L-prolinamide (162 mg).
¹H-NMR(300MHz,CDCl₃)δ:1.90-2.21(3H,m), 2.35-2.42(1H,m), 2.42(3H,s), 3.72-3.93(1H,m), 3.91(3H,s), 4.25-4.33(1H,m), 4.87 and 5.53(1H,each br d,J=7.16Hz), 5.39 and 6.75(1H,each br s), 6.67(1H,br d,J=8.44Hz), 7.11-7.15(2H,m), 7.23-7.26(2H,m), 7.63 and 7.72(1H,each br d,J=8.44Hz), 7.80(1H,s), 8.04 and 8.17(1H,each s).
[α]_{D}²⁶ -36.5 ° (c=0.2, CHCl₃).
MS (FAB)m/z:406 (M+H)⁺.

### [Example 60] (2S)-1-[1-(4-Methylphenyl)-2-(6-methyl-3-pyridyl)-1H-imidazole-4-carbonyl]pyrrolidine-2-carboxamide

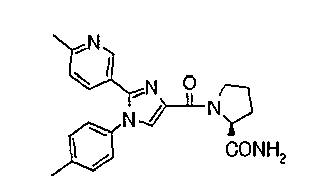

In a manner similar to that employed in Example 1, the title compound in solid form (312 mg, 62.1%) was prepared from 1-(4-methylphenyl)-2-(6-methyl-3-pyridyl)-1H-imidazole-4-carboxylic acid (400 mg) obtained in Referential Example 11 and L-prolinamide (171 mg).
¹H-NMR(300MHz,CDCl₃)δ:1.90-2.21(3H,m), 2.35-2.42(1H,m), 2.41(3H,s), 2.53(3H,s), 3.71-3.88(1H,m), 4.20-4.32 (1H,m), 4.87 and 5.53(1H,each br d,J=7.16Hz), 5.34 and 6.65(1H,br s), 7.10-7.14(2H, m) , 7.23-7.27(2H, m), 7.65 and 7.80(1H, each br d, J=8 . 63Hz), 7.82(1H, s), 7.95 (1H, s), 8.28 and 8.45(1H,each s).
[α] _{D}²⁶-50. 8° (c=0.2, CHCl₃) .
MS(FAB)m/z:390(M+H)⁺.

### [Example 61] (2S)-1-[5-(6-Methoxy-3-pyridyl)-1-(4-methylphenyl)-1H-1,2,4-triazole-3-carbonyl]pyrrolidine-2-carboxamde

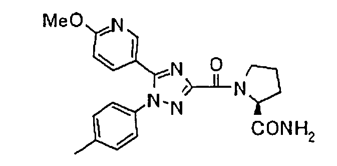

In a manner similar to that employed in Example 33, the title compound in solid form (187 mg, 57%) was prepared from 5-(6-methoxy-3-pyridyl)-1-(4-methylphenyl)-1H-1,2,4-triazole-3-carboxylic acid (248 mg) obtained in Referential Example 27 and L-prolinamide (135 mg).
¹H-NMR (400MHz, CDCl₃) δ: 1.88-2.57 (4H, m), 2.42(3Hx1/5, s), 2.43(3Hx4/5,s), 3. 83-3.95 (2Hx1/5,m), 3.94 (3H, s), 4.10-4.15(2Hx4/5,m), 4.92-4.96(1Hx4/5,m), 5.21-5.25 (1Hx1/5,m), 5.31-5.42(2Hx4/5,m), 6.25-6.33 (2Hx1/5,m), 6.73 (1H, d, J=8.7Hz), 7.23-7.35(4H,m), 7.79(1H,dd,J=2.4,8.7Hz), 8.26(1Hx1/5,d,J=2.4Hz), 8.30(1Hx4/5,d,J=2.4Hz).
[α]_{D}²⁶-8.5 (c=0.5,MeOH).
MS (ESI)m/z:407 (M+H)⁺.
Elementary analysis: as C₂₁H₂₂N₆O₃·0.25H₂O
Calculated: C,61.38;H,5.52;N,20.45.
Found: C,61.23;H,5.25;N,20.39.

### [Example 62] (2S)-1-[1-(6-Methoxy-3-pyridyl)-5-(4-methylphenyl)-1H-1,2,4-triazole-3-carbonyl]pyrrolidine-2-carboxamide

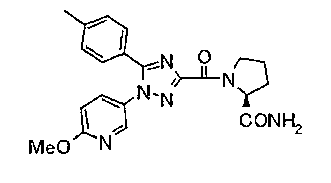

In a manner similar to that employed in Example 33, the title compound in solid form (172 mg, 53%) was prepared from 1-(6-methoxy-3-pyridyl)-5-(4-methylphenyl)-1H-1,2,4-triazole-3-carboxylic acid (248 mg) obtained in Referential Example 26 and L-prolinamide (137 mg).
¹H-NMR (400MHz, CDCl₃) δ:1.89-2.25 (3H×4/5+4H×1/5, m), 2.38(3H,s), 2.31-2.57(1H×4/5,m), 3.82-3.96(2H×1/5,m), 3.98(3H×1/5,s), 3.99(3Hx4/5,s), 4.10-4.15(2H×4/5,m), 4.92-4.96(1H×4/5,m), 5.22-5.27 (1H×1/5,m), 5.31-5.42 (2H×4/5,m), 6.32-6.38 (2H×1/5,m), 6.80-6.84 (1H,m), 7.16-7.20 (2H, m), 7.39-7.44(2H,m), 7.50-7.62(1H,m), 8.16(1H×1/5, d, J=2.7Hz), 8.19 (1H×4/5,d, J=2.7Hz) . [α]_{D}²⁵-18.1° (c=0.5, MeOH).
MS(ESI)m/z:407(M+H)⁺.
Elementary analysis: as C₂₁H₂₂N₆O₃·0.5H₂O
Calculated: C,60.71;H,5.58;N,20.23.
Found: C,60.45;H,5.35;N,20.05.

### [Example 63] 1-[2-(6-Methoxy-3-pyridyl)-1-(2-pyridyl)-1H-imidazole-4-carbonyl]-4,4-difluoropiperidine

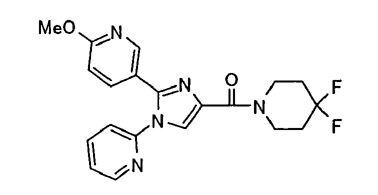

Triethylamine (697 µL) was added to 2-(6-methoxy-3-pyridyl)-1-(2-pyridyl)-1H-imidazole-4-carboxylic acid (296 mg) obtained in Referential Example 18, 1-hydroxybenzotriazole (54 mg), 1-ethyl-3-(3-dimethylaminopropyl)carbodiimide hydrochloride (288 mg), and 4,4-difluoropiperidine hydrochloride (173 mg) obtained in Referential Example 40 in methylene chloride (30 mL), followed by stirring at room temperature for 3 days. The reaction mixture was partitioned between water and chloroform. The organic layer was dried over sodium sulfate anhydrate. After a filtration step, the solvent was removed under reduced pressure, and the residue was purified by silica gel column chromatography (hexane - ethyl acetate), to thereby give the title compound as a solid product (226 mg, 57%).
¹H-NMR (400MHz, CDCl₃)δ: 1.63 (4H,s), 2. 08-2.11 (4H,m) , 3.94(3H,s), 6.71(1H, dd, J=8.5,0.7Hz), 7.16(1H,d,J=8.1Hz), 7.37-7.39(1H,m), 7.62(1H,dd,J=8.7,2.6Hz), 7.80(1H,td,J=7.8,2.0Hz), 8.00(1H,s), 8.17(1H,d,J-2.0Hz), 8.56-8.57(1H,m).
MS(ESI)m/z:400(M+H)⁺.

### [Example 64] 1-[2-(6-Methoxy-3-pyridyl)-1-(2-pyridyl)-1H-imidazole-4-carbonyl]-4-fluoropiperidine

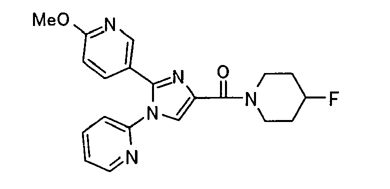

In a manner similar to that employed in Example 63, the title compound in solid form (203 mg, 53%) was prepared from 2-(6-methoxy-3-pyridyl)-1-(2-pyridyl)-1H-imidazole-4-carboxylic acid (296 mg) obtained in Example 18 and 4-fluoropiperidine hydrochloride (139 mg) obtained in Referential Example 41.
¹H-NMR(400MHz,CDCl₃)δ:1.60(2H,br s), 1.92-2.06(4H,m), 3.74-4.45(2H,m), 3.93(3H,s), 4.85-4.99(1H,m), 6.70(1H,dd,J=8.7,0.6Hz), 7.16 (1H, dd, J=8.1,1.0Hz), 7.37(1H,ddd,J=7.6,4.9,1.0Hz), 7.63(1H,dd,J=8.8,2.4Hz), 7.79(1H,td,J=7.8,1.9Hz), 7.96(1H,s), 8.17(1H,t,J=1.2Hz), 8.56 (1H, dt, J=4 . 9, 0. 9Hz) .
MS (ESI)m/z:382 (M+H)⁺.
Elementary analysis: as C₂₀H₂₀FN₅O₂
Calculated: C,62.98;H,5.29;N,18.36.
Found: C,63.02;H,5.11;N,18.18.

### [Example 65] 1-[2-(6-Methoxy-3-pyridyl)-1-(2-pyridyl)-1H-imidazole-4-carbonyl]-4-methoxypiperidine

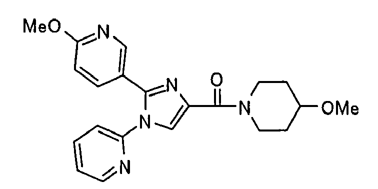

In a manner similar to that employed in Example 63, the title compound in solid form (217 mg, 55%) was prepared from 2-(6-methoxy-3-pyridyl)-1-(2-pyridyl)-1H-imidazole-4-carboxylic acid (296 mg) obtained in Referential Example 18 and 4-methoxypiperidine hydrochloride (152 mg) obtained in Referential Example 39.
¹H-NMR (400MHz, CDCl₃) δ: 1.59 (1H, s), 1.68(2H, tt, J=13.1, 4.2Hz), 1.99(2H, dt, J=21.9, 7.5Hz), 3.39(3H, s), 3.47-3.53(1H, m), 3.91(3H,s), 4.88-4.60(3H,m), 6.70(1H, t, J=4.6Hz), 7.16(1H, dd, J=8.1, 1.0Hz), 7.36(1H, ddd, J=7.6, 4.9, 1.0Hz), 7.64(1H, dt, J=8.7, 1.2Hz), 7.79(1H, td, J=7.8, 1.9Hz), 7.93(1H, d, J=1.0Hz), 8.17(1H, d, J=2.4Hz), 8.55(1H, dt, J=4.9, 0.9Hz).
MS (ESI) m/z: 394 (M+H)⁺.

### [Example 66] 1-[1-(6-Methoxy-3-pyridyl)-5-(2-pyridyl)-1H-1,2,4-triazole-3-carbonyl]-4-methylpiperazine

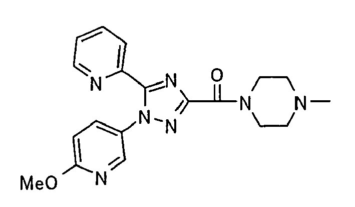

In a manner similar to that employed in Example 1, the title compound in solid form (151 mg, 46%) was prepared from 1-(6-methoxy-3-pyridyl)-5-(2-pyridyl)-1H-1,2,4-triazole-3-carboxylic acid (297 mg) obtained in Referential Example 42 and N-methylpiperazine (133 µL).
¹H-NMR (400MHz, CDCl₃) δ: 2.34 (3H,s), 2.50 (4H, dt, J=19.6, 5.1Hz), 3.89 (2H, t, J=5. 1Hz), 3.95 (2H, t, J=5.0Hz), 3.99 (3H, s), 6.81 (1H, dd, J=8.8, 0.7Hz), 7.32 (1H, ddd, J=7.7, 4.8, 1.2Hz), 7.69 (1H, dd, J=8.8, 2.9Hz), 7.82 (1H, td, J=7.8, 1.9Hz), 8.16 (1H, dt, J=7.9,1.0Hz), 8.22 (1H, dd, J =2. 7, 0.5Hz), 8.44 (1H, dq, J=4.8, 0.9Hz).
Elementary analysis: as C₁₉H₂₁N₇O₂
Calculated: C, 60.15; H, 5.58; N, 25.84.
Found: C,60.13;H,5.50;N,25.69.

### [Example 67] 1-[5-(5-Cyano-2-pyridyl)-1-(6-methoxy-3-pyridyl)-1H-1,2,4-triazole-3-carbonyl]-4,4-difluoropiperidine

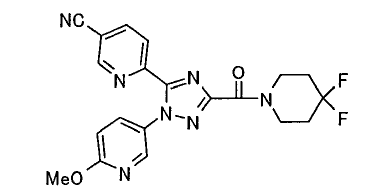

In a manner similar to that employed in Example 1, the title compound in solid form (63 mg, 25%) was prepared from 5-(5-cyano-2-pyridyl)-1-(6-methoxy-3-pyridyl)-1H-1,2,4-triazole-3-carboxylic acid (194 mg) obtained in Referential Example 43 and 4,4-difluoropiperidine hydrochloride (89 mg) obtained in Referential Example 40.
¹H-NMR (400MHz, CDCl₃) δ:2.17-2.08 (4H, m), 3.96-4.07 (7H,m), 6.85(1H, d, J=8.8Hz), 7.66(1H, dd, J=8.8, 2.7Hz), 8.11 (1H, dd, J=8.2, 2.1Hz), 8.21 (1H, d, J=2.2Hz), 8 .39(1H, dd, J=8 . 2, 0 . 9Hz), 8.67 (1H, q, J=1.0Hz).
Elementary analysis: as C₂₀ H₁₇ N₇O₂
Calculated: C,56.47;H,4.03;N,23.05.
Found: C,56.58;H,4.17;N,23.15.

### [Example 68] 1-[2-(6-Methoxy-3-pyridyl)-1-(2-pyridyl)-1H-imidazole-4-carbonyl]-4-methylpiperazine

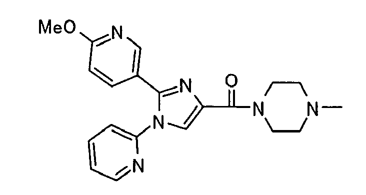

In a manner similar to that employed in Example 1, the title compound in solid form (235 mg, 61.5%) was prepared from 2-(6-methoxy-3-pyridyl)-1-(2-pyridyl)-1H-imidazole-4-carboxylic acid (300 mg) obtained in Referential Example 18 and N-methylpiperazine (134)J.L).
¹H-NMR(300MHz, CDCl₃)δ:2.34 (3H, s), 2.48-2.52(4H,m), 3.75-3.88(2H,m), 3.93 (3H, s), 4.25-4 .42 (2H,m), 6.71(1H, d, J=0.73Hz), 7.15(1H,d,J=8.07Hz), 7.34-7.39(1H,m), 7.63(1H,dd,J=8.63,2.39Hz), 7.96(1H,s), 8.16(1H,d,J=1.65Hz), 8.55(1H, d, J=4.96, 1.10Hz).
MS (FAB)m/z:379(M+H)⁺.

### [Example 69] (3S)-1-[2-(6-Methoxy-3-pyridyl)-1-(2-pyridyl)-1H-imidazole-4-carbonyl]-3-fluoropyrrolidine

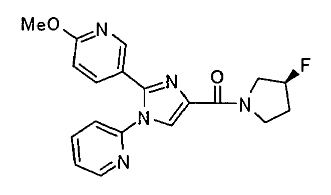

In a manner similar to that employed in Example 63, the title compound in solid form (13.0 mg, 21%) was prepared from 2-(6-methoxy-3-pyridyl)-1-(2-pyridyl)-1H-imidazole-4-carboxylic acid (50.0 mg) obtained in Referential Example 18 and (3S)-fluoropyrrolidine hydrochloride (9.1 mg) obtained in Referential Example 44.
¹H-NMR (400MHz, CDCl₃)δ: 1.99-2.37 (2H, m), 3.75-4.30(3H,m), 3,94(3H,s), 4.61(1H, q, J=13.0Hz), 5.33(1H,dd,J=52.7,16.1Hz), 6.70(1H,d,J=8.5Hz), 7.17(1H,d,J=7.8Hz), 7.37(1H,dd,J=7.4,5.0Hz), 7.65(1H,t,J=6.3Hz), 7.80 (1H, td, J=7.8,1.8Hz), 8.05 (1H,d, J=2.9Hz), 8.18(1H,d,J=2.4Hz), 8.56(1H,d,J=4.6Hz).
MS(ESI)m/z:368(M+H)⁺.

### [Example 70] 1-[2-(6-Methoxy-3-pyridyl)-1-(2-pyridyl)-1H-imidazole-4-carbonyl]-4-fluoromethylpiperidine

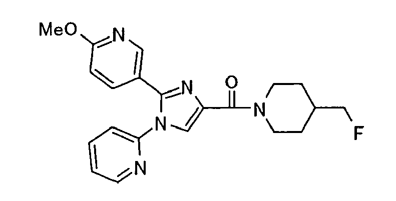

In a manner similar to that employed in Example 63, the title compound in solid form (21.4 mg, 32%) was prepared from 2-(6-methoxy-3-pyridyl)-1-(2-pyridyl)-1H-imidazole-4-carboxylic acid (50.0 mg) obtained in Referential Example 18 and 4-fluoromethylpiperidine hydrochloride (27.0 mg) obtained in Referential Example 45.
¹H-NMR (400MHz, CDCl₃)δ:1.34 (2H, dd, J=56. 9, 9. 8Hz), 1.83(2H,d,J=12.5Hz), 2.04(1H,dd,J=9.3,6.6Hz), 2.84(1H,s), 3.17 (1H, s), 3.75-3.93(3H,m), 4.31 (2H, dd, J=48.0, 6.1Hz), 4.80 (1H,s), 5.26 (1H,s), 6.70 (1H,dd, J=8.8,0.7Hz), 7.16(1H,dd, J=8.1,0.7Hz), 7.37(1H, ddd, J=7.6,4.9,1.0Hz), 7.64(1H,dd,J=8.8,2.4Hz), 7.79(1H,td,J=7.8,1.8Hz), 7.92(1H, t, J=3.8Hz), 8.17(1H,d,J=2.4Hz), 8.56(1H,dt,J=4.9,1.0Hz).
MS(ESI)m/z:396(M+H)⁺.
Elementary analysis: as C₂₁H₂₂FN₅O₂
Calculated: C,63.78;H,5.61;N,17.71.
Found: C,63.54;H,5.45;N,17.58.

### [Example 71] 1-[5-(5-Fluoro-2-pyridyl)-1-(6-methoxy-3-pyridyl)-1H-1,2,4-triazole-3-carbonyl]-4,4-difluoropiperidine

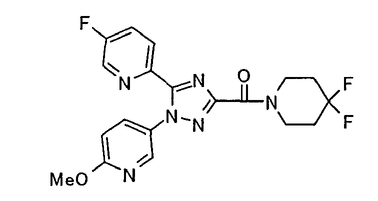

1-Ethyl-3-(3-dimethylaminopropyl)carbodiimide hydrochloride (421 mg), 1-hydroxybenzotriazole (297 mg), and 4,4-difluoropiperidine hydrochloride (287 mg) obtained in Referential Example 40 were added to 5-(5-fluoro-2-pyridyl)-1-(6-methoxy-3-pyridyl)-1H-1,2,4-triazole-3-carboxylic acid sodium salt (630 mg) obtained in Referential Example 46 in acetonitrile (10 mL), followed by stirring at room temperature for 14.5 hours. The reaction mixture was partitioned between water and a chloroform-methanol (10:1) solvent mixture. The organic layer was washed with saturated aqueous sodium hydrogencarbonate, and then dried over sodium sulfate anhydrate. After a filtration step, the solvent was removed under reduced pressure, and the residue was purified by silica gel thin-layer chromatography (chloroform - methanol), to thereby give the title compound as a solid product (237 mg, 28%).
¹H-NMR (400MHz, CDCl₃) δ: 2.10-2.14(4H, m), 3.95-4.03(7H,m), 6.81-7.00(1H, m), 7.54(1H, ddd, J=9.7, 6.9, 1.9Hz), 7.66(1H, dd, J=8.8, 2.9Hz), 8.21-8.24(2H, m), 8.28(1H, d, J=2.7Hz).
MS (ESI) m/z: 419(M+H)⁺.

### [Example 72] (3R)-1-[2-(6-Methoxy-3-pyridyl)-1-(2-pyridyl)-1H-imidazole-4-carbonyl]-3-fluoropiperidine

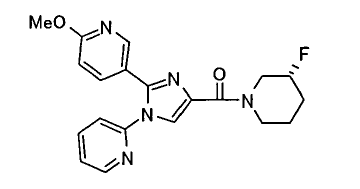

In a manner similar to that employed in Example 63, the title compound in solid form (30.0 mg, 44%) was prepared from 2-(6-methoxy-3-pyridyl)-1-(2-pyridyl)-1H-imidazole-4-carboxylic acid (52.3 mg) obtained in Referential Example 18 and (3R)-fluoropiperidine hydrochloride (25 mg) obtained in Referential Example 48.
¹H-NMR(400MHz, CDCl₃) δ:1.58 (2H, s), 1.95(3H,s), 3.50-4.25(3H,br), 3.93(3H,s), 4.73 (1H, d, J=47.6Hz), 6.70 (1H, d, J=8.8Hz), 7.16 (1H, d, J=7.8Hz), 7.37 (1H, dd, J=7.6, 4.9Hz), 7.65 (1H, d, J=8.8Hz), 7.79 (1H, td, J=7.8, 1.9Hz), 7.96 (1H, s), 8.17 (1H, d, J=2.4Hz), 8.55 (1H, dd, J=5.2, 1.3Hz).
MS(ESI)m/z:382(M+H)⁺.
Elementary analysis: as C₂₀H₂₀FN₅O₂
Calculated: C, 62.98; H, 5.29; N, 18.36; F, 5.29.
Found: C,62.79;H,5.06;N,18.28;F,5.01.

### [Example 73] 1-[5-(5-Fluoro-2-pyridyl)-1-(6-methoxy-3-pyridyl)-1H-1,2,4-triazole-3-carbonyl]-4-methylpiperazine

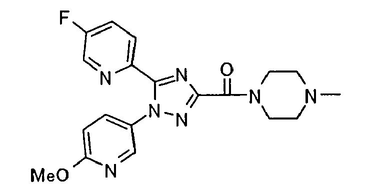

In a manner similar to that employed in Example 71, the title compound in solid form (48 mg, 6%) was prepared from 5-(5-fluoro-2-pyridyl)-1-(6-methoxy-3-pyridyl)-1H-1,2,4-triazole-3-carboxylic acid sodium salt (630 mg) obtained in Referential Example 46 and N-methylpiperazine (264 µL).
¹H-NMR(400MHz,CDCl₃)δ:2.34(3H,s), 2.50(4H,dt,J=20.2,5.1Hz), 3.88-3.89(2H,m), 3.94-3.97(2H,m), 3.99(3H,s), 6.82(1H,t,J=4.4Hz), 7.52-7.54(1H, m), 7.66(1H, dd, J=7.4,3.7Hz), 8.23-8.26(3H,m).
MS (ESI)m/z:397 (M+H)⁺.

### [Example 74] 1-[1-(6-Methoxy-3-pyridyl)-5-(5-methyl-2-pyridyl)-1H-1,2,4-triazole-3-carbonyl]-4,4-difluoropiperidine

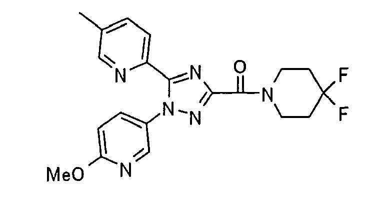

In a manner similar to that employed in Example 71, the title compound in solid form (167 mg, 40%) was prepared from 1-(6-methoxy-3-pyridyl)-5-(5-methyl-2-pyridyl)-1H-1,2,4-triazole-3-carboxylic acid (311 mg) obtained in Referential Example 47 and 4,4-difluoropiperidine hydrochloride (143 mg) obtained in Referential Example 40.
¹H-NMR(400MHz,CDCl₃)δ:1.57(3H,s), 2.11(4H,s), 2.36(3H,s), 3.93-4.00(5H,m), 4.04-4.06(2H,m), 6.81(1H,dd,J=8.8,0.7Hz), 7.60-7.64(1H, m), 7.68(1H, dd, J=8.8, 2.7Hz), 8.02 (1H, d, J=7 . 8Hz), 8.20-8.25(1H,m), 8.26-8.29(1H,m).

### [Example 75] 1-[1-(6-Methoxy-3-pyridyl)-5-(5-methyl-2-pyridyl)-1H-1,2,4-triazole-3-carbonyl]-4-methoxypiperidine

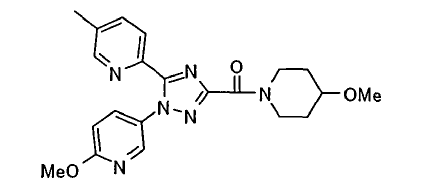

In a manner similar to that employed in Example 71, the title compound in solid form (182 mg, 50%) was prepared from 1-(6-methoxy-3-pyridyl)-5-(5-methyl-2-pyridyl)-1H-1,2,4-triazole-3-carboxylic acid (311 mg) obtained in Referential Example 47 and 4-methoxypiperidine hydrochloride (184 mg) obtained in Referential Example 39.
¹H-NMR (400MHz, CDCl₃)δ:1.65-1.78(2H,m), 1.87-2.01(2H,m), 2.35(3H,s), 3.38(3H,s), 3.48-3.55(1H,m), 3.60-3.69(2H,m), 3.98(3H,s), 4.06-4.08(2H,m), 6.80(1H,dd1,J=8.8,0.5Hz), 7.61(1H,ddd,J=8.1,1.0,0.5Hz), 7.69(1H,dd,J=8.7,2.8Hz), 8.03(1H,d,J=7.8Hz), 8.21(1H,dd,J=2.7,0.7Hz), 8.26-8.27(1H,m).
MS (ESI) : 408 (M+H)⁺.

### [Example 76] 1-[5-(5-Chloro-2-pyridyl)-1-(6-methoxy-3-pyridyl)-1H-1,2,4-triazole-3-carbonyl]-4,4-difluoropiperidine

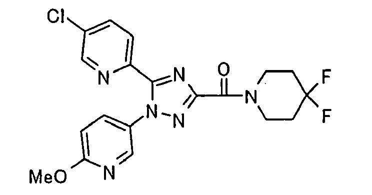

In a manner similar to that employed in Example 71, the title compound in solid form (146 mg, 53%) was prepared from 5-(5-chloro-2-pyridyl)-1-(6-methoxy-3-pyridyl)-1H-1,2,4-triazole-3-carboxylic acid (297 mg) obtained in Referential Example 49 and 4,4-difluoropiperidine hydrochloride (90.8 mg) obtained in Referential Example 40.
¹H-NMR(400MHz, CDCl₃)δ:2.11-2.13(4H, m), 3.95-4.06(7H,m), 6.83(1H, d, J=8.8Hz), 7.66(1H, dd, J=8.8, 2.7Hz), 7.81(1H, dd, J=8.5, 2.3Hz), 8.16(1H, d, J=9.1Hz), 8.21(1H, d, J=2.7Hz), 8.38(1H, d, J=2.5Hz).
MS(ESI)m/z:435(M+H)⁺.

### [Example 77] (2S)-1-[2-(6-Methoxy-3-pyridyl)-1-(2-pyridyl)-¹H-imidazole-4-carbonyl]-2-fluoromethylpyrrolidine

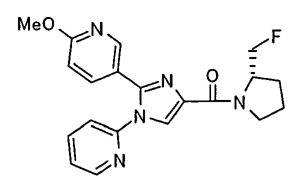

### 1) Mixture of (2S)-N-benzyl-2-fluoromethylpyrrolidine and (3R)-N-benzyl-3-fluoropiperidine

Diethylaminosulfur trifluoride (11.2 mL) was added to (2S)-N-benzyl-2-hydroxymethylpyrrolidine (10 mL) in dichloromethane (100 mL) at -78°C, followed by stirring at room temperature for 50 minutes. The reaction mixture was partitioned between saturated aqueous sodium hydrogencarbonate and methylene chloride. The organic layer was dried over magnesium sulfate anhydrate. After a filtration step, the solvent was removed under reduced pressure, and the residue was purified by silica gel column chromatography (hexane - ethyl acetate), to thereby give a 3:5 mixture of (2S)-N-benzyl-2-fluoromethylpyrrolidine and (3R)-N-benzyl-3-fluoropiperidine as an oily product (7.71 g, 71%).
¹H-NMR (400MHz, CDCl₃) δ:1.51-2.86 (5/8×8H,m), 1.51-2.96(3/8×7H,m), 3.49(3/8×1H,d,J=13.2Hz), 3.56(5/8×2H,s), 4.04(3/8×1H,d,J=13.2Hz), 4.25(3/8×1H,ddd,J=20.0,9.3,5.4Hz), 4.37(3/8×1H,ddd,J=19.7,9.2,5.4Hz), 4.54-4.70(5/8×1H,m), 7.22-7.34(5H, m).
MS(ESI)m/z:194(M+H)⁺.

### 2) Mixture of (2S)-fluoromethylpyrrolidine hydrochloride and (3R)-fluoropiperidine hydrochloride

1-Chloroethyl chloroformate (0.857 mL) was added to the above-obtained mixture of (2S)-N-benzyl-2-fluoromethylpyrrolidine and (3R)-N-benzyl-3-fluoropiperidine (1.38 g) in methylene chloride (50 mL). The resultant mixture was refluxed for 2 hours, followed by cooling in air. The reaction solvent was removed under reduced pressure. The residue was dissolved in methanol (20 mL). The resultant mixture was refluxed for 1.5 hours, followed by cooling in air. The reaction solvent was removed under reduced pressure. Diethyl ether was added to the resultant mixture. The precipitate was collected by filtration, and then dried, to thereby give a 3:5 mixture of (2S)-fluoromethylpyrrolidine hydrochloride and (3R)-fluoropiperidine hydrochloride (820 mg, 82%).
¹H-NMR(400MHz,DMSO-d₆)δ:1.58-2.06(5/8×8H,m), 1.58-3.33(3/8x6H,m), 3.78-3.85(3/8×1H,m), 4.56-4.76(3/8×2H,m), 4.98(5/8×1H, d, J=45.9Hz), 9.41(2H,br s).

### 3) Title compound

In a manner similar to that employed in Example 63, 2-(6-methoxy-3-pyridyl)-1-(2-pyridyl)-1H-imidazole-4-carboxylic acid (158 mg) obtained in Referential Example 18 and the above-obtained mixture of (2S)-fluoromethylpyrrolidine hydrochloride and (3R)-fluoropiperidine hydrochloride (89.3 mg) were employed, to thereby give (3R)-1-[2-(6-methoxy-3-pyridyl)-1-(2-pyridyl)-1H-imidazole-4-carbonyl]-3-fluoropiperidine (13 mg, 6%) obtained in Example 72 and the title compound in solid form (14 mg, 7%).
¹H-NMR (400MHz, CDCl₃) δ : 1.67-2.11 (5H, m), 3.93(3H,s), 4.13 (1H,br s), 4.55-4.85(2H,m), 6.69 (1H, d, J=8. 8Hz), 7.15-7.17 (1H,m), 7.37 (1H,br s), 7.64 (1H,br s), 7.80 (1H,br s), 8.16 (1H,br s), 8.56 (1H, d, J=5.2Hz).
MS(ESI)m/z:382(M+H)⁺.

### [Test Example 1] Inhibitory activity for platelet aggregation

Human blood was collected in the presence of 3.13% sodium citrate as an anticoagulant in a volume 1/10 the blood volume. The collected blood was centrifuged at 180xg for 10 minutes so as to separate the upper layer; i.e., platelet rich plasma (PRP) from the blood. The remaining lower layer was further centrifuged at 1,600xg for 10 minutes, and platelet poor plasma (PRP); i.e., the thus-obtained upper layer, was collected. A solution (1 µL) of the compound of the Examples was added to PRP (200 µL), and the mixture was allowed to stand at 37°C for 2 minutes. Subsequently, collagen (2 µL) was added to the resultant mixture so as to induce platelet aggregation. Percentage of platelet aggregation was measured using PAM-12C (SSR Engineering). Optical transmittance of PPP was employed as the value reflecting the state in which 100% aggregation occurred. Percent of platelet aggregation values of PRP were determined at a series of concentrations of each Example compound and IC₅₀ of each compound was calculated. Table 1 shows the results.

### [Test Example 2] Inhibitory effects on cyclooxygenase-1 (COX-1) and cyclooxygenase-2 (COX-2)

Inhibitory activity against COX-1 and COX-2 of the compounds produced in the Examples was measured by use of a COX Inhibitor Screening Assay Kit (product of Cayman Chemical Company, Catalog Nos. 560101 and 560121).

Before starting the measurement, reaction buffer, heme, arachidonic acid, SnCl₂, EIA buffer, washing buffer, prostaglandin (PG) screening EIA standard, PG screening acetylcholine esterase (AchE), tracer (chromogenic enzyme HRP conjugate), and PG screening EIA antiserum were prepared ready for use.

### (1) Production of PGF_{2α} in the presence of COX-1 or COX-2

A reaction mixture containing the compound of the Examples (50 µM) and COX-1 or COX-2 was allowed to stand at 37°C for 10 minutes. Arachidonic acid (10 µL) was added thereto, and the resultant mixture was further allowed to stand at 37°C for 2 minutes. 1N-Hydrochloric acid (50 µL) was added to the reaction mixture, to thereby stop the reaction. SnCl₂ solution (100 µL) was added thereto, and the resultant mixture was allowed to stand at room temperature for 5 minutes.

### (2) Quantitative determination of PGF_{2α} using ELISA

Antiserum (rabbit anti-PGF_{2α} antibody, 50 µL) was added to the wells of 96 well plate that had been coated with mouse anti-rabbit IgG. A solution of PGF_{2α}-containing mixture obtained above (2000-fold diluted, 50 µL) and AchE tracer (50 µL) were added to the well in this order, and the mixture was allowed to stand at room temperature for 18 hours. The wells were washed 5 times with the washing buffer to remove an excessive AchE tracer, and Ellman reagent (200 µL) was added. After leaving the plate in a dark room for 60 minutes, absorbance was measured at 405 nm.

### (3) Calculation of inhibitory activity of the compound of the Examples

A calibration curve was obtained by use of the PG screening EIA standard, and the production amount of PGF_{2α} was determined from the absorbance above. Percent inhibition against COX-1 or COX-2 at 50 µM of the compound of the Examples was calculated. Table 1 shows the results.

Notably, the amount of PGF_{2α} produced by use of a reaction mixture containing no compound of the Example was regarded as 100% in calculation of percent inhibition.

**Table 1**

| Compound | Inhibition of collagen-induced platelet aggregation, IC₅₀ (µM) | Inhibitory effect against COX-1 at 50 µM (% inhibition) | Inhibitory effect against COX-2 at 50 µM (% inhibition) |
|---|---|---|---|
| 6 | 0.33 | 28.4 | 23.4 |
| 25 | 0.71 | 3.1 | -8.7 |
| 37 | 0.27 | -3.2 | -0.8 |
| 38 | 0.32 | 3.5 | 10.7 |
| 32 | 0.41 | 5.6 | -2.2 |
| 63 | 0.02 | 3.4 | 7.5 |
| 64 | 0.06 | -1.4 | 4.5 |
| 65 | 0.12 | ND | ND |
| 66 | 0.7 | ND | ND |
| 67 | 0.22 | ND | ND |
| 71 | 0.19 | 35.2 | ND |
| 72 | 0.11 | 39.0 | ND |
| 74 | 0.021 | 46.9 | ND |

| | | | |
|---|---|---|---|
| ND: Not Determined | | | |

As is clear from Table 1, the compound (I) of the present invention, a salt thereof or a solvate thereof, or a solvate of the salt exhibited strong platelet coagulation inhibitory activity, without inhibiting COX-1 or COX-2.

## Claims

1. A compound represented by formula (I), a salt of the compound, or a solvate of the compound or the salt: 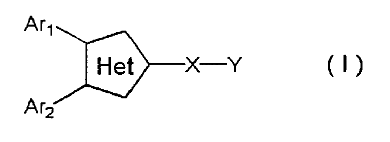 wherein the group represented by formula (1): 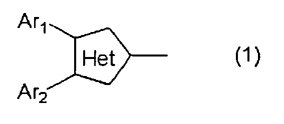 is any of the groups represented by formula (a) to (c): 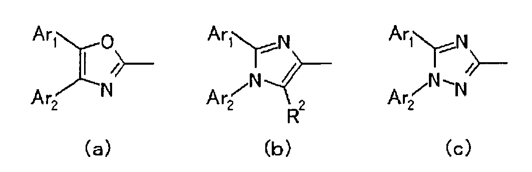 (wherein Ar₁ and Ar₂ each independently represent a 6-membered aromatic heterocyclic group which may have a substituent or a phenyl group which may have a substituent; and R² represents a group selected from among a hydrogen atom, a halogeno group, a hydroxyl group, a lower alkoxy group, and a lower alkyl group which may have a substituent);
X represents a carbonyl group or a thiocarbonyl group; and
Y represents a group represented by formula (2): 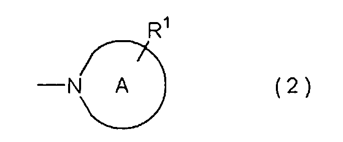 (wherein the ring structure A represents a 4- to 7-membered ring which may have, in addition to N shown in formula (2), one hetero atom selected from among N, O, and S; and the ring structure A may have a substituent represented by R¹, wherein R¹ represents 1 to 4 groups, which are identical to or different from one another, selected from among a hydroxyl group, a cyano group, an oxo group, a halogeno group, a lower alkyl group which may have a substituent, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be a substituted by a lower alkyl group, an aminosulfonyl group which may be substituted by a lower alkyl group, a 3-to 6-membered spiro-alicyclic alkyl group which may have a substituent, and a 4- to 7-membered alicyclic heterocyclic group which may have a substituent).

2. A compound as described in claim 1, a salt of the compound, or a solvate of the compound or the salt, wherein the group represented by formula (1) is a group represented by formula (b), and R¹ in formula (2) represents 1 to 4 groups, which are identical to or different from one another, selected from among a cyano group, an oxo group, a halogeno group, a lower alkyl group which has been substituted by a halogeno group, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3- to 6-membered spiro-alicyclic alkyl group which may have a substituent, and a 4-to 7-membered alicyclic heterocyclic group which may have a substituent.

3. A compound as described in claim 1 or 2, a salt of the compound, or a solvate of the compound or the salt, wherein X is a carbonyl group.

4. A compound as described in any one of claims 1 to 3, a salt of the compound, or a solvate of the compound or the salt, wherein Ar₁ in formula (I) is a pyridyl group which may have a substituent or a pyridazinyl group which may have a substituent.

5. A compound as described in any one of claims 1 to 3, a salt of the compound, or a solvate of the compound or the salt, wherein Ar₁ in formula (I) is a phenyl group which may have a substituent.

6. A compound as described in any one of claims 1 to 5, a salt of the compound, or a solvate of the compound or the salt, wherein Ar₂ in formula (I) is a pyridyl group which may have a substituent.

7. A compound as described in any one of claims 1 to 5, a salt of the compound, or a solvate of the compound or the salt, wherein Ar₂ in formula (I) is a phenyl group which may have a substituent.

8. A compound as described in any one of claims 1 to 7, a salt of the compound, or a solvate of the compound or the salt, wherein the group represented by formula (1) is a group represented by formula (a): 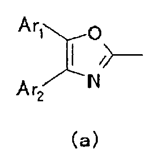 wherein Ar₁ and Ar₂ have the same meanings as described above.

9. A compound as described in any one of claims 1 to 7, a salt of the compound, or a solvate of the compound or the salt, wherein the group represented by formula (1) is a group represented by formula (b): 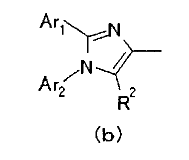 wherein Ar₁, Ar₂, and R² have the same meanings as described above.

10. A compound as described in any one of claims 1 to 7, a salt of the compound, or a solvate of the compound or the salt, wherein the group represented by formula (1) is a group represented by formula (c): 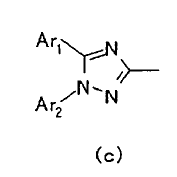 wherein Ar₁ and Ar₂ have the same meanings as described above.

11. A compound as described in any one of claims 1 to 10, a salt of the compound, or a solvate of the compound or the salt, wherein the ring structure A in formula (2) is a ring selected from among azetidine, pyrrolidine, piperidine, piperazine, morpholine, homopiperazine, and oxazepane, and R¹ represents 1 to 4 groups, which are identical to or different from one another, selected from among a hydroxyl group, a cyano group, an oxo group, a halogeno group, a lower alkyl group which may have a substituent, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3-to 6-membered spiro-alicyclic alkyl group which may have a substituent, and a 4- to 7-membered alicyclic heterocyclic group which may have a substituent.

12. A compound as described in any one of claims 1 to 10, a salt of the compound, or a solvate of the compound or the salt, wherein the ring structure A in formula (2) is a ring selected from among azetidine, pyrrolidine, piperidine, piperazine, morpholine, homopiperazine, and oxazepane, and R¹ represents 1 to 4 groups, which are identical to or different from one another, selected from among a cyano group, an oxo group, a halogeno group, a lower alkyl group which has been substituted by a halogeno group, a lower alkoxy group, an aralkyloxy group, a lower thioalkoxy group, a lower alkoxycarbonyl group, an aralkyloxycarbonyl group, a lower acyl group, a carboxyl group, a hydroxyiminocarbonyl group, an alkoxyimino group, a lower alkylsulfonyl group, an amino group which may have a substituent, a carbamoyl group which may be substituted by a lower alkyl group, an aminosulfonyl group which may be substituted by a lower alkyl group, a 3-to 6-membered spiro-alicyclic alkyl group which may have a substituent, and a 4- to 7-membered alicyclic heterocyclic group which may have a substituent.

13. A compound as described in any one of claims 1 to 10, a salt of the compound, or a solvate of the compound or the salt, wherein the ring structure A in formula (2) is a ring selected from among azetidine, pyrrolidine, piperidine, piperazine, morpholine, homopiperazine, and oxazepane, and R¹ represents 1 to 4 groups, which are identical to or different from one another, of halogeno groups or lower alkyl groups which have been substituted by a halogeno group.

14. A compound as described in any one of claims 1 to 10, a salt of the compound, or a solvate of the compound or the salt, wherein the group represented by formula (2) is a group selected from among a 3-dimethylaminoazetidin-1-yl group, a 2,2-dimethyl-3-dimethylaminoazetidin-1-yl group, a 2-hydroxymethylazetidin-1-yl group, a 2-carbamoylazetidin-1-yl group, a 2-oxopyrrolidino group, a 2-hydroxymethylpyrrolidino group, a 2-carbamoylpyrrolidino group, a 2-fluoromethylpyrrolidino group, a 3-fluoropyrrolidino group, a 2-hydroxymethylpiperidino group, a 2-carbamoylpiperidino group, a 2-methylcarbamoylpiperidino group, a 2-dimethylcarbamoylpiperidino group, a 3-fluoropiperidino group, a 4-fluoropiperidino group, a 4,4-difluoropiperidino group, a 4-fluoromethylpiperidino group, a 4-methoxypiperidino group, a 3-oxo-4-methylpiperazino group, a 4-methylpiperazino group, a 4-ethylpiperazino group, a 4-isopropylpiperazino group, a 4-cyclopropylpiperazino group, a 2,4-dimethylpiperazino group, a 3,4-dimethylpiperazino group, a 3-cyclopropyl-4-methylpiperazino group, a 3,4,5-trimethylpiperazino group, a 2,2,4-trimethylpiperazino group, a 3,3,4-trimethylpiperazino group, a 2-cyclopropanespiro-4-methylpiperazino group, a morpholino group, a 3-carbamoylmorpholino group, a 1,1-dioxothiomorpholino group, a 3-oxo-4-methylhomopiperazino group, a 5-oxo-4-methylhomopiperazino group, a 4-methylhomopiperazino group, a 4-ethylhomopiperazino group, a 4-cyclopropylhomopiperazino group, and a 1,4-oxazepan-4-yl group.

15. A drug containing a compound as recited in any one of claims 1 to 14, a salt of the compound, or a solvate of the compound or the salt.

16. A preventive and/or therapeutic agent for ischemic diseases, containing a compound as recited in any one of claims 1 to 14, a salt of the compound, or a solvate of the compound or the salt.

17. A platelet aggregation inhibitor containing a compound as recited in any one of claims 1 to 14, a salt of the compound, or a solvate of the compound or the salt.

18. A pharmaceutical composition containing a compound as recited in any one of claims 1 to 14, a salt of the compound, or a solvate of the compound or the salt, and a pharmacologically acceptable carrier therefor.

19. Use, for production of a drug, of a compound as recited in any one of claims 1 to 14, a salt of the compound, or a solvate of the compound or the salt.

20. Use, for production of a preventive and/or therapeutic agent for ischemic diseases, of a compound as recited in any one of claims 1 to 14, a salt of the compound, or a solvate of the compound or the salt.

21. Use, for production of a platelet aggregation inhibitor, of a compound as recited in any one of claims 1 to 14, a salt of the compound, or a solvate of the compound or the salt.

22. A method for treating ischemic diseases comprising administering an effective amount of a compound as recited in any one of claims 1 to 14, a salt of the compound, or a solvate of the compound or the salt.
